(19) Europäisches Patentamt
European Patent Office
Office européen des brevets

(11) EP 4 531 857 B1

(12) # EUROPEAN PATENT SPECIFICATION

(45) Date of publication and mention of the grant of the patent:
**11.03.2026 Bulletin 2026/11**

(21) Application number: **23733583.1**

(22) Date of filing: **25.05.2023**

(51) International Patent Classification (IPC):
***A61K 31/4985** (2006.01)* ***A61P 35/00** (2006.01)*
***A61P 35/02** (2006.01)* ***A61K 45/06** (2006.01)*
***A61K 31/496** (2006.01)* ***A61K 31/513** (2006.01)*
***A61K 39/00** (2006.01)* ***A61K 31/4439** (2006.01)*
***A61K 31/506** (2006.01)*

(52) Cooperative Patent Classification (CPC):
(C-Sets available)
**A61K 31/4985; A61K 31/4439; A61K 31/496; A61K 31/506; A61K 31/513; A61K 45/06; A61P 35/00; A61P 35/02** (Cont.)

(86) International application number:
**PCT/US2023/067487**

(87) International publication number:
**WO 2023/230567 (30.11.2023 Gazette 2023/48)**

(54) **GCN2 MODULATOR FOR TREATING ADVANCED SOLID TUMORS OR BLOOD CANCER**

GCN2-MODULATOR ZUR BEHANDLUNG VON FORTGESCHRITTENEN SOLIDER TUMOREN ODER BLUTKREBS

MODULATEUR DE GCN2 POUR LE TRAITEMENT D’ UN TUMEUR SOLIDE ADVANCEE OU CANCER DU SANG

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **25.05.2022 US 202263345727 P**
**20.01.2023 US 202363440297 P**
**03.02.2023 US 202363443269 P**
**30.03.2023 US 202363455861 P**

(43) Date of publication of application:
**09.04.2025 Bulletin 2025/15**

(73) Proprietor: **Hibercell, Inc.**
**New York, NY 10019 (US)**

(72) Inventors:
- **BOSE, Nandita**
**New York, New York 10019 (US)**
- **GARGANO, Michele**
**New York, New York 10019 (US)**
- **IGLESIAS, Jose**
**New York, New York 10019 (US)**
- **MULVIHILL, Mark J.**
**New York, New York 10019 (US)**
- **OLIVER-SHAFFER, Patricia**
**New York, New York 10019 (US)**
- **SURGULADZE, David**
**New York, New York 10019 (US)**
- **RAMURTHY, Savithri**
**New York, New York 10019 (US)**
- **TAMEIRE, Feven**
**New York, New York 10019 (US)**

(74) Representative: **Harris, Jennifer Lucy et al**
**Kilburn & Strode LLP**
**Lacon London**
**84 Theobalds Road**
**London WC1X 8NL (GB)**

(56) References cited:
**WO-A1-2021/222147** **WO-A1-2022/159746**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **JUN FUJIMOTO ET AL: "Identification of Novel, Potent, and Orally Available GCN2 Inhibitors with Type I Half Binding Mode", ACS MEDICINAL CHEMISTRY LETTERS, vol. 10, no. 10, 19 September 2019 (2019-09-19), US, pages 1498 - 1503, XP055663172, ISSN: 1948-5875, DOI: 10.1021/acsmedchemlett.9b00400**
- **LICARI EUGENIA ET AL: "The two faces of the Integrated Stress Response in cancer progression and therapeutic strategies", INTERNATIONAL JOURNAL OF BIOCHEMISTRY AND CELL BIOLOGY, ELSEVIER, AMSTERDAM, NL, vol. 139, 13 August 2021 (2021-08-13), XP086788050, ISSN: 1357-2725, [retrieved on 20210813], DOI: 10.1016/J.BIOCEL.2021.106059**
- **BOSE NANDITA ET AL: "Abstract 6231: Activation of GCN2 by HC-7366 results in significant antitumor efficacy as monotherapy and in combination with multiple standard of care agents in various solid cancer models | Cancer Research | American Association for Cancer Research", CANCER RES, vol. 87, no. Suppl 7, 4 April 2023 (2023-04-04), pages 6231, XP093077604, Retrieved from the Internet <URL:https://aacrjournals.org/cancerres/article/83/7_Supplement/6231/723695/Abstract-6231-Activation-of-GCN2-by-HC-7366> DOI: https://doi.org/10.1158/1538-7445.AM2023-6231**
- **PELSTER MEREDITH ET AL: "A multicenter, open-label, phase 1a/b study of HC-7366, a modulator of integrated stress response (ISR) kinase GCN2 in subjects with advanced solid tumors.", JOURNAL OF CLINICAL ONCOLOGY, vol. 40, no. 16_suppl, 1 June 2022 (2022-06-01), US, pages TPS3179 - TPS3179, XP093076990, ISSN: 0732-183X, DOI: 10.1200/JCO.2022.40.16_suppl.TPS3179**


(52) Cooperative Patent Classification (CPC): (Cont.)

C-Sets
**A61K 31/4439, A61K 2300/00;**
**A61K 31/496, A61K 2300/00;**
**A61K 31/4985, A61K 2300/00;**
**A61K 31/506, A61K 2300/00;**
**A61K 31/513, A61K 2300/00**

## Description

### CROSS REFERENCE TO RELATED APPLICATIONS

This application claims the benefit of and priority to U.S. Provisional Application Serial Nos. 63/345,727, filed May 25, 2022; 63/440,297, filed January 20, 2023; 63/443,269, filed February 3, 2023; and 63/455,861, filed March 30, 2023.

### BACKGROUND

Cancer is a leading cause of death worldwide, accounting for nearly 10 million deaths in 2020 (World Health Organization). Targeted therapy and immunotherapy have expanded the horizons for treatment of solid tumors by improving prognosis drastically. However, tumor recurrence, drug resistance, and drug intolerance continue to be major challenges in the management of advanced cancer (Wang et al., "Drug resistance and combating drug resistance in cancer," Cancer Drug Resistance, 2019, 2(2): 141-160; Chakraborty et al., "The difficulties in cancer treatment" Ecancermedicalscience, 2012, 6:ed16). Cancer cells often experience a variety of stressors in their microenvironment such as hypoxia, low pH, and deficiencies in nutrients. In order to survive harsh tumor microenvironments, cancer cells actively utilize adaptive stress pathways such as the integrated stress response (ISR) (Ye et al., "The GCN2-ATF4 pathway is critical for tumor cell survival and proliferation in response to nutrient deprivation," EMBO J., 2010, 29(12): 2082-2096; Pakos-Zebrucka et al., "The integrated stress response," EMBO Rep., 2016, 17(10): 1374-1395). The ISR consists of 4 kinases: protein kinase ribonucleic acid [RNA]-like endoplasmic reticulum kinase, protein kinase double-stranded RNA-dependent, general control nondepressible 2 (GCN2), and heme-regulated inhibitor (Donnelly et al., "The eIF2$\alpha$ kinases: their structures and functions," Cell Mol Life Sci., 2013, 70(19): 3493-3511). These four kinases sense unique stressors and phosphorylate $\alpha$-subunit of eukaryotic initiation factor 2 (eIF2$\alpha$) (Albert et al., "Adaptive Protein Translation by the Integrated Stress Response Maintains the Proliferative and Migratory Capacity of Lung Adenocarcinoma Cells," Mol Cancer Res., 2019, 17(12): 2343-2355). The high molecular weight kinase GCN2 senses amino acid deficiency as part of the ISR. Under amino acid starvation, uncharged transfer RNA accumulates and activates GCN2 (Anda et al., "Activation of Gen2 in response to different stresses," PLOS ONE, 2017, 12(8): E0182143). Phosphorylation of eIF2$\alpha$ by ISR kinases, such as GCN2, inhibits general protein synthesis during cellular stress but also promotes the translation of select mRNAs including activating transcription factor 4 (ATF4) which is a key effector of the ISR (Pakos-Zebrucka et al.). Once translated, ATF4 translocates to the nucleus and drives the expression of genes involved in adaptation to stress such as autophagy, antioxidant response, amino acid biosynthesis, and metabolism (Pakos-Zebrucka et al.; Harding et al., "An integrated stress response regulates amino acid metabolism and resistance to oxidative stress," Mol Cell, 2003, 11(3): 619-633). Other factors which activate GCN2 include ultraviolet light, viral infection, and oxidative stress (Costa-Mattioli et al., "The integrated stress response: From mechanism to disease," Science, 2020, 368(6489): eaat5314). ATF4 is important for tumor cells to maintain homeostasis of amino acid metabolism. Activation of the ISR pathway promotes tumor cell survival under nutrient deprivation (Ye et al.). GCN2/ATF4 expression is elevated in primary human liver, breast, lung, and head and neck tumors and GCN2 activation compared to normal tissue has been observed in colon, breast, and lung tumor samples.

ISR activation plays a dual role in cell fate decisions. During acute stress conditions, ISR can promote adaptation and during chronic stress conditions this pathway can turn apoptotic which results in increased phosphorylation of eIF2$\alpha$ for an extended time (Wortel et al., "Surviving Stress: Modulation of ATF4-Mediated Stress Responses in Normal and Malignant Cells," Trends Endocrinol Metabol. , 2017, 28(11): 794-806). By reducing protein synthesis or activating apoptotic pathways, prolonged activation of ISR can be harmful to cell growth (Wortel et al.; Harding et al., "Ppplr14 gene knockout reveals an essential role for translation initiation factor 2 alpha (eIF2alpha) dephosphorylation in mammalian development," Proc Natl Acad Sci USA, 2009, 106(6); 1832-1837; Münch, "The different axes of the mammalian mitochondrial unfolded protein response," BMC Biology, 2018; 16(1): 81). Persistent ISR activation as a consequence of mutation of eIF2$\alpha$ phosphatases has been shown to have a deleterious effect on embryogenesis due to inhibition of protein synthesis (Harding et al., "Ppplr14 gene knockout reveals an essential role for translation initiation factor 2 alpha (eIF2alpha) dephosphorylation in mammalian development," Proc Natl Acad Sci USA, 2009, 106(6); 1832-1837). GCN2 activation also can have antiproliferative effects through suppression of general protein synthesis and induction of cell cycle arrest preventing cells from growing during times of nutrient scarcity (Lehman et al., "Translation Upregulation of an Individual p21Cip1 Transcript Variant by GCN2 Regulates Cell Proliferation and Survival under Nutrient Stress," PLOS Genetics, 2015, 11(6): e1005212). Therefore, continuous activation of the GCN2 pathway could suppress protein synthesis and cell growth, thereby inhibiting tumor proliferation.

Thus, there remains an unmet need to develop new therapeutic strategies that utilize modulation, either activation or inhibition, of the GCN2 pathway for the treatment of a variety of cancers (e.g., advanced solid tumors and blood cancers).

## SUMMARY

The invention is set out in the appended set of claims. The references to methods of treatment in the subsequent paragraphs of this description are to be interpreted as references to the compounds, pharmaceutical compositions and medicaments of the present invention for use in a method for treatment of the human (or animal) body by therapy.

In one aspect, provided herein are methods of treating an advanced solid tumor in a subject in need thereof, comprising administering to the subject an effective amount of a compound of formula (I)

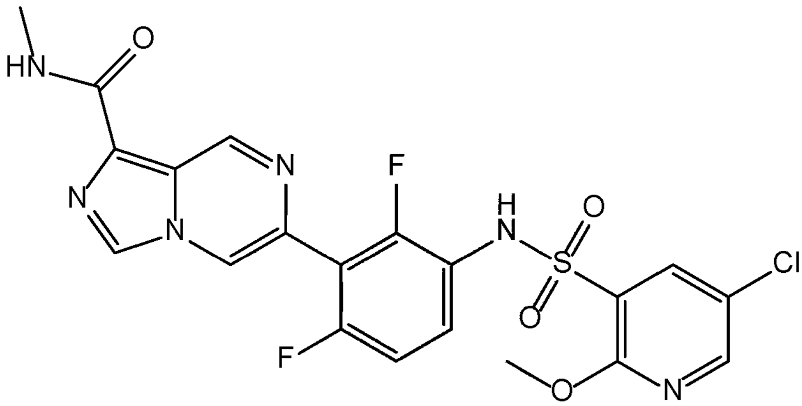

(I),

or a pharmaceutically acceptable salt thereof.

In certain embodiments, the advanced solid tumor is selected from the group consisting of squamous cell carcinoma of the head and neck, colorectal cancer, non-small cell lung cancer, renal cell carcinoma, and transitional cell carcinoma of the bladder.

In certain embodiments, the advanced solid tumor is selected from the group consisting of sarcoma, colorectal cancer, head and neck cancer, and prostate cancer.

In another aspect, provided herein are methods of treating a blood cancer in a subject in need thereof, comprising administering to the subject an effective amount of a compound of formula (I)

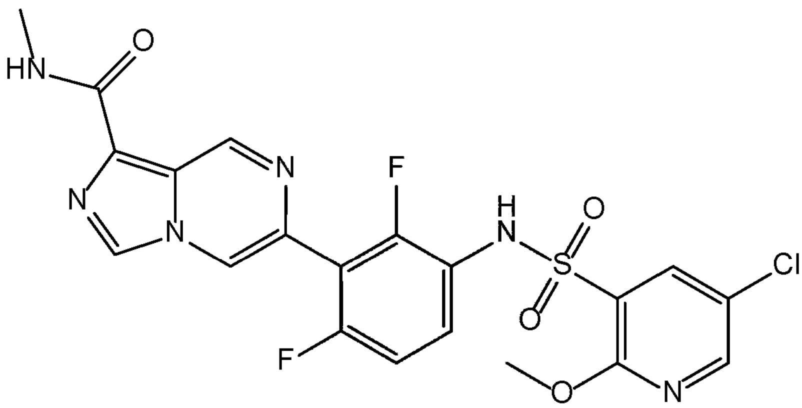

(I),

or a pharmaceutically acceptable salt thereof.

In certain embodiments, the blood cancer is a leukemia. In certain embodiments, the blood cancer is acute myeloid leukemia.

In some embodiments, the blood cancer is resistant to B-cell lymphoma inhibitors. In certain embodiments, the blood cancer is resistant to venetoclax.

In some embodiments, administering the effective amount of the compound of formula (I), or a pharmaceutically acceptable salt thereof, activates the integrated stress response pathway (ISR) in the advanced solid tumor or blood cancer. In some embodiments, the ISR activation is GCN2 dependent.

In some embodiments, administering the effective amount of the compound of formula (I), or a pharmaceutically acceptable salt thereof, induces expression of asparagine synthetase (ASNS), phosphoserine aminotransferase 1 (PSAT1), phosphoglycerate dehydrogenase (PHGDH), and/or BCL2 binding component 3 (PUMA) in the advanced solid tumor or blood cancer. In some embodiments, administering the effective amount of the compound of formula (I), or a

pharmaceutically acceptable salt thereof, reduces protein levels of S 100 calcium binding protein A8/A9 (S100A8/A9), hypoxia-inducible factor (HIF) 1α/2α, and/or glucose transporter type 1 (GLUT1) in the advanced solid tumor or blood cancer. In some embodiments, administering the effective amount of the compound of formula (I), or a pharmaceutically acceptable salt thereof, reduces mitochondrial respiration and/or glycolysis in the advanced solid tumor or blood cancer. In some embodiments, administering the effective amount of the compound of formula (I), or a pharmaceutically acceptable salt thereof, decreases myeloid restricted precursor and mature myeloid cells in the subject. In some embodiments, administering the effective amount of the compound of formula (I), or a pharmaceutically acceptable salt thereof, alters metabolites involved in amino acid metabolism, oxidative stress, the urea cycle, and/or pyrimidine biosynthesis in the advanced solid tumor or blood cancer. In some embodiments, administering the effective amount of the compound of formula (I), or a pharmaceutically acceptable salt thereof, reduces proteins involved in oxidative phosphorylation in the advanced solid tumor or blood cancer. In some embodiments, administering the effective amount of the compound of formula (I), or a pharmaceutically acceptable salt thereof, reduces activity of HIF and/or E2F transcription factor 1 (E2F1)-driven transcription in the advanced solid tumor or blood cancer. In some embodiments, administering the effective amount of the compound of formula (I), or a pharmaceutically acceptable salt thereof, increases ATF4 and/or (Jun Proto-Oncogene AP-1 Transcription Factor Subunit) JUN transcriptional activity in the advanced solid tumor or blood cancer.

In certain embodiments, administering the effective amount of the compound of formula (I), or a pharmaceutically acceptable salt thereof, comprises administering to the subject about 10 mg to about 150 mg of the compound of formula (I), or a pharmaceutically acceptable salt thereof, on a free acid equivalent weight basis. In certain embodiments, administering the effective amount of the compound of formula (I), or a pharmaceutically acceptable salt thereof, comprises administering orally to the subject about 10 mg to about 150 mg of the compound of formula (I), or a pharmaceutically acceptable salt thereof, on a free acid equivalent weight basis. In certain embodiments, administering the effective amount of the compound of formula (I), or a pharmaceutically acceptable salt thereof, comprises administering orally to the subject about 10 mg to about 150 mg of the compound of formula (I), or a pharmaceutically acceptable salt thereof, on a free acid equivalent weight basis, daily. In certain embodiments, administering the effective amount of the compound of formula (I), or a pharmaceutically acceptable salt thereof, comprises administering orally to the subject about 10 mg to about 150 mg of the compound of formula (I), or a pharmaceutically acceptable salt thereof, on a free acid equivalent weight basis, once daily. In certain embodiments, administering the effective amount of the compound of formula (I), or a pharmaceutically acceptable salt thereof, comprises administering orally to the subject about 10 mg to about 150 mg of the compound of formula (I), or a pharmaceutically acceptable salt thereof, on a free acid equivalent weight basis, once daily for 21 consecutive days.

In certain embodiments, the subject is in a fasting state. In certain embodiments, administering the effective amount of the compound of formula (I), or a pharmaceutically acceptable salt thereof, comprises administering to the subject about 10 mg to about 150 mg of the compound of formula (I), or a pharmaceutically acceptable salt thereof, on a free acid equivalent weight basis, about 1 hour before a meal or about 2 hours after a meal.

In certain embodiments, the subject has previously been administered at least one and no more than 5 prior lines of therapy.

In certain embodiments, the pharmaceutically acceptable salt is a potassium salt. In certain embodiments, the potassium salt is a hydrate. In certain embodiments, the potassium salt is a monohydrate.

In certain embodiments, the method further comprises administering an effective amount of a second therapeutic agent to the subject. In certain embodiments, the second therapeutic agent is selected from the group consisting of an immune checkpoint inhibitor, an epidermal growth factor receptor (EGFR) inhibitor, an antiangiogenic agent, venetoclax, fluorouracil, and combinations thereof.

In some embodiments, the second therapeutic agent is selected from the group consisting of an anti-vascular endothelial growth factor receptor (VEGFR) antibody, fluorouracil, a phosphoinositide 3-kinase alpha (PI3Kα) inhibitor, a mitogen-activated protein kinase kinase 1/2 (MEK1/2) inhibitor, and a hypoxia-inducible factor (HIF) inhibitor.

In some embodiments, the second therapeutic agent is venetoclax.

In some embodiments, administering the effective amount of the compound of formula (I), or a pharmaceutically acceptable salt thereof, and the venetoclax activates the integrated stress response pathway (ISR) in the advanced solid tumor or blood cancer to a greater extent than the compound of formula (I), or a pharmaceutically acceptable salt thereof, or venetoclax administered alone.

In some embodiments, the second therapeutic agent is an anti-VEGFR antibody.

In some embodiments, the second therapeutic agent is a HIF inhibitor. In certain embodiments, the second therapeutic agent is belzutifan.

In some embodiments, the second therapeutic agent is 5-fluorouracil.

In some embodiments, the second therapeutic agent is a PI3Kα inhibitor. In some embodiments, the second therapeutic agent is alpelisib.

In some embodiments, the second therapeutic agent is a MEK1/2 inhibitor. In some embodiments, the second therapeutic agent is trametinib.

In some embodiments, the second therapeutic agent is an EGFR inhibitor. In some embodiments, the second therapeutic agent is selected from osimertinib and dacomitinib.

In certain embodiments, the subject is a human. In certain embodiments, the subject is an adult human.

## BRIEF DESCRIPTION OF THE FIGURES

**FIG.** 1 is a graph showing the inhibitory effects of HC-7366 on protein kinase double-stranded RNA-dependent, general control nondepressible 2 (GCN2) activity in a biochemical assay. The data is presented as a plot of HC-7366 concentration vs. %Vehicle, where %Vehicle represents the DMSO control.

**FIG. 2** is a graph showing the effect of HC-7366 on activating transcription factor 4 (ATF4) activation in HT1080 cells using an activating transcription factor 4 (ATF4) activity assay.

**FIG. 3** is a graph showing the results of an ATF4 activity assay for HT1080 cells treated with HC-7366 and Halofuginone, demonstrating the inhibitory effect of HC-7366 on GCN2 in the ATF4 assay of **FIG. 2.**

**FIG. 4** is a graph showing the effects of HC-7366 on MOLM-16 cell viability using a CellTiter-Glo® (CTG) Assay.

**FIG. 5** is a Western blot showing the expression of various proteins, including GCN2 and ATF4, in HT1080 cells after treatment with HC-7366, with and without Halofuginone, at various concentrations, as indicated in the figure.

**FIG. 6** is a graph comparing the change in mean tumor volume over time in HT1080 tumor-bearing mice administered vehicle (e.g., 5% (v/v) DMSO and 95% (v/v) Captisol (20% w/v) in PBS (final pH 7.4)) twice daily, 0.3 mg/kg HC-7366 twice daily, 1 mg/kg HC-7366 twice daily, 3 mg/kg HC-7366 twice daily, 0.6 mg/kg HC-7366 once daily, 2 mg/kg HC-7366 once daily, or 6 mg/kg HC-7366 once daily.

**FIG. 7** is a graph comparing the change in mean tumor volume over time in HT1080 tumor-bearing mice administered vehicle (e.g., 5% (v/v) DMSO and 95% (v/v) Captisol (20% w/v) in PBS (final pH 7.4)) twice daily, 0.3 mg/kg HC-7366 twice daily, 1 mg/kg HC-7366 twice daily, or 3 mg/kg HC-7366 twice daily.

**FIG. 8** is a graph comparing the change in mean tumor volume over time in LoVo tumor-bearing mice administered vehicle (e.g., 5% (v/v) DMSO and 95% (v/v) Captisol (20% w/v) in PBS (final pH 7.4)) twice daily, 0.3 mg/kg HC-7366 twice daily, 1 mg/kg HC-7366 twice daily, 3 mg/kg HC-7366 twice daily, 10 mg/kg HC-7366 twice daily, or 30 mg/kg HC-7366 twice daily.

**FIG. 9** is a graph comparing the change in mean tumor volume over time in LoVo tumor-bearing mice administered vehicle (e.g., 5% (v/v) DMSO and 95% (v/v) Captisol (20% w/v) in PBS (final pH 7.4)) twice daily, 0.3 mg/kg HC-7366 twice daily, 1 mg/kg HC-7366 twice daily, or 3 mg/kg HC-7366 twice daily.

**FIG. 10** is a graph comparing the change in mean tumor volume over time in DLD-1 tumor-bearing mice administered vehicle (e.g., 5% (v/v) DMSO and 95% (v/v) Captisol (20% w/v) in PBS (final pH 7.4)) twice daily, 0.3 mg/kg HC-7366 twice daily, 1 mg/kg HC-7366 twice daily, 3 mg/kg HC-7366 twice daily, 10 mg/kg HC-7366 twice daily, or 30 mg/kg HC-7366 twice daily.

**FIG. 11** is a graph comparing the change in mean tumor volume over time in FaDu tumor-bearing mice administered vehicle (e.g., 5% (v/v) DMSO and 95% (v/v) Captisol (20% w/v) in PBS (final pH 7.4)) twice daily, 0.3 mg/kg HC-7366 twice daily, 1 mg/kg HC-7366 twice daily, 3 mg/kg HC-7366 twice daily, or 10 mg/kg HC-7366 twice daily.

**FIG. 12** is a graph comparing the change in mean tumor volume over time in FaDu tumor-bearing mice administered vehicle (e.g., 5% (v/v) DMSO and 95% (v/v) Captisol (20% w/v) in PBS (final pH 7.4)) twice daily, 0.3 mg/kg HC-7366 twice daily, 1 mg/kg HC-7366 twice daily, or 3 mg/kg HC-7366 twice daily.

**FIG. 13** is a graph comparing the change in mean tumor volume over time in FaDu tumor-bearing mice administered vehicle (e.g., 5% (v/v) DMSO and 95% (v/v) Captisol (20% w/v) in PBS (final pH 7.4)) twice daily, 0.3 mg/kg HC-7366 twice daily, 1 mg/kg HC-7366 twice daily, 3 mg/kg HC-7366 twice daily, 10 mg/kg HC-7366 twice daily, or 30 mg/kg HC-7366 twice daily.

**FIG. 14** is a graph comparing the change in mean tumor volume over time in LNCaP tumor-bearing mice administered vehicle (e.g., 5% (v/v) DMSO and 95% (v/v) Captisol (20% w/v) in PBS (final pH 7.4)) twice daily, 0.3 mg/kg HC-7366 twice daily, 1 mg/kg HC-7366 twice daily, 3 mg/kg HC-7366 twice daily, 10 mg/kg HC-7366 twice daily, or 30 mg/kg HC-7366 twice daily.

**FIG. 15** is a graph comparing the change in mean tumor volume over time in TM00298 (PDX) tumor-bearing mice administered with vehicle (e.g., 5% (v/v) DMSO and 95% (v/v) Captisol (20% w/v) in PBS (final pH 7.4)) twice daily, 3 mg/kg HC-7366 twice daily, or 30 mg/kg HC-7366 twice daily.

**FIG. 16** is a graph comparing the change in tumor volume over time in MOLM-16 tumor-bearing mice administered vehicle (e.g., 5% (v/v) DMSO and 95% (v/v) Captisol (20% w/v) in PBS (final pH 7.4)) twice daily, 0.25 mg/kg HC-7366 twice daily, 0.5 mg/kg HC-7366 twice daily, 1 mg/kg HC-7366 twice daily, or 2 mg/kg HC-7366 twice daily.

**FIG. 17** is a graph comparing the change in tumor volume over time in KG-1 tumor-bearing mice administered vehicle (e.g., 5% (v/v) DMSO and 95% (v/v) Captisol (20% w/v) in PBS (final pH 7.4)) twice daily, 0.3 mg/kg HC-7366 twice

daily, 1 mg/kg HC-7366 twice daily, 3 mg/kg HC-7366 twice daily, 10 mg/kg HC-7366 twice daily, or 30 mg/kg HC-7366 twice daily.

**FIG. 18** is a graph comparing the change in tumor volume over time in Kasumi-1 tumor-bearing mice administered vehicle (e.g., 5% (v/v) DMSO and 95% (v/v) Captisol (20% w/v) in PBS (final pH 7.4)) twice daily, 3 mg/kg HC-7366 twice daily, or 30 mg/kg HC-7366 twice daily.

**FIG. 19** is a graph comparing the change in tumor volume over time in OCI-AML2 tumor-bearing mice administered vehicle (e.g., 5% (v/v) DMSO and 95% (v/v) Captisol (20% w/v) in PBS (final pH 7.4)) twice daily, 0.3 mg/kg HC-7366 twice daily, 1 mg/kg HC-7366 twice daily, 3 mg/kg HC-7366 twice daily, 10 mg/kg HC-7366 twice daily, or 30 mg/kg HC-7366 twice daily.

**FIG. 20** is a graph comparing the change in tumor volume over time in MV4-11 tumor-bearing mice administered vehicle (e.g., 5% (v/v) DMSO and 95% (v/v) Captisol (20% w/v) in PBS (final pH 7.4)) twice daily, 0.3 mg/kg HC-7366 twice daily, 1 mg/kg HC-7366 twice daily, 3 mg/kg HC-7366 twice daily, 10 mg/kg HC-7366 twice daily, or 30 mg/kg HC-7366 twice daily.

**FIG. 21** is an image of immunohistochemical (IHC) assays of ASNS expressed in KG-1 tumors collected from the treatment groups described in **FIG. 17.** The tumors were collected on day 27 after beginning treatment (end of study).

**FIG. 22** is a plot showing the total intensity per unit of area of the immunohistochemical assays described in **FIG. 21.** ****$p<0.0001$, one-way ANOVA.

**FIG. 23** is an image of IHC assays of PSAT1 expressed in KG-1 tumors collected from the treatment groups described in **FIG. 17.** The tumors were collected on day 27 after beginning treatment (end of study).

**FIG. 24** is a plot showing the total intensity per unit of area of the immunohistochemical assays described in **FIG. 23.** *$p<0.05$, ****$p<0.0001$, one-way ANOVA.

**FIG. 25** is an image of IHC assays of PHGDH expressed in KG-1 tumors collected from the treatment groups described in **FIG. 17.** The tumors were collected on day 27 after beginning treatment (end of study).

**FIG. 26** is a plot showing the total intensity per unit of area of the immunohistochemical assays described in **FIG. 25.** ****$p<0.0001$, one-way ANOVA.

**FIG. 27** is an image of IHC assays of S100A8/A9 expressed in KG-1 tumors collected from the treatment groups described in **FIG. 17.** The tumors were collected on day 27 after beginning treatment (end of study).

**FIG. 28** is a plot showing the percentage of S100A8/A9+ cells in the tumors described in **FIG. 27.** *$p<0.05$, **$p<0.01$, one-way ANOVA.

**FIG. 29** is an image of IHC assays of ASNS expressed in DLD-1 tumors collected from the treatment groups described in **FIG. 10.** The tumors were collected on day 20 after beginning treatment (end of study).

**FIG. 30** is a plot showing the % positive by area of the IHC assays described in **FIG. 29.** *$p<0.05$, **$p<0.01$, ***$p<0.001$, one-way ANOVA.

**FIG. 31** is an image of IHC assays of PSAT1 expressed in DLD-1 tumors collected from the treatment groups described in **FIG. 10.** The tumors were collected on day 20 after beginning treatment (end of study).

**FIG. 32** is a plot showing the % positive by area of the IHC assays described in **FIG. 31.** ****$p<0.0001$, one-way ANOVA.

**FIG. 33** is an image of IHC assays of PUMA expressed in DLD-1 tumors collected from the treatment groups described in **FIG. 10.** The tumors were collected on day 20 after beginning treatment (end of study).

**FIG. 34** is a plot showing the % positive by area of the IHC assays described in **FIG. 33.** ****$p<0.0001$, one-way ANOVA.

**FIG. 35** is an image of IHC assays of HIf1$\alpha$ and HIf2$\alpha$ expressed in DLD-1 tumors collected from the treatment groups described in **FIG. 10.**

**FIG. 36** is a plot showing the expression of HIf1$\alpha$ (% positive cells) in the immunohistochemical assays described in **FIG. 35.** *$p<0.05$, ***$p<0.001$, ****$p<0.0001$, one-way ANOVA.

**FIG. 37** is a plot showing the expression of HIf2$\alpha$ (% positive cells) in the immunohistochemical assays described in **FIG. 35.** ***$p<0.001$, ****$p<0.0001$, one-way ANOVA.

**FIG. 38** is an image of IHC assays of HIF1$\alpha$ and HIF2$\alpha$ expressed in DLD-1 tumor-bearing mice administered vehicle (e.g., 5% (v/v) DMSO and 95% (v/v) Captisol (20% w/v) in PBS (final pH 7.4)) twice daily or 1 mg/kg HC-7366 twice daily.

**FIG. 39** is a plot showing the expression of HIf1$\alpha$ (% positive cells) in the immunohistochemical assays described in **FIG. 38.** **$p<0.01$, one-way ANOVA.

**FIG. 40** is an image of IHC assays of HIf1$\alpha$ and HIf2$\alpha$ expressed in FaDu tumors collected from the treatment groups described in **FIG. 13** as well as a treatment group administered 30 mg/kg HC-7366 twice daily.

**FIG. 41** is a plot showing the expression of HIf1$\alpha$ (% positive cells) in the immunohistochemical assays described in **FIG. 40.** **$p<0.01$, one-way ANOVA.

**FIG. 42** is a plot showing the expression of HIf2$\alpha$ (% positive cells) in the immunohistochemical assays described in **FIG. 40.** ***$p<0.001$, ****$p<0.0001$ one-way ANOVA.

**FIG. 43** is a graph comparing the change in tumor volume over time in MV4-11 tumors treated with vehicle (e.g., 5% (v/v) DMSO and 95% (v/v) Captisol (20% w/v) in PBS (final pH 7.4)) twice daily, 3 mg/kg HC-7366 twice daily, 30 mg/kg HC-7366 twice daily, 50 mg/kg venetoclax once daily, 3 mg/kg HC-7366 twice daily + 50 mg/kg venetoclax once daily, or 30 mg/kg HC-7366 twice daily + 50 mg/kg venetoclax once daily.

**FIG. 44** is an image of IHC assays of ASNS expressed in MV4-11 tumors collected from the treatment groups described in **FIG. 43.**

**FIG. 45** is a plot showing the expression of ASNS (% positive by area) of the immunohistochemical assays described in **FIG. 44.** ***p<0.001, ****p<0.0001, one-way ANOVA.

**FIG. 46** is an image of IHC assays of PHGDH expressed in MV4-11 tumors collected from the treatment groups described in **FIG. 43.**

**FIG. 47** is a plot showing the expression of PHGDH (% positive by area) of the immunohistochemical assays described in **FIG. 46.** **p<0.01, ****p<0.0001, one-way ANOVA.

**FIG. 48** is an image of IHC assays of PUMA expressed in MV4-11 tumors collected from the treatment groups described in **FIG. 43.**

**FIG. 49** is a plot showing the expression of PUMA (% positive by area) of the immunohistochemical assays described in **FIG. 48.** *p<0.05, ****p<0.0001, one-way ANOVA.

**FIG. 50** is an image of IHC assays of S100A8/A9 expressed in MV4-11 tumors collected from the treatment groups described in **FIG. 43.**

**FIG. 51** is a plot showing the percentage of S100A8/A9+ cells in the tumors described in **FIG. 50.** *p<0.05, **p<0.01, one-way ANOVA.

**FIG. 52** is an image of IHC assays of HIf2α and GLUT1 expressed in 786-O tumors collected from 786-O tumor-bearing mice administered vehicle (e.g., 5% (v/v) DMSO and 95% (v/v) Captisol (20% w/v) in PBS (final pH 7.4)) twice daily, 2 mg/kg HC-7366 twice daily, DC-101 15 mg/kg twice weekly, or 2 mg/kg HC-7366 twice daily + DC-101 15 mg/kg twice weekly.

**FIG. 53** is a plot showing the expression of HIf2α (% positive cells) in the immunohistochemical assays described in **FIG. 52.** *p<0.05, ****p<0.0001, one-way ANOVA.

**FIG. 54** is a plot showing the expression of GLUT1 (% positive by area) in the immunohistochemical assays described in **FIG. 52.** ****p<0.0001, one-way ANOVA.

**FIG. 55** is an image of IHC assays of HIf2α and GLUT1 expressed in A498 tumors collected from A498 tumor-bearing mice administered vehicle (e.g., 5% (v/v) DMSO and 95% (v/v) Captisol (20% w/v) in PBS (final pH 7.4)) twice daily, 2 mg/kg HC-7366 twice daily, DC-101 15 mg/kg twice weekly, or 2 mg/kg HC-7366 twice daily + DC-101 15 mg/kg twice weekly.

**FIG. 56** is a plot showing the expression of HIf2α (% positive cells) in the immunohistochemical assays described in **FIG. 55.** ***p<0.001, one-way ANOVA.

**FIG. 57** is a plot showing the expression of GLUT1 (% positive by area) in the immunohistochemical assays described in **FIG. 55.**

**FIG. 58** is a graph comparing the change in tumor volume over time in 786-O tumor-bearing mice administered vehicle (e.g., 5% (v/v) DMSO and 95% (v/v) Captisol (20% w/v) in PBS (final pH 7.4)) twice daily, 3 mg/kg HC-7366 twice daily, 0.1 mg/kg PT-2977 (belzutifan) twice daily, or 3 mg/kg HC-7366 twice daily + 0.1 mg/kg PT-2977 (belzutifan) twice daily.

**FIG. 59** is an image of IHC assays of HIF1α in head and neck tumors from human patients treated with 10 mg HC-7366 (102-101), colorectal tumors from human patients treated with 10 mg HC-7366 (102-201), and colorectal tumors from human patients treated with 20 mg HC-7366 (101-201).

**FIG. 60** is a plot showing the expression of HIf2α+ cells (% total cells) of the immunohistochemical assays described in **FIG. 59.**

**FIG. 61** is a graph comparing the change in mean tumor volume over time in DLD-1 tumor-bearing mice administered vehicle (e.g., 5% (v/v) DMSO and 95% (v/v) Captisol (20% w/v) in PBS (final pH 7.4)) twice daily, 3 mg/kg HC-7366 twice daily, 30 mg/kg HC-7366 twice daily, 20 mg/kg DC-101 biweekly, 20 mg/kg DC-101 biweekly + 3 mg/kg HC-7366 twice daily, or 20 mg/kg DC-101 biweekly + 30 mg/kg HC-7366 twice daily.

**FIG. 62** is a graph comparing the change in mean tumor volume over time in DLD-1 tumor-bearing mice administered vehicle (e.g., 5% (v/v) DMSO and 95% (v/v) Captisol (20% w/v) in PBS (final pH 7.4)) twice daily, 3 mg/kg HC-7366 twice daily, 30 mg/kg HC-7366 twice daily, 75 mg/kg 5-fluorouracil once weekly, 75 mg/kg 5-fluorouracil once weekly + 3 mg/kg HC-7366 twice daily, or 75 mg/kg 5-fluorouracil once weekly + 30 mg/kg HC-7366 twice daily.

**FIG. 63** is a graph comparing the change in mean tumor volume over time in HCT116 tumor-bearing mice administered vehicle (e.g., 5% (v/v) DMSO and 95% (v/v) Captisol (20% w/v) in PBS (final pH 7.4)) twice daily, 3 mg/kg HC-7366 twice daily, 30 mg/kg HC-7366 twice daily, 50 mg/kg alpelisib, 50 mg/kg alpelisib + 3 mg/kg HC-7366 twice daily, or 50 mg/kg alpelisib + 30 mg/kg HC-7366 twice daily.

**FIG. 64** is a graph comparing the change in mean tumor volume over time in HCT116 tumor-bearing mice

administered vehicle (e.g., 5% (v/v) DMSO and 95% (v/v) Captisol (20% w/v) in PBS (final pH 7.4)) twice daily, 3 mg/kg HC-7366 twice daily, 30 mg/kg HC-7366 twice daily, trametinib 1 mg/kg, trametinib 1 mg/kg + 3 mg/kg HC-7366 twice daily, or trametinib 1 mg/kg + 30 mg/kg HC-7366 twice daily.

**FIG. 65** is a graph showing the effects of HC-7366 on MOLM-16 GCN2 wild-type cell viability as measured by CTG assay 24 and 48 hours after treatment with HC-7366, compared to cells treated with vehicle (DMSO).

**FIG. 66** is a graph showing the effects of HC-7366 on MOLM-16 GCN2 CRISPR knockout cell viability as measured by CTG assay 24 and 48 hours after treatment with HC-7366, compared to cells treated with vehicle (DMSO).

**FIG. 67** is a graph showing the expression of GCN2 in the MOLM-16 GCN2 wild-type cells and MOLM-16 GCN2 CRISPR knockout cells described in **FIG. 65** and **FIG. 66,** respectively, post treatment with HC-7366.

**FIG. 68** is a graph showing the expression of ATF4 in the MOLM-16 GCN2 wild-type cells and MOLM-16 GCN2 CRISPR knockout cells described in **FIG. 65** and **FIG. 66,** respectively, post treatment with HC-7366.

**FIG. 69** is a graph showing the expression of ASNS in the MOLM-16 GCN2 wild-type cells and MOLM-16 GCN2 CRISPR knockout cells described in **FIG. 65** and **FIG. 66,** respectively, post treatment with HC-7366.

**FIG. 70** is a graph showing the expression of PSAT1 in the MOLM-16 GCN2 wild-type cells and MOLM-16 GCN2 CRISPR knockout cells described in **FIG. 65** and **FIG. 66,** respectively, post treatment with HC-7366.

**FIG. 71** is a graph showing the effects of HC-7366 on FaDu GCN2 wild-type cell viability as measured by CTG assay 72 and 96 hours after treatment with HC-7366, compared to cells treated with vehicle (DMSO).

**FIG. 72** is a graph showing the effects of HC-7366 on FaDu GCN2 CRISPR knockout cell viability as measured by CTG assay 72 and 96 hours after treatment with HC-7366, compared to cells treated with vehicle (DMSO).

**FIG. 73** is a graph showing the expression of pGCN2 in the FaDu wild-type cells and FaDu GCN2 CRISPR knockout cells described in **FIG. 71** and **FIG. 72,** respectively, post treatment with HC-7366.

**FIG. 74** is a graph showing the expression of GCN2 in the FaDu wild-type cells and FaDu GCN2 CRISPR knockout cells described in **FIG. 71** and **FIG. 72,** respectively, post treatment with HC-7366.

**FIG. 75** is a graph showing the expression of ATF4 in the FaDu GCN2 wild-type cells and FaDu CRISPR knockout cells described in **FIG. 71** and **FIG. 72,** respectively, post treatment with HC-7366.

**FIG. 76** is a graph showing the expression of ASNS in the FaDu GCN2 wild-type cells and FaDu GCN2 CRISPR knockout cells described in **FIG. 71** and **FIG. 72,** respectively, post treatment with HC-7366.

**FIG. 77** is a graph showing the absorbance of lysates from HEK 293 GCN2 wild-type cells over a sucrose gradient treated with either vehicle (DMSO) or 100 nM HC-7366 for 16 hours. The polysome to monosome ratio is also provided.

**FIG. 78** is a graph showing the absorbance of lysates from HEK 293 GCN2 CRISPR knockout cells over a sucrose gradient treated with either vehicle (DMSO) or 100 nM HC-7366 for 16 hours. The polysome to monosome ratio is also provided.

**FIG. 79** is a gel showing $\alpha$-puromycin staining of newly synthesized proteins at 100 nM HC-7366.

**FIG. 80** is a Western blot showing ISR marker levels post treatment with HC-7366.

**FIG. 81** is a graph showing the effects of HC-7366 on CTG-2229 cell viability as measured by CTG assay after 6 days of treatment with HC-7366.

**FIG. 82** is a graph showing the effects of HC-7366 on CTG-3680 cell viability as measured by CTG assay after 6 days of treatment with HC-7366.

**FIG. 83** is a graph showing the effects of HC-7366 on CTG-3667 cell viability as measured by CTG assay after 6 days of treatment with HC-7366.

**FIG. 84** is a graph showing the effects of HC-7366 on CTG-2456 cell viability as measured by CTG assay after 6 days of treatment with HC-7366.

**FIG. 85** is a graph showing the effects of HC-7366 on CTG-2457 cell viability as measured by CTG assay after 6 days of treatment with HC-7366.

**FIG. 86** is a graph showing the effects of HC-7366 on CTG-2454 cell viability as measured by CTG assay after 6 days of treatment with HC-7366.

**FIG. 87** is a series of t-distributed stochastic neighbor embedding (tSNE) plots showing the distribution of AML stem cells, myeloid restricted precursor cells, mature myeloid cells, non-myeloid cells, and other cell types of terminal samples collected from bone marrow (BM), whole blood (WB), and spleen (SP) of female NCG immunocompromised mice bearing a primary (P1) human acute myeloid leukemia (AML) xenotransplant TumorGraft model and treated with vehicle, 1 mg/kg HC-7366 twice daily for 28 days, 10 mg/kg HC-7366 twice daily for 28 days, 30 mg/kg HC-7366 twice daily for 28 days, or 100 mg/kg venetoclax once daily for 28 days.

**FIG. 88** is a graph showing the oxygen consumption rate (pmol/min) over time of MOLM-16 cells treated with vehicle (DMSO), 0.001 $\mu$M HC-7366, 0.1 $\mu$M HC-7366, or 10 $\mu$M HC-7366.

**FIG. 89** is a graph showing the extracellular acidification rate (ECAR) (mpH/min) over time of MOLM-16 cells treated with vehicle (DMSO), 0.001 $\mu$M HC-7366, 0.1 $\mu$M HC-7366, or 10 $\mu$M HC-7366.

**FIG. 90** is a heat map showing amino acid levels in MOLM-16 tumors treated with 0.3 mg/kg HC-7366, 1 mg/kg

HC-7366, 3 mg/kg HC-7366, 10 mg/kg HC-7366, or 30 mg/kg HC-7366 for 4 days normalized to vehicle control.

**FIG. 91** is a graph showing the scaled intensity of aspartate levels in the treatment groups described in **FIG. 90.**

**FIG. 92** is a graph showing the scaled intensity of cysteine levels in the treatment groups described in **FIG. 90.**

**FIG. 93** is a graph showing the scaled intensity of methionine levels in the treatment groups described in **FIG. 90.**

**FIG. 94** is a heat map showing oxidative stress marker levels in MOLM-16 tumors treated with 0.3 mg/kg HC-7366, 1 mg/kg HC-7366, 3 mg/kg HC-7366, 10 mg/kg HC-7366, or 30 mg/kg HC-7366 for 4 days normalized to vehicle control.

**FIG. 95** is schematic showing molecular pathways involved in glutathione production.

**FIG. 96** is a graph showing the scaled intensity of S-adenosylmethionine (SAM) levels in the treatment groups described in **FIG. 94.**

**FIG.** 97. is a graph showing the scaled intensity of oxidized glutathione (GSSG) levels in the treatment groups described in **FIG. 94.**

**FIG. 98** is a graph showing the scaled intensity of S-adenosylhomocysteine (SAH) levels in the treatment groups described in **FIG. 94.**

**FIG. 99** is a graph showing the scaled intensity of ophthalmate levels in the treatment groups described in **FIG. 94.**

**FIG. 100** is a graph showing the scaled intensity of cysteine levels in the treatment groups described in **FIG. 94.**

**FIG. 101** is a graph showing the scaled intensity of cystathionine levels in the treatment groups described in **FIG. 94.**

**FIG. 102** is a heat map showing pyrimidine synthesis metabolite levels in MOLM-16 tumors treated with 0.3 mg/kg HC-7366, 1 mg/kg HC-7366, 3 mg/kg HC-7366, 10 mg/kg HC-7366, or 30 mg/kg HC-7366 for 4 days normalized to vehicle control.

**FIG. 103** is a graph showing the scaled intensity of orotidine levels in the treatment groups described in **FIG. 102.**

**FIG. 104.** is a graph showing the scaled intensity of orotate levels in the treatment groups described in **FIG. 102.**

**FIG. 105** is a graph showing the scaled intensity of dihydroorotate levels in the treatment groups described in **FIG. 102.**

**FIG. 106** is a graph showing the scaled intensity of UMP levels in the treatment groups described in **FIG. 102.**

**FIG. 107** is a heat map showing aspartate metabolism marker levels in MOLM-16 tumors treated with 0.3 mg/kg HC-7366, 1 mg/kg HC-7366, 3 mg/kg HC-7366, 10 mg/kg HC-7366, or 30 mg/kg HC-7366 for 4 days normalized to vehicle control.

**FIG. 108** is schematic showing molecular pathways involved in aspartate metabolism.

**FIG. 109** is a graph showing the scaled intensity of ornithine levels in the treatment groups described in **FIG. 107.**

**FIG. 110** is a graph showing the scaled intensity of citrulline levels in the treatment groups described in **FIG. 107.**

**FIG. 111** is a graph showing the scaled intensity of asparagine levels in the treatment groups described in **FIG. 107.**

**FIG. 112** is a graph showing the scaled intensity of 5-methylthioadenosine (MTA) levels in the treatment groups described in **FIG. 107.**

**FIG. 113** is a graph showing the scaled intensity of arginine in the treatment groups described in **FIG. 107.**

**FIG. 114** is a graph showing the scaled intensity of aspartate in the treatment groups described in **FIG. 107.**

**FIG. 115** is a graph showing change in tumor amino acid levels in FaDu tumor-bearing mice treated with 1 mg/kg HC-7366 twice daily, 3 mg/kg HC-7366 twice daily, or 30 mg/kg HC-7366 twice daily for 4 days normalized to vehicle control. Statistically significant changes are marked with an asterisk ($p \leq 0.05$).

**FIG. 116** is a graph showing change in plasma amino acid levels in FaDu tumor-bearing mice treated with 1 mg/kg HC-7366 twice daily, 3 mg/kg HC-7366 twice daily, or 30 mg/kg HC-7366 twice daily for 4 days normalized to vehicle control. Statistically significant changes are marked with an asterisk ($p \leq 0.05$).

**FIG. 117** is a graph showing change in tumor gamma glutamyl amino acid levels in FaDu tumor-bearing mice treated with 1 mg/kg HC-7366 twice daily, 3 mg/kg HC-7366 twice daily, or 30 mg/kg HC-7366 twice daily for 4 days normalized to vehicle control. Statistically significant changes are marked with an asterisk ($p \leq 0.05$).

**FIG. 118** is a graph showing change in plasma gamma glutamyl amino acid levels in FaDu tumor-bearing mice treated with 1 mg/kg HC-7366 twice daily, 3 mg/kg HC-7366 twice daily, or 30 mg/kg HC-7366 twice daily for 4 days normalized to vehicle control. Statistically significant changes are marked with an asterisk ($p \leq 0.05$).

**FIG. 119** is a graph showing change in tumor urea cycle marker levels in FaDu tumor-bearing mice treated with 1 mg/kg HC-7366 twice daily, 3 mg/kg HC-7366 twice daily, or 30 mg/kg HC-7366 twice daily for 4 days normalized to vehicle control. Statistically significant changes are marked with an asterisk ($p \leq 0.05$).

**FIG. 120** is a graph showing change in plasma urea cycle marker levels in FaDu tumor-bearing mice treated with 1 mg/kg HC-7366 twice daily, 3 mg/kg HC-7366 twice daily, or 30 mg/kg HC-7366 twice daily for 4 days normalized to vehicle control. Statistically significant changes are marked with an asterisk ($p \leq 0.05$).

**FIG. 121** is a graph showing change in tumor pyrimidine synthesis marker levels in FaDu tumor-bearing mice treated with 0.3 mg/kg HC-7366 twice daily, 1 mg/kg HC-7366 twice daily, or 3 mg/kg HC-7366 twice daily for 4 days normalized to vehicle control. Statistically significant changes are marked with an asterisk ($p \leq 0.05$).

**FIG. 122** is a graph showing change in tumor oxidative stress marker levels in FaDu tumor-bearing mice treated with 1 mg/kg HC-7366 twice daily, 3 mg/kg HC-7366 twice daily, or 30 mg/kg HC-7366 twice daily for 4 days normalized to vehicle control. Statistically significant changes are marked with an asterisk ($p \leq 0.05$).

**FIG. 123** is a heat map showing intracellular amino acid levels in GCN2 wild type and GCN2 CRISPR knockout FaDu cells treated with vehicle (DMSO) or 0.1 μM HC-7366.

**FIG. 124** is a graph showing the GCN2 expression in GCN2 wild type and GCN2 CRISPR knockout FaDu cells treated with vehicle (DMSO) or 0.1 μM HC-7366.

**FIG. 125 is** a graph showing the ATF4 expression in GCN2 wild type and GCN2 CRISPR knockout FaDu cells treated with vehicle (DMSO) or 0.1 μM HC-7366.

**FIG. 126** is a schematic showing pathway analysis (IPA) of differentially expressed proteins in FaDu tumors, which predicted strong repression of the oxidative phosphorylation pathway with 3 mg/kg HC-7366, but not 30 mg/kg HC-7366.

**FIG. 127** is a heat map showing the fold change in oxidative phosphorylation proteins in FaDu tumor-bearing mice treated with 3 mg/kg HC-7366 or 30 mg/kg HC-7366.

**FIG. 128** is a graph comparing the change in mean tumor volume over time in DLD-1 tumor-bearing mice administered vehicle (e.g., 5% (v/v) DMSO and 95% (v/v) Captisol (20% w/v) in PBS (final pH 7.4)) twice daily, 0.3 mg/kg HC-7366 twice daily, 1 mg/kg HC-7366 twice daily, 3 mg/kg HC-7366 twice daily, 10 mg/kg HC-7366 twice daily, or 30 mg/kg HC-7366 twice daily.

**FIG. 129** is a chart showing the change in expression of upstream regulators of differentially expressed genes in the treatment groups described in **FIG. 128.**

**FIG. 130** is a plot showing the results of IHC staining of Ki67 positive cells in tumor sections of the treatment groups described in **FIG. 128,** *p<0.05, **p<0.01, one-way ANOVA.

**FIG. 131** is a graph comparing the change in mean tumor volume over time in NCI-H1975 tumor-bearing mice administered vehicle (e.g., 0.5% methyl cellulose (MC), 10 μL/g) twice daily, 3 mg/kg HC-7366 twice daily, osimertinib 2.5 mg/kg once daily, or 3 mg/kg HC-7366 twice daily + osimertinib 2.5 mg/kg once daily.

**FIG. 132** is a graph comparing the % body weight change over time in NCI-H1975 tumor-bearing mice administered vehicle (e.g., 0.5% MC, 10 μL/g) twice daily, 3 mg/kg HC-7366 twice daily, osimertinib 2.5 mg/kg once daily, or 3 mg/kg HC-7366 twice daily + osimertinib 2.5 mg/kg once daily.

**FIG. 133** is a graph comparing the change in mean tumor volume over time in NCI-H1975 tumor-bearing mice administered vehicle (e.g., 0.5% MC, 10 μL/g) twice daily, 3 mg/kg HC-7366 twice daily, dacomitinib 15 mg/kg once daily, or 3 mg/kg HC-7366 twice daily + dacomitinib 15 mg/kg once daily.

**FIG. 134** is a graph comparing the % body weight change over time in NCI-H1975 tumor-bearing mice administered vehicle (e.g., 0.5% MC, 10 μL/g) twice daily, 3 mg/kg HC-7366 twice daily, dacomitinib 15 mg/kg once daily, or 3 mg/kg HC-7366 twice daily + dacomitinib 15 mg/kg once daily.

**FIG. 135** is a graph comparing the change in tumor volume over time in MFE280 tumor-bearing mice administered vehicle twice daily, 0.5 mg/kg HC-7366 twice daily, 2 mg/kg HC-7366 twice daily, 1 mg/kg PT-2977 (belzutifan) twice daily, 0.5 mg/kg HC-7366 twice daily + 1 mg/kg PT-2977 twice daily, or 2 mg/kg HC-7366 twice daily + 1 mg/kg PT-2977 twice daily.

## DETAILED DESCRIPTION

As generally described herein, the present disclosure provides methods of treating an advanced solid tumor (e.g., advanced squamous cell carcinoma of the head and neck, colorectal cancer, non-small cell lung cancer (NSCLC), renal cell carcinoma, and transitional cell carcinoma of the bladder) in a subject in need thereof. The present disclosure also provides methods of treating a blood cancer (e.g., acute myeloid leukemia (AML)) in a subject in need thereof. The methods described herein generally comprise administering to the subject an effective amount of a compound of formula (I), or a pharmaceutically acceptable salt thereof.

### Definitions

To facilitate an understanding of the present invention, a number of terms and phrases are defined below.

Unless defined otherwise, all technical and scientific terms used herein have the same meaning as commonly understood by one of ordinary skill in the art to which this invention belongs. The abbreviations used herein have their conventional meaning within the chemical and biological arts. The chemical structures and formulae set forth herein are constructed according to the standard rules of chemical valency known in the chemical arts.

Throughout the description, where compositions and kits are described as having, including, or comprising specific components, or where processes and methods are described as having, including, or comprising specific steps, it is contemplated that, additionally, there are compositions and kits of the present invention that consist essentially of, or consist of, the recited components, and that there are processes and methods according to the present invention that consist essentially of, or consist of, the recited processing steps.

In the application, where an element or component is said to be included in and/or selected from a list of recited

elements or components, it should be understood that the element or component can be any one of the recited elements or components, or the element or component can be selected from a group consisting of two or more of the recited elements or components.

Where reference is made to a particular compound, that compound can be used in various embodiments of compositions of the present invention and/or in methods of the present invention, unless otherwise understood from the context. In other words, within this application, embodiments have been described and depicted in a way that enables a clear and concise application to be written and drawn, but it is intended and will be appreciated that embodiments may be variously combined or separated without parting from the present teachings and invention(s). For example, it will be appreciated that all features described and depicted herein can be applicable to all aspects of the invention(s) described and depicted herein.

The articles "a" and "an" are used in this disclosure to refer to one or more than one (i.e., to at least one) of the grammatical object of the article, unless the context is inappropriate. By way of example, "an element" means one element or more than one element.

The term "and/or" is used in this disclosure to mean either "and" or "or" unless indicated otherwise.

It should be understood that the expression "at least one of" includes individually each of the recited objects after the expression and the various combinations of two or more of the recited objects unless otherwise understood from the context and use. The expression "and/or" in connection with three or more recited objects should be understood to have the same meaning unless otherwise understood from the context.

The use of the term "include," "includes," "including," "have," "has," "having," "contain," "contains," or "containing," including grammatical equivalents thereof, should be understood generally as open-ended and non-limiting, for example, not excluding additional unrecited elements or steps, unless otherwise specifically stated or understood from the context.

Where the use of the term "about" is before a quantitative value, the present invention also includes the specific quantitative value itself, unless specifically stated otherwise. As used herein, the term "about" refers to a $\pm 10\%$ variation from the nominal value unless otherwise indicated or inferred from the context.

At various places in the present specification, variable or parameters are disclosed in groups or in ranges. It is specifically intended that the description include each and every individual subcombination of the members of such groups and ranges. For example, an integer in the range of 0 to 40 is specifically intended to individually disclose 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, and 40, and an integer in the range of 1 to 20 is specifically intended to individually disclose 1, 2, 3, 4, 5, 6, 7, 8, 9, 10, 11, 12, 13, 14, 15, 16, 17, 18, 19, and 20.

The use of any and all examples, or exemplary language herein, for example, "such as" or "including," is intended merely to illustrate better the present invention and does not pose a limitation on the scope of the invention unless claimed. No language in the specification should be construed as indicating any non-claimed element as essential to the practice of the present invention.

As a general matter, compositions specifying a percentage are by weight unless otherwise specified. Further, if a variable is not accompanied by a definition, then the previous definition of the variable controls.

As used herein, "pharmaceutical composition" or "pharmaceutical formulation" refers to the combination of an active agent with a carrier, inert or active, making the composition especially suitable for diagnostic or therapeutic use in vivo or ex vivo.

"Pharmaceutically acceptable" means approved or approvable by a regulatory agency of the federal or a state government or the corresponding agency in countries other than the United States, or that is listed in the U.S. Pharmacopoeia or other generally recognized pharmacopoeia for use in animals, and more particularly, in humans.

As used herein, "pharmaceutically acceptable salt" refers to any salt of an acidic or a basic group that may be present in a compound of the present invention (e.g., a compound of formula (I)), which salt is compatible with pharmaceutical administration.

As is known to those of skill in the art, "salts" of compounds may be derived from inorganic or organic acids and bases. Examples of acids include, but are not limited to, hydrochloric, hydrobromic, sulfuric, nitric, perchloric, fumaric, maleic, phosphoric, glycolic, lactic, salicylic, succinic, toluene-p-sulfonic, tartaric, acetic, citric, methanesulfonic, ethanesulfonic, formic, benzoic, malonic, naphthalene-2-sulfonic and benzenesulfonic acid. Other acids, such as oxalic, while not in themselves pharmaceutically acceptable, may be employed in the preparation of salts useful as intermediates in obtaining the compounds described herein and their pharmaceutically acceptable acid addition salts.

Examples of bases include, but are not limited to, alkali metal (e.g., sodium and potassium) hydroxides, alkaline earth metal (e.g., magnesium and calcium) hydroxides, ammonia, and compounds of formula $NW^{4+}$, wherein W is $C_{1-4}$ alkyl, and the like.

Examples of salts include, but are not limited to, acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, flucoheptanoate, glycerophosphate, hemisulfate, heptanoate, hexanoate, hydrochloride,

hydrobromide, hydroiodide, 2-hydroxyethanesulfonate, lactate, maleate, methanesulfonate, 2-naphthalenesulfonate, nicotinate, oxalate, pamoate, pectinate, persulfate, phenylpropionate, picrate, pivalate, propionate, succinate, tartrate, thiocyanate, tosylate, undecanoate, and the like. Other examples of salts include anions of the compounds of the present invention compounded with a suitable cation such as $Na^-$, $K^+$, $Ca^{2+}$, $NH^{4+}$, and $NW^{4+}$ (where W can be a $C_{1-4}$ alkyl group), and the like.

For therapeutic use, salts of the compounds of the present invention (e.g., a compound of formula (I)) are contemplated as being pharmaceutically acceptable. However, salts of acids and bases that are non-pharmaceutically acceptable may also find use, for example, in the preparation or purification of a pharmaceutically acceptable compound.

As used herein, "pharmaceutically acceptable excipient" refers to a substance that aids the administration of an active agent to and/or absorption by a subject and can be included in the compositions of the present invention without causing a significant adverse toxicological effect on the patient. Non-limiting examples of pharmaceutically acceptable excipients include water, NaCl, normal saline solutions, such as a phosphate buffered saline solution, emulsions (e.g., such as an oil/water or water/oil emulsions), lactated Ringer's, normal sucrose, normal glucose, binders, fillers, disintegrants, lubricants, coatings, sweeteners, flavors, salt solutions (such as Ringer's solution), alcohols, oils, gelatins, carbohydrates, fatty acid esters, and colors, and the like. Such preparations can be sterilized and, if desired, mixed with auxiliary agents such as lubricants, preservatives, stabilizers, wetting agents, emulsifiers, salts for influencing osmotic pressure, buffers, coloring, and/or aromatic substances and the like that do not deleteriously react with the compounds of the invention. For examples of excipients, see Martin, Remington's Pharmaceutical Sciences, 15th Ed., Mack Publ. Co., Easton, PA (1975).

The term "AUC" refers to the area under the time/plasma concentration curve after administration of the compound of formula (I), or pharmaceutically acceptable salt thereof, or a pharmaceutical composition described herein. $AUC_{0\text{-infinity}}$ denotes the area under the plasma concentration versus time curve from time 0 to infinity. $AUC_{0\text{-}t}$ denotes the area under the plasma concentration versus time curve from time 0 to time t. It should be appreciated that AUC values can be determined by known methods in the art.

A "subject" to which administration is contemplated includes, but is not limited to, humans (i.e., a male or female of any age group, e.g., a pediatric subject (e.g., infant, child, adolescent) or adult subject (e.g., young adult, middle-aged adult or senior adult)) and/or a non-human animal, e.g., a mammal such as primates (e.g., cynomolgus monkeys, rhesus monkeys), cattle, pigs, horses, sheep, goats, rodents, cats, and/or dogs. In certain embodiments, the subject is a human. In certain embodiments, the subject is an adult human. In certain embodiments, the subject is a non-human animal.

The term "$C_{max}$" refers to the maximum concentration of a therapeutic agent in the blood (e.g., plasma) following administration of the therapeutic agent (e.g., the compound of formula (I), or a pharmaceutically acceptable salt thereof) or a pharmaceutical composition comprising the therapeutic agent (e.g., a pharmaceutical composition described herein).

The term "$t_{max}$" refers to the time in hours when $C_{max}$ is achieved following administration of a therapeutic agent (e.g., a compound of formula (I), or a pharmaceutically acceptable salt thereof) or a pharmaceutical composition comprising the therapeutic agent (e.g., a pharmaceutical composition described herein).

As used herein, "solid dosage form" means a pharmaceutical dose(s) in solid form, e.g., tablets, capsules, granules, powders, sachets, reconstitutable powders, dry powder inhalers and chewables.

As used herein, "administering" means oral administration, administration as a suppository, topical contact, intravenous administration, parenteral administration, intraperitoneal administration, intramuscular administration, intralesional administration, intrathecal administration, intracranial administration, intranasal administration or subcutaneous administration, or the implantation of a slow-release device, e.g., a miniosmotic pump, to a subject. Administration is by any route, including parenteral and transmucosal (e.g., buccal, sublingual, palatal, gingival, nasal, vaginal, rectal, or transdermal). Parenteral administration includes, e.g., intravenous, intramuscular, intraarterial, intradermal, subcutaneous, intraperitoneal, intraventricular, and intracranial. Other modes of delivery include, but are not limited to, the use of liposomal formulations, intravenous infusion, transdermal patches, etc. By "co-administer" it is meant that a composition described herein is administered at the same time, just prior to, or just after the administration of one or more additional therapies (e.g., anti-cancer agent, chemotherapeutic, or immunotherapy). The compound of formula (I), or a pharmaceutically acceptable salt thereof, can be administered alone or can be co-administered to the patient. Coadministration is meant to include simultaneous or sequential administration of the compound individually or in combination (more than one compound or agent). Thus, the preparations can also be combined, when desired, with other active substances (e.g., to reduce metabolic degradation).

As used herein, "fasting state" means at least 1 hour before food or at least 2 hours after food is consumed by a subject.

The terms "disease," "disorder," and "condition" are used interchangeably herein.

As used herein, and unless otherwise specified, the terms "treat," "treating" and "treatment" contemplate an action that occurs while a subject is suffering from the specified disease, disorder or condition, which reduces the severity of the disease, disorder or condition, or retards or slows the progression of the disease, disorder or condition (e.g., "therapeutic treatment").

In general, an "effective amount" of a compound (e.g., a compound of formula (I), or a pharmaceutically acceptable salt thereof) refers to an amount sufficient to elicit the desired biological response, e.g., to treat an advanced solid tumor and/or a blood cancer. As will be appreciated by those of ordinary skill in this art, the effective amount of a compound of the disclosure may vary depending on such factors as the desired biological endpoint, the pharmacokinetics of the compound, the disease being treated, the mode of administration, and the age, weight, health, and condition of the subject.

**Compound**

The compound of formula (I), as depicted below, is a selective GCN2 modulator (e.g., activation or inhibition of GCN2), and also known as 6-(3-((5-chloro-2-methoxypyridine)-3-sulfonamido)-2,6-difluorophenyl)-N-methylimidazo [1,5-a]pyrazine-1-carboxamide:

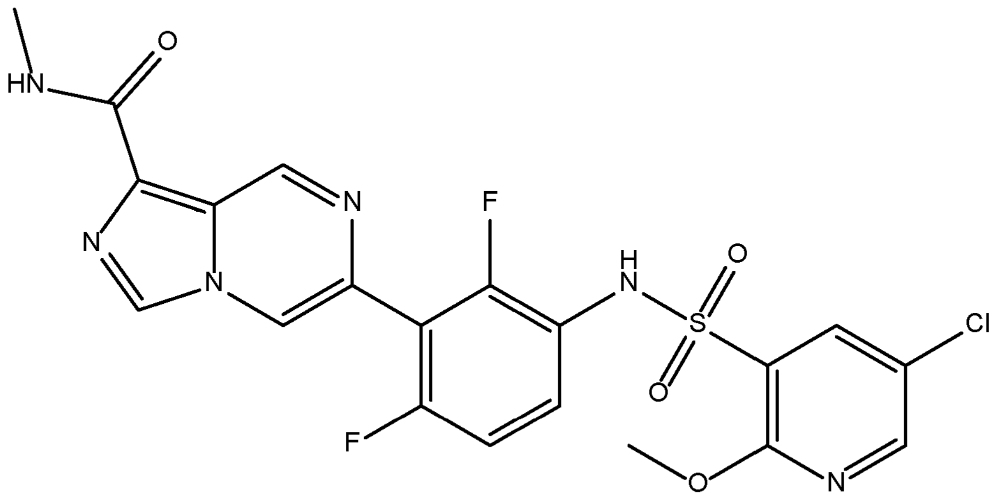

(I).

The compound of formula (I) is also referred to as HC-7366 throughout the present disclosure. A method of chemically synthesizing the compound of formula (I) is described in Example 1.

In one aspect, provided herein is a method of administering the compound of formula (I), or a pharmaceutically acceptable salt thereof, for the treatment of an advanced solid tumor (e.g., squamous cell carcinoma of the head and neck, colorectal cancer, NSCLC, renal cell carcinoma, and transitional cell carcinoma of the bladder) in a subject in need thereof.

In another aspect, provided herein is a method of administering the compound of formula (I), or a pharmaceutically acceptable salt thereof, for the treatment of a blood cancer (e.g., AML) in a subject in need thereof.

In various embodiments, provided herein is a method of administering a pharmaceutically acceptable salt of the compound of formula (I) for the treatment of an advanced solid tumor (e.g., squamous cell carcinoma of the head and neck, colorectal cancer, NSCLC, renal cell carcinoma, and transitional cell carcinoma of the bladder) in a subject in need thereof.

In various embodiments, provided herein is a method of administering a pharmaceutically acceptable salt of the compound of formula (I) for the treatment of a blood cancer (e.g., AML) in a subject in need thereof.

In certain embodiments, the pharmaceutically acceptable salt of the compound of formula (I) is a potassium salt. In certain embodiments, the potassium salt of the compound of formula (I) is a hydrate. In certain embodiments, the potassium salt of the compound of formula (I) is a monohydrate. A method of preparing a potassium salt of the compound of formula (I) is described in Example 2.

**Pharmaceutical Compositions**

Provided herein are pharmaceutical compositions generally comprising a compound of formula (I), or a pharmaceutically acceptable salt thereof, and one or more pharmaceutically acceptable excipients.

In one aspect, provided herein is a method of administering a pharmaceutical composition comprising a compound of formula (I), or a pharmaceutically acceptable salt thereof, and one or more pharmaceutically acceptable excipients, for the treatment of an advanced solid tumor (e.g., squamous cell carcinoma of the head and neck, colorectal cancer, NSCLC, renal cell carcinoma, and transitional cell carcinoma of the bladder) in a subject in need thereof.

In another aspect, provided herein is a method of administering a pharmaceutical composition comprising a compound of formula (I), or a pharmaceutically acceptable salt thereof, and one or more pharmaceutically acceptable excipients, for the treatment of a blood cancer (e.g., AML) in a subject in need thereof.

In another aspect, provided herein is a method of administering a pharmaceutical composition comprising a pharmaceutically acceptable salt of a compound of formula (I) and one or more pharmaceutically acceptable excipients, for the treatment of an advanced solid tumor (e.g., squamous cell carcinoma of the head and neck, colorectal cancer, NSCLC, renal cell carcinoma, and transitional cell carcinoma of the bladder) in a subject in need thereof.

In another aspect, provided herein is a method of administering a pharmaceutical composition comprising a pharmaceutically acceptable salt of a compound of formula (I) and one or more pharmaceutically acceptable excipients, for the treatment of a blood cancer (e.g., AML) in a subject in need thereof.

In various embodiments, provided herein is a pharmaceutical composition comprising an effective amount of a compound of formula (I), or a pharmaceutically acceptable salt thereof, and one or more pharmaceutically acceptable excipients.

In various embodiments, provided herein is a pharmaceutical composition comprising an effective amount of a pharmaceutically acceptable salt of a compound of formula (I) and one or more pharmaceutically acceptable excipients.

In certain embodiments, the amount of the compound of formula (I), or a pharmaceutically acceptable salt thereof, in a pharmaceutical composition described herein is about 10 mg to about 150 mg, about 20 mg to about 150 mg, about 40 mg to about 150 mg, about 75 mg to about 150 mg, about 125 mg to about 150 mg, about 10 mg to about 125 mg, about 10 mg to about 75 mg, about 10 mg to about 40 mg, about 10 mg to about 20 mg, about 20 mg to about 125 mg, about 20 mg to about 75 mg, about 20 mg to about 40 mg, about 40 mg to about 125 mg, about 40 mg to about 75 mg, or about 75 mg to about 125 mg, on a free acid equivalent weight basis. In certain embodiments, the amount of the compound of formula (I), or a pharmaceutically acceptable salt thereof, in a pharmaceutical composition described herein is about 10 mg to about 150 mg on a free acid equivalent weight basis.

In certain embodiments, the amount of the compound of formula (I), or a pharmaceutically acceptable salt thereof, in a pharmaceutical composition described herein is about 10 mg, about 15 mg, about 20 mg, about 25 mg, about 30 mg, about 35 mg, about 40 mg, about 45 mg, about 50 mg, about 60 mg, about 70 mg, about 80 mg, about 90 mg, about 100 mg, about 125 mg, or about 150 mg, on a free acid equivalent weight basis. In certain embodiments, the amount of the compound of formula (I), or a pharmaceutically acceptable salt thereof, in a pharmaceutical composition described herein is about 10 mg on a free acid equivalent weight basis. In certain embodiments, the amount of the compound of formula (I), or a pharmaceutically acceptable salt thereof, in a pharmaceutical composition described herein is about 20 mg on a free acid equivalent weight basis. In certain embodiments, the amount of the compound of formula (I), or a pharmaceutically acceptable salt thereof, in a pharmaceutical composition described herein is about 40 mg on a free acid equivalent weight basis. In certain embodiments, the amount of the compound of formula (I), or a pharmaceutically acceptable salt thereof, in a pharmaceutical composition described herein is about 75 mg on a free acid equivalent weight basis. In certain embodiments, the amount of the compound of formula (I), or a pharmaceutically acceptable salt thereof, in a pharmaceutical composition described herein is about 125 mg on a free acid equivalent weight basis. In certain embodiments, the amount of the compound of formula (I), or a pharmaceutically acceptable salt thereof, in a pharmaceutical composition described herein is about 150 mg on a free acid equivalent weight basis.

In certain embodiments, the amount of the pharmaceutically acceptable salt of a compound of formula (I) in a pharmaceutical composition described herein is about 10 mg to about 150 mg, about 20 mg to about 150 mg, about 40 mg to about 150 mg, about 75 mg to about 150 mg, about 125 mg to about 150 mg, about 10 mg to about 125 mg, about 10 mg to about 75 mg, about 10 mg to about 40 mg, about 10 mg to about 20 mg, about 20 mg to about 125 mg, about 20 mg to about 75 mg, about 20 mg to about 40 mg, about 40 mg to about 125 mg, about 40 mg to about 75 mg, or about 75 mg to about 125 mg, on a free acid equivalent weight basis. In certain embodiments, the amount of the pharmaceutically acceptable salt of a compound of formula (I) in a pharmaceutical composition described herein is about 10 mg to about 150 mg on a free acid equivalent weight basis.

In certain embodiments, the amount of the pharmaceutically acceptable salt of a compound of formula (I) in a pharmaceutical composition described herein is about 10 mg, about 15 mg, about 20 mg, about 25 mg, about 30 mg, about 35 mg, about 40 mg, about 45 mg, about 50 mg, about 60 mg, about 70 mg, about 80 mg, about 90 mg, about 100 mg, about 125 mg, or about 150 mg, on a free acid equivalent weight basis. In certain embodiments, the amount of the pharmaceutically acceptable salt of a compound of formula (I) in a pharmaceutical composition described herein is about 10 mg on a free acid equivalent weight basis. In certain embodiments, the amount of the pharmaceutically acceptable salt of a compound of formula (I) in a pharmaceutical composition described herein is about 20 mg on a free acid equivalent weight basis. In certain embodiments, the amount of the pharmaceutically acceptable salt of a compound of formula (I) in a pharmaceutical composition described herein is about 40 mg on a free acid equivalent weight basis. In certain embodiments, the amount of the pharmaceutically acceptable salt of a compound of formula (I) in a pharmaceutical composition described herein is about 75 mg on a free acid equivalent weight basis. In certain embodiments, the amount of the pharmaceutically acceptable salt of a compound of formula (I) in a pharmaceutical composition described herein is about 125 mg on a free acid equivalent weight basis. In certain embodiments, the amount of the pharmaceutically acceptable salt of a compound of formula (I) in a pharmaceutical composition described herein is about 150 mg on a free acid equivalent weight basis.

In various embodiments, provided herein are pharmaceutical compositions comprising:

(i) about 10 mg to about 150 mg of a compound of formula (I), or a pharmaceutically acceptable salt thereof, on a free acid equivalent weight basis; and
(ii) one or more pharmaceutically acceptable excipients.

In various embodiments, provided herein are pharmaceutical compositions comprising:

(i) about 10 mg to about 150 mg of a pharmaceutically acceptable salt of the compound of formula (I), on a free acid equivalent weight basis; and
(ii) one or more pharmaceutically acceptable excipients.

In another aspect, provided herein are pharmaceutical compositions comprising about 10 mg to about 150 mg of a compound of formula (I), or a pharmaceutically acceptable salt thereof, on a free acid equivalent weight basis, and one or more pharmaceutically acceptable excipients, for the treatment of an advanced solid tumor (e.g., squamous cell carcinoma of the head and neck, colorectal cancer, NSCLC, renal cell carcinoma, and transitional cell carcinoma of the bladder) in a subject in need thereof.

In another aspect, provided herein are pharmaceutical compositions comprising about 10 mg to about 150 mg of a compound of formula (I), or a pharmaceutically acceptable salt thereof, on a free acid equivalent weight basis, and one or more pharmaceutically acceptable excipients, for the treatment of a blood cancer (e.g., AML) in a subject in need thereof.

In another aspect, provided herein are pharmaceutical compositions comprising about 10 mg to about 150 mg of a pharmaceutically acceptable salt of the compound of formula (I), on a free acid equivalent weight basis, and one or more pharmaceutically acceptable excipients, for the treatment of a blood cancer (e.g., AML) in a subject in need thereof.

In another aspect, provided herein are pharmaceutical compositions comprising about 10 mg to about 150 mg of a pharmaceutically acceptable salt of the compound of formula (I), on a free acid equivalent weight basis, and one or more pharmaceutically acceptable excipients, for the treatment of an advanced solid tumor (e.g., squamous cell carcinoma of the head and neck, colorectal cancer, NSCLC, renal cell carcinoma, and transitional cell carcinoma of the bladder) in a subject in need thereof.

In certain embodiments, the pharmaceutically acceptable salt of the compound of formula (I) is a potassium salt. In certain embodiments, the potassium salt of the compound of formula (I) is a hydrate. In certain embodiments, the potassium salt of the compound of formula (I) is a monohydrate.

The pharmaceutical compositions described herein can be administered by a variety of routes including, but not limited to, oral (enteral) administration, parenteral (by injection) administration, rectal administration, transdermal administration, intradermal administration, intrathecal administration, subcutaneous (SC) administration, intravenous (IV) administration, intramuscular (IM) administration, and intranasal administration. In certain embodiments, the pharmaceutical compositions described herein are administered orally.

The pharmaceutical compositions described herein may also be administered chronically ("chronic administration"). Chronic administration refers to administration of a compound or pharmaceutical composition thereof over an extended period of time, *e.g.,* for example, over 3 months, 6 months, 1 year, 2 years, 3 years, 5 years, *etc.,* or may be continued indefinitely, for example, for the rest of the subject's life. In certain embodiments, the chronic administration is intended to provide a constant level of the compound in the blood, e.g., within the therapeutic window over the extended period of time.

The pharmaceutical compositions described herein may be presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions.

In certain embodiments, the pharmaceutical compositions provided herein are administered to the patient as a solid dosage form. In certain embodiments, the solid dosage form is a capsule.

In certain embodiments, the pharmaceutical composition is an immediate release capsule formulation of HC-7366 potassium salt monohydrate. In some embodiments, the capsule is a hard gelatin capsule. In some embodiments, the capsule comprises one or more of lactose monohydrate, microcrystalline cellulose, croscarmellose sodium, colloidal silicon dioxide, and magnesium stearate. In some embodiments, the capsule comprises each of lactose monohydrate, microcrystalline cellulose, croscarmellose sodium, colloidal silicon dioxide, and magnesium stearate. In some embodiments, the capsule comprises 10 mg, 25 mg, or 100 mg of HC-7366 potassium salt monohydrate on a free acid equivalent weight basis.

Although the descriptions of pharmaceutical compositions provided herein are principally directed to pharmaceutical compositions which are suitable for administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and/or perform such modification with ordinary experimentation. General considerations in the formulation and/or manufacture of pharmaceutical compositions can be found, for example, in Remington: The Science and Practice of Pharmacy 21st ed., Lippincott Williams & Wilkins, 2005.

**Methods of Use and Treatment**

Provided herein are methods of treating a solid tumor in a subject in need thereof. The methods generally comprise administering to the subject an effective amount of a compound of formula (I), or a pharmaceutically acceptable salt thereof; or a pharmaceutical composition described herein. In certain embodiments, the solid tumor is an advanced solid tumor.

Solid tumors the compound of formula (I), or a pharmaceutically acceptable salt thereof, is contemplated to be useful in treating include, but are not limited to, pancreatic cancer; bladder cancer; colorectal cancer; breast cancer, including metastatic breast cancer; prostate cancer, including androgen-dependent and androgen-independent prostate cancer; kidney or renal cancer, including, e.g., metastatic renal cell carcinoma; hepatocellular cancer; lung cancer, including, e.g., NSCLC, bronchioloalveolar carcinoma (BAC), and adenocarcinoma of the lung; ovarian cancer, including, e.g., progressive epithelial or primary peritoneal cancer; cervical cancer; gastric cancer; esophageal cancer; head and neck cancer, including, e.g., squamous cell carcinoma of the head and neck; melanoma; neuroendocrine cancer, including metastatic neuroendocrine tumors; brain tumors, including, e.g., glioma, anaplastic oligodendroglioma, adult glioblastoma multiforme, and adult anaplastic astrocytoma; bone cancer; and soft tissue sarcoma, hepatic carcinoma, rectal cancer, penile carcinoma, vulval cancer, thyroid cancer, salivary gland carcinoma, endometrial or uterine carcinoma, hepatoma, hepatocellular cancer, liver cancer, gastric or stomach cancer including gastrointestinal cancer, cancer of the peritoneum, squamous carcinoma of the lung, gastroesophageal cancer, biliary tract cancer, gall bladder cancer, colorectal/appendiceal cancer, and squamous cell cancer (e.g., epithelial squamous cell cancer).

In certain embodiments, the advanced solid tumor is selected from the group consisting of squamous cell carcinoma of the head and neck, colorectal cancer, NSCLC, renal cell carcinoma, and transitional cell carcinoma of the bladder.

In certain embodiments, the advanced solid tumor is a sarcoma. In certain embodiments, the advanced solid tumor is colorectal cancer. In certain embodiments, the advanced solid tumor is a head and neck cancer. In certain embodiments, the advanced solid tumor is renal cell carcinoma. In certain embodiments, the advanced solid tumor is prostate cancer. In certain embodiments, the advanced solid tumor is NSCLC. In certain embodiments, the advanced solid tumor is endometrial cancer.

In certain embodiments, the advanced solid tumor is selected from the group consisting of sarcoma, colorectal cancer, head and neck cancer, and prostate cancer.

Also provided herein are methods of treating a blood cancer in a subject in need thereof. The methods generally comprise administering to the subject an effective amount of a compound of formula (I), or a pharmaceutically acceptable salt thereof.

In certain embodiments, the blood cancer is selected from the group consisting of multiple myeloma, leukemia, and lymphoma. In certain embodiments, the leukemia is selected from the group consisting of chronic lymphocytic leukemia, chronic myelocytic leukemia, acute lymphoblastic leukemia, and AML.

In certain embodiments, the blood cancer is leukemia. In certain embodiments, the blood cancer is AML.

In some embodiments, the blood cancer is resistant to B-cell lymphoma inhibitors. In certain embodiments, the blood cancer is resistant to venetoclax.

In some embodiments, administering the effective amount of the compound of formula (I), or a pharmaceutically acceptable salt thereof, activates the integrated stress response pathway (ISR) in the advanced solid tumor or blood cancer. In some embodiments, the ISR activation is GCN2 dependent.

In some embodiments, administering the effective amount of the compound of formula (I), or a pharmaceutically acceptable salt thereof, induces expression of ASNS, PSAT1, PHGDH, argininosuccinate synthase 1 (ASS 1), and/or PUMA in the advanced solid tumor or blood cancer. In some embodiments, administering the effective amount of the compound of formula (I), or a pharmaceutically acceptable salt thereof, reduces protein levels of S 100A8/A9 in the advanced solid tumor or blood cancer. In some embodiments, administering the effective amount of the compound of formula (I), or a pharmaceutically acceptable salt thereof, reduces mitochondrial respiration in the advanced solid tumor or blood cancer. In some embodiments, administering the effective amount of the compound of formula (I), or a pharmaceutically acceptable salt thereof, decreases myeloid restricted precursor and mature myeloid cells in the subject. In some embodiments, administering the effective amount of the compound of formula (I), or a pharmaceutically acceptable salt

thereof, alters metabolites involved in amino acid metabolism, oxidative stress, the urea cycle, and/or pyrimidine biosynthesis in the advanced solid tumor or blood cancer. In some embodiments, administering the effective amount of the compound of formula (I), or a pharmaceutically acceptable salt thereof, reduces proteins involved in oxidative phosphorylation in the advanced solid tumor or blood cancer. In some embodiments, administering the effective amount of the compound of formula (I), or a pharmaceutically acceptable salt thereof, reduces activity of HIF and/or E2F1-driven transcription in the advanced solid tumor or blood cancer. In some embodiments, HIF and/or E2F1-driven transcription includes expression of metaphase-anaphase transition genes. In some embodiments, administering the effective amount of the compound of formula (I), or a pharmaceutically acceptable salt thereof, increases ATF4 and/or JUN transcriptional activity in the advanced solid tumor or blood cancer.

In certain embodiments, administering the effective amount of the compound of formula (I), or a pharmaceutically acceptable salt thereof, comprises administering to the subject about 10 mg to about 150 mg, about 15 mg to about 150 mg, about 20 mg to about 150 mg, about 25 mg to about 150 mg, about 30 mg to about 150 mg, about 35 mg to about 150 mg, about 40 mg to about 150 mg, about 45 mg to about 150 mg, about 50 mg to about 150 mg, about 55 mg to about 150 mg, about 60 mg to about 150 mg, about 65 mg to about 150 mg, about 70 mg to about 150 mg, about 75 mg to about 150 mg, about 80 mg to about 150 mg, about 85 mg to about 150 mg, about 90 mg to about 150 mg, about 95 mg to about 150 mg, about 100 mg to about 150 mg, about 105 mg to about 150 mg, about 110 mg to about 150 mg, about 115 mg to about 150 mg, about 120 mg to about 150 mg, about 125 mg to about 150 mg, about 130 mg to about 150 mg, about 135 mg to about 150 mg, about 140 mg to about 150 mg, or about 145 mg to about 150 mg of the compound of formula (I), or a pharmaceutically acceptable salt thereof, on a free acid equivalent weight basis.

In certain embodiments, administering the effective amount of the compound of formula (I), or a pharmaceutically acceptable salt thereof, comprises administering to the subject about 10 mg to about 150 mg, about 15 mg to about 145 mg, about 20 mg to about 140 mg, about 25 mg to about 135 mg, about 30 mg to about 135 mg, about 35 mg to about 130 mg, about 40 mg to about 125 mg, about 45 mg to about 120 mg, about 50 mg to about 115 mg, about 55 mg to about 110 mg, about 60 mg to about 105 mg, about 65 mg to about 100 mg, about 70 mg to about 95 mg, about 75 mg to about 90 mg, or about 80 to about 85 mg of the compound of formula (I), or a pharmaceutically acceptable salt thereof, on a free acid equivalent weight basis.

In certain embodiments, administering the effective amount of the compound of formula (I), or a pharmaceutically acceptable salt thereof, comprises administering to the subject about 10 mg to about 75 mg, about 15 mg to about 75 mg, about 20 mg to about 75 mg, about 25 mg to about 75 mg, about 30 mg to about 75 mg, about 35 mg to about 75 mg, about 40 mg to about 75 mg, about 45 mg to about 75 mg, about 50 mg to about 75, about 55 mg to about 75 mg, about 60 mg to about 75 mg, about 65 mg to about 75 mg, or about 70 mg to about 75 mg of the compound of formula (I), or a pharmaceutically acceptable salt thereof, on a free acid equivalent weight basis.

In certain embodiments, administering the effective amount of the compound of formula (I), or a pharmaceutically acceptable salt thereof, comprises administering to the subject about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, about 100 mg, about 105 mg, about 110 mg, about 115 mg, about 120 mg, about 125 mg, about 130 mg, about 135 mg, about 140 mg, about 145 mg, or about 150 mg of the compound of formula (I), or a pharmaceutically acceptable salt thereof, on a free acid equivalent weight basis.

In certain embodiments, administering the effective amount of the compound of formula (I), or a pharmaceutically acceptable salt thereof, comprises administering to the subject about 10 mg of the compound of formula (I), or a pharmaceutically acceptable salt thereof, on a free acid equivalent weight basis. In certain embodiments, administering the effective amount of the compound of formula (I), or a pharmaceutically acceptable salt thereof, comprises administering to the subject about 20 mg of the compound of formula (I), or a pharmaceutically acceptable salt thereof, on a free acid equivalent weight basis. In certain embodiments, administering the effective amount of the compound of formula (I), or a pharmaceutically acceptable salt thereof, comprises administering to the subject about 40 mg of the compound of formula (I), or a pharmaceutically acceptable salt thereof, on a free acid equivalent weight basis. In certain embodiments, administering the effective amount of the compound of formula (I), or a pharmaceutically acceptable salt thereof, comprises administering to the subject about 75 mg of the compound of formula (I), or a pharmaceutically acceptable salt thereof, on a free acid equivalent weight basis. In certain embodiments, administering the effective amount of the compound of formula (I), or a pharmaceutically acceptable salt thereof, comprises administering to the subject about 125 mg of the compound of formula (I), or a pharmaceutically acceptable salt thereof, on a free acid equivalent weight basis. In certain embodiments, administering the effective amount of the compound of formula (I), or a pharmaceutically acceptable salt thereof, comprises administering to the subject about 150 mg of the compound of formula (I), or a pharmaceutically acceptable salt thereof, on a free acid equivalent weight basis.

In certain embodiments, administering the effective amount of the compound of formula (I), or a pharmaceutically acceptable salt thereof, comprises administering to the subject at least about 10 mg, at least about 15 mg, at least about 20 mg, at least about 25 mg, at least about 30 mg, at least about 35 mg, at least about 40 mg, at least about 45 mg, at least about 50 mg, at least about 55 mg, at least about 60 mg, at least about 65 mg, at least about 70 mg, at least about 75 mg, at

least about 80 mg, at least about 85 mg, at least about 90 mg, at least about 95 mg, at least about 100 mg, at least about 105 mg, at least about 110 mg, at least about 115 mg, at least about 120 mg, at least about 125 mg, at least about 130 mg, at least about 135 mg, at least about 140 mg, or at least about 145 mg of the compound of formula (I), or a pharmaceutically acceptable salt thereof, on a free acid equivalent weight basis.

In certain embodiments, the compound of formula (I), or a pharmaceutically acceptable salt thereof, is administered orally to the subject. In certain embodiments, the compound of formula (I), or a pharmaceutically acceptable salt thereof, is administered orally to the subject daily. In certain embodiments, the compound of formula (I), or a pharmaceutically acceptable salt thereof, is administered orally to the subject once daily. In certain embodiments, the compound of formula (I), or a pharmaceutically acceptable salt thereof, is administered orally to the subject twice daily.

In certain embodiments, the compound of formula (I), or a pharmaceutically acceptable salt thereof, is administered orally to the subject once daily for 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, or 36 consecutive days. In certain embodiments, the compound of formula (I), or a pharmaceutically acceptable salt thereof, is administered orally to the subject once daily for 21 consecutive days.

In certain embodiments, the compound of formula (I), or a pharmaceutically acceptable salt thereof, is administered orally to the subject once daily for at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, or 36 consecutive days. In certain embodiments, the compound of formula (I), or a pharmaceutically acceptable salt thereof, is administered orally to the subject once daily for at least 21 consecutive days.

In certain embodiments, administering the effective amount of the compound of formula (I), or a pharmaceutically acceptable salt thereof, comprises administering to the subject about 10 mg to about 150 mg of the compound of formula (I), or a pharmaceutically acceptable salt thereof, on a free acid equivalent weight basis.

In certain embodiments, administering the effective amount of the compound of formula (I), or a pharmaceutically acceptable salt thereof, comprises administering orally to the subject about 10 mg to about 150 mg of the compound of formula (I), or a pharmaceutically acceptable salt thereof, on a free acid equivalent weight basis.

In certain embodiments, administering the effective amount of the compound of formula (I), or a pharmaceutically acceptable salt thereof, comprises administering orally to the subject about 10 mg to about 150 mg of the compound of formula (I), or a pharmaceutically acceptable salt thereof, on a free acid equivalent weight basis, daily.

In certain embodiments, administering the effective amount of the compound of formula (I), or a pharmaceutically acceptable salt thereof, comprises administering orally to the subject about 10 mg to about 150 mg of the compound of formula (I), or a pharmaceutically acceptable salt thereof, on a free acid equivalent weight basis, once daily.

In certain embodiments, administering the effective amount of the compound of formula (I), or a pharmaceutically acceptable salt thereof, comprises administering orally to the subject about 10 mg to about 150 mg of the compound of formula (I), or a pharmaceutically acceptable salt thereof, on a free acid equivalent weight basis, once daily for 21 consecutive days.

In certain embodiments, the subject is in a fasting state. In certain embodiments, the subject is not in a fasting state. In certain embodiments, administering the effective amount of the compound of formula (I), or a pharmaceutically acceptable salt thereof, comprises administering to the subject the effective amount about 1 hour before a meal or about 2 hours after a meal.

In certain embodiments, administering the effective amount of the compound of formula (I), or a pharmaceutically acceptable salt thereof, comprises administering to the subject about 10 mg to about 150 mg of the compound of formula (I), or a pharmaceutically acceptable salt thereof, on a free acid equivalent weight basis, about 1 hour before a meal or about 2 hours after a meal.

In certain embodiments, the subject has previously been administered at least one prior line of therapy. In certain embodiments, the subject has previously been administered fewer than five prior lines of therapy. In certain embodiments, the subject has previously been administered one, two, three, or 4 prior lines of therapy. In certain embodiments, the subject has not been administered a prior line of therapy.

In certain embodiments, the subject has previously been administered at least one and no more than 5 prior lines of therapy.

Prior lines of therapy include, but are not limited to, surgery, radiation therapy (e.g., external beam radiation therapy or internal radiation therapy), chemotherapy (e.g., alkylating agents, nitrosoureas, anti-metabolites, plant alkaloids and natural products, anti-tumor antibiotics, hormonal agents, and biological response modifiers), gene therapy, DNA therapy, viral therapy (e.g., oncolytic virus therapy), RNA therapy, adjuvant therapy, and immunotherapy (e.g., immune checkpoint inhibition, adoptive cell therapies, (e.g., tumorinfiltrating lymphocyte therapy, engineered T-cell receptor therapy, CAR T-cell therapy, natural killer cell therapy), or monoclonal antibodies).

In certain embodiments, the methods include administering an effective amount of a pharmaceutically acceptable salt of the compound of formula (I). In certain embodiments, the pharmaceutically acceptable salt is a potassium salt. In certain embodiments, the potassium salt is a hydrate. In certain embodiments, the potassium salt is a monohydrate.

In certain embodiments, the methods described herein further comprise administering an effective amount of a

second therapeutic agent to the subject. In certain embodiments, the second therapeutic agent is selected from the group consisting of a checkpoint inhibitor, an EGFR inhibitor, an antiangiogenic agent, venetoclax, fluorouracil, and combinations thereof.

In some embodiments, the second therapeutic agent is selected from the group consisting of an anti-VEGFR antibody, fluorouracil, a PI3K$\alpha$ inhibitor, a MEK1/2 inhibitor, and a hypoxia-inducible factor (HIF) inhibitor.

In some embodiments, the second therapeutic agent is venetoclax.

In some embodiments, the second therapeutic agent is fluorouracil. In some embodiments, the second therapeutic agent is 5-fluorouracil.

In some embodiments, administering the effective amount of the compound of formula (I), or a pharmaceutically acceptable salt thereof, and the venetoclax activates the integrated stress response pathway (ISR) in the advanced solid tumor or blood cancer to a greater extent than the compound of formula (I), or a pharmaceutically acceptable salt thereof, or venetoclax administered alone.

In some embodiments, extent of ISR activation may be determined by measurement of ISR activation markers described herein (e.g., ASNS, PSAT1, PHGDH, ASS1 and/or PUMA) in the tumor or cancer using the experimental methods described herein.

In certain embodiments, the second therapeutic agent is a checkpoint inhibitor. In certain embodiments, the second therapeutic agent is a PD-1 or PD-L1 inhibitor. In certain embodiments, the second therapeutic agent is selected from the group consisting of nivolumab, pembrolizumab, atezolizumab, avelumab, durvalumab, cemiplimab, and dostarlimab.

In certain embodiments, the second therapeutic agent is an EGFR inhibitor. In some embodiments, the EGFR inhibitor is selected from the group consisting of erlotinib, gefitinib, afatinib, osimertinib, and dacomitinib. In some embodiments, the EGFR inhibitor is selected from osimertinib and dacomitinib. In certain embodiments, the EGFR inhibitor is osimertinib. In certain embodiments, the EGFR inhibitor is dacomitinib. In some embodiments, the second therapeutic agent is selected from osimertinib and dacomitinib. In certain embodiments, the second therapeutic agent is osimertinib. In certain embodiments, the second therapeutic agent is dacomitinib.

In certain embodiments, the second therapeutic agent is an antiangiogenic agent. In certain embodiments, the antiangiogenic agent is a VEGFR inhibitor. In certain embodiments, the antiangiogenic is an anti-VEGFR antibody. In certain embodiments, the anti-VEGFR antibody is an anti-vascular endothelial growth factor receptor 2 (VEGFR2) antibody. In certain embodiments, the anti-VEGFR2 antibody is ramucirumab or bevacizumab. In certain embodiments, the anti-VEGFR2 antibody is ramucirumab. In certain embodiments, the anti-VEGFR2 antibody is bevacizumab.

In certain embodiments, the second therapeutic agent is a VEGFR inhibitor. In certain embodiments, the VEGFR inhibitor is selected from the group consisting of sunitinib, axitinib, Lenvatinib, tivozanib, pazopanib, cabozantinib, and ramucirumab. In certain embodiments, the second therapeutic agent is an anti-VEGFR antibody. In certain embodiments, the anti-VEGFR antibody is an anti-VEGFR2 antibody. In certain embodiments, the anti-VEGFR2 antibody is ramucirumab or bevacizumab. In certain embodiments, the anti-VEGFR2 antibody is ramucirumab. In certain embodiments, the anti-VEGFR2 antibody is bevacizumab.

In some embodiments, the second therapeutic agent is a HIF inhibitor. In certain embodiments, the HIF inhibitor is belzutifan.

In some embodiments, the second therapeutic agent is a PI3K$\alpha$ inhibitor. In some embodiments, the PI3K$\alpha$ inhibitor is selected from the group consisting of copanlisib and alpelisib. In certain embodiments, the PI3K$\alpha$ inhibitor is alpelisib. In some embodiments, the second therapeutic agent is alpelisib.

In some embodiments, the second therapeutic agent is a MEK1/2 inhibitor. In some embodiments, the MEK1/2 inhibitor is selected from the group consisting of binimetinib, cobimetinib, selumetinib, and trametinib. In certain embodiments, the MEK1/2 inhibitor is trametinib. In some embodiments, the second therapeutic agent is trametinib.

In some embodiments, the second therapeutic agent is an EGFR inhibitor. In some embodiments, the second therapeutic agent is selected from osimertinib and dacomitinib.

In another aspect, provided herein is a method of treating a blood cancer in a subject in need thereof, comprising administering to the subject an effective amount of a compound of formula (I), or a pharmaceutically acceptable salt thereof; or a pharmaceutical composition described herein, and an effective amount of venetoclax. In some embodiments, the blood cancer is AML.

In another aspect, provided herein is a method of treating a solid tumor (e.g., a solid tumor/advanced solid tumor described herein) in a subject in need thereof, comprising administering to the subject an effective amount of a compound of formula (I), or a pharmaceutically acceptable salt thereof; or a pharmaceutical composition described herein, and an effective amount of a second therapeutic agent selected from the group consisting of an anti-VEGFR2 antibody, 5-fluorouracil, alpelisib, and trametinib.

In another aspect, provided herein is a method of treating a solid tumor (e.g., a solid tumor/advanced solid tumor described herein) in a subject in need thereof, comprising administering to the subject an effective amount of a compound of formula (I), or a pharmaceutically acceptable salt thereof; or a pharmaceutical composition described herein, and an

effective amount of a VEGFR inhibitor. In some embodiments, the VEGFR inhibitor is an anti-VEGFR antibody. In some embodiments, the solid tumor is selected from renal cell carcinoma and colorectal cancer.

In another aspect, provided herein is a method of treating a solid tumor (e.g., a solid tumor/advanced solid tumor described herein) in a subject in need thereof, comprising administering to the subject an effective amount of a compound of formula (I), or a pharmaceutically acceptable salt thereof; or a pharmaceutical composition described herein, and an effective amount of an anti-VEGFR antibody. In some embodiments, the anti-VEGFR antibody is an anti-VEGFR2 antibody. In some embodiments, the solid tumor is selected from renal cell carcinoma and colorectal cancer.

In another aspect, provided herein is a method of treating a solid tumor (e.g., a solid tumor/advanced solid tumor described herein) in a subject in need thereof, comprising administering to the subject an effective amount of a compound of formula (I), or a pharmaceutically acceptable salt thereof; or a pharmaceutical composition described herein, and an effective amount of a HIF inhibitor. In certain embodiments, the HIF inhibitor is belzutifan. In some embodiments, the solid tumor is renal cell carcinoma. In some embodiments, the solid tumor is endometrial cancer.

In another aspect, provided herein is a method of treating a solid tumor (e.g., a solid tumor/advanced solid tumor described herein) in a subject in need thereof, comprising administering to the subject an effective amount of a compound of formula (I), or a pharmaceutically acceptable salt thereof; or a pharmaceutical composition described herein, and an effective amount of 5-fluorouracil. In some embodiments, the solid tumor is colorectal cancer.

In another aspect, provided herein is a method of treating a solid tumor (e.g., a solid tumor/advanced solid tumor described herein) in a subject in need thereof, comprising administering to the subject an effective amount of a compound of formula (I), or a pharmaceutically acceptable salt thereof; or a pharmaceutical composition described herein, and an effective amount of a PI3K$\alpha$ inhibitor. In some embodiments, the PI3K$\alpha$ inhibitor is alpelisib. In some embodiments, the solid tumor is colorectal cancer.

In another aspect, provided herein is a method of treating a solid tumor (e.g., a solid tumor/advanced solid tumor described herein) in a subject in need thereof, comprising administering to the subject an effective amount of a compound of formula (I), or a pharmaceutically acceptable salt thereof; or a pharmaceutical composition described herein, and an effective amount of a MEK1/2 inhibitor. In some embodiments, the MEK1/2 inhibitor is trametinib. In some embodiments, the solid tumor is colorectal cancer.

In another aspect, provided herein is a method of treating a solid tumor (e.g., a solid tumor/advanced solid tumor described herein) in a subject in need thereof, comprising administering to the subject an effective amount of a compound of formula (I), or a pharmaceutically acceptable salt thereof; or a pharmaceutical composition described herein, and an effective amount of an EGFR inhibitor. In some embodiments, the EGFR inhibitor is selected from osimertinib and dacomitinib. In some embodiments, the solid tumor is NSCLC.

In another aspect, provided herein are methods of treating a solid tumor (e.g., a solid tumor/advanced solid tumor described herein) in a subject in need thereof, comprising administering to the subject any one of the pharmaceutical compositions described herein to the subject.

In another aspect, provided herein are methods of treating a blood cancer (e.g., a blood cancer described herein) in a subject in need thereof, comprising administering to the subject any one of the pharmaceutical compositions described herein to the subject.

In certain embodiments, the subject is a human. In certain embodiment, the subject is an adult human.

## EXAMPLES

In order that the disclosure described herein may be more fully understood, the following examples are set forth. The synthetic and biological examples described in this application are offered to illustrate the compounds, pharmaceutical compositions, and methods provided herein and are not to be construed in any way as limiting their scope.

### Example 1: Synthesis of 6-(3-((5-chloro-2-methoxypyridine)-3-sulfonamido)-2,6-difluorophenyl)-N-methylimidazo[1,5-a]pyrazine-1-carboxamide (Compound of Formula (I))

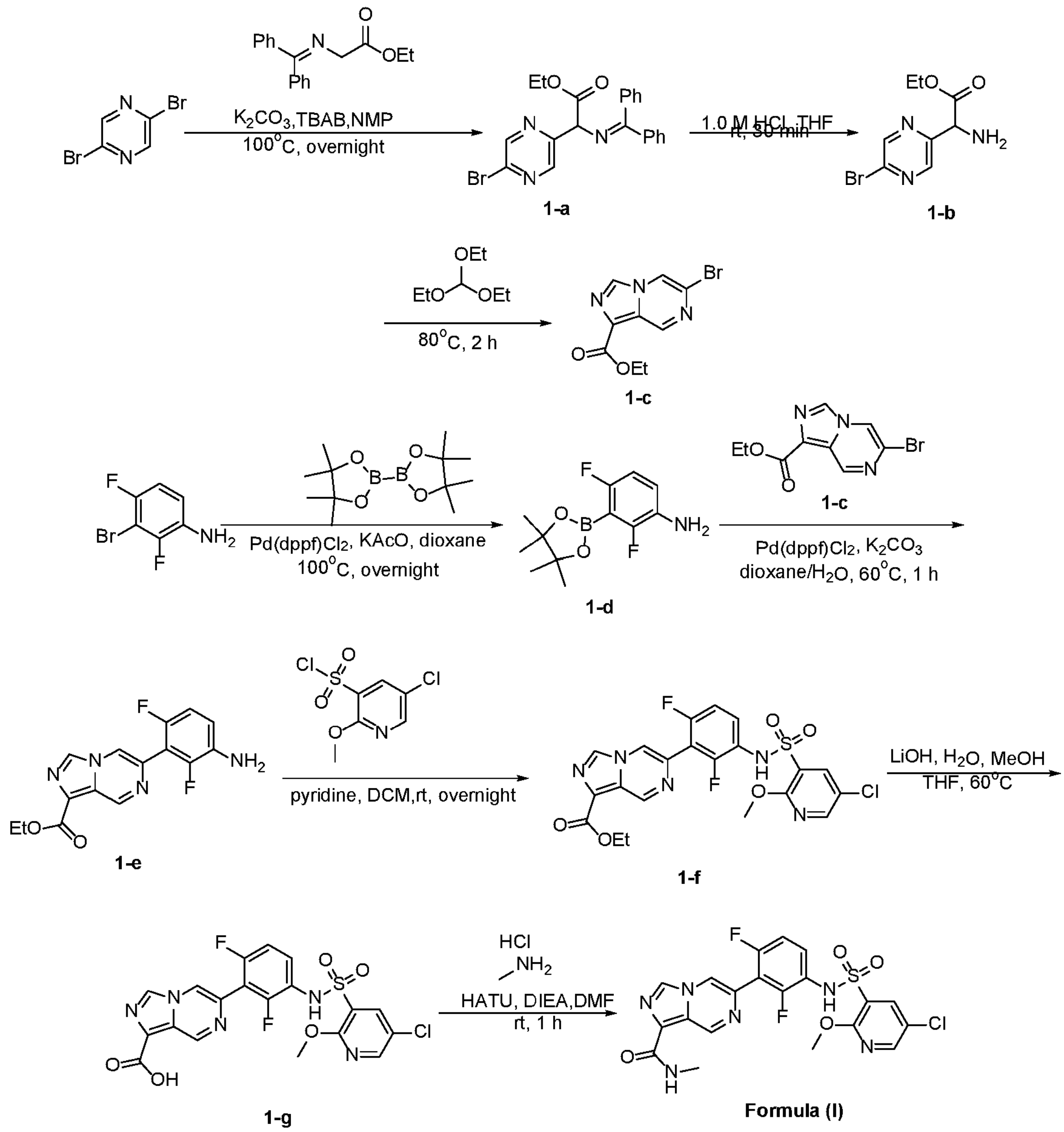


1-a, 1-b, 1-c, 1-d, 1-e, 1-f, 1-g, Formula (I)

Synthesis of 1-a: ethyl 2-(5-bromopyrazin-2-yl)-2-[(diphenylmethylidene) amino] acetate

2,5-Dibromopyrazine (10 g, 42 mmol, 1 equiv.), ethyl 2-[(diphenylmethylidene)amino]acetate (11.8 g, 44 mmol, 1.05 equiv.), tetrabutylammonium bromide (TBAB) (13.6 g, 42 mmol, 1 equiv.) and $K_2CO_3$ (17.4 g, 126 mmol, 3 equiv.) in (N-methyl-2-pyrrolidone) NMP (200 mL) were stirred overnight at 100 °C in an oil bath. The reaction mixture was cooled and filtered. The filtrate was diluted with 200 mL of water. The resulting solution was extracted with 2 x 200 mL of ethyl acetate and the organic layers combined. The resulting mixture was washed with 2 x 200 ml of water. The mixture was dried over anhydrous sodium sulfate and concentrated. The residue was applied to a silica gel column, eluting with ethyl acetate/petroleum ether (PE) (1/10). The collected fractions were combined and concentrated to give ethyl 2-(5-bromopyrazin-2-yl) -2-[(diphenylmethylidene)amino]acetate (8 g, 45% yield) as a yellow solid.
LCMS (ES, m/z): $[M+H]^+$ : 424

Synthesis of 1-b: ethyl 2-amino-2-(5-bromopyrazin-2-yl) acetate

Into a 250 mL round-bottom flask, was placed ethyl 2-(5-bromopyrazin-2-yl)-2-[(diphenylmethylidene)amino] acetate (8 g, 18.8 mmol, 1 equiv.), tetrahydrofuran (THF) (10 mL) and HCl (aqueous, 1 M) (20 mL). The resulting solution was stirred for 30 min at 25 °C. The solution formed was diluted with 50 mL of water and extracted with 2 x 50 mL of dichloromethane. The aqueous layers were adjusted to pH 8 with $NH_3.H_2O$ and further extracted with 3 x 50 mL of dichloromethane. The combined organic layers were dried over anhydrous sodium sulfate and concentrated. Ethyl 2-amino-2-(5-bromopyrazin-2-yl)acetate (4.7 g, 96% yield) was isolated as a yellow solid which was used in next step directly without further purification.
LCMS (ES, m/z): $[M+H]^+$ : 260

Synthesis of 1-c: ethyl 6-bromoimidazo [1,5-a]pyrazine-1-carboxylate

Into a 50 mL round-bottom flask, was placed ethyl 2-amino-2-(5-bromopyrazin-2-yl) acetate (4.2 g, 0.02 mol, 1 equiv.) and triethyl orthoformate (20 mL). The resulting solution was stirred for 2 h at 80 °C in an oil bath. The reaction mixture was cooled, and the solids collected by filtration. Air drying gave ethyl 6-bromoimidazo [1,5-a]pyrazine-1-carboxylate (2.2 g, 50% yield) as a brown solid.
LCMS (ES, m/z): $[M+H]^+$ : 270

Synthesis of 1-d: 2,4-difluoro-3-(4,4,5,5-tetramethyl-1,3,2-dioxaborolan-2-yl)aniline

3-Bromo-2,4-difluoroaniline (10 g, 48 mmol, 1 equiv.), [1,1'-Bis(diphenylphosphino)ferrocene]dichloropalladium(II) (Pd(dppf)$Cl_2$) (3.5 g, 4.8 mmol, 0.1 equiv.), bis(pinacolato)diboron (18.3 g, 72 mmol, 1.5 equiv.) and potassium acetate (KOAc) (14.2 g, 144.2 mmol, 3 equiv.) were dissolved in dioxane (240 mL). The resulting solution was stirred overnight at 100 °C in an oil bath. The reaction mixture was cooled, and the solids removed by filtration. The filtrate was concentrated and diluted with dichloromethane (DCM) (100 mL), then washed with 2 x 100 mL of water and 100 mL of brine. The organic phase was dried over anhydrous sodium sulfate and concentrated under reduced pressure. The residue was applied to a silica gel column, eluting with ethyl acetate/petroleum ether (1/10). 2,4-Difluoro-3-(4,4,5,5-tetramethyl-1,3,2-dioxaborolan-2-yl)aniline (8 g, 65% yield) was isolated as a yellow solid.
LCMS (ES, m/z): $[M+H]^+$ : 256

Synthesis of 1-e: ethyl 6-(3-amino-2,6-difluorophenyl)imidazo[1,5-a]pyrazine-1-carboxylate

Ethyl 6-bromoimidazo[1,5-a]pyrazine-1-carboxylate (500 mg, 1.9 mmol, 1 equiv.), 2,4-difluoro-3- (4,4,5,5-tetramethyl-1,3,2-dioxaborolan-2-yl)aniline (708 mg, 2.8 mmol, 1.5 equiv.), Pd(dppf)$Cl_2$ (135 mg, 0.2 mmol, 0.1 equiv.), $K_2CO_3$ (767 mg, 5.6 mmol, 3 equiv) in dioxane (10 mL) and $H_2O$ (2 mL) were stirred for 1 h at 60 °C in an oil bath. The reaction mixture was cooled, diluted with water (20 ml) and extracted with 3 x 20 mL of dichloromethane. The organic layers were dried over anhydrous sodium sulfate and concentrated. The residue was applied to a silica gel column and eluted with ethyl acetate/PE (1/2). Ethyl 6-(3-amino-2,6-difluorophenyl)imidazo[1,5-a]pyrazine-1-carboxylate (200 mg 34% yield) was isolated as a brown solid.
LCMS (ES, m/z): $[M+H]^+$ : 319

Synthesis of 1-f: ethyl 6-[3-(5-chloro-2-methoxypyridine-3-sulfonamido)-2,6-difluorophenyl]imidazo[1,5-a]pyrazine-1-carboxylate

Ethyl 6-(3-amino-2, 6-difluorophenyl)imidazo[1,5-a]pyrazine-1- carboxylate (150 mg, 0.5 mmol, 1 equiv.) in DCM (5 mL) was treated with pyridine (186 mg, 2.3 mmol, 5 equiv.), then 5-chloro-2-methoxypyridine-3-sulfonyl chloride (137 mg, 0.6 mmol, 1.2 equiv.). The resulting solution was stirred overnight. The resulting mixture was concentrated and purified by Flash-Prep-HPLC with the following conditions: Column, WelFlashTM C18-I, Spherical C18 20-40 μm; mobile phase: 0.1% Formic Acid/ 5-70% MeCN over 15 min; Detector, 254 & 220 nm. Ethyl 6-[3-(5-chloro-2- methoxypyridine-3-sulfonamido)-2,6-difluorophenyl]imidazo[1,5-a]pyrazine-1-carboxylate (320 mg 97% yield) was isolated as a yellow solid.
LCMS (ES, m/z): $[M+H]^+$ : 524

Synthesis of 1-g: 6-[3-(5-chloro-2-methoxypyridine-3-sulfonamido)-2,6-difluorophenyl]imidazo[1,5-a]pyrazine-1-carboxylic acid

Ethyl 6-[3-(5-chloro-2-methoxypyridine-3-sulfonamido)-2,6-difluorophenyl]imidazo[1,5-a]pyrazine-1-carboxylate (200 mg, 0.4 mmol, 1 equiv), methanol (MeOH) (2 mL), THF (2 mL), $H_2O$ (2 mL) and LiOH (27 mg, 1.1 mmol, 3 equiv) were stirred for 1 h at 60 °C in an oil bath. After concentration, the crude product was purified by Flash-Prep-high performance liquid chromatography (HPLC) with the following conditions: Column, WelFlashTM C18-I, Spherical C18 20-40 μm; mobile phase: 5-60% acetonitrile (MeCN)/0.1% ammonia over 15 min; Detector, 254 nm. 6-[3-(5-Chloro-2-methoxypyridine-3-sulfonamido)-2,6- difluorophenyl]imidazo[1,5-a] pyrazine-1-carboxylic acid (170 mg, 90% yield) was isolated as a yellow solid.
LCMS (ES, m/z): $[M+H]^+$ : 496

Synthesis of 6-[3-(5-chloro-2-methoxypyridine- 3-sulfonamido)-2,6-difluorophenyl]-N-methylimidazo[1,5-a]pyrazine-1-carboxamide

6-[3-(5-Chloro-2-methoxypyridine-3-sulfonamido)-2,6-difluorophenyl]imidazo[1,5-a]pyrazine-1-carboxylic acid (170 mg, 0.3 mmol, 1 equiv) in N,N-dimethylformamide (DMF) (4 mL) was treated with diisopropylethylamine (DIEA) (133 mg, 1 mmol, 3 equiv), methylamine hydrochloride (16 mg, 0.5 mmol, 1.5 equiv) and 1-[Bis(dimethylamino)methylene]-1H-1,2,3-triazolo[4,5-b]pyridinium 3-oxide hexafluorophosphate (HATU) (195 mg, 0.5 mmol, 1.5 equiv). The resulting solution was stirred for 1 hr, then concentrated. The crude product was purified by Flash-Prep-HPLC with the following conditions: Column, WelFlashTM C18-I, Spherical C18 20-40 μm, 120 g; mobile phase: 5-60% MeCN/0.1% formic acid over 20 min. 6-[3-(5-Chloro-2-methoxypyridine-3-sulfonamido)-2,6-difluorophenyl]-N-methylimidazo[1,5-a]pyrazine-1-carboxamide (43 mg, 25% yield) was isolated as an off-white solid.

Liquid chromatography/mass spectrometry (LCMS) (ES, m/z): $[M+H]^+$ : 509
$^1$H nuclear magnetic resonance spectroscopy (NMR) (300 MHz, DMSO-$d_6$) $\delta$ 10.46 (s, 1H), 9.51 (d, $J$ = 1.6 Hz, 1H), 8.66 (d, $J$ = 5.0 Hz, 2H), 8.51 (d, $J$ = 2.6 Hz, 1H), 8.43 (d, $J$ = 4.9 Hz, 1H), 8.09 (d, $J$ = 2.6 Hz, 1H), 7.42 (td, $J$ = 8.8, 5.8 Hz, 1H), 7.25 (td, $J$ = 9.3, 1.4 Hz, 1H), 3.92 (s, 3H), 2.84 (d, $J$= *4.7* Hz, 3H).

**Example 2: Preparation of the Compound of Formula (I) Potassium Salt**

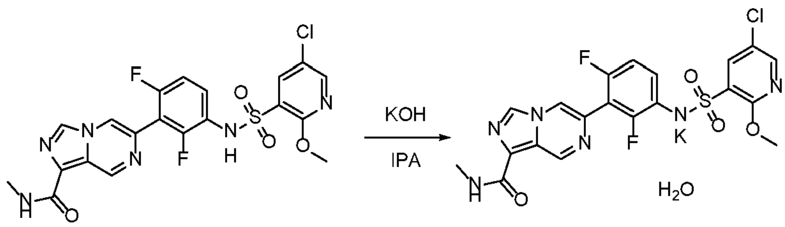


To a stirred mixture of 6-(3-((5-chloro-2-methoxypyridine)-3-sulfonamido)-2,6-difluorophenyl)-N-methylimidazo [1,5-a]pyrazine-1-carboxamide (as prepared in Example 1) in aqueous isopropyl alcohol (IPA) was slowly added 1.1 equivalents of an aqueous KOH solution and the solution heated. The resulting mixture was cooled, the solid collected, washed with IPA/water, and then dried under heat and vacuum to afford potassium ((5-chloro-2-methoxypyridin-3-yl) sulfonyl)(2,4-difluoro-3-(1-(methylcarbamoyl)imidazo[1,5-a]pyrazin-6-yl)phenyl)amide.
$^{19}$F NMR (400 MHz, d6-DMSO): -129.62 and -127.72 ppm.

**Example 3: Biochemical** Assay

GCN2 protein was obtained from Carna Biosciences (GCN2 cat#: 05-153). The protein was diluted in assay buffer (ThermoFisher Scientific, #PV6135), 2 mM dithiothreitol (DTT), to obtain a final concentration of 2 nM and 5 μL was plated in a 384-well white assay plate. HC-7366 was serially diluted to 11 concentrations by 3-fold dilution in dimethyl sulfoxide (DMSO) and 10 nL of stock was plated into 384 well white assay plate. DMSO was used as a vehicle control. Green fluorescent protein (GFP)-eIF2α protein was obtained from ThermoFisher (cat# PV4809). The protein was diluted in assay buffer to a 2x concentration of 200 nM along with 300 mM ATP (final concentration of 100 nM GFP-eIF2α and 150 μM ATP) in the presence of 2 mM DTT and a 5 μL aliquot was added to each well containing the GCN2 protein and HC-7366. The plate was incubated in the dark at 25 °C for 1.5 hours, shaking at 1250 rpm. Tb-anti P-eIF2α (ThermoFisher cat# PV4810) were diluted to a concentration of 4 nM with 20 mM ethylenediaminetetraacetic acid (EDTA) (final concentration of 2 nM Tb-anti P-eIF2α and 10 nM EDTA) in time-resolved fluorescence energy transfer (TR-FRET) Dilution Buffer (ThermoFisher cat# PV3574). 10 μL of the Tb-anti P-eIF2α solution was added to the TR-FRET reaction. The plate was incubated in the dark for 2 h at 25 °C shaking at 600 rpm. The FRET signal from the plate was read on a Envision (PerkinElmer) plate reader:

Label 1: Excitation: 340 nm, bandwidth 30 nm; Emission: 495 nm, bandwidth 10 nm. Lag time: 100 μsec. Integration time: 400 μsec. Flashes: 30.
Label 2: Excitation: 340 nm, bandwidth 30 nm; Emission: 520 nm, bandwidth 25 nm. Lag time: 100 μsec. Integration time: 400 μsec. Flashes: 30

The data were analyzed using XLfit, as shown in FIG. 1. The graph shows data depicted as %Vehicle, which is the

DMSO control.

**FIG. 1** depicts the experimental results for HC-7366 in the GCN2 biochemical assay described above showing the GCN2 inhibitory activity of the compound.

### Example 4: Cell Based Assays

HEK293-ATF4-Luc cells were with culture medium $1.5e^5$ cells/mL and 25 μL of cell suspension was added into each well of 384-well cell culture plate (Corning, CLS3570-50EA) as designated and transferred into 37 °C - 5% CO2 incubator (Thermo Scientific) overnight for cell attachment. HC-7366 was serially diluted to 11 concentrations by 3-fold dilution in DMSO and 25 nL of stock was plated into cell plate by Echo550 (Labcyte, Echo550), then incubated for 30 minutes at 37°C. After HC-7366 treatment, cells were treated with either DMSO or 12.5 nM Halofuginone to activate GCN2 for 6 hours. After 6 hours, 25 μL of One-Glo reagent (Promega, cat#: E6120) was added into each well to be detected (at 1:1 to culture medium). Then the plates were placed at room temperature for 10 min followed by luciferase luminescence reading on EnVision (PerkinElmer). The data were analyzed by XLfit (v5.3.1.3), equation 201.

**FIG. 2** depicts the experimental results for HC-7366 in the ATF4 activity assay described above for cells treated with HC-7366 only, showing the effect of HC-7366 on ATF4 activation in cells. The concentrations that showed ATF4 activation aligned to the concentrations where ATF4 protein expression was observed in HT1080 cells in **FIG. 5.** n=1 or n=2 represent different runs that were done on the same day. **FIG. 3** depicts experimental results for HC-7366 for cells treated with HC-7366 and Halofuginone demonstrating the GCN2 inhibitory effect of GCN2 in cells in the ATF4 activity assay described above.

*CellTiter-Glo® (CTG) Viability assay in MOLM-16 cells*

MOLM-16 cells were obtained from DSMZ (#ACC 555) and were cultured in RPMI 1640 (Gibco, #11875119) with 20% FBS (Invitrogen, #10099141C) in a cell culture incubator (Thermo Scientific) set at 37 °C - 5% $CO_2$, 95% relative humidity. 30 μL Cell suspension (2k cells/well) was added to each well in 384-well cell culture plates (Corning, #CLS3764-100EA). The plates were then incubated overnight. HC-7366 was diluted in 10 concentrations by 3-fold dilution in 384pp-plate using TECAN EVO200. 40 nL of the HC-7366 stock were transferred to 384-well cell culture plate using Echo550 to a final concentration ranging from 1-10 μ. The plates were incubated for 72 hr in incubator at 37 °C, 5% $CO_2$. Viability was assessed by adding 30 μL CellTiter-Glo® 2.0 Assay (Promega, G9243) was added to each well. The plates were then incubated at room temperature (RT) for 30 min. The luminescent signals were read by Envision (PerkinElmer) plate reader. The data were analyzed by XLfit (v5.3.1.3), equation 201.

**FIG. 4** depicts the experimental results for HC-7366 in the cell viability assay described above.

### Example 5: Western Blot Analysis

Cell lysates were obtained from HT1080 cells treated with HC-7366 (0 to 10 μM) for 30 min followed by treatment with either DMSO or 100 nM Halofuginone to activate GCN2 and subjected to western blot analysis.

*Reagents*

Fisher Bioreagent (cat#: BP2471-1) 10X Tris-buffered saline (TBS)
Fisher Tween 20 (cat#: BP337-100 lot 10817)
BioRad Blotting grade blocker (milk) (cat#: 170-6404)
Pierce SuperSignal West Femto (cat#: 34095)

*Primary antibodies*

Anti-phospho-GCN2-T899 Rabbit Monoclonal, Abcam cat#: ab75836
Anti-GCN2 Rabbit Monoclonal, Abcam cat #ab134053
Anti-p-PERK Rabbit antibody, Eli Lilly
Anti-PERK Rabbit Monoclonal, Cell Signaling Technology cat#:3192
Anti p-PKR Rabbit Polyclonal, ThermoFisher Scientific cat # 44-668G
Anti PKR Rabbit Polyclonal, ThermoFisher Scientific cat #: 700286

Anti-p-eIF2α S51 Rabbit Monoclonal, Cell Signaling Technology cat#: 3398
Anti-eIF2α Rabbit, Cell Signaling Technology cat#: 9722
Anti-ATF4 Rabbit Monoclonal, Cell Signaling Technology cat#: 11815
Anti-β-actin Mouse Monoclonal- Sigma cat#: A5441
Jackson Immunoresearch Secondary antibodies 1:5000 in 5% milk
HRP goat anti-rabbit, cat#: 111-035-046 lot 105262
HRP goat anti-mouse, cat#: 115-035-071 lot 100214

*Sample Preparation*

The HT1080 cells were harvested and counted and diluted to the desired density. 2 mL of cell suspension was added into each well of 6-well cell culture plate. The plates were transferred into 37°C, 5% $CO_2$ incubator overnight. The cells were treated as described above. The media was aspirated, and cells were washed with ice cold phosphate buffered saline (PBS). Ice cold 1x radioimmunoprecipitation assay (RIPA) lysis buffer supplemented with protease and phosphatase inhibitors was added to the cell pellets, on ice. After 30 min on ice the cell lysates were centrifuged at 4°C, 12,000 rpm for 15 minutes and the supernatant collected. Protein concentration was determined using BCA kit. The cell lysates were mixed with 5X sodium dodecyl sulfate (SDS) loading buffer and denatured at 95°C for 5 minutes.

*Western Blot*

30 μg of protein was loaded in 4-12% Bis-Tris Gels in 1X MOPS buffer and run at 120 V for 120 min. After the electrophoresis was completed, the separated protein was transferred to a nitrocellulose membrane using transblot system for 90 min, 300 mA, then the membrane was blocked with (tris-buffered saline) TBS buffer (5% bovine serum albumin (BSA)) for 1 hr at room temperature. The membrane was exposed to the primary antibodies overnight in cold room in TBS buffer (1% BSA). The membrane was then washed with TBS-Tween for 3x 10 min at R/T, incubated with secondary antibodies in TBS buffer (1% BSA) for 1 hr at RT. The membrane was washed with TBS-Tween for 3X 10 min at R/T and the imaging was acquired with Li-COR imaging system.

**FIG. 5** depicts the experimental results for HC-7366 in the western blot analysis described above. HC-7366 showed activation of GCN2 pathway at low concentrations. Halofuginone acts as a GCN2 agonist by inhibiting prolyl-tRNA synthetase. Cells treated with halofuginone showed activation of GCN2 and ATF4. The GCN2 modulator HC-7366 inhibited halofuginone induced p-GCN2 and ATF4 in a dose dependent manner. This result demonstrates the GCN2 modulatory nature of HC-7366, where it acts as an inhibitor of pGN2 and ATF4 in the presence of Halofuginone but activates the GCN2 pathway by itself.

**Example 6: In Vivo Tumor Growth Inhibition (TGI) Studies**

Effect of HC-7366 on xenograft tumor growth was investigated in several models listed below.

**1. Study design**

*1.1 Animals*

Cell lines and Species & Strain of mice used:

**MOLM-16 (human AML cells):** Female 6-8 weeks old nonobese diabetic/severe combined immunodeficiency (NOD SCID) mice were inoculated subcutaneously on the right flank with MOLM-16 tumor cells (1 x $10^7$) in RPMI 1640 without serum for tumor development. The treatments were started when the mean tumor size reached approximately 220 $mm^3$ at Day 14 after cell inoculation and continued until end of study by twice daily oral gavage dosing of HC-7366 at listed doses (vehicle, 0.25 mg/kg HC-7366, 0.5 mg/kg HC-7366, 1 mg/kg HC-7366, or 2 mg/kg HC-7366, **FIG. 16**)

**HT1080 (human fibrosarcoma cells):** Female 6-8 weeks old BALB/c nude mice were inoculated subcutaneously on the right flank with HT1080 tumor cells (1 x $10^7$) in 0.1 ml of EMEM medium without serum for tumor development. The treatments were started when the mean tumor size reached approximately 109 $mm^3$ at Day 4 post inoculation and continued until end of study by oral gavage dosing of HC-7366 at listed doses (vehicle, 0.3 mg/kg HC-7366 BID (two times per day), 1 mg/kg HC-7366 BID, 3 mg/kg HC-7366 BID, 0.6 mg/kg HC-7366 QD (one time per day), 2 mg/kg HC-7366 QD, or 6 mg/kg HC-7366 QD, **FIGs. 6-7).**

**LoVo (human colorectal cancer cells):** Female 6-8 weeks old BALB/c nude mice were inoculated subcutaneously on the right flank with LoVo tumor cells (5 x $10^6$) in 0.1 ml of EMEM medium without serum for tumor development. The treatments were started when the mean tumor size reached approximately 243 $mm^3$ at Day 16 post inoculation and

continued until end of study by twice daily oral gavage dosing of HC-7366 at listed doses (vehicle, 0.3 mg/kg HC-7366, 1 mg/kg HC-7366, 3 mg/kg HC-7366, 10 mg/kg HC-7366, or 30 mg/kg HC-7366, **FIGs. 8-9).**

**DLD-1 (human colorectal cancer cells):** Female 6-8 weeks old BALB/c nude mice were inoculated subcutaneously on the right flank with DLD-1 tumor cells (5 x $10^6$) in 0.1 ml of RPMI1640 medium without serum for tumor development. The treatments were started when the mean tumor size reached approximately 195 $mm^3$ at Day 7 post inoculation and continued until end of study by twice daily oral gavage dosing of HC-7366 at listed doses (vehicle, 0.3 mg/kg HC-7366, 1 mg/kg HC-7366, 3 mg/kg HC-7366, 10 mg/kg HC-7366, or 30 mg/kg HC-7366, **FIG. 10).** For the time course study, tumors were harvested at Days 4, 7 and 14. Tumors were then used for IHC and RNAseq experiments (see Example 21).

**FaDu (human head and neck cancer cells):** Female 6-8 weeks old BALB/c nude mice were inoculated subcutaneously on the right flank with FaDu tumor cells (5 x $10^6$) in 0.1 ml of EMEM medium without serum for tumor development. The treatments were started when the mean tumor size reached approximately 194 $mm^3$ at Day 21 post inoculation and continued until end of study by twice daily oral gavage dosing of HC-7366 at listed doses (vehicle, 0.3 mg/kg HC-7366, 1 mg/kg HC-7366, 3 mg/kg HC-7366, 10 mg/kg HC-7366, or 30 mg/kg HC-7366, **FIG. 11-13).**

**KG-1 (human AML cancer cells):** Female 6-8 weeks old NOD SCID mice were inoculated subcutaneously on the right flank with KG1 tumor cells (5 x $10^6$) in RPMI 1640 without serum for tumor development. The treatments were started when the mean tumor size reached approximately 194 $mm^3$ at Day 20 after cell inoculation and continued until end of study by twice daily oral gavage dosing of HC-7366 at listed doses (vehicle, 0.3 mg/kg HC-7366, 1 mg/kg HC-7366, 3 mg/kg HC-7366, 10 mg/kg HC-7366, or 30 mg/kg HC-7366, **FIG. 17).**

**Kasumi-1 (human AML cancer cells):** Female 6-8 weeks old CB17 SCID mice were inoculated subcutaneously on the right flank with KG1 tumor cells (1 x $10^7$) in RPMI 1640 without serum for tumor development. The treatments were started when the mean tumor size reached approximately 151 $mm^3$ at Day 29 after cell inoculation and continued until end of study by twice daily oral gavage dosing of HC-7366 at listed doses (vehicle, 3 mg/kg HC-7366, or 30 mg/kg HC-7366, **FIG. 18**).

**OCI-AML2 (human AML cancer cells):** Female 6-8 weeks old NOD SCID mice were inoculated subcutaneously on the right flank with OCI-AML2 tumor cells (5 x $10^6$) in RPMI 1640 without serum for tumor development. The treatments were started when the mean tumor size reached approximately 149 $mm^3$ at Day 7 after cell inoculation and continued until end of study by twice daily oral gavage dosing of HC-7366 at listed doses (vehicle, 0.3 mg/kg HC-7366, 1 mg/kg HC-7366, 3 mg/kg HC-7366, 10 mg/kg HC-7366, or 30 mg/kg HC-7366, **FIG. 19).**

**MV4-11 (human AML cancer cells):** Female 6-8 weeks old BALB/c nude mice were inoculated subcutaneously on the right flank with MV4-11 tumor cells (1 x $10^7$) in RPMI 1640 without serum for tumor development. The treatments were started when the mean tumor size reached approximately 150 $mm^3$ at Day 13 after cell inoculation and continued until end of study by twice daily oral gavage dosing of HC-7366 at listed doses (vehicle, 0.3 mg/kg HC-7366, 1 mg/kg HC-7366, 3 mg/kg HC-7366, 10 mg/kg HC-7366, or 30 mg/kg HC-7366, **FIG. 20).** For venetoclax combination study, venetoclax was dissolved in 0.5% carboxymethylcellulose (CMC) and co-administered at 50 mg/kg dose once daily (see Example 8).

**786-O (human renal cell carcinoma cancer cells):** Female 6-8 weeks old BALB/c nude mice were inoculated subcutaneously on the right flank with 786-O tumor cells (5 x $10^6$) in 0.1 ml of RPMI1640 medium without serum for tumor development. The treatments were started when the mean tumor size reached approximately 189 $mm^3$ after three weeks post inoculation and continued until end of study by twice daily oral gavage dosing of HC-7366 at listed doses (vehicle or 2 mg/kg HC-7366, **FIGs. 52-54**). For the DC101 combination study, DC101 (murine monoclonal anti-VEGFR2 antibody) was dosed at 15 mg/kg twice a week by intraperitoneal injection.

**A498 (human renal cell carcinoma cancer cells):** Female 6-8 weeks old NOD SCID mice were inoculated subcutaneously on the right flank with A498 tumor cells (5 x $10^6$) in 0.1 ml of MEM medium without serum for tumor development. The treatments were started when the mean tumor size reached approximately 349.44 $mm^3$ after four weeks post inoculation and continued until end of study by twice daily oral gavage dosing of HC-7366 at listed doses (vehicle or 2 mg/kg HC-7366, **FIGs. 55-57).** DC101 was dosed at 15 mg/kg twice a week by intraperitoneal injection.

**LNCaP (human prostate cancer cells):** Male 6-8 weeks old NOD SCID mice were inoculated subcutaneously on the right flank with LNCaP tumor cells (1 x $10^7$) in 0.1 ml with Matrigel (1: 1) in RPMI1640 with 10 % fetal bovine serum (FBS) for tumor development. The treatments were started when the mean tumor size reached approximately 207 $mm^3$ at Day 14 post inoculation and continued until end of study by twice daily oral gavage dosing of HC-7366 at listed doses (vehicle, 0.3 mg/kg HC-7366, 1 mg/kg HC-7366, 3 mg/kg HC-7366, 10 mg/kg HC-7366, or 30 mg/kg HC-7366 twice daily, **FIG. 14).**

**TM00298 (human prostate cancer cells):** Male 6-8 weeks old NSG mice were inoculated subcutaneously with 1 $mm^3$ TM00298 tumor fragments. The treatments were started when the mean tumor size reached approximately 123 $mm^3$ and continued until end of study by twice daily oral gavage dosing of HC-7366 at listed doses (vehicle, 3 mg/kg HC-7366, or 30 mg/kg HC-7366, **FIG. 15).**

**NCI-H1975 (human non-small cell lung cancer cells):** Female 7-8 weeks old Athymic Nude-Foxn1nu mice were inoculated subcutaneously on the right flank with NCI-H1975 tumor cells (2.5 x $10^6$) in 0.1 ml of RPMI1640 medium

without serum for tumor development. Single agent HC-7366 was dosed at 3 mg/kg, osimertinib at 2.5 mg/kg and dacomitinib at 15 mg/kg. HC-7366 was combined with either osimertinib or dacomitinib. The vehicle for the groups was 0.5% methyl cellulose. The treatments were started when the mean tumor size reached approximately 205 $mm^3$ at Day 22 post inoculation and continued until end of study. HC-7366 was given twice daily by oral gavage whereas osimertinib or dacomitinib were administered once a day by oral gavage **(FIGs. 131 and 133).**

**HCT116 (human colorectal cancer cells):** Female 7-8 weeks old Athymic Nude-Foxn1nu mice were inoculated subcutaneously on the right flank with HCT116 tumor cells (5 x $10^6$) in 0.1 ml of McCoy's 5a medium without serum for tumor development. Single agent HC-7366 was dosed at 3 or 30 mg/kg (twice daily, orally), Trametinib at 1 mg/kg (once a day, orally) and Alpelisib at 50 mg/kg (once a day, orally). HC-7366 was combined with either Trametinib or Alpelisib. The treatments were started when the mean tumor size reached approximately 270 $mm^3$ at Day 18 post inoculation and continued until end of study **(FIGs 63-64).**

**MFE280 (human endometrial cancer cells):** Female 7-8 weeks old Athymic Nude-Foxn1nu mice were inoculated subcutaneously on the right flank with MFE280 tumor cells (10 x $10^6$) in 0.2 ml of PBS for tumor development. Single agent HC-7366 was dosed at 0.5 or 2 mg/kg (twice daily, orally), PT2977 at 1 mg/kg (twice daily, orally). HC-7366 at 0.5 or 2 mg/kg was combined with PT2977. The treatments were started when the mean tumor size reached approximately 191 $mm^3$ at Day 28 post inoculation and continued until end of study **(FIG. 135).**

1.1.2 Supplier: Beijing AniKeeper Biotech Co., Ltd.
1.1.3 Age: 6 to 8 weeks
1.1.4 Sex: Female
1.1.5 Body Weight: 16 to 22 g

*1.2 Animal Maintenance*

1.2.1 Quarantine: Animals were quarantined for 7 days before the study. The general health of the animals was evaluated by a veterinarian, and complete health checks were performed. Animals with abnormalities were excluded prior the study. General procedures for animal care and housing were in accordance with the standard, Commission on Life Sciences, National Research Council, standard operating procedures (SOPs).

1.2.2 Housing: General procedures for animal care and housing were in accordance with the standard, Commission on Life Sciences, National Research Council, standard operating procedures (SOPs). All the procedures related to animal handling, care and animal research methods used in this study were performed according to guidelines approved by the Institutional Animal Care and Use Committee (IACUC) of Pharmaron following the guidance of the Association for Assessment and Accreditation of Laboratory Animal Care (AAALAC). The mice were kept in laminar flow rooms at constant temperature and humidity with 3-5 mice in each cage. Animals were housed in polycarbonate cage, which is in the size of 300 $\times$ 180 $\times$ 150 $mm^3$ and in an environmentally monitored, well-ventilated room maintained at a temperature of (22 $\pm$ 3 °C) and a relative humidity of 40%-80%. Fluorescent lighting was provided illumination approximately 12 hours per day. The bedding material was soft wood, which was changed once per week.

1.2.3 Animal ID: Each animal was assigned an identification number; the following identification method was applied. Each cage card was labeled with such information as study number, group, sex, dose, animal number, initiation date, study director and telephone number. Individual animal was identified by ear coding.

1.2.4 Diet: Animals had free access to irradiation sterilized dry granule food during the entire study period.

1.2.5 Water: Sterile drinking water in a bottle was available to all animals ad libitum during the quarantine and study periods. The bottle and the stopper with attached sipper tube were autoclaved prior to use. Samples of water from the animal facility were analyzed and results of water analysis were retained in the facility records and were reviewed by the veterinarian, or designee, to assure that no known contaminants were present that could interfere with or affect the outcome of studies.

*1.3 Groups and Treatments*

Groups and treatments were started when the mean tumor volume reached about 100-250 $mm^3$. Based on the tumor volume and body weight, mice were assigned to respective groups such that the average starting tumor size is the same for each treatment group.

*1.4 Vehicle*

Vehicle for HC-7366: 5% (v/v) DMSO and 95% (v/v) Captisol (20% w/v) in PBS (final pH 7.4) unless noted otherwise.

**2. Experimental method and measurement parameters**

*2.1 Method for Tumor Inoculation*

Each mouse was inoculated subcutaneously on the right flank or orthotopically into the mammary fat pad with tumor cells in a total volume of 0.1 ml for tumor development. The treatment was started when the mean tumor size reached approximately 100-250 $mm^3$. Animals were randomized based on tumor volume in the groups of 8-10 animals, typically including a vehicle control and treatment groups at various dose levels.

*2.2 Measurement Parameters*

For routine monitoring, all study animals were monitored not only for tumor growth but also behavior such as mobility, food and water consumption (by cage side checking only), body weight (BW), eye/hair matting and any other abnormal effect. Any mortality and/or abnormal clinical signs were recorded.

2.2.1 Body Weight (BW): Body weights of all animals were measured and recorded three times weekly throughout the study. Body weight change, expressed in %, is calculated using the following formula:

$$\text{BW change (\%)} = (\text{BW}_{\text{Day X}} / \text{BW}_{\text{Day 0}}) \times 100$$

2.2.2 Tumor Measurements: The measurement of tumor size was conducted three times weekly with a caliper and recorded. The tumor volume ($mm^3$) was estimated using the formula: TV = $a \times b^2/2$, where "a" and "b" is long and short diameters of a tumor, respectively. Tumor volume was calculated using the formula [tumor volume ($mm^3$) = $\pi/6$ x length x width$^2$).

The %T/C (ratio between the tumor volume in the treated group and in the control group) was calculated by the formula tumor growth inhibition (TGI) = (1- $\Delta T/\Delta C$) x 100%, if $\Delta T > 0$. $\Delta T$, mean tumor volume of the drug-treated group on the observation day of the study - mean tumor volume of the drug-treated group on initial day of dosing; $\Delta C$, mean tumor volume of the control group on the observation day of the study - mean tumor volume of the control group on initial day of dosing. Regression was calculated using the formula = 100 x $\Delta T$/T initial, if $\Delta T < 0$. Animals with 0 $mm^3$ tumor volume (no measurable tumor) for three consecutive measurements were considered as complete responders and animals with 50% tumor regressions were partial responders.

*2.8 Data Acquisition and Statistical Analysis*

2.8.1 Data Acquisition: Protocol-required measurements and observations were recorded manually on appropriate forms.

2.8.2 Statistical Analysis: All statistical tests were conducted, and the level of significance was set at 5% or $P < 0.05$. The group means and standard deviation were calculated for all measurement parameters as study designed. One-way analysis of variance (ANOVA) was applied among the groups.

HC-7366 showed potent single agent activity in solid tumors including regressions and complete responses. HC-7366 showed up to 80% tumor growth inhibition at both 1 and 3 mg/kg in HT1080 fibrosarcoma model tumor bearing mice. HC-7366 showed 94% tumor growth inhibition at both 1 and 3 mg/kg in LoVo colorectal model tumor bearing mice. HC-7366 showed ~78% tumor growth inhibition at 1 mg/kg and 3 mg/kg in DLD-1 colorectal tumor model. HC-7366 showed 33% tumor regression at 1 mg/kg in FaDu head and neck squamous model tumor bearing mice and significant TGI at all doses. HC-7366 showed ~61-65% tumor growth inhibition at ≥ 3 mg/kg in LNCaP androgen receptor positive prostate tumor model. HC-7366 showed ~70% tumor growth inhibition at both 3 mg/kg and 30 mg/kg in TM00298 androgen receptor positive patient-derived prostate tumor model. All treatments were well tolerated by body weight.

**FIGs. 6-15 and 135** depict solid tumor growth inhibition for HC-7366 in the TGI study described above.

HC-7366 showed potent single agent activity in AML models. HC-7366 showed complete eradication of MOLM-16 xenograft tumors at 2 mg/kg whereas other doses showed tumor growth inhibition. HC-7366 at 1 and 3 mg/kg showed tumor stasis in KG-1. HC-7366 at 3 and 30 mg/kg exhibited 73% and 77% TGI in Kasumi-1, respectively. HC-7366 at 10 and 30 mg/kg showed about 47% and 51% TGI in OCI-AML2, respectively. HC-7366 showed 45% TGI at 30 mg/kg in MV4-11.

**FIGs. 16-20** depict the AML tumor growth inhibition for HC-7366 in the TGI study described above. The FAB classification of each AML is shown. HC-7366 showed robust responses in M0-M2 subtypes of AML.

**Example 7: Tumor Pharmacodynamic Analysis**

*Immunohistochemistry*

Immunohistochemical analysis was carried out on tumors that were collected at the end of the study described in Example 6. Formalin-fixed paraffin-embedded samples were sectioned at 5 μm thickness and mounted on Superfrost Plus microscope slides (Fisher Scientific). All immunohistochemical (IHC) staining was performed on Bond Rx autostainer (Leica Biosystems) using the conventional TSA-amplified detection system. Primary antibodies used were as follows: α-ASNS (Cell Signaling Technology #92479), α-PUMA (Cell Signaling Technology #98672), α-PSAT1 (Proteintech #10501-1-AP), α-PHGDH (Proteintech #14719-1-AP), HIF-1α-Novus Biologicals NB100-479, HIF-2α- Novus Biologicals NB100-122SS, and GLUT-1-EMB Biosciences 07-1401. TSA-conjugated Alexa488, Alexa 568 and Alexa647 fluorophores from Invitrogen (#B40953, #B40956, and #B40958) were used at 1:500 dilution. Slides were counterstained with DAPI and cover slipped using Mowiol anti-fade mounting media (Sigma #D2522).

*Image analysis for immunohistochemistry*

Slides were imaged with Aperio Versa 200 (Leica Biosystems) whole-slide scanner using 10x/0.32NA objective, and the data were analyzed using custom-written macros in ImageJ/FIJI (NIH). Briefly, 150 μm of outer edges of the tumor sections and necrotic regions (determined by lack of 4',6-diamidino-2-phenylindole (DAPI) signal or presence of condensed DNA) were eliminated from the analysis. Gaussian-blurred DAPI channel was used to measure the area of the tumor tissue. Scans of immune-staining were processed to remove background, then total intensity value above appropriate threshold levels was measured and normalized to tissue area. All graphing and statistics (one-way ANOVA and pair-wise comparisons) were performed using Prism GraphPad.

*S100A8 and S100A9 Immunohistochemistry staining and analysis*

Slides were deparaffinized, rehydrated and heat mediated antigen retrieval was conducted with AR6 buffer (Akoya). Slides were blocked with Roche diagnostics antibody diluent (Fisher) and stained with primary antibodies for 1 hr at 110 rpm. After washing, horseradish peroxidase (HRP)-conjugated secondary antibodies were added for 10 mins at 110 rpm. After washing, OPAL detection dye (Akoya) was added for a static 10 min incubation. Antigen retrieval was performed post OPAL staining, followed by DAPI staining and slide covering. Multispectral images were captured on the Polaris imaging system (Akoya) and spectral unmixing, cell segmentation and phenotyping was performed using Inform Tissue Finder software (Akoya). Imaging data was converted into .csv files and imported into Flowjo for analysis of S100A8/A9 percent positive cells. Primary antibodies used were rabbit anti-human/mouse S100A8/A9 (Clone RM1038, Abcam ab288715, paired with Opal 570).

HC-7366 treated KG-1 tumors were evaluated for ISR markers by IHC at the end of study (day 27) using the methods described above. HC-7366 potently induced ISR in KG-1 tumors. HC-7366 potently induced ASNS **(FIGs. 21 and 22),** PSAT1 **(FIGs. 23 and 24),** and PHGDH **(FIGs. 25 and 26).** The highest induction was observed at 1 and 3 mg/kg dose of HC-7366. HC-7366 potently reduced protein levels of S100A8/A9 in KG-1 tumors at 1 and 3 mg/kg doses **(FIGs. 27 and 28).** PUMA levels were not changed by HC-7366 in this model.

HC-7366 treated FaDu tumors were evaluated for HIF1α and HIF2α by IHC at the end of study using the methods described above. HC-7366 significantly reduced both HIF1α and HIF2α protein levels in FaDu tumors. Expression pattern of HIF1α and HIF2α was mutually exclusive **(FIGs. 40-42).**

HC-7366 treated DLD-1 tumors were evaluated for ASNS, PSAT1, PUMA, HIF1α, and HIF2α by IHC at the end of study (day 20) using the methods described above. HC-7366 potently induced ASNS, PSAT1, and PUMA in a bell-shaped manner. HC-7366 significantly reduced both HIF1α and HIF2α protein levels in FaDu tumors **(FIGs. 29-39).** HIF2α was expressed at very low levels in the tumor model. HC-7366 potently activated GCN2/ISR in tumors.

**Example 8: Combination of HC-7366 and Venetoclax in MV4-11 Cells**

MV4-11 tumors were collected at the end of the venetoclax combination study described in Example 6. Tumor growth curves are provided of MV4-11 tumors treated with single agent HC-7366 and venetoclax or combination **(FIG. 43).** HC-7366 with venetoclax showed robust combination benefit resulting in regressions. HC-7366 had 34% TGI as a single agent. Venetoclax monotherapy showed 62% TGI. However, HC-7366 at the GCN2 activating dose of 3 mg/kg showed 26% tumor regression when combined with venetoclax.

Immunohistochemical analysis was performed on the tumors according to the procedures described in Example 7. Immunohistochemical ISR marker analysis of the end-of-study tumors showed that HC-7366 at 3 mg/kg caused potent activation of ISR markers such as ASNS **(FIGs. 44 and 45),** PHGDH **(FIGs. 46 and 47)** and PUMA **(FIG. 48 and 49),** which were further increased with venetoclax combination suggesting hyperactivation of ISR. S100A8/A9 levels were significantly reduced with single agent treatment of both HC-7366 and venetoclax, and the combination of venetoclax and

HC-7366 robustly decreased S100A8/A9 levels further **(FIGs. 50 and 51).** PSAT1 levels did not change.

**Example 9: Combination of HC-7366 and anti-VEGFR antibody in 786-O, A498, and DLD-1 Tumors**

786-O and A498 tumors were collected at the end of the DC101 combination studies described in Example 6. IHC analysis was performed on the tumors according to the procedures described in Example 7.

HC-7366 treated 786-O tumors were evaluated for HIF2$\alpha$ and GLUT1 by IHC. HIF2$\alpha$ significantly decreased with both HC-7366 and DC101 single agent treatment and in combination. GLUT1 levels were significantly reduced with single agent HC-7366 or in combination with DC101 **(FIGs. 52-54).** The tumor model did not express HIF1$\alpha$.

HC-7366 treated A498 tumors were evaluated for HIF2$\alpha$ and GLUT1 by IHC. HIF2$\alpha$ significantly increased with DC101 single agent treatment. No significant changes in GLUT1 levels were observed in any of the groups **(FIGs. 55-57).** The tumor model did not express HIF1$\alpha$.

The effect of DC101 in combination with HC-7366 in DLD-1 tumor-bearing mice was evaluated using protocols analogous to those described in Example 6. Mice were treated with 3 or 30 mg/kg HC-7366, twice daily by oral gavage. DC101 was administered at 20 mg/kg twice weekly and with twice daily dosing via intraperitoneal injection. HC-7366 at 3 and 30 mg/kg inhibited tumor growth in DLD-1 colorectal model with TGI% values of ~65% and ~21%, respectively, while DC101 resulted in a TGI% value of ~55%. A significant combination benefit was observed when 3 mg/kg HC-7366 was combined with DC101 (~76% tumor growth inhibition), but no combination benefits were observed with 30 mg/kg **(FIG. 61).**

**Example 10: Combination of HC-7366 and belzutifan (PT-2977)**

Female 6-8 weeks old BALB/c nude mice were inoculated subcutaneously on the right flank with 786-O tumor cells (5 x $10^6$) in 0.1 ml of RPMI1640 medium without serum for tumor development (see Example 6). The treatments were started when the mean tumor size reached approximately 200 $mm^3$ after three weeks post inoculation and continued until end of study by twice daily oral gavage dosing of HC-7366 at listed doses. PT-2977 was dosed at 0.1 mg/kg twice a day by oral gavage till end of study.

Tumor growth curves are provided of 786-O tumors treated with single agent HC-7366 and belzutifan or combination **(FIG. 58).** HC-7366 and PT-2977 as single agents exhibited comparable antitumor efficacy in this model with TGI% values of ~78% and ~70% , respectively. Significant combination antitumor benefit was observed when HC-7366 was combined with PT2977 resulting in average tumor regression of ~50%.

Female 7-8 weeks old Athymic Nude-Foxn1nu mice were inoculated subcutaneously on the right flank with MFE280 tumor cells (10 x $10^6$) in 0.2 ml of PBS for tumor development (see Example 6). Single agent HC-7366 was dosed at 0.5 or 2 mg/kg (twice daily, orally), PT2977 at 1 mg/kg (twice daily, orally). HC-7366 at 0.5 or 2 mg/kg was combined with PT2977. The treatments were started when the mean tumor size reached approximately 191 $mm^3$ at Day 28 post inoculation and continued until end of study.

Tumor growth curves are provided of MFE280 tumors treated with single agent HC-7366 and belzutifan or combination **(FIG. 135).** Single agent HC-7366 showed 45% and 53% tumor growth inhibition at 0.5 mg/kg and 2 mg/kg, respectively. PT2977 at 1 mg/kg showed 53% tumor growth inhibition. HC-7366 0.5 mg/kg + PT2977 1 mg/kg showed 97% tumor growth inhibition. HC-7366 2 mg/kg + PT2977 1 mg/kg showed 17% tumor regression. Treatments were well tolerated as determined by body weight measurements.

**Example 11: Combination of HC-7366 and 5-fluorouracil**

The effect of 5-fluorouracil in combination with HC-7366 in DLD-1 tumor-bearing mice was evaluated using protocols analogous to those described in Example 6. Mice were treated with 3 or 30 mg/kg HC-7366, twice daily by oral gavage. 5-fluorouracil was administered at 75 mg/kg once a week, twice daily via intravenous injection. HC-7366 at 3 and 30 mg/kg inhibited tumor growth in DLD-1 colorectal model with TGI% values of ~65% and ~21%, respectively, while 5-fluorouracil resulted in a TGI% value of ~68%. There was a significant combination benefit observed when 3 mg/kg HC-7366 was combined with DC101 (~88% tumor growth inhibition; tumor stasis), but no combination benefits were observed with 30 mg/kg **(FIG. 62).**

**Example 12: Combination of HC-7366 and PI3K$\alpha$ or MEK1/2 inhibitors**

HCT-116 tumor growth was monitored in alpelisib and trametinib combination studies described in Example 6.

HC-7366 showed a trend (~38% TGI) for efficacy at 3 mg/kg dose in HCT116 colorectal cancer model, while 30 mg/kg HC-7366 did not reduce tumor growth. Significant combination benefit was observed when alpelisib was co-administered with either dose of HC-7366 **(FIG. 63).**

HC-7366 did not show efficacy in HCT116 at either 3 or 30 mg/kg but had significant combination benefit when

trametinib was co-administered with 3 mg/kg dose of HC-7366 **(FIG. 64).**

All treatments were well tolerated as measured by body weight.

**Example 13: Combination of HC-7366 and EGFR inhibitors**

NCI-H1975 tumor growth was monitored in the osimertinib and dacomitinib combination studies described in Example 6.

HC-7366 at 3 mg/kg showed significant anti-tumor efficacy with a TGI of 59%, while osimertinib at 2.5 mg/kg resulted in a TGI value of 95% with 1/10 partial response. HC-7366 + osimertinib showed combination benefits resulting in average tumor regression of ~90% with 2/10 complete responses and 8/10 partial responses **(FIG. 131).**

Dacomitinib alone at 15 mg/kg resulted in tumor stasis followed by tumor growth with a TGI% value of 36% at the end of the study without statistical significance. HC-7366 + dacomitinib showed combination benefits with a TGI% value of ~99% and 1/10 partial response **(FIG. 133).** Animals in the dacomitinib-containing groups showed significant body weight loss. As a result, animals in these groups were given dosing holidays. No treatment related body weight loss was observed in the HC-7366 and vehicle groups.

**Example 14: GCN2 Dependence of HC-7366 Mediated Effects**

*CTG Assay*

MOLM-16 cells were obtained from DSMZ (#ACC 555) and were cultured in RPMI 1640 (Gibco, #11875119) with 20% FBS (Invitrogen, #10099141C) at 37 °C and 5% $CO_2$. MOLM-16 GCN2 CRISPR knockout cells (sgGCN2) were generated and compared to control cells (sgControl). For viability experiments, cells were plated in 384-well cell culture plates and incubated overnight. Compounds were diluted in 8 concentrations by 3-fold dilution in 384pp-plate using TECAN EVO200 to a final concentration ranging from 1-10 μM. The plates were incubated for 24 or 48 hr and viability was assessed by adding CellTiter-Glo® 2.0 Assay according to the manufacturer's protocol (Promega, G9243) to each well. The plates were then incubated at RT for 30 min. The luminescent signals were read by Envision (PerkinElmer) plate reader and analyzed by Prism.

FaDu cells were obtained from ATCC (#HTB-43) and were cultured in Eagle's Minimum Essential Medium, Catalog No. 30-2003 at 37 °C and 5% $CO_2$. FaDu GCN2 CRISPR knockout cells (sgGCN2) or control cells (sgControl) were generated. For viability experiments, cells were plated in 384-well cell culture plates and incubated overnight. Compounds were diluted in 10 concentrations by 3-fold dilution in 384pp-plate using TECAN EVO200 to a final concentration ranging from 1-10 μM. The plates were incubated for 72 or 96 hr and viability was assessed by adding CellTiter-Glo® 2.0 Assay according to the manufacturer's protocol (Promega, G9243) to each well. The plates were then incubated at RT for 30 min. The luminescent signals were read by Envision (PerkinElmer) plate reader and analyzed by Prism

HC-7366 reduced viability in vitro and induced ISR in a GCN2-dependent manner in MOLM-16 cells. HC-7366 potently reduced viability at lower concentrations in a U-shaped manner **(FIG. 65).** This response was reversed in GCN2 CRISPR knockout cells **(FIG. 66).**

HC-7366 reduced viability *in vitro* and induced ISR in a GCN2-dependent manner in FaDu cells. HC-7366 potently reduced viability in FaDu control cells **(FIG. 71).** This response was reversed in GCN2 CRISPR knockout cells **(FIG. 72).**

*Jess Protein analysis*

Cell lysates were obtained from MOLM-16 sgControl or sgGCN2 cells treated with HC-7366 (0, 0.01, 0.1 and 10 μM) for 24 hr. Ice cold 1x radioimmunoprecipitation assay (RIPA) lysis buffer supplemented with protease and phosphatase inhibitors was added to the cell pellets, on ice. After 30 min on ice, the cell lysates were centrifuged at 4 °C, 12,000 rpm for 15 minutes and the supernatant collected. Protein concentration was determined using BCA kit. The cell lysates were mixed with 5X sodium dodecyl sulfate (SDS) loading buffer and denatured at 95 °C for 5 minutes. 1 μg lysate was run on Jess capillary and results were analyzed by compass simple western software.

Cell lysates were obtained from FaDu sgControl or sgGCN2 cells treated with HC-7366 (0, 0.01, 0.1 and 1 μM) for 72 hr. Ice cold 1x radioimmunoprecipitation assay (RIPA) lysis buffer supplemented with protease and phosphatase inhibitors was added to the cell pellets, on ice. After 30 min on ice the cell lysates were centrifuged at 4 °C, 12,000 rpm for 15 minutes and the supernatant was collected. Protein concentration was determined using BCA kit. The cell lysates were mixed with 5X SDS loading buffer and denatured at 95°C for 5 minutes. 1 μg lysate was run on Jess capillary and results were analyzed by compass simple western software. The following primary antibodies were used: p-GCN2 T899 (Abcam # ab75836), GCN2 (Cell Signaling # 3302), ATF4 (Cell Signaling # 11815) and ASNS (Proteintech # 14681). Anti-rabbit

secondary antibody was from ProteinSimple # 042-206.

The protein expression of GCN2 in wild type MOLM-16 cells (WT, sgControl) and CRISPR knockout (sgGCN2) cells were measured by JESS as described above **(FIG. 67).** HC-7366-mediated activation of ISR was assessed by observing levels of ATF4 **(FIG. 68)** and its downstream targets ASNS **(FIG. 69)** and PSAT1 **(FIG. 70),** which showed a GCN2-dependent increase.

The protein expression of GCN2 in wild type (WT) FaDu cells (WT, sgControl) and CRISPR knockout (sgGCN2) cells were measured by JESS as described above **(FIGs. 73 and 74).** HC-7366-mediated activation of ISR was assessed by observing levels of ATF4 **(FIG. 75)** and its downstream target ASNS **(FIG. 76),** which showed a GCN2-dependent increase with HC-7366 at 0.01 and 0.1 μM concentration.

*Polysome profiling*

Polysome profiling was performed in HEK 293 GCN2 WT or HEK293 GCN2 knockout cells as previously described in Johannes and Sarnow et al., RNA, 1998. Cells were treated with HC-7366 for 16 hours and then collected for polysome profiling.

The effects of HC-7366 on protein synthesis were evaluated by polysome profiling of GCN2 WT and CRISPR knockout HEK 293 cells. HC-7366 reduced polysome to monosome ratio, which reflects reduced translation in GCN2 WT cells but not in GCN2 knockout cells **(FIGs. 77 and 78).** HC-7366 potently reduced puromycin labeling of newly synthesized proteins at 100 nM which activates GCN2/ISR pathway as measured by increased levels of p-eIF2α and ATF4 **(FIGs. 79 and 80).**

HC-7366 reduces viability in vitro and induces ISR in a GCN2-dependent manner.

**Example 15: Ex Vivo Patient-Derived Xenograft (PDX) Study with HC-7366**

HC-7366 (4.57-10,000 nM) was evaluated in 30 patient-derived Champions' human AML models, ex vivo in 2D cell cultures. Cell viability was assayed after treating PDX models with DMSO or HC-7366 for 6 days using a plate-based luminescence assay (Cell Titer Glo). HC-7366 potently reduced viability of primary AML patient-derived xenograft (PDX) cells.

11 PDX models were sensitive responders with $EC_{50}$ of < 100 nM. 12 PDX modes were moderate responders with $EC_{50}$ 100-350 nM. 5 PDX models were resistant responders with $EC_{50}$ 924-2253 nM. Only two models showed no response to HC-7366. $EC_{50}$ values were calculated to the bottom of the curve and are provided below. Viability curves are provided for the models CTG-2229 **(FIG. 81),** CTG-3680 **(FIG. 82),** CTG-3667 **(FIG. 83),** CTG-2456 **(FIG. 84),** CTG-2457 **(FIG. 85),** and CTG-2454 **(FIG. 86).**

| PDX MODEL | HC-7366 $EC_{50}$ (uM) |
|---|---|
| CTG-2456 | 31.8 |
| CTG-2229 | 34.0 |
| CTG-2455 | 35.7 |
| CTG-2236 | 36.5 |
| CTG-2227 | 39.9 |
| CTG-2775 | 43.4 |
| CTG-3440 | 55.2 |
| CTG-2234 | 65.5 |
| CTG-3673 | 65.6 |
| CTG-2232 | 84.3 |
| CTG-2774 | 96.9 |
| CTG-3441 | 101.7 |
| CTG-2233 | 101.7 |
| CTG-2235 | 103.1 |
| CTG-3679 | 106.7 |
| CTG-2238 | 122.8 |

(continued)

| PDX MODEL | HC-7366 $EC_{50}$ (uM) |
|---|---|
| CTG-3674 | 128.6 |
| CTG-2453 | 179.5 |
| CTG-3438 | 196.2 |
| CTG-3680 | 230.8 |
| CTG-2457 | 247.8 |
| CTG-2700 | 333.1 |
| CTG-3439 | 349.4 |
| CTG-3667 | 924.9 |
| CTG-2239 | 1166 |
| CTG-2228 | 1771 |
| CTG-2240 | 2220 |
| CTG-2704 | 2253 |
| CTG-2454 | >10000 |
| CTG-2701 | ND |

**Example 16: AML PDX In Vivo Study**

Sub-lethally irradiated NCG mice were inoculated with $2x10^6$ human AML cells of model CTG-2229. Mice were monitored post AML inoculation to assess human AML engraftment using huCD45/muCD45/huCD33/huCD3 antibodies and BD TruCountTM beads. When individual animals had ≥ 20% live huCD45 cells in the bone marrow, they were randomized to treatment cohorts. For the efficacy study, animals were administered (n = 13/group) with vehicle control, HC-7366 at 1 mg/kg, 10 mg/kg or 30 mg/kg PO/BID×28, or Venetoclax at 100 mg/kg PO/QD×28. Terminal blood, spleen, and bone marrow samples were collected for flow cytometry **(FIG. 87).** Tolerability was assessed by body weight loss, lethality, and clinical signs of adverse treatment-related side effects. Body weights were measured twice weekly.

1 mg/kg HC-7366 significantly decreased myeloid restricted precursor ($CD34^+CD33^+$) and mature myeloid cells ($CD34^-CD33^+$) in all tissues. HC-7366 was most pronounced in bone marrow when compared to venetoclax.

**Example 17: HC-7366-mediated effects on oxygen consumption rate (OCR) and metabolomics**

MOLM-16 cells were treated with 0.001, 0.1 or 10 μM HC-7366 for 16 hrs and the Seahorse XF Glycolysis Stress Test (ECAR; extracellular acidification rate) and Seahorse XF Mito stress test (OCR; oxygen consumption rate) (Agilent technologies, CA, USA) were performed.

HC-7366 significantly reduced OCR **(FIG. 88)** and glycolysis **(FIG. 89)** with the strongest effect observed at the activating 0.1 μM concentration. Collectively, these results show that HC-7366 reduces both mitochondrial oxygen consumption rate and glycolysis leading to reduced ATP production.

**Example 18: HC-7366 Mediated Effects on Metabolomics in Tumor Xenograft Models**

MOLM-16 xenograft tumors were treated with Vehicle or HC-7366 (0.3, 1, 3, 10 or 30 mg/kg) for four days, and snap frozen tumors were collected for metabolomic analysis (n=12). Metabolic analysis was performed at Metabolon on the LC/MS/MS and Polar LC platforms. Data was normalized to Vehicle group and Welch's two-sample t-test was performed for statistical analysis. Heat maps show metabolite levels normalized to vehicle control **(FIGs. 90, 94, 102, and 107).**

HC-7366 significantly altered multiple metabolites in MOLM-16 tumors.

HC-7366 significantly elevated levels of most amino acids. Some amino acid levels were decreased with HC-7366; this included aspartate and cysteine **(FIGs. 90-93).** HC-7366 significantly reduced glutathione levels (GSH and GSSG) and enhanced numerous γ-glutamyl-amino acids suggesting that the HC7366-treated tumors attempted to increase glutathione production, potentially in response to increased oxidative stress in the cells **(FIGs. 94-101).** HC-7366 significantly reduced metabolites involved in pyrimidine synthesis **(FIGs. 102-106).** HC-7366 significantly reduced

aspartate metabolism and polyamine-related metabolites (putrescine, spermine, 5-methylthioadenosine (MTA)) at all doses **(FIGs. 107-114).** Generally, the most changes in metabolites occurred at 1 and 3 mg/kg doses.

FaDu tumor bearing mice were treated with vehicle or HC-7366 (1, 3, or 30 mg/kg) twice a day orally for three days, and on the morning of Day 4 an AM dose was administered and tumors and plasma were collected 1hr after dose. Tumor and plasma samples were extracted and split into equal parts for analysis on LC/MS/MS and Polar LC platforms. Proprietary software was used to match ions to an in-house library of standards for metabolite identification and for metabolite quantitation by peak area integration (Metabolon). Proteomic analysis on tumors was performed at MS Bioworks. The equivalent of 3 μg of peptide from each pooled fraction was analyzed by nano LC-MS/MS with a Waters M-Class HPLC system interfaced to a ThermoFisher Fusion Lumos mass spectrometer. Histograms show metabolite levels of treatment groups normalized to vehicle control **(FIGs. 115-122).**

HC-7366 significantly elevated levels of several amino acids both in tumor and plasma. HC-7366 also significantly increased several gamma glutamyl amino acid levels in both tumor and plasma. HC-7366 significantly increased levels of arginine, arginosuccinate and citrulline in tumors and significantly increased urea levels in the plasma. HC-7366 significantly reduced pyrimidine synthesis metabolites in tumors. HC-7366 significantly reduced oxidized glutathione levels but did not alter reduced glutathione levels in tumor.

**Example 19: HC-7366 Mediated Effects on Metabolomics in Cells**

Wild type and GCN2 CRISPR knockout FaDu cells were treated with 0.1 μM for 9 hours, and metabolomics were evaluated.

Treatment of wild type FaDu cells with 0.1 μM HC-7366 for 9 h resulted in an enhanced level of many amino acids in a GCN2-dependent manner. Basal levels of Ala, Asp, Glu, Gly, Pro, Thr, and Asn were reduced in GCN2 CRISPR knockout cells indicating that GCN2 regulates the synthesis of several amino acids following HC-7366 treatment. This result confirmed the *in vivo* FaDu data (see Example 18) showing enhanced free amino acids in tumor and plasma and highlighted that GCN2 mediates this effect during HC-7366 treatment. GCN2 wild type cells and GCN2 CRISPR Knockout cells were treated with 0.1 μM HC-7366 which induced ATF4 in a GCN2 dependent manner **(FIGs. 123-125).**

**Example 20: Effect of HC-7366 on Oxidative Phosphorylation in FaDu Xenograft Model**

FaDu tumor bearing mice were treated with HC-7366 at 3 and 30 mg/kg twice a day for four days. Tumors were harvested for non-targeted proteomic analysis.

Pathway analysis (IPA) of differentially expressed proteins in FADU tumors predicted strong repression of the oxidative phosphorylation pathway with 3 mg/kg, but not with 30 mg/kg of HC-7366 **(FIG. 126).**

Several protein subunits of the electron transport chain were decreased in the 3 mg/kg treatment group (relative to vehicle). Fewer oxidative phosphorylation proteins were changed after treatment with 30 mg/kg (relative to vehicle), and some increased **(FIG. 127).** The data suggest that the activating dose of 3 mg/kg HC-7366 inhibits mitochondrial respiration in tumors, representing a possible mechanism of action that is unique to this dose.

**Example 21: RNAseq Analysis of DLD-1 Tumor Xenograft Model Treated with HC-7366**

DLD-1 tumor bearing mice were treated with HC-7366 at 0.3, 1, 3, 10 and 30 mg/kg twice a day for 14 days. Tumors were harvested for RNAseq analysis at Day 4, Day 7 and Day 14 **(FIG. 128).**

Analysis of upstream regulators of differentially expressed genes showed that ATF4 was significantly induced at all doses of HC-7366. Certain transcription factors associated with efficacy. These included ARID1A, SMAD4 and E2F1 **(FIG. 129).** The analysis showed reduction of HIF1α, SMAD4 and E2F1 signature while enhancing ATF4 and ARID1A signature.

IHC staining of Ki67 positive cells in DLD-1 tumor sections showed that HC-7366 significantly reduced Ki67 positive cancer cells on Day 4 at the most efficacious doses of 1 and 3 mg/kg but not at the less efficacious doses of 0.3, 10 or 30 mg/kg **(FIG. 130).**

**Example 22: A Multicenter, Open-label, Phase 1a/b study of the Compound of Formula (I) in Subjects with Advanced Solid Tumors**

A first-in-human, multicenter, open-label, Phase 1a/b dose-escalation and dose-expansion study to establish the maximum tolerated dose (MTD), recommended phase II dose (RP2D), and evaluate the safety and tolerability of once daily (QD) oral dosing of HC-7366 potassium salt monohydrate in a dose-escalating fashion in subjects with advanced solid tumors was conducted. Up to 36 subjects were enrolled into the Phase 1a dose-escalation part of the study. Approximately 50% of all subjects enrolled in this study were subjects with squamous cell carcinoma of the head and neck

(SCCHN), colorectal cancer (CRC), non-small cell lung cancer (NSCLC), and transitional cell carcinoma of the bladder (TCC). Subjects with other solid tumor types were eligible provided study selection criteria were met and that they did not exceed 50% of all enrolled subjects. The study was conducted in the United States at approximately 3 to 5 sites. This Phase 1a/b study followed a traditional 3+3 design. The starting dose level was 10 mg QD, escalating to 20, 40, 75, 125, and 150 mg QD as safety allowed. All doses were administered in the fasting state with water at least 1 hour before food or at least 2 hours after food. The Phase 1b dose-expansion part involved cohort expansion at up to 2 dose levels selected from the dose-escalation data by the safety monitoring committee (SMC), to obtain additional safety and preliminary efficacy information. Each cohort in Phase 1b enrolled 15 subjects. The study can be expanded into a Phase 2 study via protocol amendment which assesses the dose and tumor type(s) selected in Phase 1a/b as the most appropriate for further clinical development. Subjects were dosed until unacceptable toxicity, disease progression per immune-related Response Evaluation Criteria in Solid Tumors (iRECIST), discontinuation of treatment for other protocol-allowed reason (e.g., subject refusal), any other administrative reasons, or after 2 years of treatment, whichever occurs first. For scheduling purposes, dosing occurred in 3-week cycles and computed tomography (CT) scans were conducted once every 6 weeks with the first postbaseline scan after 6 weeks of dosing (precycle 3). Subjects spent Cycle 1/Day 1 (C1D1) in the clinic followed by an overnight stay for safety monitoring and pharmacokinetic (PK) sampling. Subjects were hospitalized for administration of the first 2 doses: C1D1 and Cycle 1/Day 2 (C1D2) (as local coronavirus disease 19 restrictions allow). After the initial hospital stay at the start of study, subjects were seen in outpatient clinic on Days 8, 15, and 21 of Cycle 1 and thereafter, on the first day of each cycle for physical and laboratory assessments, adverse event (AE) and dosing compliance monitoring, and PK C3-C6; the End of Treatment visit is also in-person in outpatient clinic. An overnight stay for Cycle 1/Day 21 (C1D21) to C2D1 was optional. Subjects who discontinued before the first postbaseline CT scan for reasons other than disease progression, a treatment-related AE, or dose-limiting toxicity (DLT) prior to completion of the DLT-evaluation period were replaced to ensure an adequate safety assessment at each dose level.

***(1) Dose Escalation Scheme***

There are six dose-escalation levels:

| Dose Level | HC-7366 Dose (QD) |
|---|---|
| 1 (Starting Dose) | 10 mg |
| 2 | 20 mg |
| 3 | 40 mg |
| 4 | 75 mg |
| 5 | 125 mg |
| 6 | 150 mg |

Dose-escalation followed a traditional 3+3 design. A minimum of 3 subjects were enrolled in each cohort sequentially, with expansion to 6 subjects in each cohort as needed to determine the DLT. For each cohort, the first subject was a sentinel subject. Sentinel subjects were dosed and followed for 4 days to assess safety and tolerability. If deemed safe and well tolerated, the remainder of the cohort (N=2) was enrolled. If none of the first 3 subjects in a cohort experienced a study treatment-related DLT during the first 21 days (DLT-evaluation period), the next cohort was enrolled. If 1 of 3 subjects experienced a study treatment-related DLT, the same cohort was expanded to 6 subjects. If 1 of 6 subjects experienced a study treatment-related DLT, the next cohort was enrolled. If at any time during a cohort, ≥2 subjects experienced a study treatment-related DLT, the MTD is exceeded, additional enrollment within the cohort was ceased, and dose-escalation stopped. The MTD was defined as the dose level below which the study treatment-related DLT is observed in ≥33% (i.e., ≥2 of 6) of subjects in a cohort. If there were 2 DLTs in Dose Level 1, Dose Level -1 was enrolled. Before applying the dose-escalation rules, 3 subjects in a given dose level must have received a minimum of 75% of the planned dose and have been evaluated for toxicity, unless one or more subjects experiences a DLT within the first 21 days. If the first 3 DLT-evaluable subjects within a cohort experienced no DLTs during the DLT-evaluation period, the next cohort was enrolled. In the case a subject does not receive a minimum of 75% of the planned dose, for any reason other than a DLT (i.e., lost to follow-up), the subject was replaced.

A decision to dose escalate was made on the basis of all observations in all subjects in the respective cohort. The DLT-evaluable subjects included subjects that experienced AEs and serious AEs related to the disease, but not treatment. Even if the predefined criteria were not been met, the investigators and sponsor may have jointly decided to add additional subjects to a given dose level (no more than 6 evaluable subjects per cohort) or not to dose escalate on the basis of all

available data.

Dose-escalation guidelines for sequential cohorts are summarized below:

| Subjects with Treatment-Related DLT at a Given Dose Level | Dose-escalation* |
|---|---|
| 0 of 3 | Proceed to next dose cohort level |
| 1 of 3 | Enroll 3 additional subjects at this dose level (6 total evaluable subjects) |
| ≥2 of 3 | Dose-escalation stops. MTD is established as the next lower dose level |
| 1 of 6 | Proceed to next dose cohort level |
| ≥2 of 6 | Dose-escalation stops. MTD is established as the next lower dose level |
| ≤1 of 6 at the highest evaluated dose level | MTD not achieved. Maximum administered dose obtained |

***(2) Dose-limiting Toxicity***

Dose-limiting toxicities w defined as 1 of the following events occurring during the DLT-evaluation period (i.e., up to the end of Cycle 1, 21 days after the first administration of HC-7366):

Nonhematologic:

- Any Grade 3 or higher nonhematologic toxicity, according to the National Cancer Institute Common Terminology Criteria for Adverse Events (NCI-CTCAE) v 5.0, except for any of the following:
    - ◦ Grade > 3 nausea or vomiting that resolves to Grade 1 or 2 in 72 hours or less with optimal medical management.
    - ◦ Grade 3 fatigue < 5 days.
    - ◦ Isolated Grade 3 laboratory abnormalities that are not associated with clinical signs or symptoms and are reversed with appropriate maximal medical intervention within 3 days.
- Grade ≥ 2 cardiotoxicity.
- Grade ≥ 2 pituitary toxicity (hypopituitarism).
- Any symptomatic congestive heart failure, decreased left ventricular ejection fraction of 50% or less based on multigated acquisition scan or echocardiogram, or QT interval corrected for heart rate (QTc) prolongation to greater than 500 msec.
- Any treatment-related Grade 3 or higher nonhematologic laboratory abnormality with the following exceptions:
    - ◦ Grade 3 or higher amylase or lipase that is not associated with symptoms or clinical manifestations of pancreatitis.
    - ◦ Grade 3 or higher electrolyte abnormality that lasts up to 72 hours, is not clinically complicated, and resolves spontaneously or responds to conventional medical interventions.
- Delay of Cycle 2 treatment start by > 2 weeks due to unresolved treatment-related Grade ≥ 3 nonhematologic toxicity.

Hematologic:

- Grade 4 thrombocytopenia for ≥ 7 days or Grade 3 or 4 thrombocytopenia associated with bleeding or requiring platelet transfusion.
- Grade 4 neutropenia for ≥ 7 days.
- Any grade neutropenic fever (> 38.5 °C).

- Grade 4 anemia or Grade 3 or 4 anemia requiring blood transfusion.
- Note: Grade 3 or 4 lymphopenia are not considered DLTs, but is recorded as part of the overall safety evaluation.

Other:

- More than 25% of prescribed doses are missed due to any unresolved, treatment-related toxicity.

An AE is ascribed as related to the study drug except where a clear relationship to the underlying disease or recognized comorbidities is evident.

Any subject who experiences a treatment-related DLT is immediately removed from study treatment. Additional subject cohorts are not enrolled at the subsequent dosing level until all subjects at the initial (or previous) dosing levels complete all planned treatment for Cycle 1 (as 3 weeks of oral QD dosing at the assigned dose level) and are able to start Cycle 2 with no more than a 2-week delay.

***(3) Recommended Phase 2 Dose***

The RP2D and administration duration of HC-7366 was determined in discussion with the SMC along with the sponsor, considering the available data on safety, PK, and efficacy. The RP2D did not exceed the MTD. If the MTD was not reached at the end of the Phase 1a/b study, the RP2D was determined by the SMC along with sponsor after a review of the study data.

***(4) Number of Subjects***

Up to 66 subjects (Phase 1a: N = up to 36 subjects; Phase 1b: N = 30 subjects).

***(5) Diagnosis and Criteria for Inclusion***

**Criteria for Inclusion:** Each subject must:

1. Have a signed an informed consent form prior to any study-specific procedures or treatment.
2. Be ≥ 18 years of age (male or female) at the time of consent.
3. Have 1 of the following tumor types with qualifying characteristics, and have received at least 1 and no more than 5 prior lines of therapy:

   a. SCCHN
   b. CRC
   c. NSCLC
   d. TCC
   e. Other solid tumors (e.g., carcinoma of unknown primary) with the exception of rapidly progressing neoplasms (e.g., pancreatic cancer, glioblastoma, hepatocellular carcinoma).

   Subjects do not need to have progressed through all possible available therapies with known clinical benefit for their respective cancers to participate in this study. Subjects with SCCHN, CRC, NSCLC, and TCC are a priority and should constitute as whole, at least 50% of the enrolled population. Enrollment of all other will be capped when reaching a combined 50%, in order to maintain 18 slots for subjects with SCCHN, CRC, NSCLC, and TCC .
4. Have at least 1 radiologically measurable lesion as per Response Evaluation Criteria in Solid Tumors (RECIST) v 1.1 defined as a lesion that is at least 10 mm in longest diameter or lymph node that is at least 15 mm in short axis imaged by CT scan or magnetic resonance imaging and obtained by imaging within 28 days prior to study treatment. Tumor lesions situated in a previously irradiated area are considered measurable if progression has been demonstrated in such lesions
5. Have resolution of all previous treatment-related toxicities to Grade 1 severity or lower, except for stable sensory neuropathy (≤ Grade 2) and alopecia. If the subject received major surgery or radiation therapy of >30 Gy, they must have recovered from the toxicity and/or complications from the intervention
6. If subjects were previously treated with immune checkpoint inhibitors, at least 4 weeks must have elapsed since the last dose, and toxicities resolved as above
7. Subjects must have at least one biopsiable lesion at baseline. Biopsies in this clinical study conform to American

Society of Clinical Oncology's Ethical Framework for Including Research Biopsies in Oncology Clinical Trials. Provided there are suitable and accessible lesions, no biopsy contraindications, minimal risk of complications and a positive informed decision, subjects are willing to provide fresh tissue for biomarker analysis, and, based on the adequacy of the tissue sample quality, for assessment of biomarker status. Two biopsies are necessary: at baseline (within 15 days prior to study Day 1) and at the time of the first response assessment CT scan at Cycle 3/Day 1 (+7 days). Newly obtained biopsy specimens are preferred to archived samples and formalin-fixed, paraffin-embedded block specimens are preferred to slides.

8. Have Eastern Cooperative Oncology Group performance status of 0 or 1 and sustained between screening and initiation of dosing on Day 1.

9. Have no swallowing difficulties that would prevent compliance with oral dosing.

10. Have not experienced > 10% body weight loss in the previous 4 weeks.

11. Have a serum albumin level > 3 g/DL.

12. Have life expectancy of 3 months or greater as determined by the treating physician.

13. Have adequate organ function on Day 1, as defined by meeting all of the following criteria:

a. Total bilirubin ≤ 1.5 × upper limit of normal (ULN) OR direct bilirubin ≤ ULN for subjects with total bilirubin levels > 1.5 x ULN.

b. Aspartate aminotransferase and alanine aminotransferase ≤ 2.5 × ULN or ≤ 5 × ULN for subjects with known hepatic metastases.

14. Have adequate renal function on Day 1, as defined by creatinine ≤ 1.5 × ULN and creatinine clearance ≥ 60 mL/min, as per the below Cockcroft Gault formula

$$eC_{Cr} = \frac{(140 - \mathrm{Age}) \times \mathrm{Mass\ (in\ kilograms)} \times [0.85\ if\ Female]}{72 \times \mathrm{Serum\ Creatinine\ (in\ mg/dL)}}$$

15. Have adequate hematologic function on Day 1, as defined by meeting all of the following criteria:

a. Hemoglobin ≥ 9 g/dL (uncorrected by red blood cell transfusion or erythropoietin support).

b. Absolute neutrophil count ≥ 1.5 × $10^9$/L.

c. Platelet count ≥ 100 × $10^9$/L.

16. Have adequate coagulation function on Day 1, as defined by either of the following criteria:

a. International normalized ratio (INR) <1.5 × ULN OR for subjects receiving warfarin or low molecular weight heparin, the subject must, in the investigator's opinion, be clinically stable with no evidence of active bleeding while receiving anticoagulant therapy. The INR for these subjects may exceed 1.5 × ULN if that is the goal of anticoagulant therapy.

b. Activated partial thromboplastin time < 1.5 × ULN unless subject is receiving anticoagulant therapy, provided prothrombin time or partial thromboplastin time is within therapeutic range of intended use of anticoagulants.

17. Have normal or adequately controlled pan-endocrine function (pituitary, adrenal, thyroid, pancreatic, gonadal). Subjects on hormonal supplementation must be stable at their treatment doses.

18. Female subjects of childbearing potential must have a negative urine or serum pregnancy test within 72 hours prior to receiving the first dose of study medication. If the urine test is positive or cannot be confirmed as negative, a serum pregnancy test is required

19. Female subjects of childbearing potential must be willing to use an adequate form of contraception from the signing of the informed consent form (ICF) until 90 days after the last dose of study medication.

20. Female subjects must agree not to breastfeed and not to donate ova starting at screening and throughout the study treatment, and for 90 days after the final administration of study drug.

21. Male subjects with a pregnant or breastfeeding partner(s) must agree to remain abstinent or use a condom for the duration of the pregnancy or for the time their partner is breastfeeding throughout the study treatment and for 90 days after the final administration of study drug.

22. Male subjects must not donate sperm during the treatment period and for at least 90 days after the final administration of the study drug.

23. Male subjects with female partner(s) of childbearing potential must agree to use a condom with spermicide during the treatment period and for at least 90 days after the final administration of the study drug.

24. Be willing and have the ability to comply with scheduled visits (including geographical proximity), treatment plans, laboratory tests, and other study procedures.

**Criteria for Exclusion:** Subject must not meet any of the following criteria:

1. Had prior chemotherapy, targeted small molecule therapy, or radiation therapy within 2 weeks prior to the first dose of study treatment or who has not recovered from adverse reactions due to a previously administered agent or major surgery.
2. Is currently participating and receiving study therapy or has participated in a study of an investigational agent and received study therapy or used an investigational device within 4 weeks of the first dose of treatment.
3. Has a diagnosis of immunodeficiency or receiving systemic steroid therapy or any other form of immunosuppressive therapy within 7 days prior to the first dose of study treatment. The use of physiologic doses of corticosteroids may be approved after consultation with the sponsor.
4. Has known history of active tuberculosis.
5. Has known history of human immunodeficiency virus (HIV) (HIV 1/2 antibodies).
6. Has known active hepatitis B (e.g., hepatitis B surface antigen reactive) or hepatitis C (e.g., hepatitis C virus ribonucleic acid [RNA] [qualitative]) infection.
7. Has been diagnosed with severe acute respiratory syndrome coronavirus-2 infection confirmed by real-time polymerase chain reaction (PCR) test as per the local guidelines at screening and positive by PCR within 7 days prior to the first dose of study treatment.
8. Has a history of clinically severe autoimmune disease, or history of organ transplant.
9. Has a history of retinitis or photosensitive skin disorders including (but not limited to) erythema multiforme, atopic eczema, psoriasis, viral exanthemata, pemphigus, and dermatitis herpetiformis.
10. Has known additional malignancy that is progressing or required active treatment within the previous 5 years. Exceptions include basal cell carcinoma or squamous cell carcinoma of the skin that has undergone potentially curative therapy, superficial bladder cancer, or in situ cervical cancer. Subjects with other malignancies are eligible if they were cured by surgery alone or surgery plus radiotherapy and have been continuously disease-free for at least 5 years.
11. Has known active central nervous system metastases and/or carcinomatous meningitis. Subjects with previously treated brain metastases may participate provided they are stable (without evidence of disease progression by imaging for at least 4 weeks prior to the first dose of study treatment and any neurologic symptoms have returned to baseline), have no evidence of new or enlarging brain metastases, and are not using systemic steroids for at least 7 days prior to study treatment. This exception does not include carcinomatous meningitis which is excluded regardless of clinical stability.
12. Has a history of interstitial lung disease, pneumonitis within 12 months prior to screening, or current pneumonitis.
13. Has an active infection requiring systemic therapy.
14. Has a history or current evidence of any condition, therapy, or laboratory abnormality that might confound the results of the study, interfere with the subject's participation for the full duration of the study, or is not in the best interest of the subject to participate, in the opinion of the treating investigator.
15. Has a clinically significant cardiovascular disease such as unstable angina, myocardial infarction, or acute coronary syndrome, symptomatic or uncontrolled arrhythmia, congestive heart failure, baseline electrocardiogram (ECG) abnormalities, including, but not limited to, QTc prolongation to greater than 470 ms, or any Class III or IV cardiac disease as defined by the New York Heart Association Functional Classification.
16. Has overt or latent disorders of the exocrine pancreas (such as acute or chronic pancreatitis of any etiology) or chronic (including autoimmune) gastrointestinal disorders such as Crohn's disease, ulcerative colitis, rheumatoid arthritis, lupus, scleroderma, Sjogren's syndrome, and polyarteritis nodosa.
17. Has a known psychiatric or substance abuse disorder(s) that would interfere with informed consent or cooperation with the requirements of the study.
18. Is pregnant or breastfeeding or expecting to conceive children within the projected duration of the study, starting with the screening visit through 90 days after the final administration of the study drug.
19. Is a first-degree relative of the investigator, staff, or study sponsor.

***(6) Investigation Medicinal Products, Dose, and Mode of Administration***

HC-7366 potassium salt monohydrate capsules.

Dose levels: 10, 20, 40, 75, 125, and 150 mg QD.

Administration route: oral, in a fasted state with water.

***(7) Duration of Subject Participation in the Study***

Each subject was treated for a maximum of 2 years and followed for a maximum of 2 years.

Subjects continued to be dosed until unacceptable toxicity or documented disease progression per iRECIST, discontinuation of treatment for other protocol-allowed reason (e.g., subject request), any other administrative reasons, or after 2 years of treatment, whichever occurred first. Treatment of subjects beyond 2 years was at the discretion of the physician and the sponsor, in consultation with the subject.

***(8) Endpoints***

**Primary Endpoints:**

The primary endpoints of this study are as follows:

- Determination of MTD and RP2D.
- Safety and tolerability
    - Occurrence of DLTs.
    - Number and severity of treatment-emergent adverse events (TEAEs) and treatment-related TEAEs according to NCI-CTCAE v 5.0.
    - Incidence of TEAEs leading to premature discontinuation.
    - Incidence of laboratory abnormalities, based on NCI-CTCAE grades of hematology, serum chemistry, and urinalysis test results.
    - Incidence of abnormalities observed in 12-lead ECG parameters.
    - Incidence of abnormalities observed in vital signs measurements.

**Secondary Endpoints:**

The secondary PK endpoints for this study include, as appropriate, the following:

- Area under the plasma concentration versus time curve from time 0 until last.
- measurable concentration ($AUC_{0\text{-}last}$).
- Area under the plasma concentration versus time curve from time 0 to 24 hours post-dose ($AUC_{0\text{-}24}$).
- Area under the plasma concentration versus time curve from time 0 extrapolated to infinity ($AUC_{0\text{-}\infty}$).
- Area under the plasma concentration versus time curve over a dosing interval ($AUC_{0\text{-}t}$).
- Maximum observed plasma concentration (Cmax).
- Time of the maximum observed plasma concentration (tmax).
- Apparent total clearance (CL/F).
- Apparent volume of distribution during the terminal phase (Vz/F).
- Apparent terminal elimination half-life ($t_{1/2}$).
- Accumulation ratio based on $AUC_{0\text{-}t}$ ($AR_{AUC}$).
- Linearity ratio (LR).

The secondary efficacy endpoints for this study are as follows:

- Overall response rate (ORR).
- Duration of response (DOR).
- Time to treatment failure (TTF).
- Progression-free survival (PFS).
- Overall survival (OS).

Note: Overall response rate, DOR, and PFS is assessed using both RECIST v 1.1 and iRECIST criteria.

**Exploratory Endpoints:**

- PD markers: Levels of ctDNA and CTC.
    - Molecular analyses using whole exome sequencing or RNA sequencing will be explored.
- Immunophenotyping in blood samples (serum and cellular markers, RNA, cytokines), including markers of stress and immune activation.
- Local antitumor effects (microscopic residual disease, apoptosis/necrosis) and microenvironment changes in tumor biopsy.
- The CYP3A induction potential of HC-7366, the ratio of 4β-hydroxycholesterol to total cholesterol on C1D1 and C1D21 as an endogenous marker of CYP3A induction.

**Statistical Methods:**

As this is an exploratory study to characterize the MTD, RP2D, safety/tolerability, a preliminary PD, formal hypothesis testing is not performed. Descriptive statistics of parameters of interest are presented by dose level. Safety data is presented in tabular format by system organ class and preferred term, severity, and frequency of events for each cohort dose level.

**Determination of Sample Size:**

The maximum sample size for the Phase 1a part of the study was 36 subjects. No more than 6 subjects were treated at each dose level. The sample size for Phase 1b is 30 subjects, with 15 subjects each in the 2 dose cohorts chosen for expansion.

**Pharmacokinetics:**

Noncompartmental PK analysis was performed on individual plasma concentration data using a validated software platform such as Phoenix WinNonlin. HC-7366 plasma concentrations and PK parameters will be listed and summarized using descriptive statistics. Dose proportionality may be assessed graphically in conjunction with the power model as appropriate.

**Pharmacodynamics:**

Pharmacodynamic biomarker data such as integrated stress response activation marker and immune-related changes was listed and summarized using descriptive statistics, as appropriate. Figures showing the relationship between PD, PK, and clinical efficacy data were generated as appropriate.

**General Considerations:**

Descriptive statistics and table/graphic representations were performed and presented by dose level (cohort). Statistics include, but are not limited to, counts, percentages, rates, means, medians, ranges, and variabilities, and any statistical analysis are exploratory.
The demographics and baseline characteristics of the subjects including age, sex, race, ethnicity, weight, baseline disease diagnosis, and medical conditions were summarized by dose level using descriptive statistics.

**Safety:**

Safety parameters were listed and summarized using descriptive statistics. Safety variables include incidence of TEAEs, laboratory data, vital signs, 12-lead ECG results, and physical examination findings. All safety analyses were based on the Safety Population.

**Efficacy:**

The efficacy parameters are ORR, DOR, TTF, PFS, and OS. ORR is defined as number of subjects with

confirmed responses of complete response (CR) or partial response (PR), divided by the total number of treated subjects with measurable disease at baseline. DOR is defined as the time from the first observation of a PR or CR to the time of radiologically documented progression. Tumor response status is defined according to RECIST v 1.1 and iRECIST. To perform an evaluation of antitumor activity, best overall response and ORR is tabulated by overall frequency distribution. Median DOR is summarized for those subjects with confirmed responses, using Kaplan-Meier methods; PFS is similarly summarized. Listings of individual tumor measurements, tumor burden, and % changes in tumor burden are provided. Changes in tumor burden are presented graphically by waterfall plots. The analysis of TTF, PFS, and OS is the same as for DOR.

**Interim:**

Dose-limiting toxicities are reviewed by the SMC when the planned number of subjects complete their DLT observation period using the dose-escalation rules in. The SMC reviews available clinical, PK, and/or PD data as appropriate.

Tumor biopsies collected from colorectal cancer and head and neck cancer study patients at the time of screening and at cycle 3 were analyzed for HIF1α expression by IHC. Profound inhibition of HIF1α expression in patients at the lowest starting doses of 10 mg and 20 mg was observed at cycle 3 **(FIGs. 59 and 60).**

Multiplex immunohistochemistry (IHC) and imaging of patient biopsies: Slides were deparaffinized, rehydrated and heat mediated antigen retrieval was conducted with AR9 buffer (Akoya). Slides were blocked with Roche diagnostics antibody diluent (Fisher) and stained with primary antibodies for 1 hour at 110 rpm. HRP-conjugated secondary antibodies were added for 10 mins at 110 rpm followed by 10 mins with OPAL detection dye (Akoya). For multiplex staining, this process was repeated starting at the antigen retrieval stage. Upon completion of multiplex staining rounds, slides were counterstained with DAPI and cover slipped using Vectashield vibrance mounting media (Vector H-1700). Multispectral Images were captured on the Polaris imaging system (Akoya) and spectral unmixing and cell segmentation was performed using Phenochart and Inform Tissue Finder software (Akoya). Imaging data was converted into .csv files and imported into Flowjo for immune phenotyping. Primary antibodies used were rabbit anti-human HIF1α (Polyclonal, Novus NB100-122, paired with Opal 690) and rabbit anti-human HIF2α (Polyclonal, Novus NB100-479, paired with Opal 570). Secondary antibodies used was HRP-goat anti-rabbit (Jackson Immunoresearch 111-035-144).

## Claims

1. A compound of formula (I)

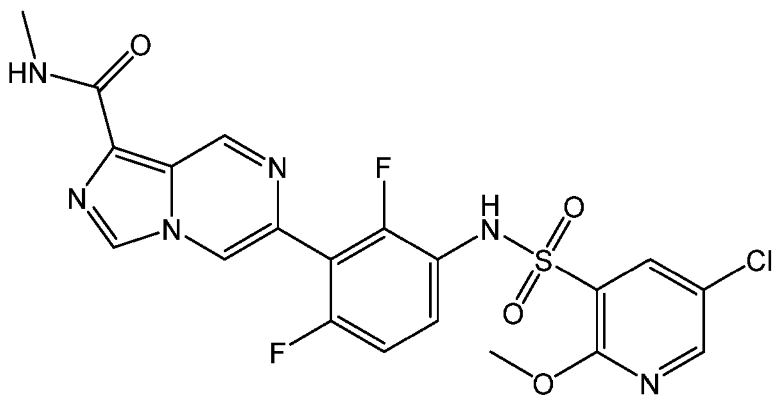

(I),

or a pharmaceutically acceptable salt thereof, for use in a method of treating an advanced solid tumor or a blood cancer in a subject in need thereof, comprising administering to the subject an effective amount of the compound or the pharmaceutically acceptable salt thereof.

2. The compound or pharmaceutically acceptable salt thereof for use according to claim 1, wherein administering the effective amount of the compound or pharmaceutically acceptable salt thereof activates the integrated stress response pathway (ISR) in the advanced solid tumor or blood cancer, optionally
wherein the ISR activation is GCN2 dependent.

3. The compound or pharmaceutically acceptable salt thereof for use according to claim 1 or claim 2, wherein

administering an effective amount of the compound or pharmaceutically acceptable salt thereof:

i. induces expression of ASNS, PSAT1, PHGDH, and/or PUMA in the advanced solid tumor or blood cancer;
ii. reduces protein levels of S100A8/A9, HIF1α, HIF2α, and/or GLUT1 in the advanced solid tumor or blood cancer;
iii. reduces mitochondrial respiration and/or glycolysis in the advanced solid tumor or blood cancer;
iv. decreases myeloid restricted precursor and mature myeloid cells in the subject;
v. alters metabolites involved in amino acid metabolism, oxidative stress, the urea cycle, and/or pyrimidine biosynthesis in the advanced solid tumor or blood cancer;
vi. reduces proteins involved in oxidative phosphorylation in the advanced solid tumor or blood cancer;
vii. reduces activity of HIF and/or E2F1-driven transcription in the advanced solid tumor or blood cancer; and/or
viii. increases ATF4 and/or JUN transcriptional activity in the advanced solid tumor or blood cancer.

**4.** The compound or pharmaceutically acceptable salt thereof for use according to any preceding claim, wherein 10 mg to 150 mg of the compound or pharmaceutically acceptable salt thereof, on a free acid equivalent weight basis, is administered to a subject, optionally
orally and/or once daily, optionally
once daily for 21 consecutive days, optionally
about 1 hour before a meal or about 2 hours after a meal.

**5.** The compound or pharmaceutically acceptable salt thereof for use according to any preceding claim, wherein the subject is in a fasting state.

**6.** The compound or pharmaceutically acceptable salt thereof for use according to any preceding claim, wherein the subject has previously been administered at least one and no more than five prior lines of therapy.

**7.** The compound or pharmaceutically acceptable salt thereof for use according to any preceding claim, wherein the pharmaceutically acceptable salt is a potassium salt, optionally
a hydrate or a monohydrate of the potassium salt.

**8.** The compound or pharmaceutically acceptable salt thereof for use according to any preceding claim, wherein the method further comprises administering an effective amount of a second therapeutic agent to the subject, optionally

wherein the second therapeutic agent is selected from the group consisting of an immune checkpoint inhibitor, an EGFR inhibitor, an antiangiogenic agent, venetoclax, fluorouracil, and combinations thereof, or
wherein the second therapeutic agent is selected from the group consisting of an anti-VEGFR antibody, fluorouracil, a PI3Kα inhibitor, a MEK1/2 inhibitor, and a hypoxia-inducible factor (HIF) inhibitor, optionally an anti-VEGFR antibody, a HIF inhibitor, a PI3Kα inhibitor, a MEK1/2 inhibitor or an EGFR inhibitor.

**9.** The compound or pharmaceutically acceptable salt thereof for use according to claim 8, wherein the second therapeutic agent is belzutifan, 5-fluorouracil, alpelisib, trametinib, osimertinib, dacomitinib or venetoclax.

**10.** The compound or pharmaceutically acceptable salt thereof for use according to claim 9, wherein the second therapeutic agent is venetoclax and optionally
wherein administering the effective amount of the compound or pharmaceutically acceptable salt thereof, and the venetoclax activates the integrated stress response pathway (ISR) in the advanced solid tumor or blood cancer to a greater extent than the compound or pharmaceutically acceptable salt thereof, or venetoclax administered alone.

**11.** The compound or pharmaceutically acceptable salt thereof for use according to any preceding claim, wherein the subject is a human, optionally
an adult human.

**12.** The compound or pharmaceutically acceptable salt thereof for use according to any preceding claim, wherein the advanced solid tumor is selected from the group consisting of squamous cell carcinoma of the head and neck, colorectal cancer, non-small cell lung cancer, renal cell carcinoma, and transitional cell carcinoma of the bladder, and optionally
wherein the advanced solid tumor is selected from the group consisting of sarcoma, colorectal cancer, head and neck cancer, and prostate cancer.

13. The compound or pharmaceutically acceptable salt thereof for use according to any preceding claim, wherein the blood cancer is a leukemia, optionally
acute myeloid leukemia, and optionally
wherein the blood cancer is resistant to B-cell lymphoma inhibitors.

14. The compound or pharmaceutically acceptable salt thereof for use according to any one of claims 1 to 11 or 13, wherein the blood cancer is resistant to venetoclax.

## Patentansprüche

1. Verbindung der Formel (I)

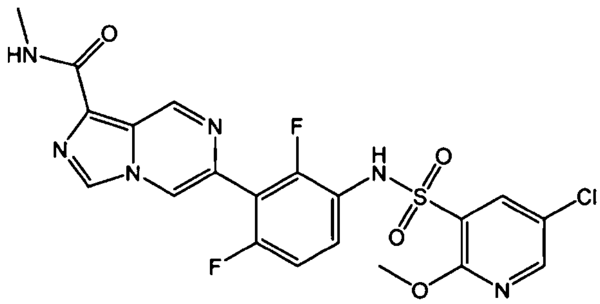

(I)

oder pharmazeutisch unbedenkliches Salz davon zur Verwendung bei einem Verfahren zur Behandlung eines fortgeschrittenen soliden Tumors oder einer Blutkrebserkrankung bei einem behandlungsbedürftigen Individuum, umfassend Verabreichen einer wirksamen Menge der Verbindung oder des pharmazeutisch unbedenklichen Salzes davon an das Individuum.

2. Verbindung oder pharmazeutisch unbedenkliches Salz davon zur Verwendung nach Anspruch 1, wobei Verabreichen der wirksamen Menge der Verbindung oder des pharmazeutisch unbedenklichen Salzes davon den ISR(Integrated Stress Response)-Weg bei dem fortgeschrittenen soliden Tumor oder der Blutkrebserkrankung aktiviert, gegebenenfalls wobei die ISR-Aktivierung GCN2-abhängig ist.

3. Verbindung oder pharmazeutisch unbedenkliches Salz davon zur Verwendung nach Anspruch 1 oder Anspruch 2, wobei Verabreichen einer wirksamen Menge der Verbindung bzw. des pharmazeutisch unbedenklichen Salzes davon:

i. die Expression von ASNS, PSAT1, PHGDH und/oder PUMA bei dem fortgeschrittenen soliden Tumor oder der Blutkrebserkrankung induziert;
ii. die Proteinspiegel von S100A8/A9, HIF1$\alpha$, HIF2a und/oder GLUT1 bei dem fortgeschrittenen soliden Tumor bzw. der Blutkrebserkrankung senkt;
iii. die mitochondriale Atmung und/oder Glykolyse bei dem fortgeschrittenen soliden Tumor bzw. der Blutkrebserkrankung verringert;
iv. eingeschränkte myeloische Vorläufer- und reife myeloische Zellen bei dem Individuum vermindert;
v. Metaboliten, die an Aminosäuremetabolismus, oxidativem Stress, dem Harnstoffzyklus und/oder Pyrimidin-Biosynthese bei dem fortgeschrittenen soliden Tumor bzw. der Blutkrebserkrankung beteiligt sind, verändert;
vi. Proteine, die an oxidativer Phosphorylierung bei dem fortgeschrittenen soliden Tumor bzw. der Blutkrebserkrankung beteiligt sind, reduziert;
vii. die Aktivität von HIF und/oder E2F1-gesteuerter Transkription bei dem fortgeschrittenen soliden Tumor bzw. der Blutkrebserkrankung verringert; und/oder
viii. die ATF4- und/oder JUN-Transkriptionsaktivität bei dem fortgeschrittenen soliden Tumor bzw. der Blutkrebserkrankung erhöht.

4. Verbindung oder pharmazeutisch unbedenkliches Salz davon zur Verwendung nach einem vorhergehenden Anspruch, wobei 10 mg bis 150 mg der Verbindung bzw. des pharmazeutisch unbedenklichen Salzes davon, auf einer Freie-Säure-Äquivalent-Gewichtsbasis, einem Individuum gegebenenfalls

oral und/oder einmal täglich, gegebenenfalls
einmal täglich, 21 aufeinanderfolgende Tage, gegebenenfalls
etwa 1 Stunde vor einer Mahlzeit oder etwa 2 Stunden nach einer Mahlzeit, verabreicht werden.

**5.** Verbindung oder pharmazeutisch unbedenkliches Salz davon zur Verwendung nach einem vorhergehenden Anspruch, wobei sich das Individuum in einem nüchternen Zustand befindet.

**6.** Verbindung oder pharmazeutisch unbedenkliches Salz davon zur Verwendung nach einem vorhergehenden Anspruch, wobei dem Individuum bereits mindestens eine und nicht mehr als fünf frühere Therapielinien verabreicht wurden.

**7.** Verbindung oder pharmazeutisch unbedenkliches Salz davon zur Verwendung nach einem vorhergehenden Anspruch, wobei es sich bei dem pharmazeutisch unbedenklichen Salz um ein Kaliumsalz, gegebenenfalls
ein Hydrat oder ein Monohydrat des Kaliumsalzes, handelt.

**8.** Verbindung oder pharmazeutisch unbedenkliches Salz davon zur Verwendung nach einem vorhergehenden Anspruch, wobei das Verfahren ferner Verabreichen einer wirksamen Menge eines zweiten Therapeutikums an das Individuum umfasst, gegebenenfalls

wobei das zweite Therapeutikum aus der Gruppe bestehend aus einem Immuncheckpoint-Inhibitor, einem EGFR-Inhibitor, einem Angiogenesehemmer,
Venetoclax, Fluoruracil und Kombinationen davon ausgewählt ist oder
wobei das zweite Therapeutikum aus der Gruppe bestehend aus einem Anti-VEGFR-Antikörper, Fluoruracil, einem PI3K$\alpha$-Inhibitor, einem MEK1/2-Inhibitor und einem Inhibitor des hypoxie-induzierbaren Faktors (HIF), gegebenenfalls einem Anti-VEGFR-Antikörper, einem HIF-Inhibitor, einem PI3K$\alpha$-Inhibitor, einem MEK1/2-Inhibitor oder einem EGFR-Inhibitor ausgewählt ist.

**9.** Verbindung oder pharmazeutisch unbedenkliches Salz davon zur Verwendung nach Anspruch 8, wobei es sich bei dem zweiten Therapeutikum um Belzutifan, 5-Fluoruracil, Alpelisib, Trametinib, Osimertinib, Dacomitinib oder Venetoclax handelt.

**10.** Verbindung oder pharmazeutisch unbedenkliches Salz davon zur Verwendung nach Anspruch 9, wobei es sich bei dem zweiten Therapeutikum um Venetoclax handelt und gegebenenfalls
wobei Verabreichen der wirksamen Menge der Verbindung bzw. des pharmazeutisch unbedenklichen Salzes davon und des Venetoclax den ISR(Integrated Stress Response)-Weg bei dem fortgeschrittenen soliden Tumor oder der Blutkrebserkrankung in einem größeren Ausmaß aktiviert als die/das allein verabreichte Verbindung bzw. pharmazeutisch unbedenkliche Salz davon bzw. Venetoclax.

**11.** Verbindung oder pharmazeutisch unbedenkliches Salz davon zur Verwendung nach einem vorhergehenden Anspruch, wobei es sich bei dem Individuum um einen Menschen handelt, gegebenenfalls
einen erwachsenen Menschen.

**12.** Verbindung oder pharmazeutisch unbedenkliches Salz davon zur Verwendung nach einem vorhergehenden Anspruch, wobei der fortgeschrittene solide Tumor aus der Gruppe bestehend aus Plattenepithelkarzinom des Kopfes und Halses, Kolorektalkarzinom, nichtkleinzelligem Lungenkrebs, Nierenzellkarzinom und Übergangszellkarzinom der Blase ausgewählt ist und gegebenenfalls
wobei der fortgeschrittene solide Tumor aus der Gruppe bestehend aus Sarkom, Kolorektalkarzinom, Krebs an Kopf und Hals und Prostatakrebs ausgewählt ist.

**13.** Verbindung oder pharmazeutisch unbedenkliches Salz davon zur Verwendung nach einem vorhergehenden Anspruch, wobei es sich bei der Blutkrebserkrankung um eine Leukämie handelt, gegebenenfalls
akute myeloische Leukämie handelt und gegebenenfalls wobei die Blutkrebserkrankung resistent gegen B-Zell-Lymphom-Inhibitoren ist.

**14.** Verbindung oder pharmazeutisch unbedenkliches Salz davon zur Verwendung nach einem der Ansprüche 1 bis 11 oder 13, wobei die Blutkrebserkrankung resistent gegen Venetoclax ist.

## Revendications

1. Composé de formule (I)

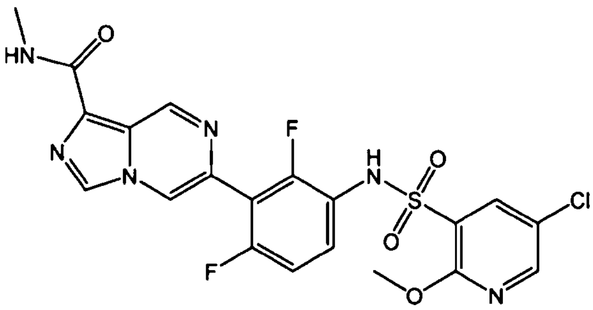

(I),

ou sel pharmaceutiquement acceptable de celui-ci, pour utilisation dans un procédé de traitement d'une tumeur solide avancée ou un cancer du sang chez un sujet qui en a besoin, comprenant l'administration au sujet d'une quantité efficace du composé ou du sel pharmaceutiquement acceptable de celui-ci.

2. Composé ou sel pharmaceutiquement acceptable de celui-ci pour utilisation selon la revendication 1, dans lequel l'administration de la quantité efficace du composé ou du sel pharmaceutiquement acceptable de celui-ci active la voie intégrée de réponse au stress (ISR) dans la tumeur solide avancée ou le cancer du sang, éventuellement dans lequel l'activation d'ISR est dépendante de GCN2.

3. Composé ou sel pharmaceutiquement acceptable de celui-ci pour utilisation selon la revendication 1 ou la revendication 2, dans lequel l'administration d'une quantité efficace du composé ou d'un sel pharmaceutiquement acceptable de celui-ci :

   i. induit l'expression d'ASNS, PSAT1, PHGDH, et/ou PUMA dans la tumeur solide avancée ou le cancer du sang ;
   ii. réduit les taux de protéine de S100A8/A9, HIF1$\alpha$, HIF2a, et/ou GLUT1 dans la tumeur solide avancée ou le cancer du sang ;
   iii. réduit la respiration mitochondriale et/ou la glycolyse dans la tumeur solide avancée ou le cancer du sang ;
   iv. diminue le précurseur myéloïde restreint et les cellules myéloïdes matures chez le sujet ;
   v. altère des métabolites impliqués dans le métabolisme des acides aminés, le stress oxydatif, le cycle de l'urée, et/ou la biosynthèse de la pyrimidine dans la tumeur solide avancée ou le cancer du sang ;
   vi. réduit des protéines impliquées dans la phosphorylation oxydative dans la tumeur solide avancée ou le cancer du sang ;
   vii. réduit l'activité de la transcription conduite par HIF et/ou E2F1 dans la tumeur solide avancée ou le cancer du sang ; et/ou
   viii. augmente l'activité transcriptionnelle de ATF4 et/ou JUN dans la tumeur solide avancée ou le cancer du sang.

4. Composé ou sel pharmaceutiquement acceptable de celui-ci pour utilisation selon l'une quelconque des revendications précédentes, dans lequel 10 mg à 150 mg du composé ou d'un sel pharmaceutiquement acceptable de celui-ci, sur la base du poids d'équivalent d'acide libre, sont administrés à un sujet, éventuellement

   par voie orale et/ou une fois par jour, éventuellement
   une fois par jour pendant 21 jours consécutifs, éventuellement
   environ 1 heure avant un repas ou environ 2 heures après un repas.

5. Composé ou sel pharmaceutiquement acceptable de celui-ci pour utilisation selon l'une quelconque des revendications précédentes, dans lequel le sujet est à jeun.

6. Composé ou sel pharmaceutiquement acceptable de celui-ci pour utilisation selon l'une quelconque des revendications précédentes, dans lequel le sujet a précédemment reçu au moins une et pas plus de cinq lignes de thérapie précédentes.

7. Composé ou sel pharmaceutiquement acceptable de celui-ci pour utilisation selon l'une quelconque des revendications précédentes, dans lequel le sel pharmaceutiquement acceptable est un sel de potassium, éventuellement un hydrate ou un monohydrate du sel de potassium.

**8.** Composé ou sel pharmaceutiquement acceptable de celui-ci pour utilisation selon l'une quelconque des revendications précédentes, dans lequel le procédé comprend en outre l'administration d'une quantité efficace d'un deuxième agent thérapeutique au sujet, éventuellement

dans lequel le deuxième agent thérapeutique est choisi dans le groupe constitué par un inhibiteur de point de contrôle immunitaire, un inhibiteur d'EGFR, un agent antiangiogenèse,
le vénétoclax, le fluorouracile, et leurs combinaisons, ou
dans lequel le deuxième agent thérapeutique est choisi dans le groupe constitué par un anticorps anti-VEGFR, le fluorouracile, un inhibiteur de PI3Kα, un inhibiteur de MEK1/2, et un inhibiteur de facteur hypoxiviable (HIF), éventuellement un anticorps anti-VEGFR, un inhibiteur de HIF, un inhibiteur de PI3Kα, un inhibiteur de MEK1/2 ou un inhibiteur d'EGFR.

**9.** Composé ou sel pharmaceutiquement acceptable de celui-ci pour utilisation selon la revendication 8, dans lequel le deuxième agent thérapeutique est le belzutifan, le 5-fluorouracile, l'alpélisib, le tramétinib, l'osimertinib, le dacomitinib ou le vénétoclax.

**10.** Composé ou sel pharmaceutiquement acceptable de celui-ci pour utilisation selon la revendication 9, dans lequel le deuxième agent thérapeutique est le vénétoclax et éventuellement
dans lequel l'administration de la quantité efficace du composé ou d'un sel pharmaceutiquement acceptable de celui-ci, et du vénétoclax active la voie intégrée de réponse au stress (ISR) dans la tumeur solide avancée ou le cancer du sang dans une plus grande mesure que le composé ou un sel pharmaceutiquement acceptable de celui-ci, ou le vénétoclax administré seul.

**11.** Composé ou sel pharmaceutiquement acceptable de celui-ci pour utilisation selon l'une quelconque des revendications précédentes, dans lequel le sujet est un humain, éventuellement
un humain adulte.

**12.** Composé ou sel pharmaceutiquement acceptable de celui-ci pour utilisation selon l'une quelconque des revendications précédentes, dans lequel la tumeur solide avancée est choisie dans le groupe constitué par le carcinome à cellules squameuses de la tête et du cou, le cancer colorectal, le cancer du poumon non à petites cellules, le carcinome de cellules rénales, et le carcinome de cellules transitionnelles de la vessie, et éventuellement
dans lequel la tumeur solide avancée est choisie dans le groupe constitué par le sarcome, le cancer colorectal, le cancer de la tête et du cou, et le cancer de la prostate.

**13.** Composé ou sel pharmaceutiquement acceptable de celui-ci, pour utilisation selon l'une quelconque des revendications précédentes, dans lequel le cancer du sang est une leucémie, éventuellement

une leucémie myéloïde aiguë, et éventuellement
dans lequel le cancer du sang est résistant à des inhibiteurs de lymphome de cellules B.

**14.** Composé ou sel pharmaceutiquement acceptable de celui-ci pour utilisation selon l'une quelconque des revendications 1 à 11 ou 13, dans lequel le cancer du sang est résistant au vénétoclax.

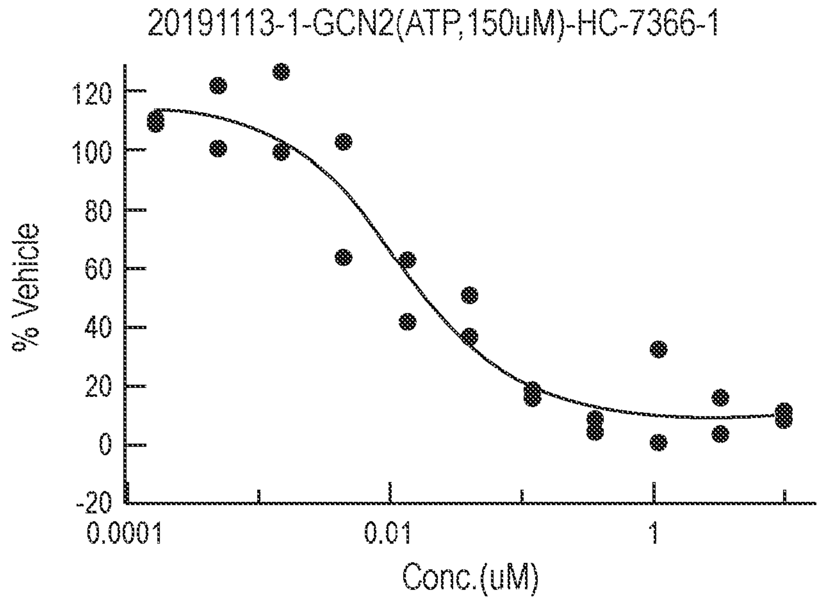
20191113-1-GCN2(ATP,150uM)-HC-7366-1
120
100
80
60
40
20
0
-20
% Vehicle
0.0001
0.01
1
Conc.(uM)

FIG. 1

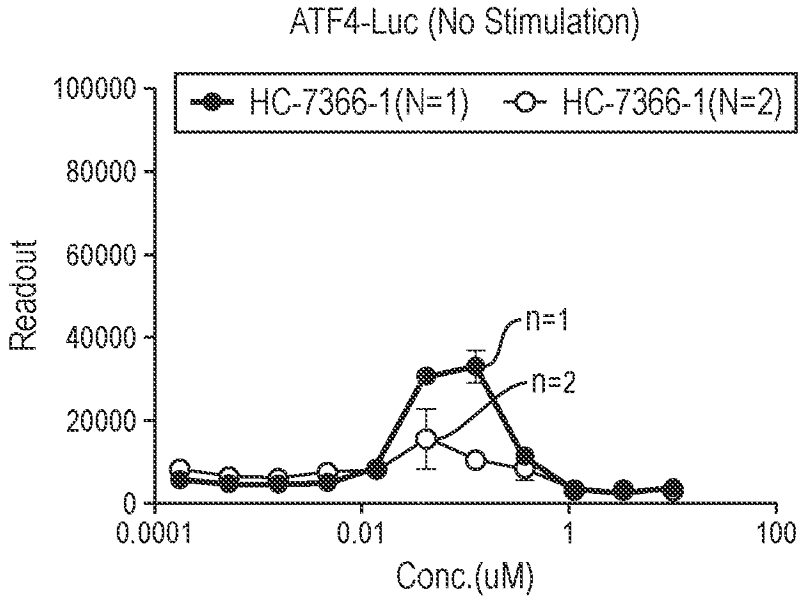
ATF4-Luc (No Stimulation)
HC-7366-1(N=1)
HC-7366-1(N=2)
Readout
100000
80000
60000
40000
20000
0
n=1
n=2
0.0001
0.01
1
100
Conc.(uM)

FIG. 2

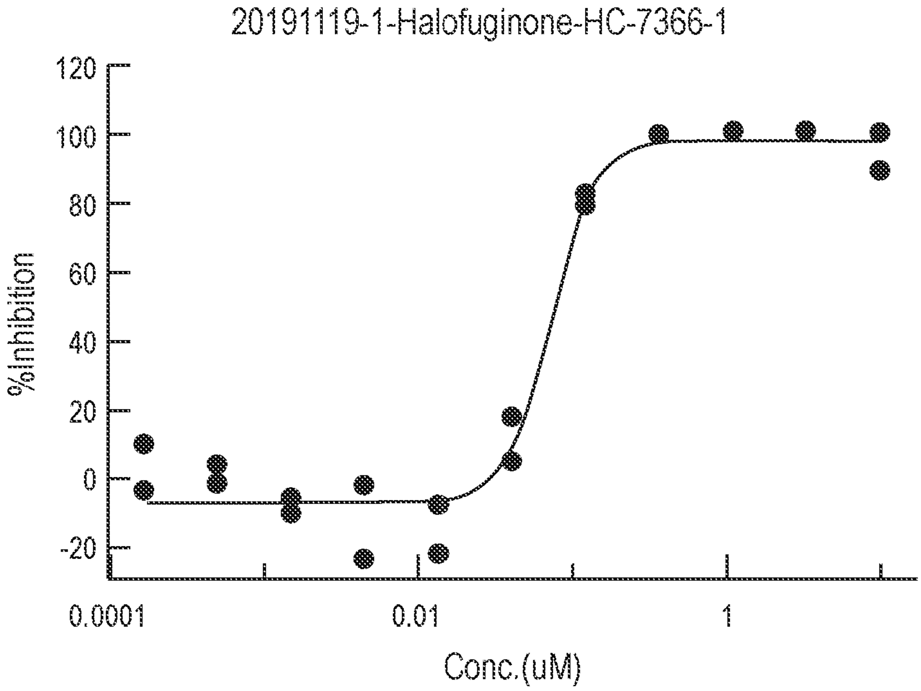
20191119-1-Halofuginone-HC-7366-1
120
100
80
60
40
20
0
-20
%Inhibition
0.0001
0.01
1
Conc.(uM)

FIG. 3

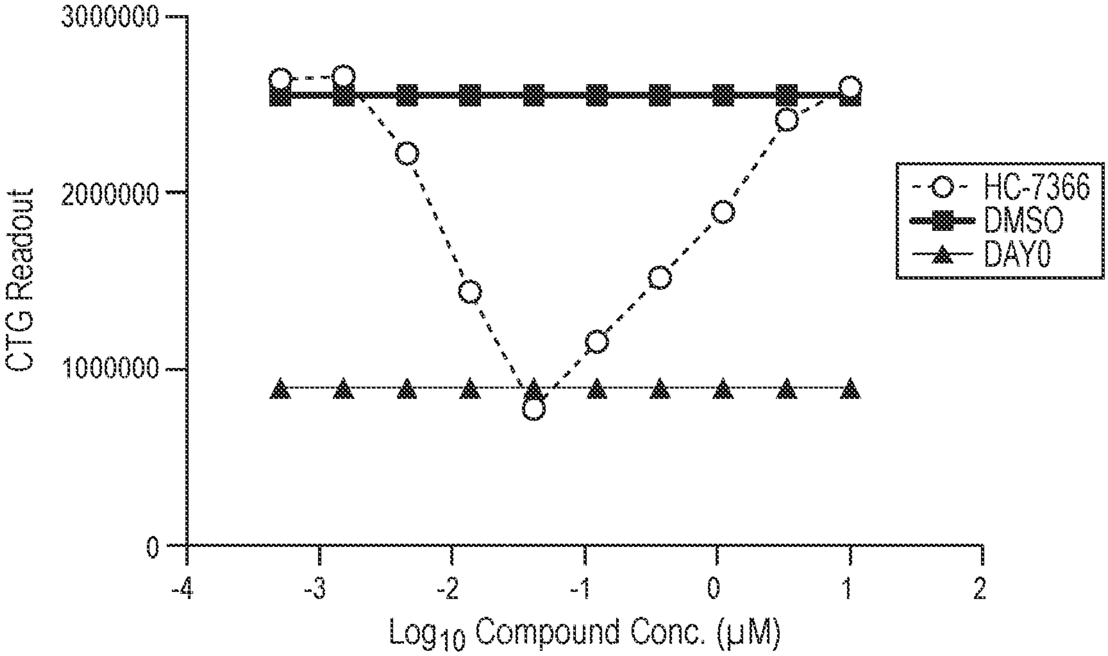
3000000
2000000
1000000
0
CTG Readout
-4
-3
-2
-1
0
1
2
Log10 Compound Conc. (μM)
HC-7366
DMSO
DAY0

FIG. 4

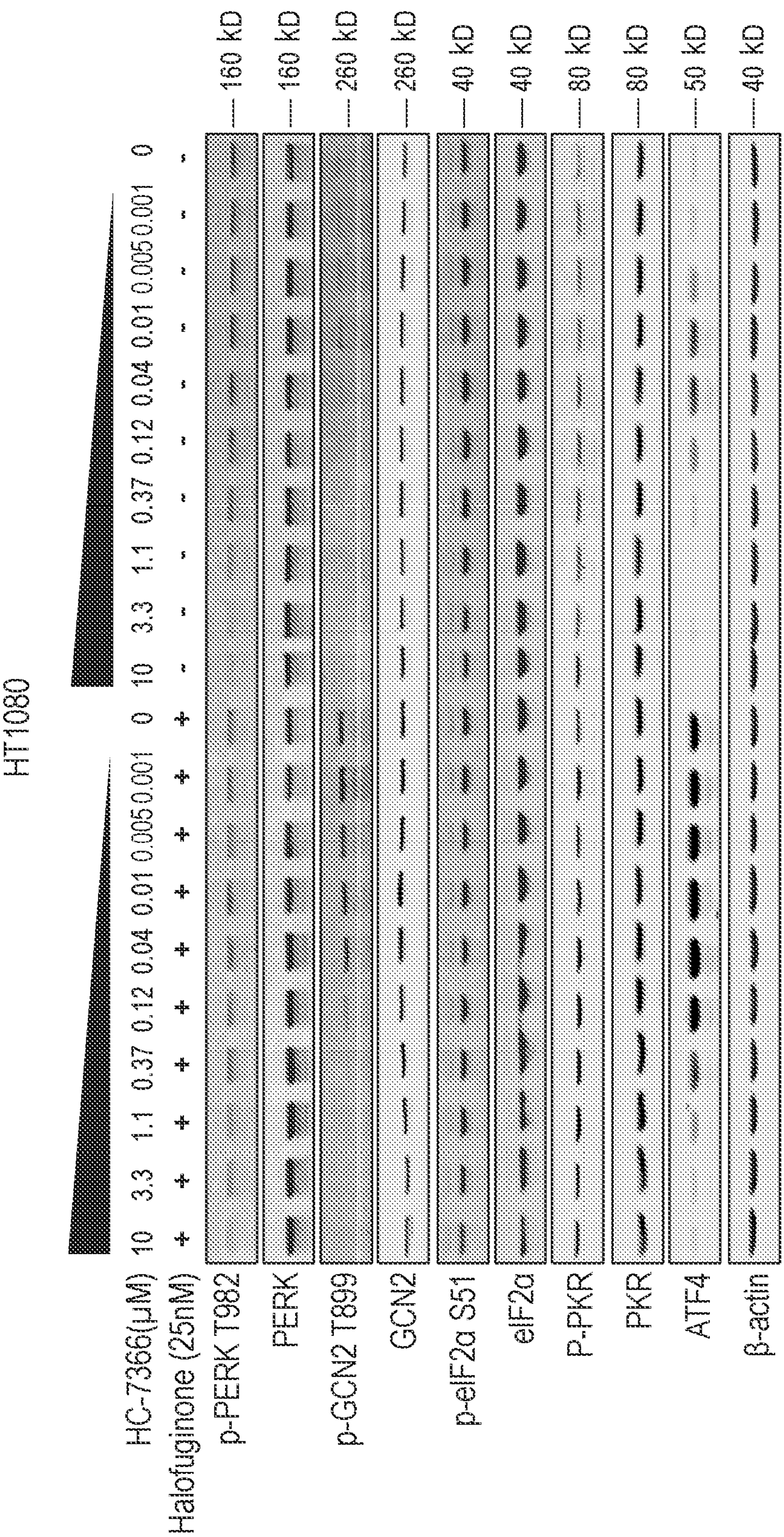
HT1080
HC-7366(μM) 10 3.3 1.1 0.37 0.12 0.04 0.01 0.005 0.001 0 10 3.3 1.1 0.37 0.12 0.04 0.01 0.005 0.001 0
Halofuginone (25nM) + + + + + + + + + + - - - - - - - - - -
p-PERK T982
PERK
p-GCN2 T899
GCN2
p-eIF2α S51
eIF2α
P-PKR
PKR
ATF4
β-actin
160 kD
160 kD
260 kD
260 kD
40 kD
40 kD
80 kD
80 kD
50 kD
40 kD

FIG. 5

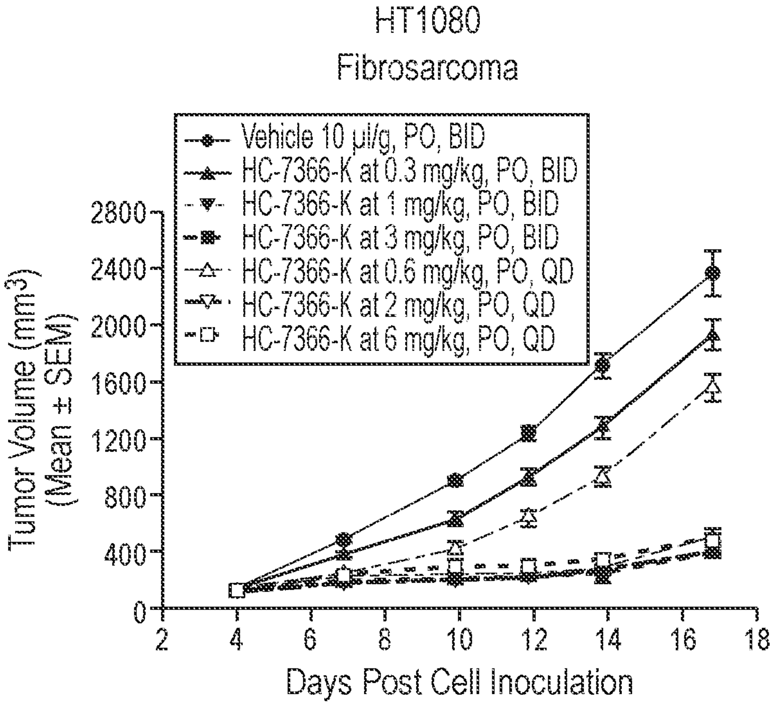
HT1080
Fibrosarcoma
Vehicle 10 μl/g, PO, BID
HC-7366-K at 0.3 mg/kg, PO, BID
HC-7366-K at 1 mg/kg, PO, BID
HC-7366-K at 3 mg/kg, PO, BID
HC-7366-K at 0.6 mg/kg, PO, QD
HC-7366-K at 2 mg/kg, PO, QD
HC-7366-K at 6 mg/kg, PO, QD
Tumor Volume (mm3)
(Mean ± SEM)
0
400
800
1200
1600
2000
2400
2800
2
4
6
8
10
12
14
16
18
Days Post Cell Inoculation

FIG. 6

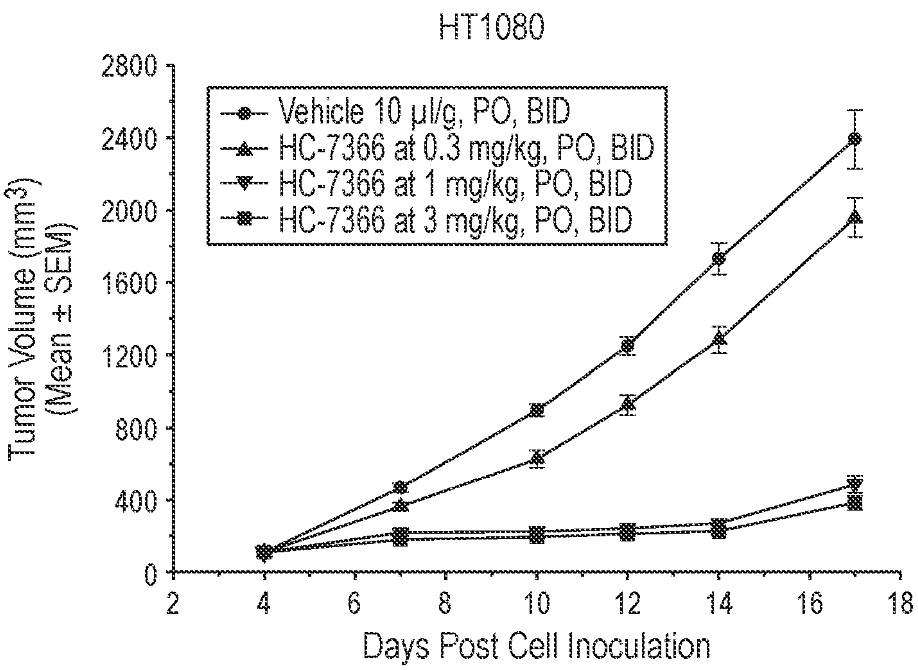
HT1080
Vehicle 10 μl/g, PO, BID
HC-7366 at 0.3 mg/kg, PO, BID
HC-7366 at 1 mg/kg, PO, BID
HC-7366 at 3 mg/kg, PO, BID
Tumor Volume (mm3)
(Mean ± SEM)
0
400
800
1200
1600
2000
2400
2800
2
4
6
8
10
12
14
16
18
Days Post Cell Inoculation

FIG. 7

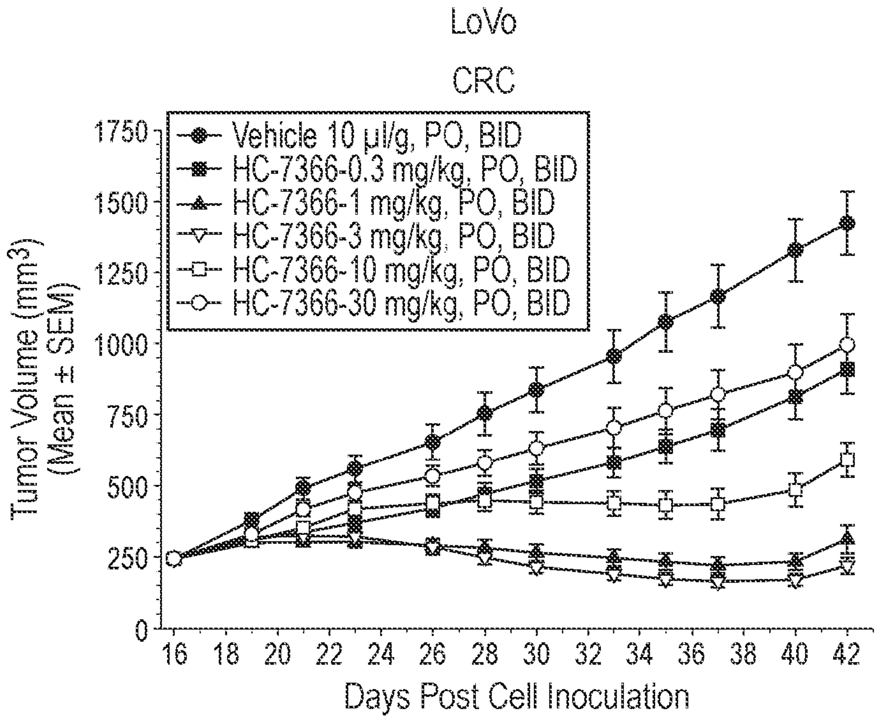
LoVo
CRC
Vehicle 10 µl/g, PO, BID
HC-7366-0.3 mg/kg, PO, BID
HC-7366-1 mg/kg, PO, BID
HC-7366-3 mg/kg, PO, BID
HC-7366-10 mg/kg, PO, BID
HC-7366-30 mg/kg, PO, BID
Tumor Volume (mm3) (Mean ± SEM)
0
250
500
750
1000
1250
1500
1750
16 18 20 22 24 26 28 30 32 34 36 38 40 42
Days Post Cell Inoculation

FIG. 8

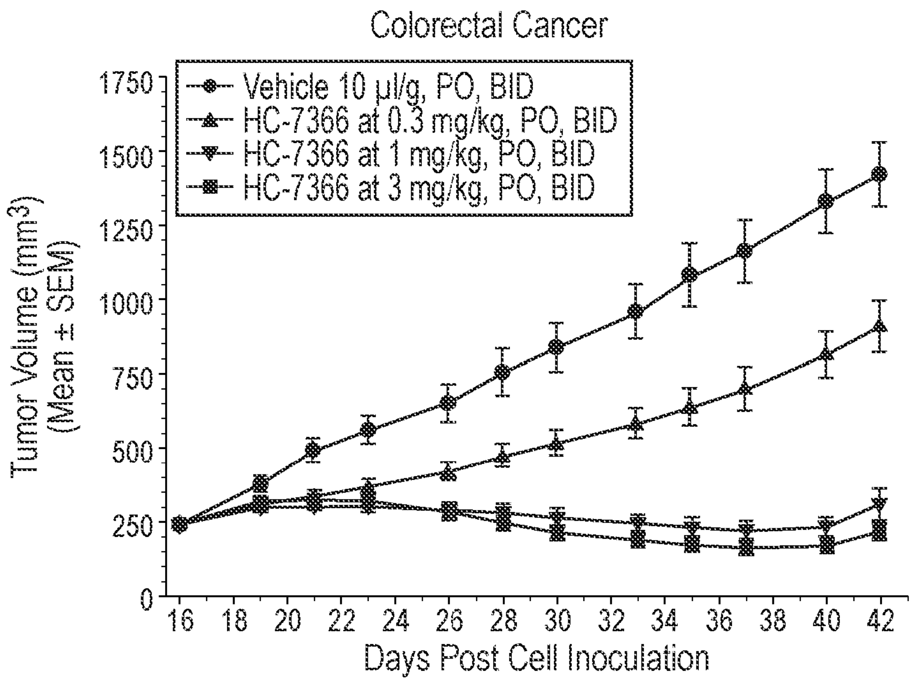
Colorectal Cancer
Vehicle 10 µl/g, PO, BID
HC-7366 at 0.3 mg/kg, PO, BID
HC-7366 at 1 mg/kg, PO, BID
HC-7366 at 3 mg/kg, PO, BID
Tumor Volume (mm3) (Mean ± SEM)
0
250
500
750
1000
1250
1500
1750
16 18 20 22 24 26 28 30 32 34 36 38 40 42
Days Post Cell Inoculation

FIG. 9

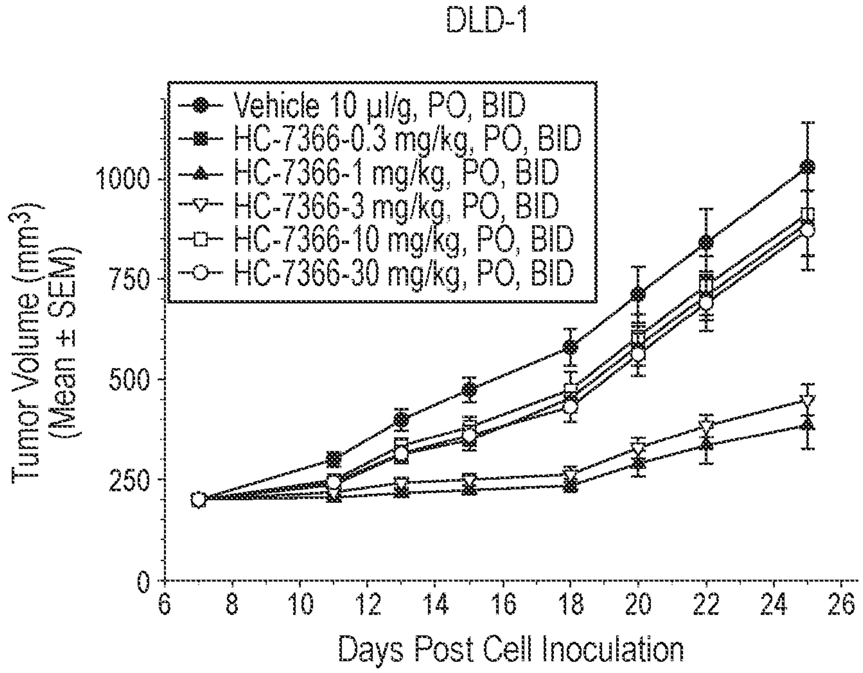
DLD-1
Vehicle 10 µl/g, PO, BID
HC-7366-0.3 mg/kg, PO, BID
HC-7366-1 mg/kg, PO, BID
HC-7366-3 mg/kg, PO, BID
HC-7366-10 mg/kg, PO, BID
HC-7366-30 mg/kg, PO, BID
Tumor Volume (mm3)
(Mean ± SEM)
0
250
500
750
1000
6
8
10
12
14
16
18
20
22
24
26
Days Post Cell Inoculation

FIG. 10

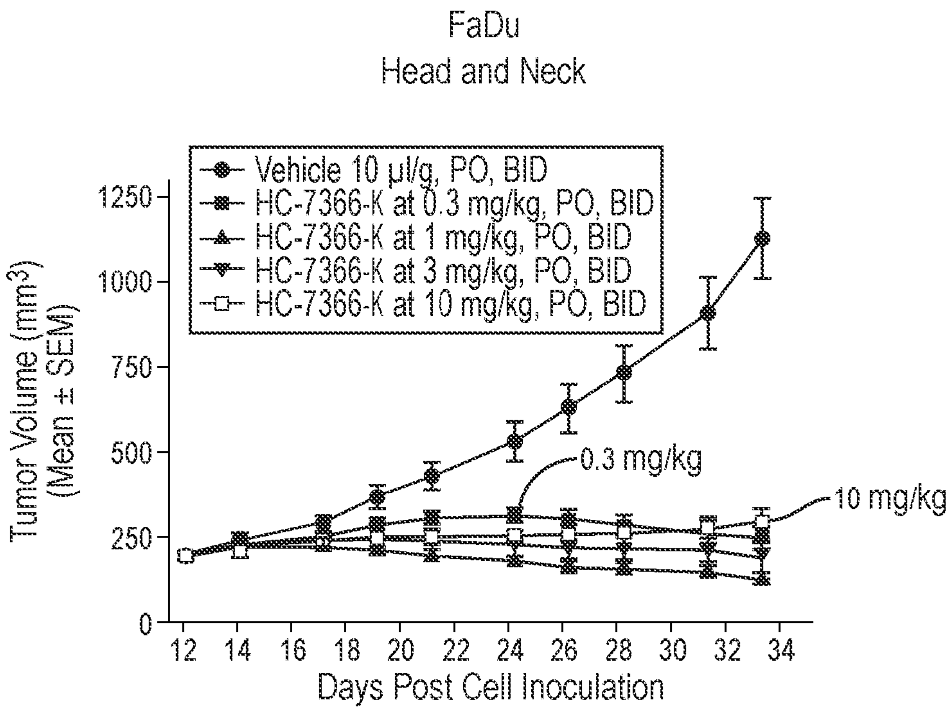
FaDu
Head and Neck
Vehicle 10 µl/g, PO, BID
HC-7366-K at 0.3 mg/kg, PO, BID
HC-7366-K at 1 mg/kg, PO, BID
HC-7366-K at 3 mg/kg, PO, BID
HC-7366-K at 10 mg/kg, PO, BID
0.3 mg/kg
10 mg/kg
Tumor Volume (mm3)
(Mean ± SEM)
0
250
500
750
1000
1250
12
14
16
18
20
22
24
26
28
30
32
34
Days Post Cell Inoculation

FIG. 11

Head and Neck

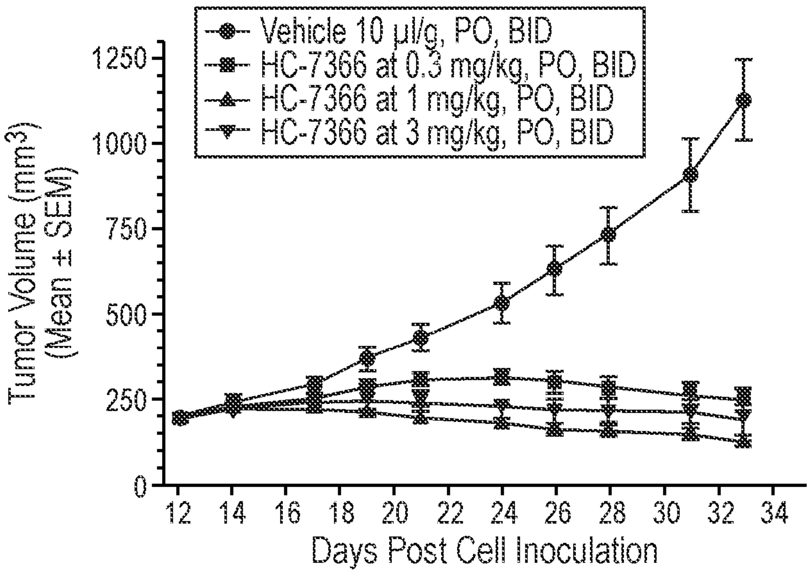


FIG. 12

Head and Neck
FaDu

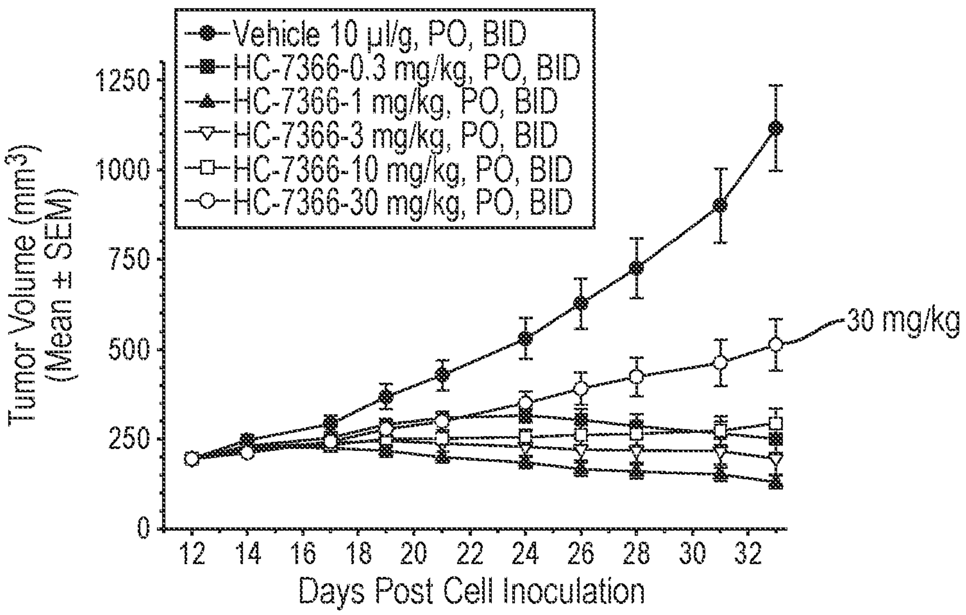


FIG. 13

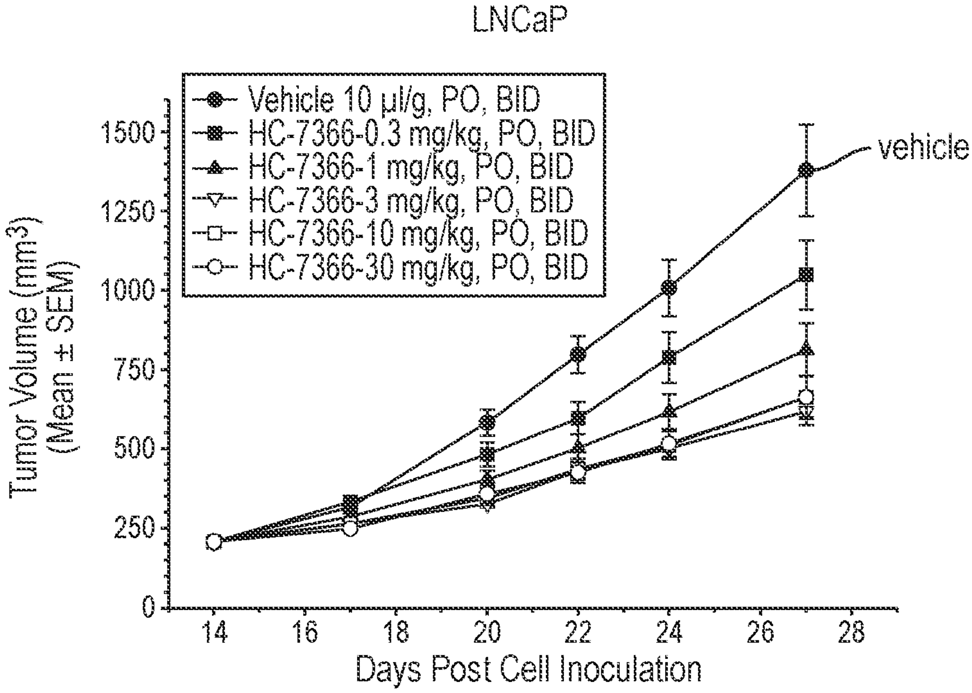
LNCaP
Vehicle 10 µl/g, PO, BID
HC-7366-0.3 mg/kg, PO, BID
HC-7366-1 mg/kg, PO, BID
HC-7366-3 mg/kg, PO, BID
HC-7366-10 mg/kg, PO, BID
HC-7366-30 mg/kg, PO, BID
vehicle
Tumor Volume (mm3) (Mean ± SEM)
0
250
500
750
1000
1250
1500
14
16
18
20
22
24
26
28
Days Post Cell Inoculation

FIG. 14

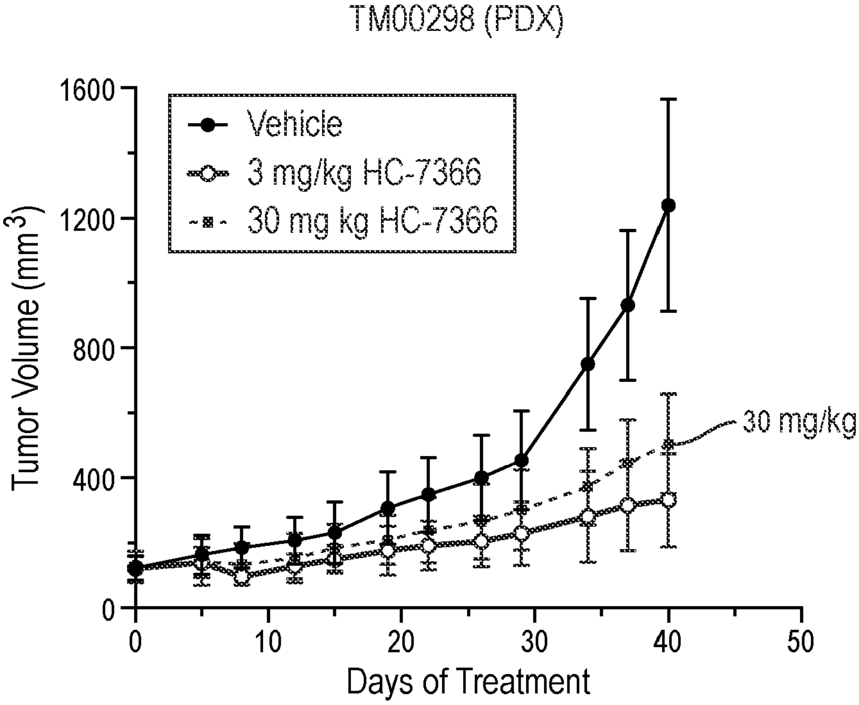
TM00298 (PDX)
Vehicle
3 mg/kg HC-7366
30 mg kg HC-7366
30 mg/kg
Tumor Volume (mm3)
0
400
800
1200
1600
0
10
20
30
40
50
Days of Treatment

FIG. 15

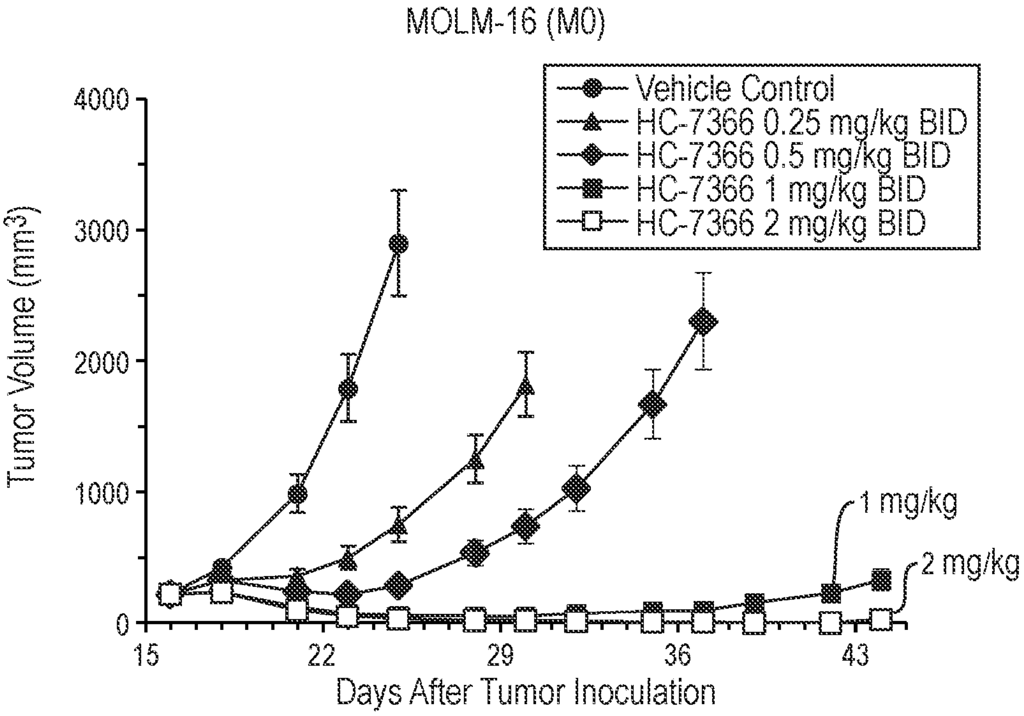
MOLM-16 (M0)
Vehicle Control
HC-7366 0.25 mg/kg BID
HC-7366 0.5 mg/kg BID
HC-7366 1 mg/kg BID
HC-7366 2 mg/kg BID
Tumor Volume (mm3)
4000
3000
2000
1000
0
15
22
29
36
43
Days After Tumor Inoculation
1 mg/kg
2 mg/kg

FIG. 16

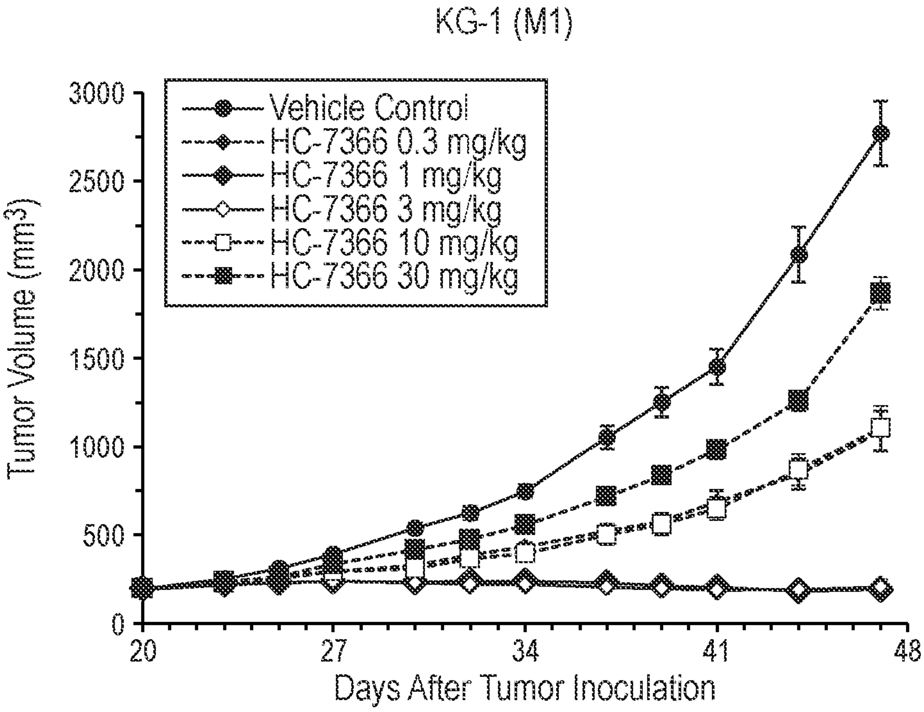
KG-1 (M1)
Vehicle Control
HC-7366 0.3 mg/kg
HC-7366 1 mg/kg
HC-7366 3 mg/kg
HC-7366 10 mg/kg
HC-7366 30 mg/kg
Tumor Volume (mm3)
3000
2500
2000
1500
1000
500
0
20
27
34
41
48
Days After Tumor Inoculation

FIG. 17

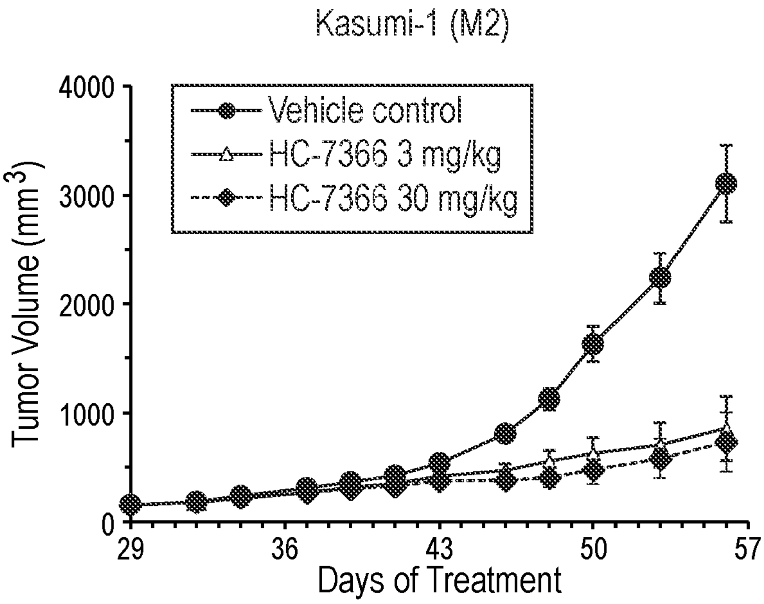
Kasumi-1 (M2)
Vehicle control
HC-7366 3 mg/kg
HC-7366 30 mg/kg
4000
3000
2000
1000
0
Tumor Volume (mm3)
29
36
43
50
57
Days of Treatment

FIG. 18

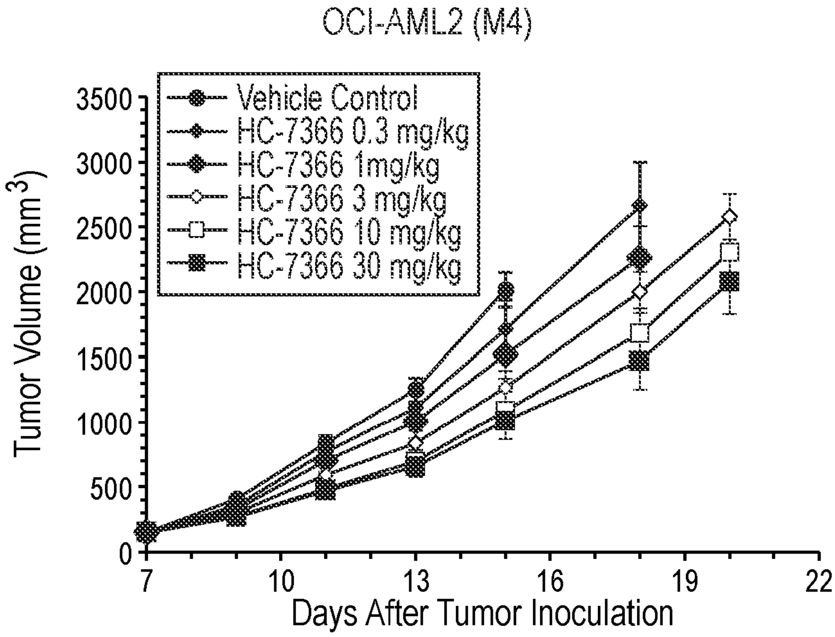
OCI-AML2 (M4)
Vehicle Control
HC-7366 0.3 mg/kg
HC-7366 1mg/kg
HC-7366 3 mg/kg
HC-7366 10 mg/kg
HC-7366 30 mg/kg
3500
3000
2500
2000
1500
1000
500
0
Tumor Volume (mm3)
7
10
13
16
19
22
Days After Tumor Inoculation

FIG. 19

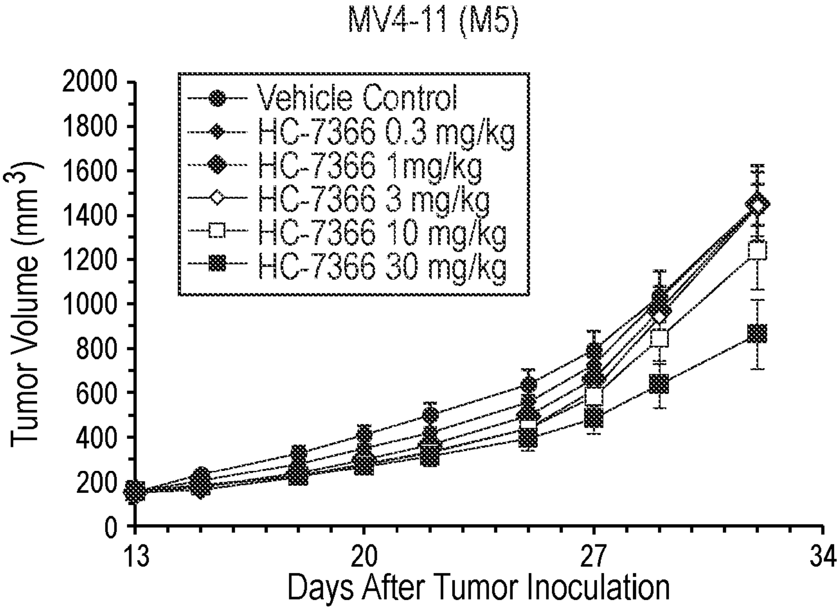
MV4-11 (M5)
Vehicle Control
HC-7366 0.3 mg/kg
HC-7366 1mg/kg
HC-7366 3 mg/kg
HC-7366 10 mg/kg
HC-7366 30 mg/kg
Tumor Volume (mm3)
0
200
400
600
800
1000
1200
1400
1600
1800
2000
13
20
27
34
Days After Tumor Inoculation

FIG. 20

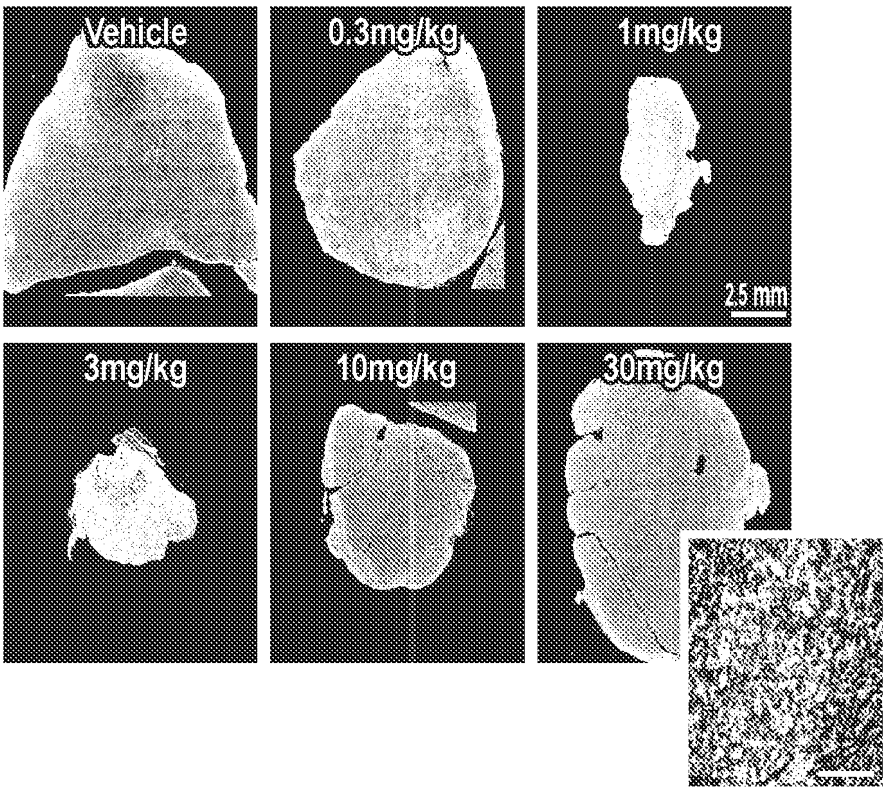
Vehicle
0.3mg/kg
1mg/kg
2.5 mm
3mg/kg
10mg/kg
30mg/kg

FIG. 21

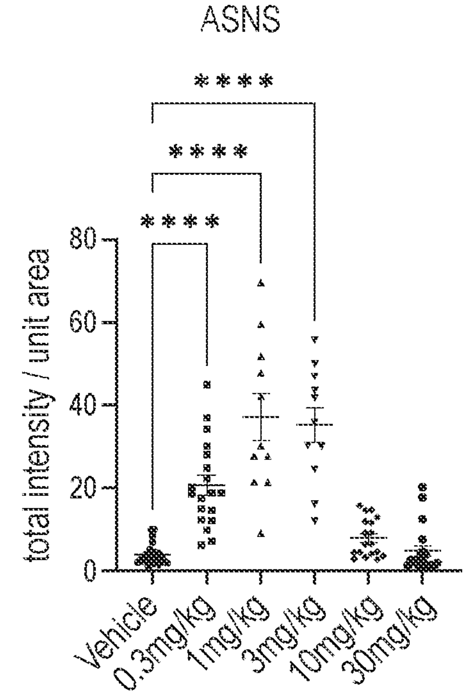


FIG. 22

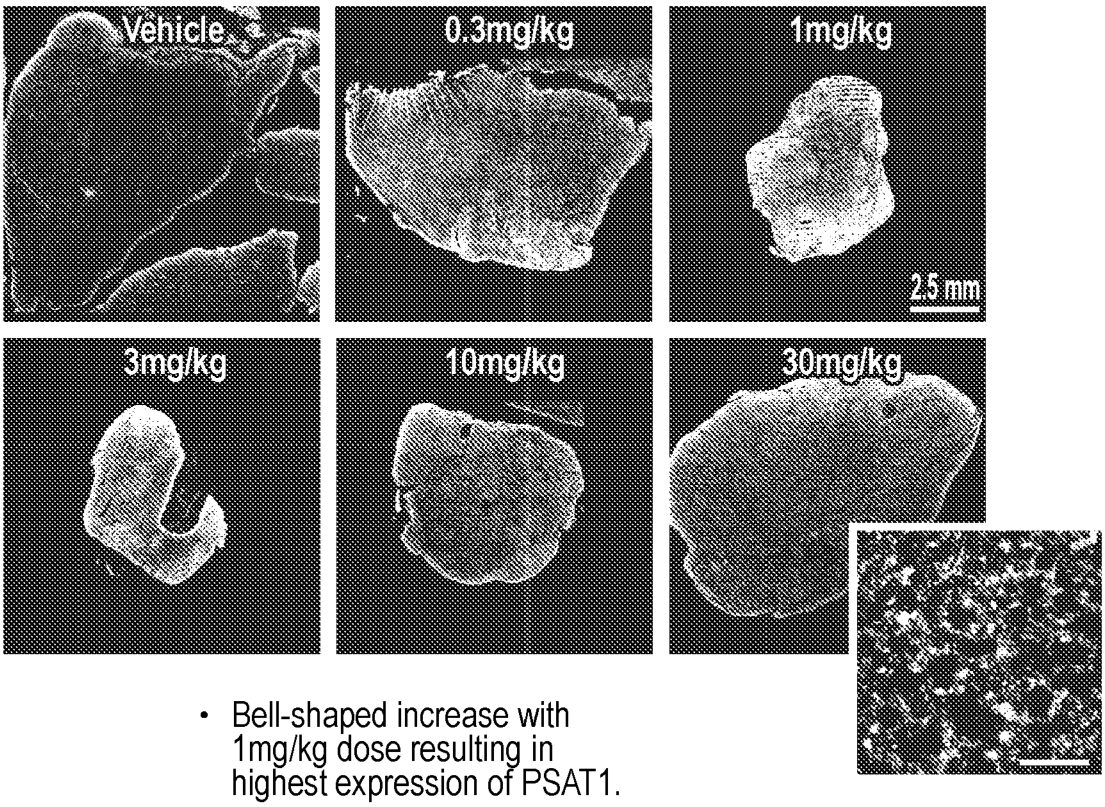


- Bell-shaped increase with 1mg/kg dose resulting in highest expression of PSAT1.

FIG. 23

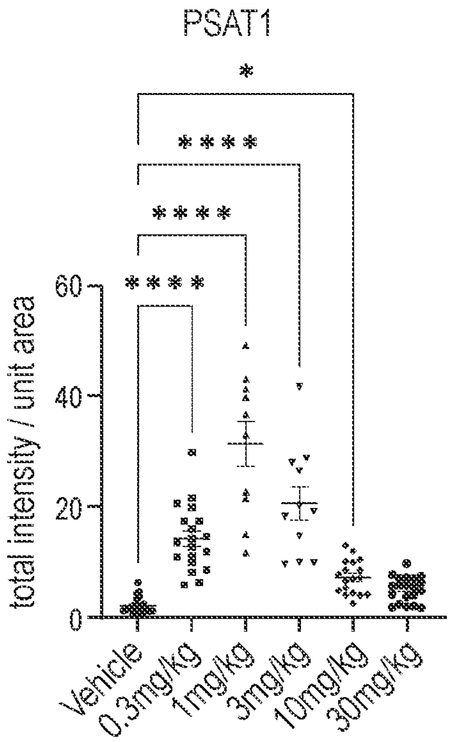
PSAT1
*
****
****
****
total intensity / unit area
60
40
20
0
Vehicle
0.3mg/kg
1mg/kg
3mg/kg
10mg/kg
30mg/kg

FIG. 24

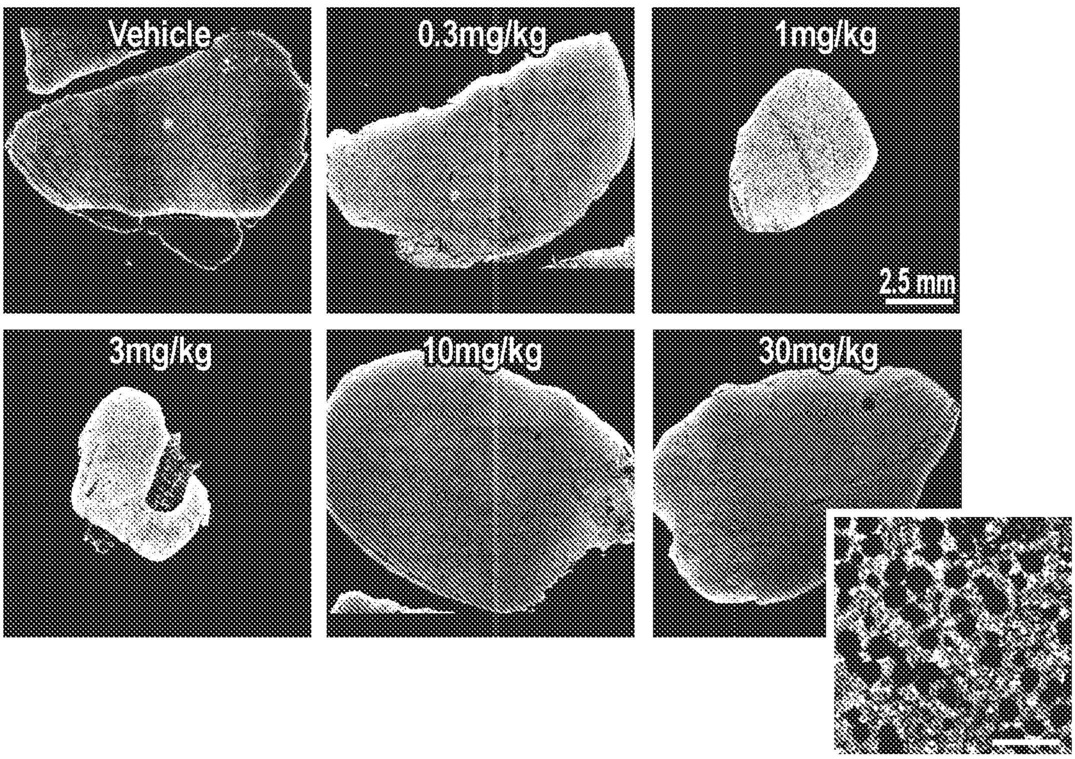
Vehicle
0.3mg/kg
1mg/kg
2.5 mm
3mg/kg
10mg/kg
30mg/kg

FIG. 25

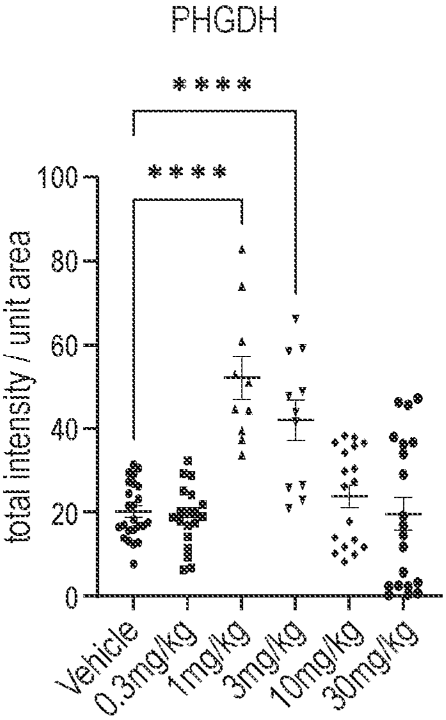
PHGDH
****
****
total intensity / unit area
100
80
60
40
20
0
Vehicle
0.3mg/kg
1mg/kg
3mg/kg
10mg/kg
30mg/kg

FIG. 26

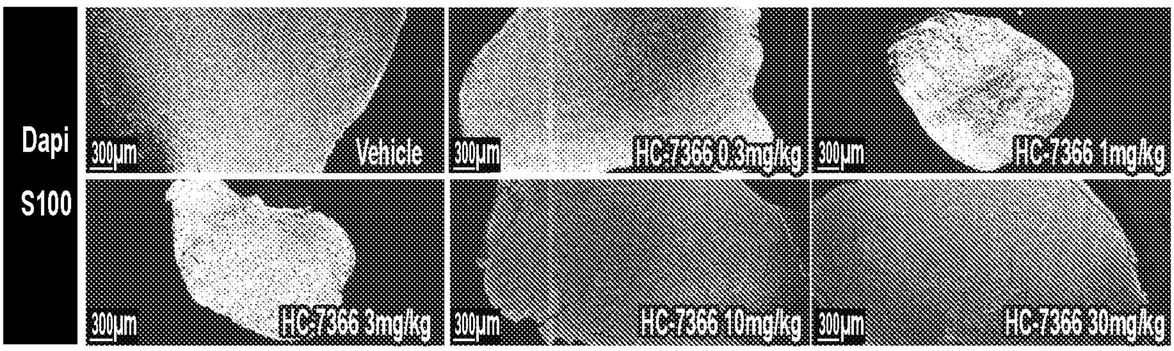
Dapi
S100
300μm
Vehicle
300μm
HC-7366 0.3mg/kg
300μm
HC-7366 1mg/kg
300μm
HC-7366 3mg/kg
300μm
HC-7366 10mg/kg
300μm
HC-7366 30mg/kg

FIG. 27

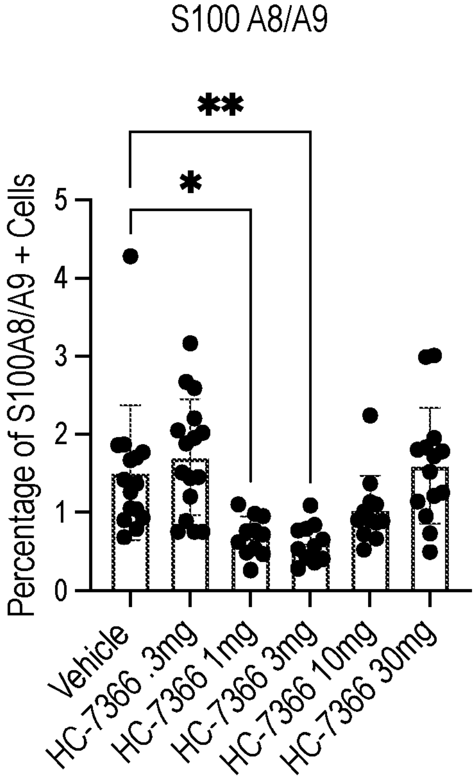
S100 A8/A9
**
*
Percentage of S100A8/A9 + Cells
0
1
2
3
4
5
Vehicle
HC-7366 .3mg
HC-7366 1mg
HC-7366 3mg
HC-7366 10mg
HC-7366 30mg

FIG. 28

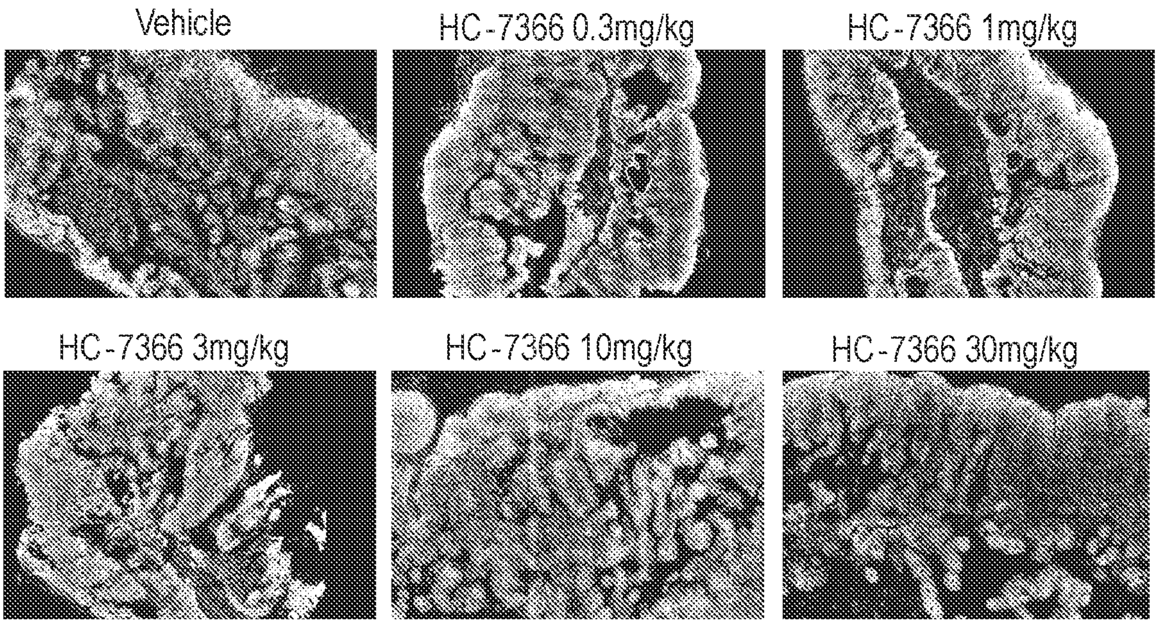
Vehicle
HC-7366 0.3mg/kg
HC-7366 1mg/kg
HC-7366 3mg/kg
HC-7366 10mg/kg
HC-7366 30mg/kg

FIG. 29

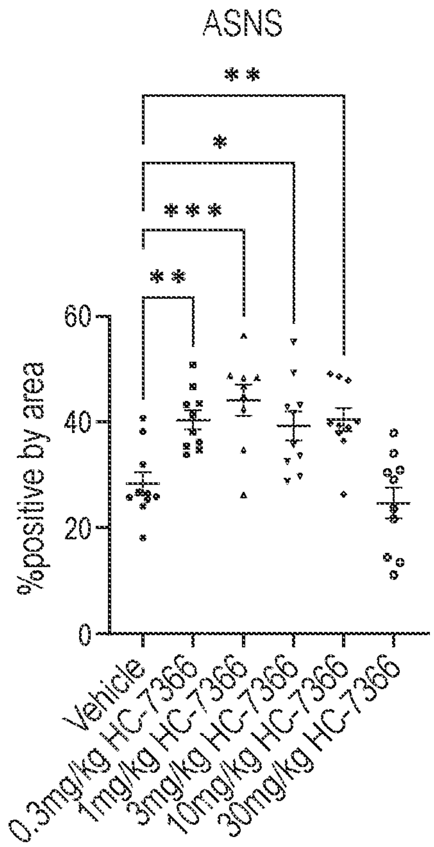
ASNS
**
*
***
**
60
40
20
0
%positive by area
Vehicle
0.3mg/kg HC-7366
1mg/kg HC-7366
3mg/kg HC-7366
10mg/kg HC-7366
30mg/kg HC-7366

FIG. 30

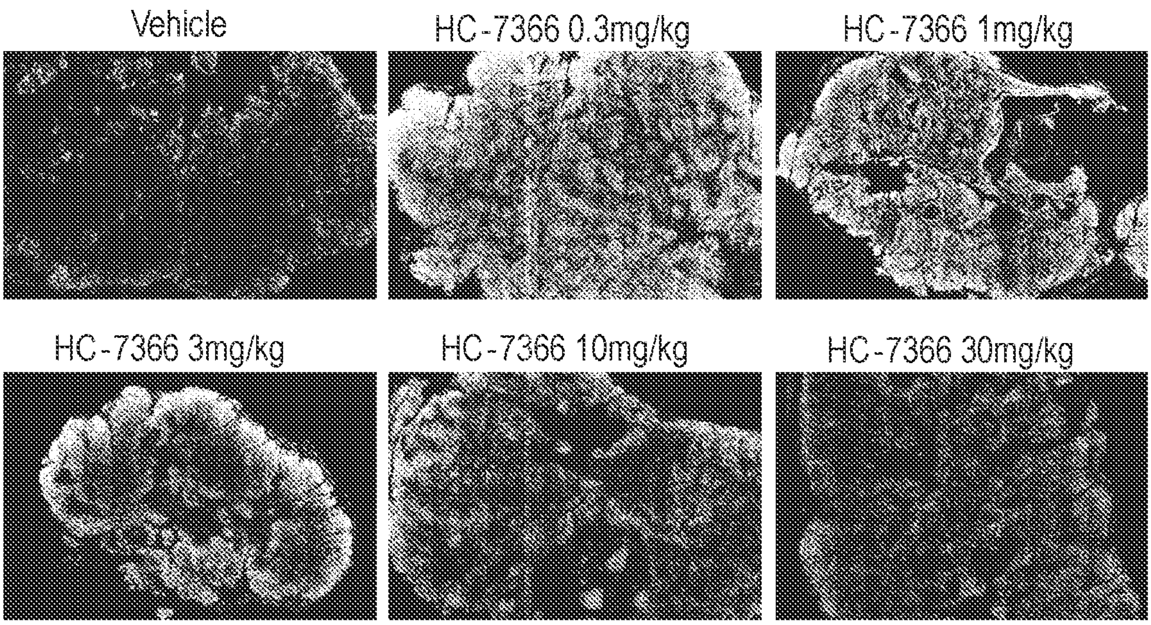
Vehicle
HC-7366 0.3mg/kg
HC-7366 1mg/kg
HC-7366 3mg/kg
HC-7366 10mg/kg
HC-7366 30mg/kg

FIG. 31

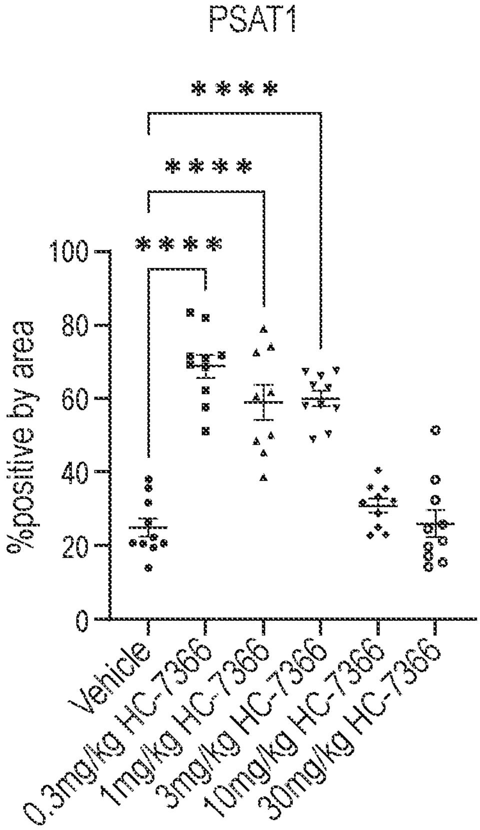
PSAT1
****
****
****
100
80
60
40
20
0
%positive by area
Vehicle
0.3mg/kg HC-7366
1mg/kg HC-7366
3mg/kg HC-7366
10mg/kg HC-7366
30mg/kg HC-7366

FIG. 32

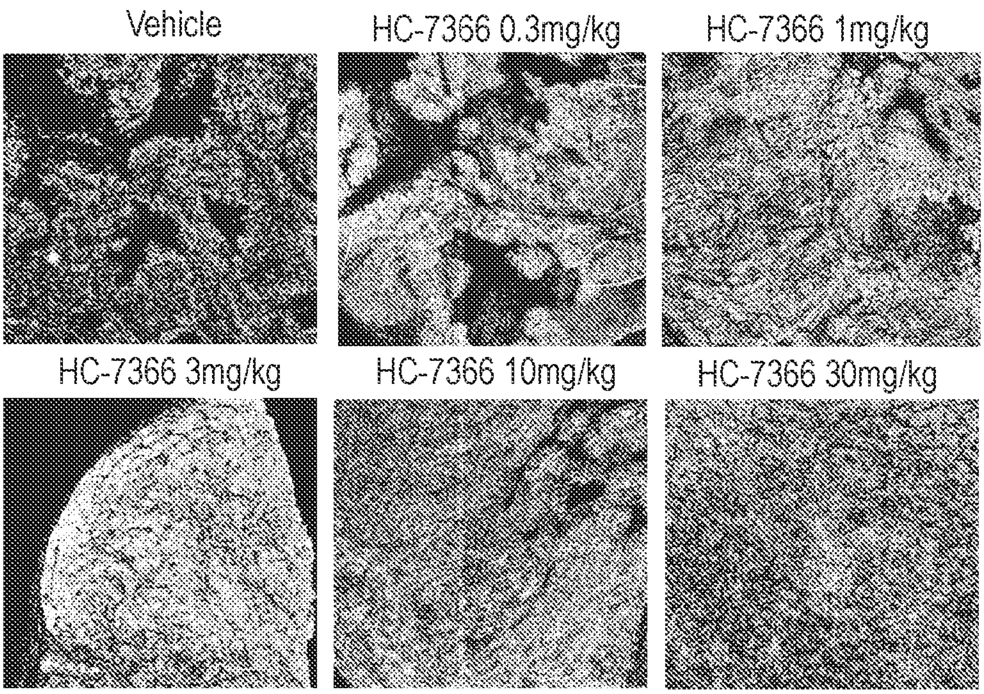
Vehicle
HC-7366 0.3mg/kg
HC-7366 1mg/kg
HC-7366 3mg/kg
HC-7366 10mg/kg
HC-7366 30mg/kg

FIG. 33

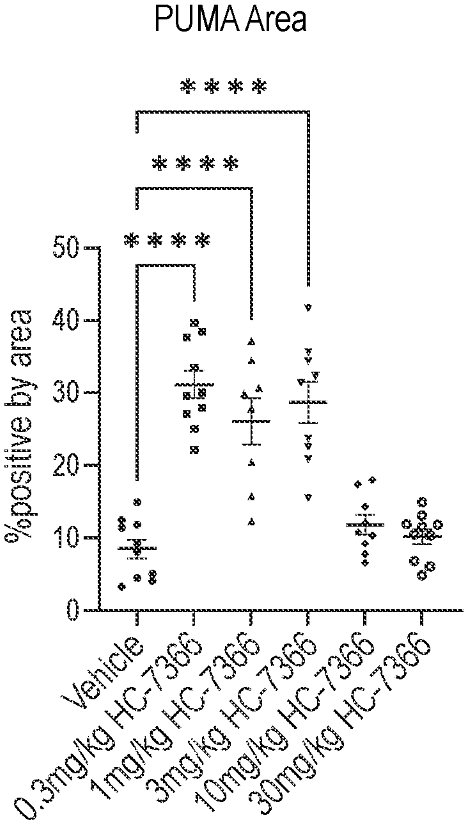
PUMA Area
****
****
****
%positive by area
50
40
30
20
10
0
Vehicle
0.3mg/kg HC-7366
1mg/kg HC-7366
3mg/kg HC-7366
10mg/kg HC-7366
30mg/kg HC-7366

FIG. 34

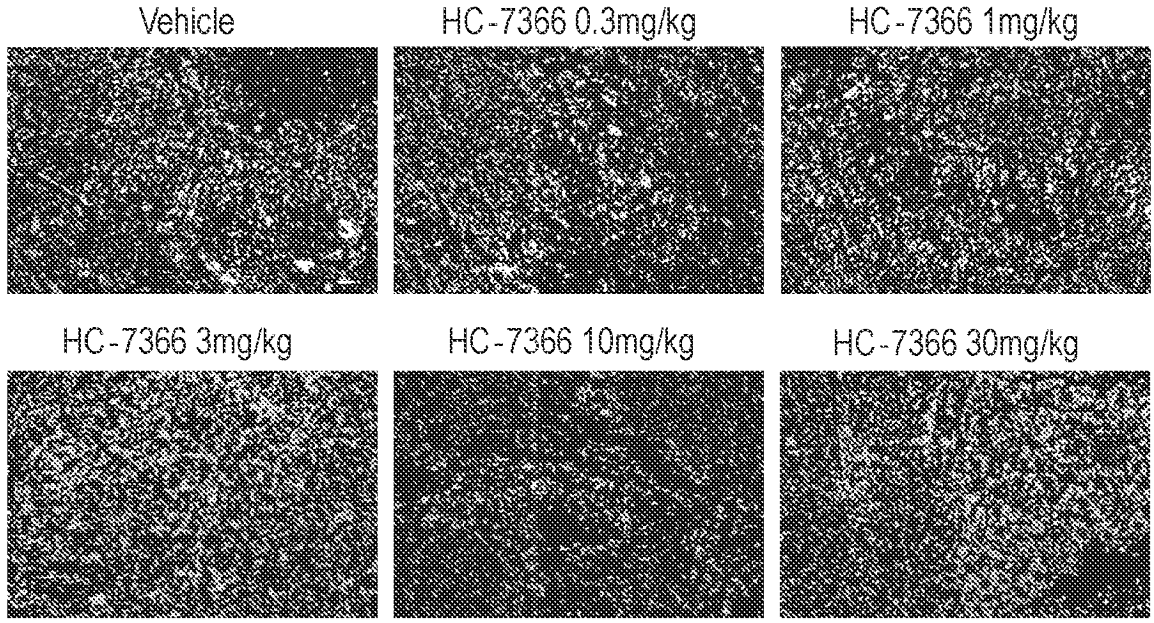
Vehicle
HC-7366 0.3mg/kg
HC-7366 1mg/kg
HC-7366 3mg/kg
HC-7366 10mg/kg
HC-7366 30mg/kg

FIG. 35

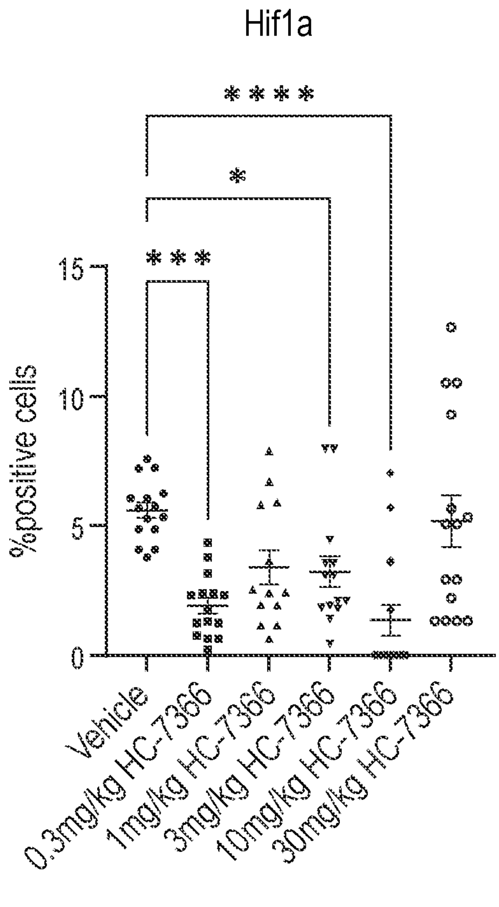
Hif1a
****
*
***
15
10
5
0
%positive cells
Vehicle
0.3mg/kg HC-7366
1mg/kg HC-7366
3mg/kg HC-7366
10mg/kg HC-7366
30mg/kg HC-7366

FIG. 36

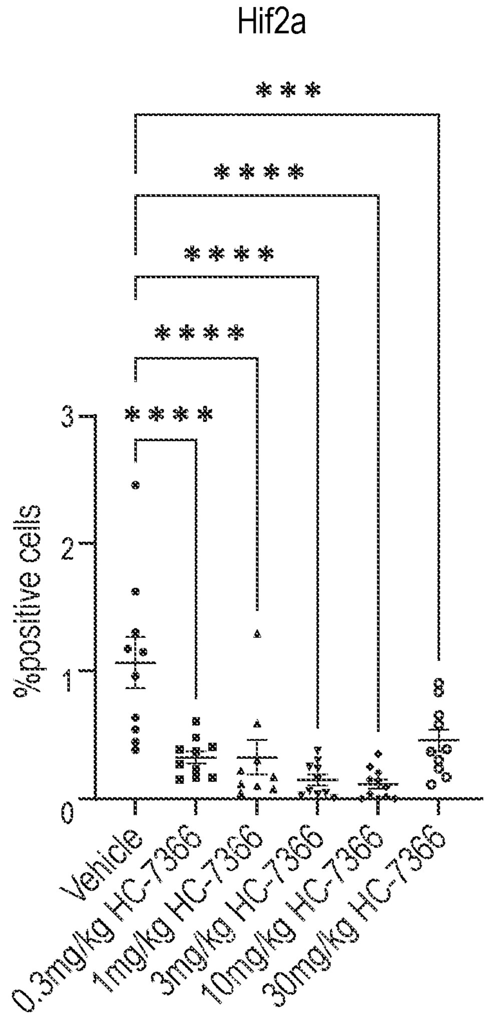
Hif2a
***
****
****
****
****
%positive cells
3
2
1
0
Vehicle
0.3mg/kg HC-7366
1mg/kg HC-7366
3mg/kg HC-7366
10mg/kg HC-7366
30mg/kg HC-7366

FIG. 37

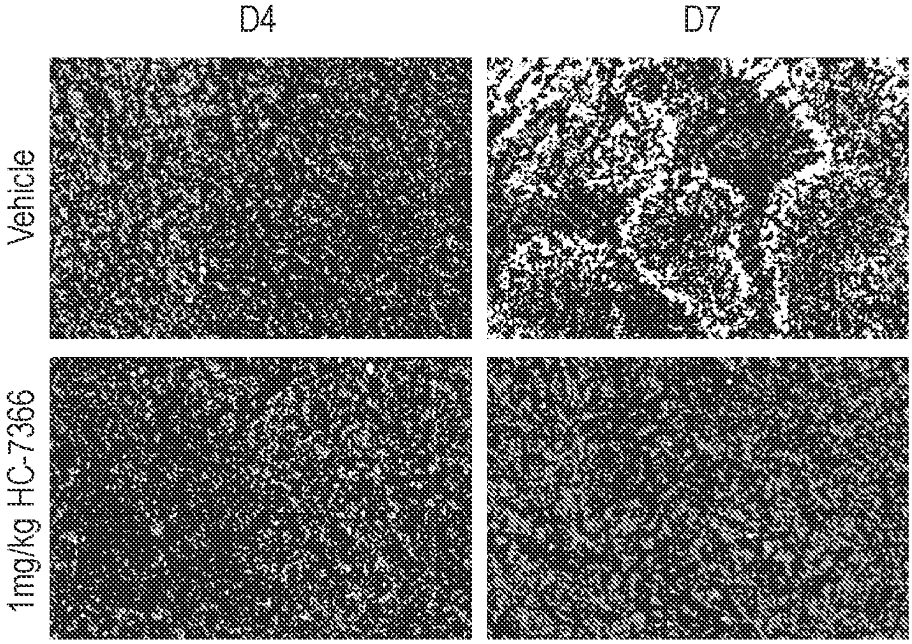
D4
D7
Vehicle
1mg/kg HC-7366

FIG. 38

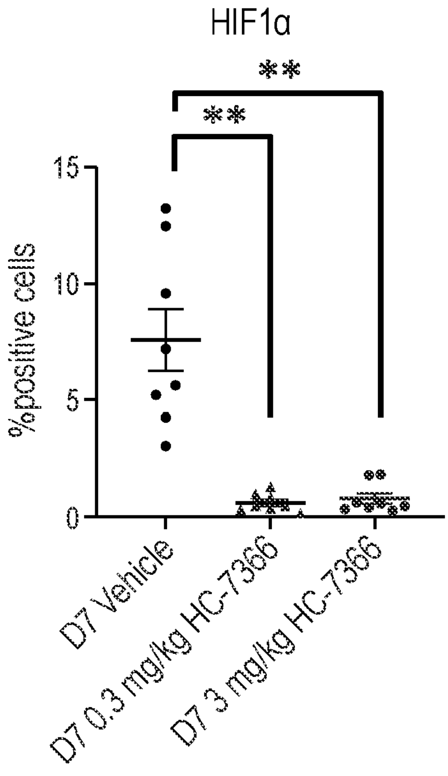
HIF1α
**
**
15
10
5
0
%positive cells
D7 Vehicle
D7 0.3 mg/kg HC-7366
D7 3 mg/kg HC-7366

FIG. 39

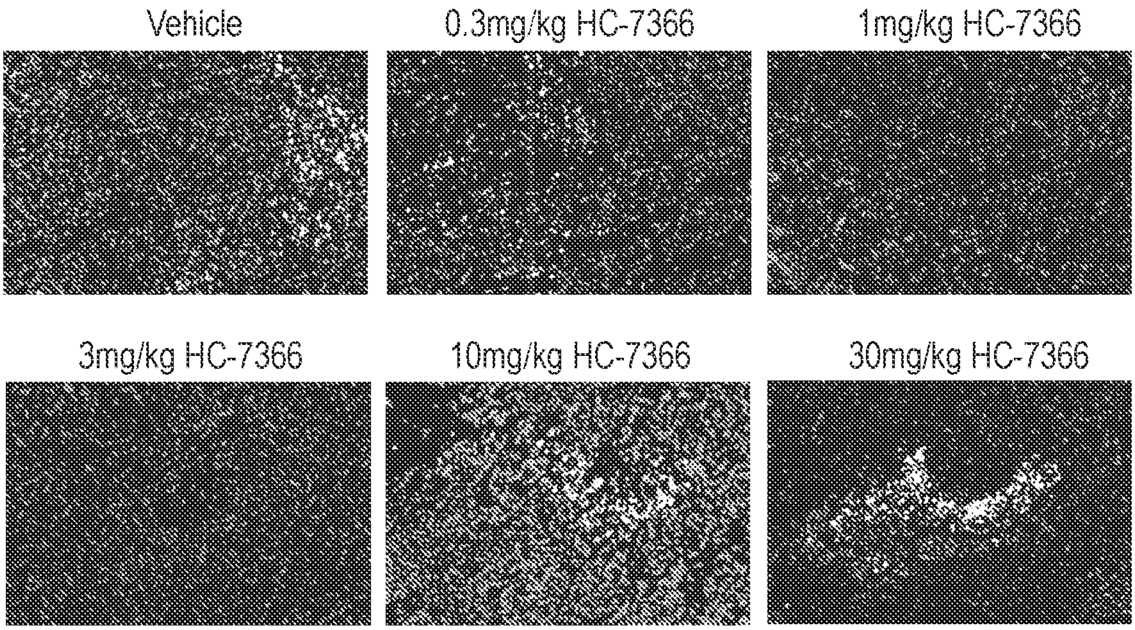
Vehicle
0.3mg/kg HC-7366
1mg/kg HC-7366
3mg/kg HC-7366
10mg/kg HC-7366
30mg/kg HC-7366

FIG. 40

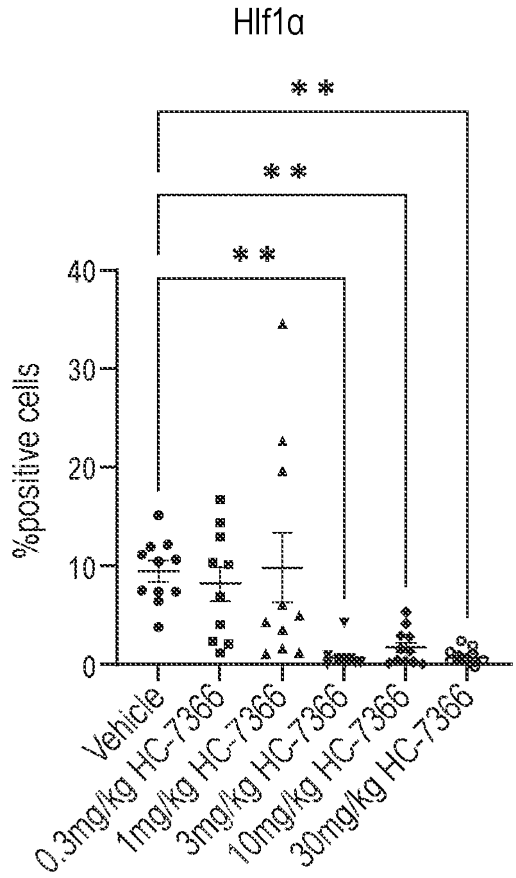
Hlf1α
**
**
**
%positive cells
40
30
20
10
0
Vehicle
0.3mg/kg HC-7366
1mg/kg HC-7366
3mg/kg HC-7366
10mg/kg HC-7366
30mg/kg HC-7366

FIG. 41

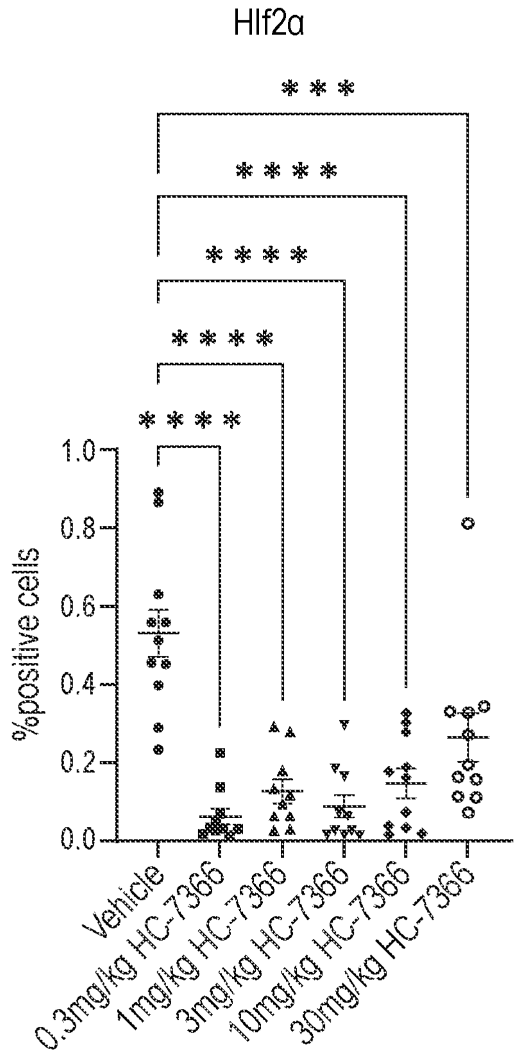
HIf2α
***
****
****
****
****
%positive cells
1.0
0.8
0.6
0.4
0.2
0.0
Vehicle
0.3mg/kg HC-7366
1mg/kg HC-7366
3mg/kg HC-7366
10mg/kg HC-7366
30mg/kg HC-7366

FIG. 42

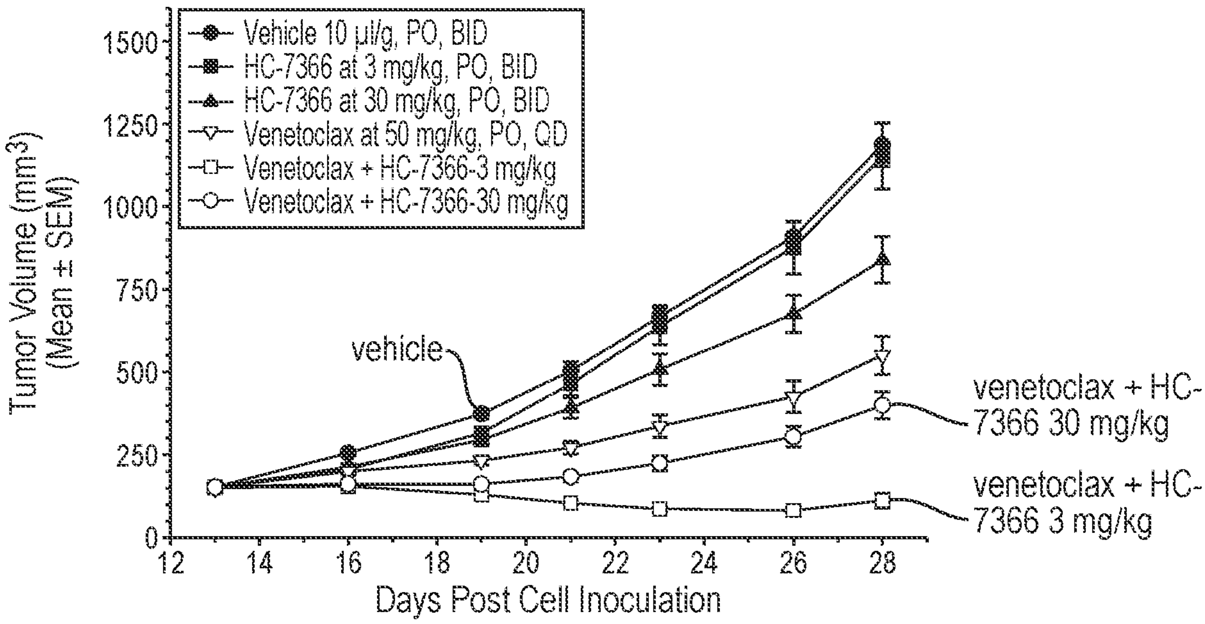
Tumor Volume (mm³) (Mean ± SEM)
Days Post Cell Inoculation
Vehicle 10 µl/g, PO, BID
HC-7366 at 3 mg/kg, PO, BID
HC-7366 at 30 mg/kg, PO, BID
Venetoclax at 50 mg/kg, PO, QD
Venetoclax + HC-7366-3 mg/kg
Venetoclax + HC-7366-30 mg/kg
vehicle
venetoclax + HC-7366 30 mg/kg
venetoclax + HC-7366 3 mg/kg
0
250
500
750
1000
1250
1500
12
14
16
18
20
22
24
26
28

FIG. 43

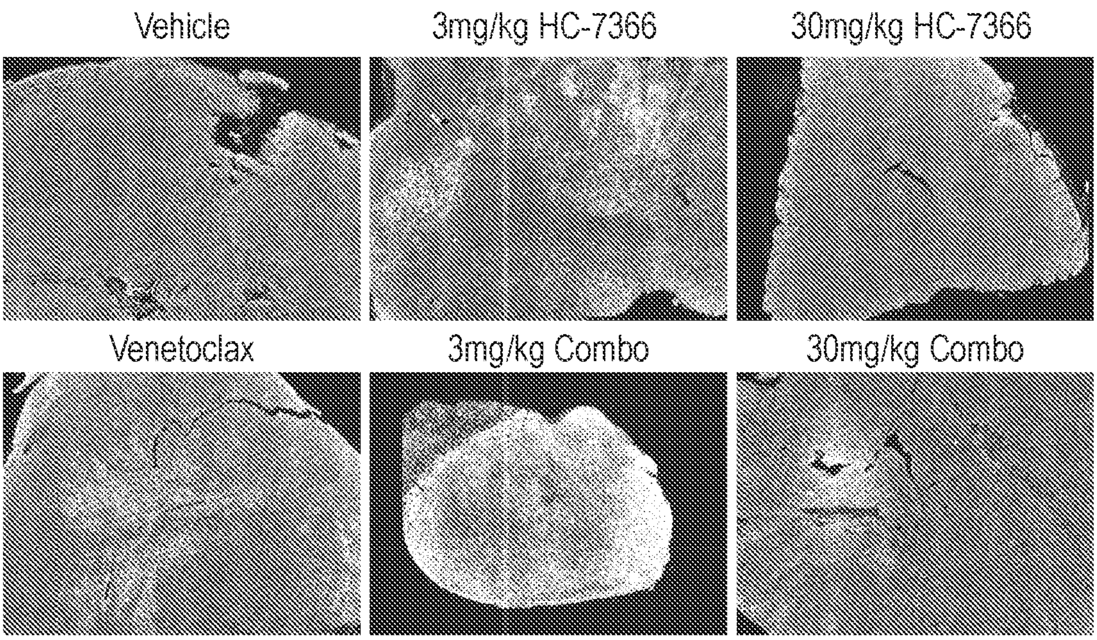
Vehicle
3mg/kg HC-7366
30mg/kg HC-7366
Venetoclax
3mg/kg Combo
30mg/kg Combo

FIG. 44

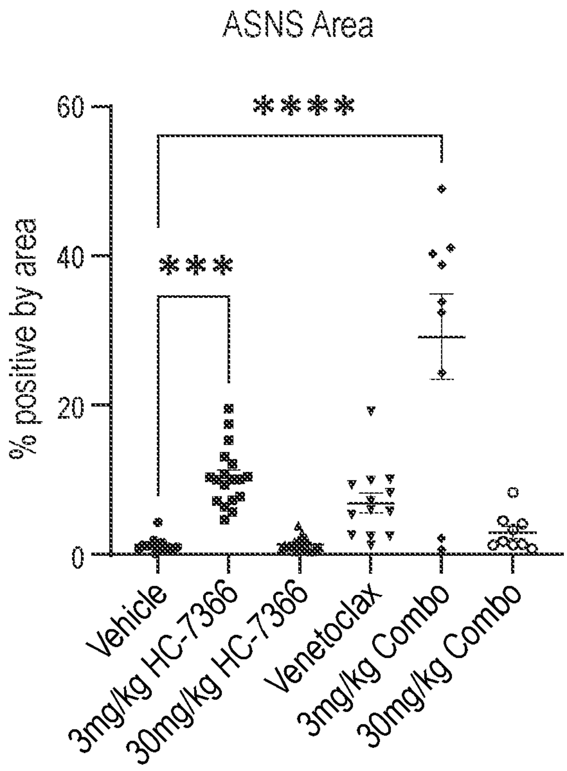
ASNS Area
****
***
60
40
20
0
% positive by area
Vehicle
3mg/kg HC-7366
30mg/kg HC-7366
Venetoclax
3mg/kg Combo
30mg/kg Combo

FIG. 45

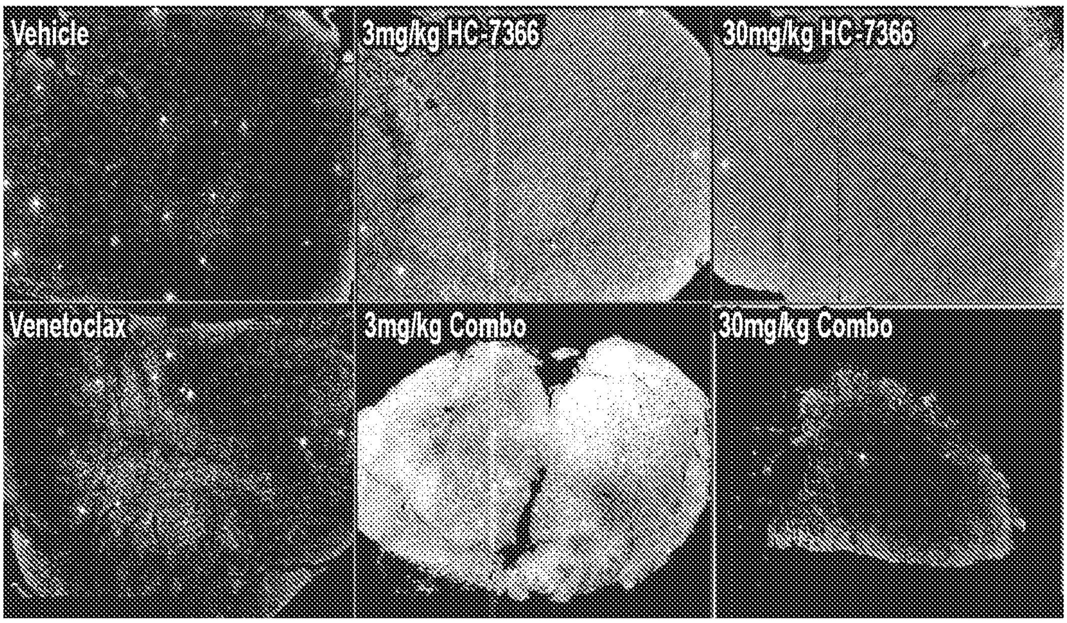
Vehicle
3mg/kg HC-7366
30mg/kg HC-7366
Venetoclax
3mg/kg Combo
30mg/kg Combo

FIG. 46

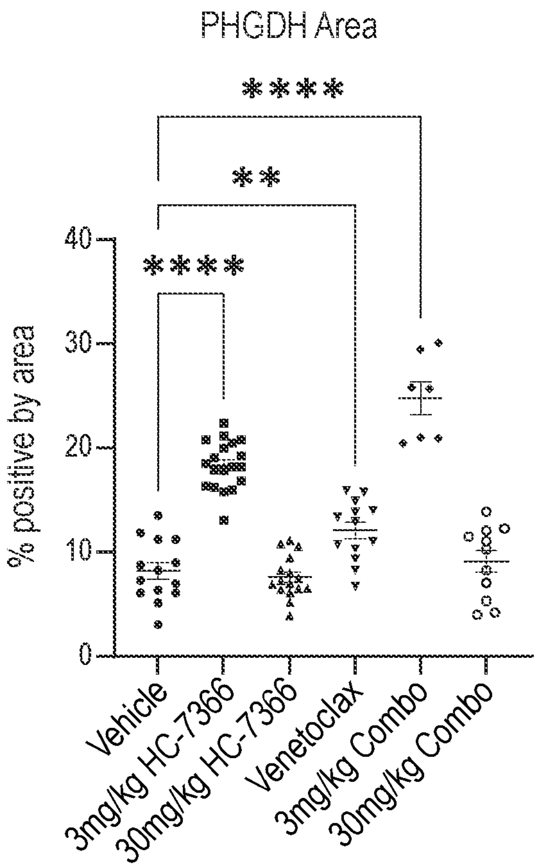
PHGDH Area
****
**
****
% positive by area
40
30
20
10
0
Vehicle
3mg/kg HC-7366
30mg/kg HC-7366
Venetoclax
3mg/kg Combo
30mg/kg Combo

FIG. 47

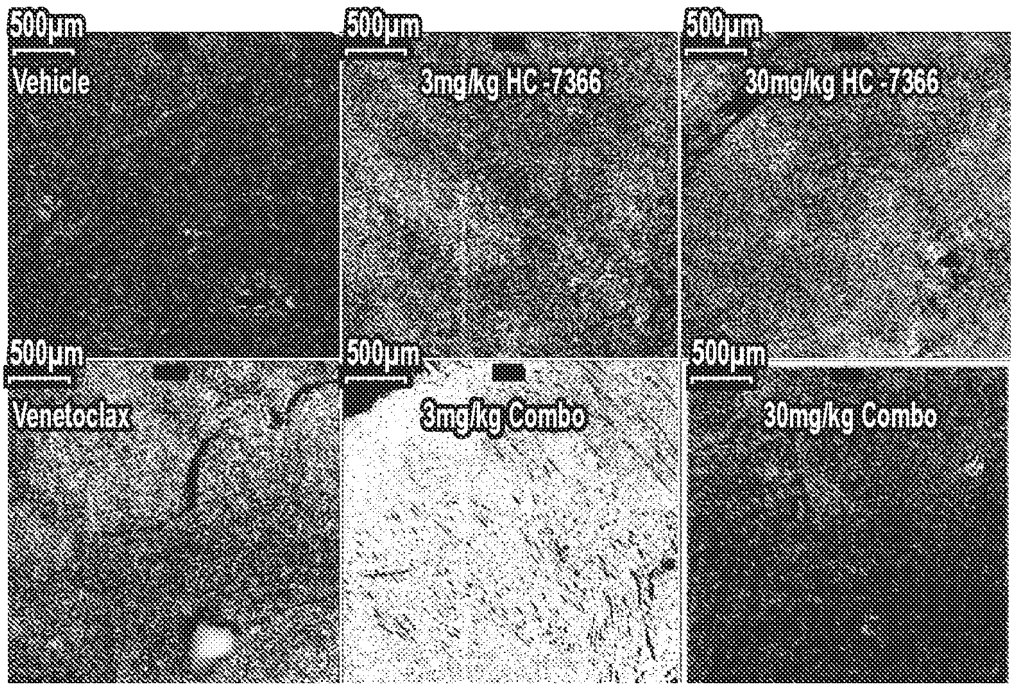
500μm
Vehicle
500μm
3mg/kg HC -7366
500μm
30mg/kg HC -7366
500μm
Venetoclax
500μm
3mg/kg Combo
500μm
30mg/kg Combo

FIG. 48

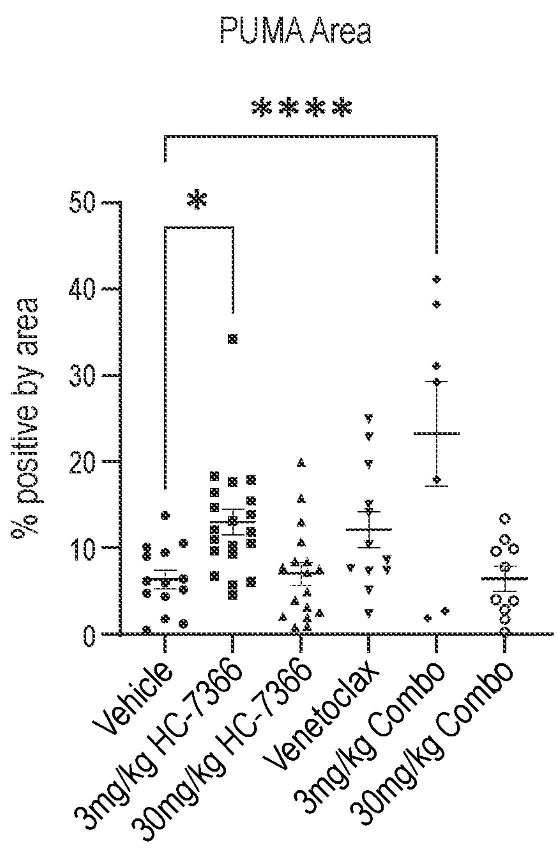
PUMA Area
****
*
% positive by area
50
40
30
20
10
0
Vehicle
3mg/kg HC-7366
30mg/kg HC-7366
Venetoclax
3mg/kg Combo
30mg/kg Combo

FIG. 49

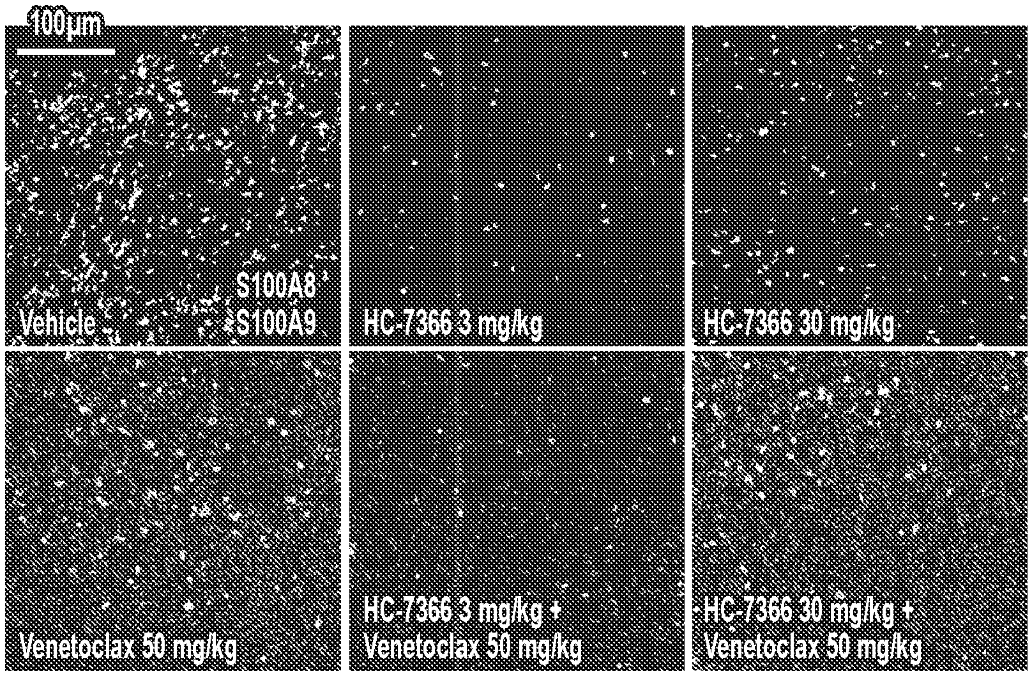
100μm
S100A8
S100A9
Vehicle
HC-7366 3 mg/kg
HC-7366 30 mg/kg
Venetoclax 50 mg/kg
HC-7366 3 mg/kg +
Venetoclax 50 mg/kg
HC-7366 30 mg/kg +
Venetoclax 50 mg/kg

FIG. 50

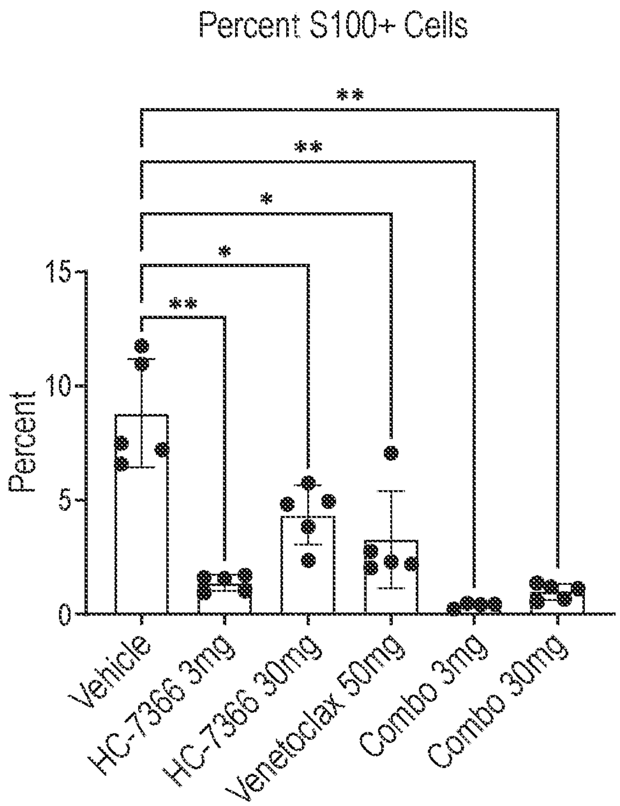
Percent S100+ Cells
**
**
*
*
**
15
10
5
0
Percent
Vehicle
HC-7366 3mg
HC-7366 30mg
Venetoclax 50mg
Combo 3mg
Combo 30mg

FIG. 51

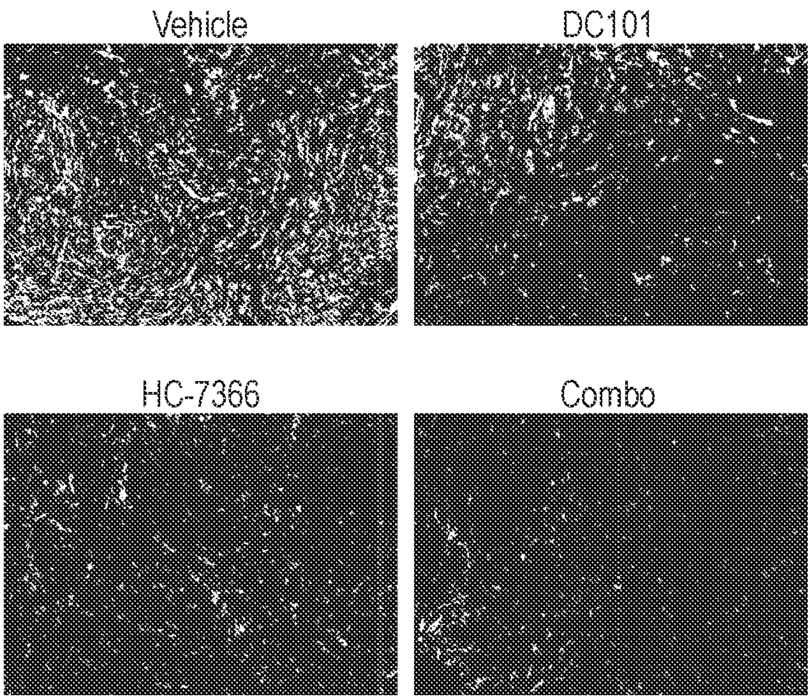
Vehicle
DC101
HC-7366
Combo

FIG. 52

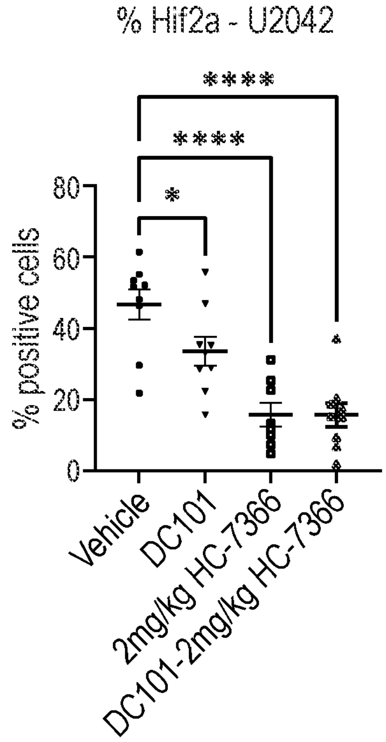
% Hif2a - U2042
****
****
*
80
60
40
20
0
% positive cells
Vehicle
DC101
2mg/kg HC-7366
DC101-2mg/kg HC-7366

FIG. 53

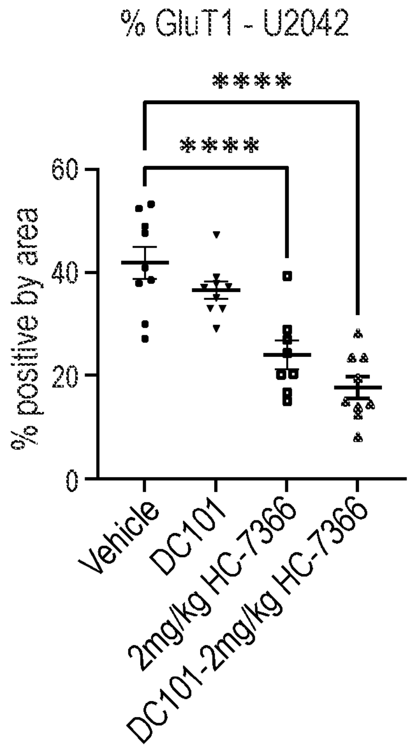
% GluT1 - U2042
****
****
60
40
20
0
% positive by area
Vehicle
DC101
2mg/kg HC-7366
DC101-2mg/kg HC-7366

FIG. 54

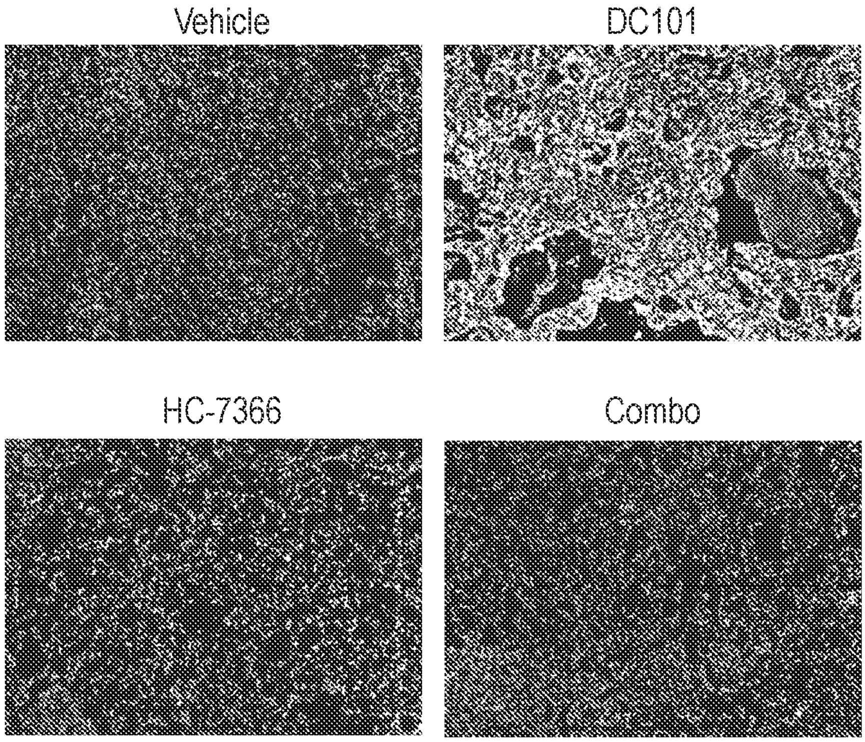
Vehicle
DC101
HC-7366
Combo

FIG. 55

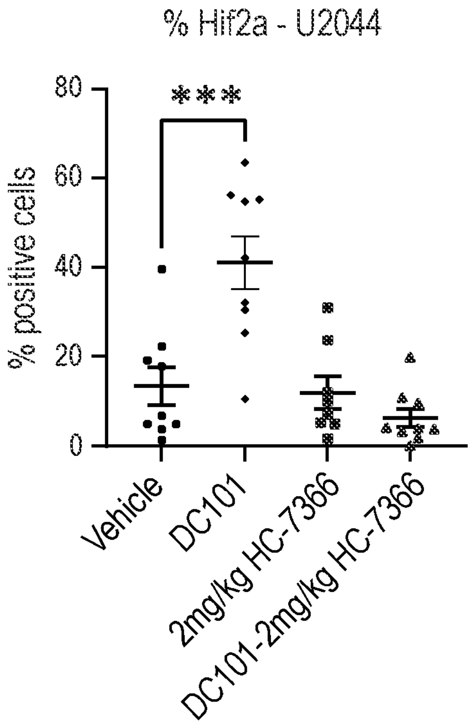
% Hif2a - U2044
80
60
40
20
0
% positive cells
***
Vehicle
DC101
2mg/kg HC-7366
DC101-2mg/kg HC-7366

FIG. 56

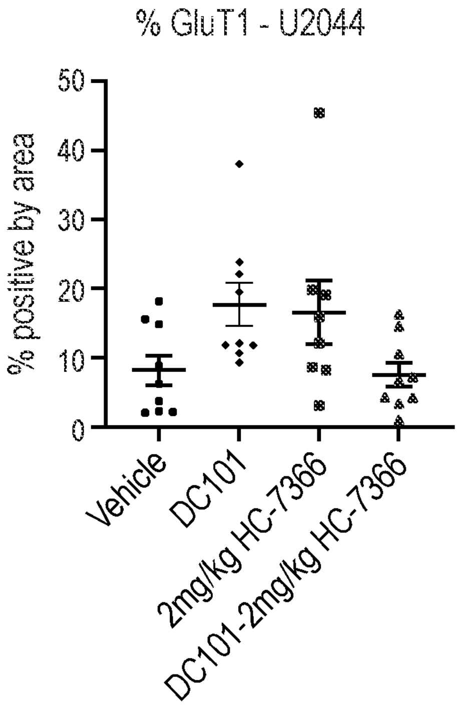
% GluT1 - U2044
% positive by area
50
40
30
20
10
0
Vehicle
DC101
2mg/kg HC-7366
DC101-2mg/kg HC-7366

FIG. 57

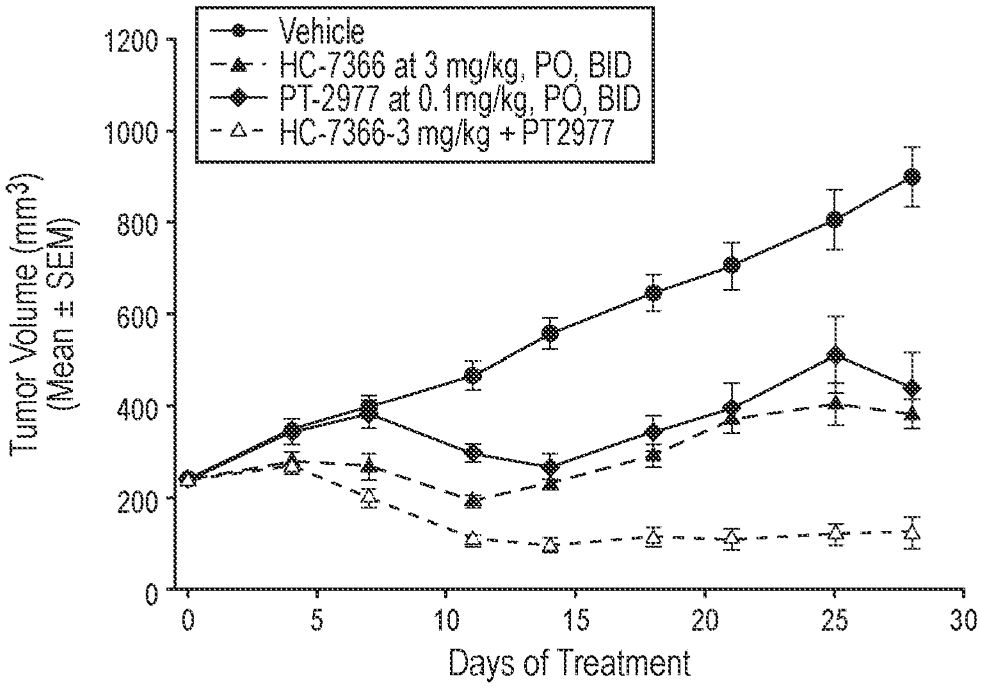
Vehicle
HC-7366 at 3 mg/kg, PO, BID
PT-2977 at 0.1mg/kg, PO, BID
HC-7366-3 mg/kg + PT2977
Tumor Volume (mm3)
(Mean ± SEM)
1200
1000
800
600
400
200
0
0
5
10
15
20
25
30
Days of Treatment

FIG. 58

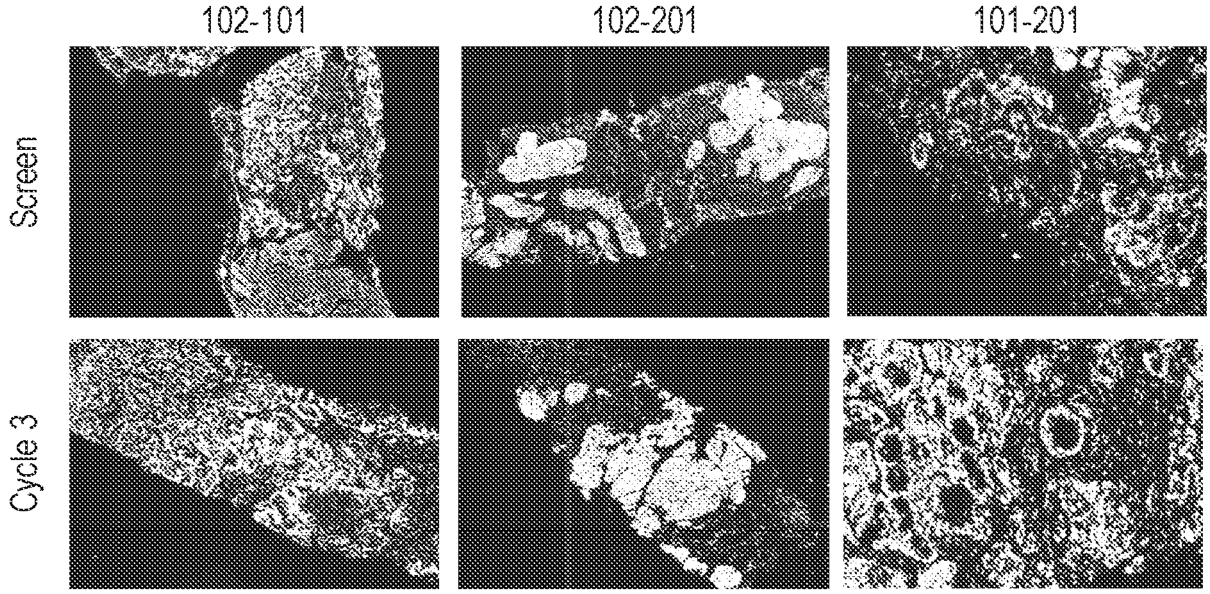


FIG. 59

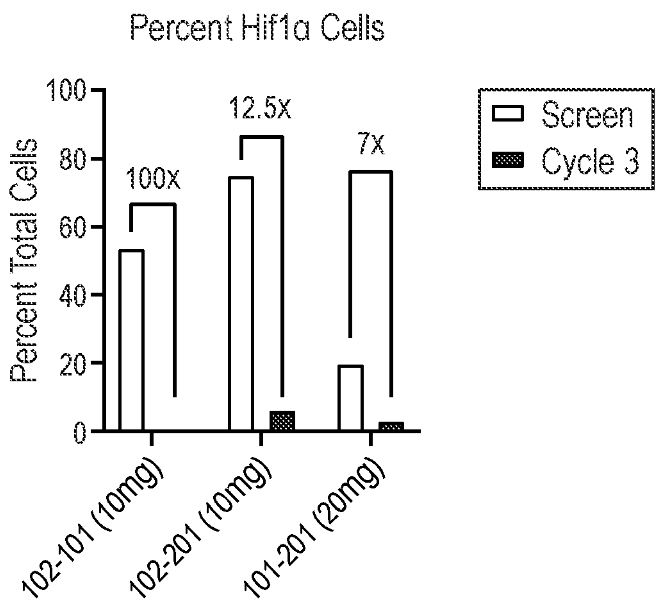


102-101 (H&N) - 10 mg
102-201 (CRC) - 10 mg
101-201 (CRC) - 20 mg

FIG. 60

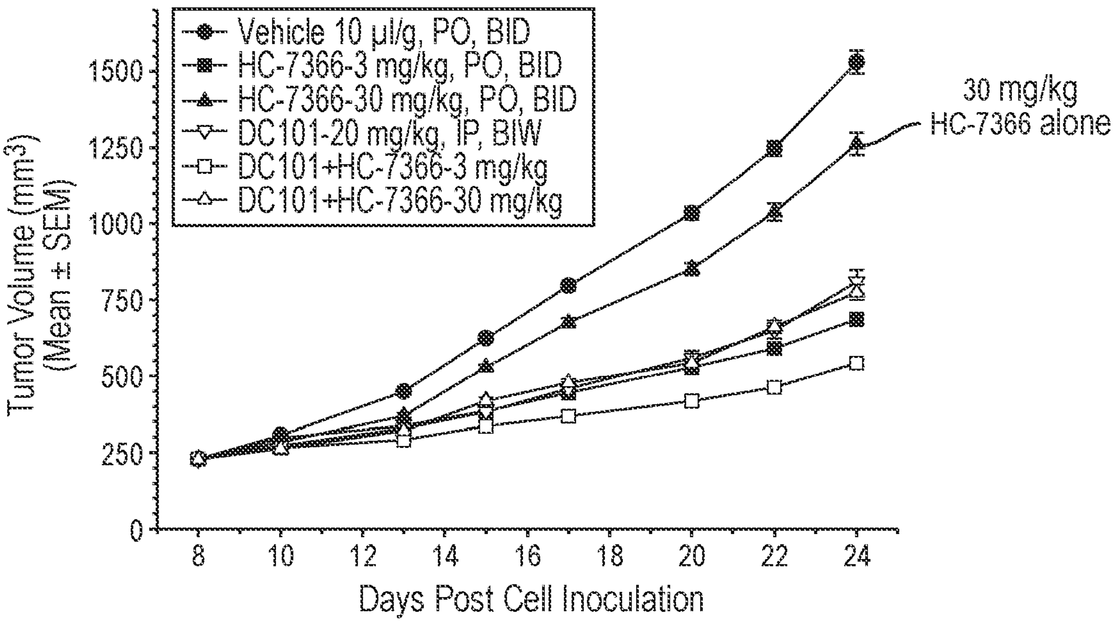
Vehicle 10 µl/g, PO, BID
HC-7366-3 mg/kg, PO, BID
HC-7366-30 mg/kg, PO, BID
DC101-20 mg/kg, IP, BIW
DC101+HC-7366-3 mg/kg
DC101+HC-7366-30 mg/kg
30 mg/kg
HC-7366 alone
Tumor Volume (mm3)
(Mean ± SEM)
0
250
500
750
1000
1250
1500
8
10
12
14
16
18
20
22
24
Days Post Cell Inoculation

FIG. 61

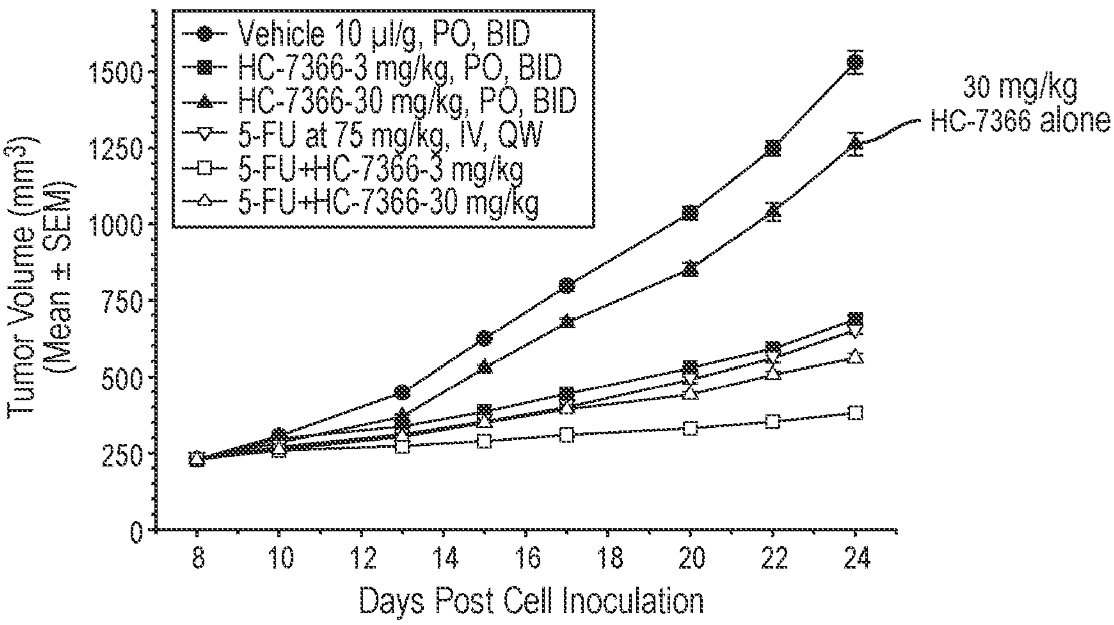
Vehicle 10 µl/g, PO, BID
HC-7366-3 mg/kg, PO, BID
HC-7366-30 mg/kg, PO, BID
5-FU at 75 mg/kg, IV, QW
5-FU+HC-7366-3 mg/kg
5-FU+HC-7366-30 mg/kg
30 mg/kg
HC-7366 alone
Tumor Volume (mm3)
(Mean ± SEM)
0
250
500
750
1000
1250
1500
8
10
12
14
16
18
20
22
24
Days Post Cell Inoculation

FIG. 62

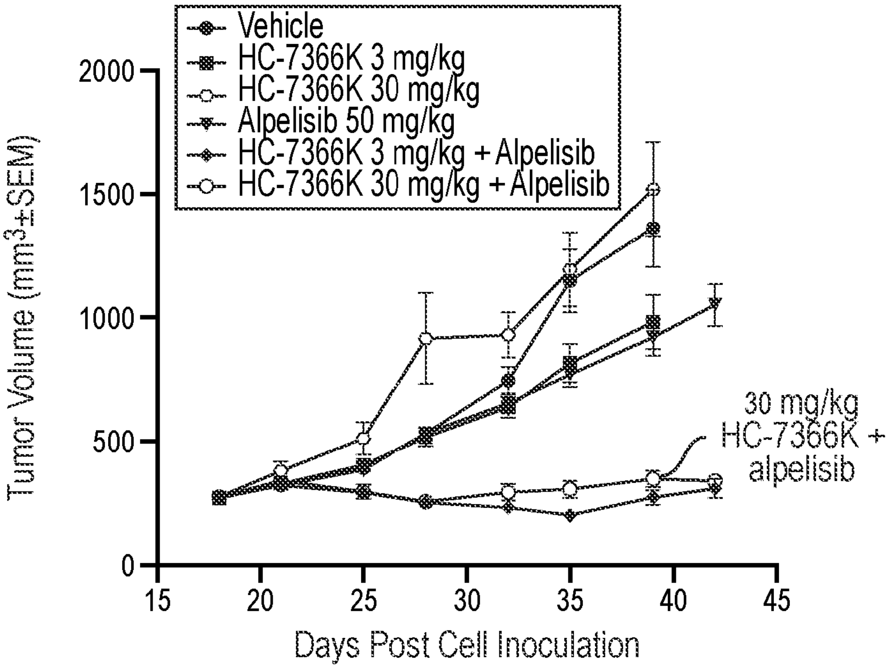
Vehicle
HC-7366K 3 mg/kg
HC-7366K 30 mg/kg
Alpelisib 50 mg/kg
HC-7366K 3 mg/kg + Alpelisib
HC-7366K 30 mg/kg + Alpelisib
Tumor Volume (mm3±SEM)
2000
1500
1000
500
0
15
20
25
30
35
40
45
Days Post Cell Inoculation
30 mg/kg
HC-7366K +
alpelisib

FIG. 63

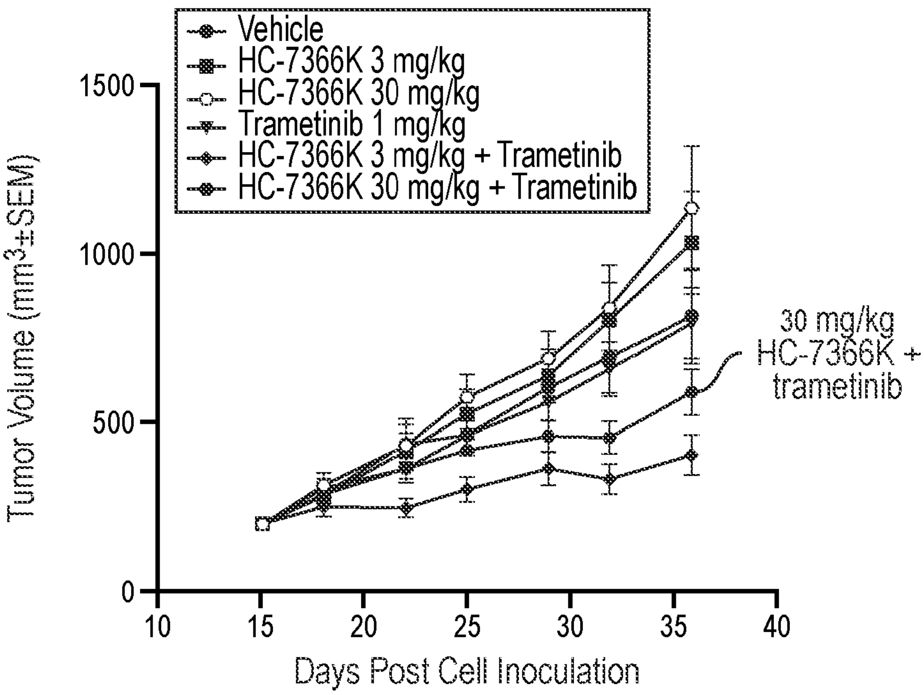
Vehicle
HC-7366K 3 mg/kg
HC-7366K 30 mg/kg
Trametinib 1 mg/kg
HC-7366K 3 mg/kg + Trametinib
HC-7366K 30 mg/kg + Trametinib
Tumor Volume (mm3±SEM)
1500
1000
500
0
10
15
20
25
30
35
40
Days Post Cell Inoculation
30 mg/kg
HC-7366K +
trametinib

FIG. 64

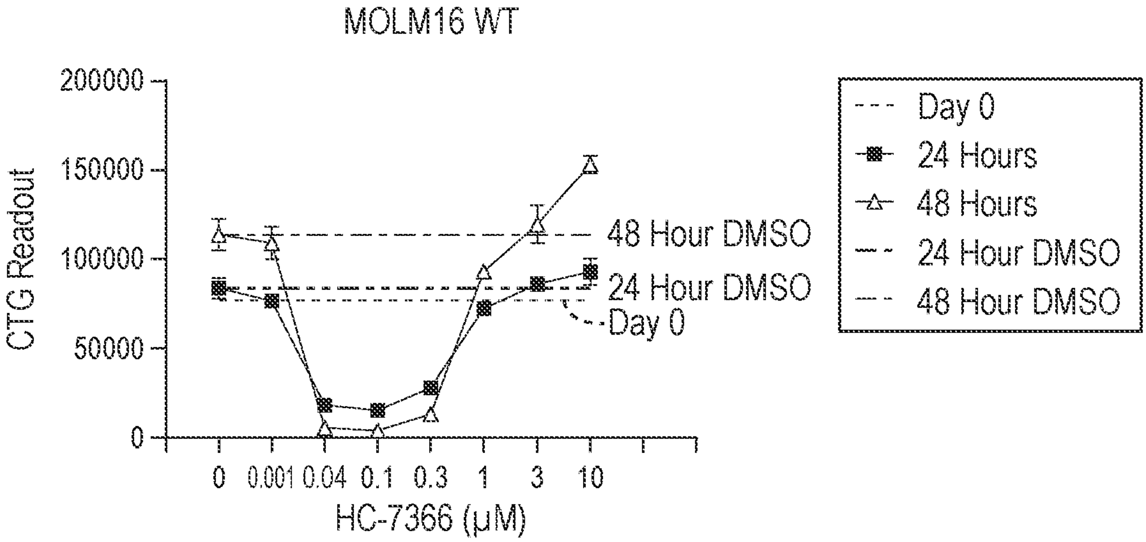
MOLM16 WT
CTG Readout
200000
150000
100000
50000
0
48 Hour DMSO
24 Hour DMSO
Day 0
0
0.001
0.04
0.1
0.3
1
3
10
HC-7366 (μM)
Day 0
24 Hours
48 Hours
24 Hour DMSO
48 Hour DMSO

FIG. 65

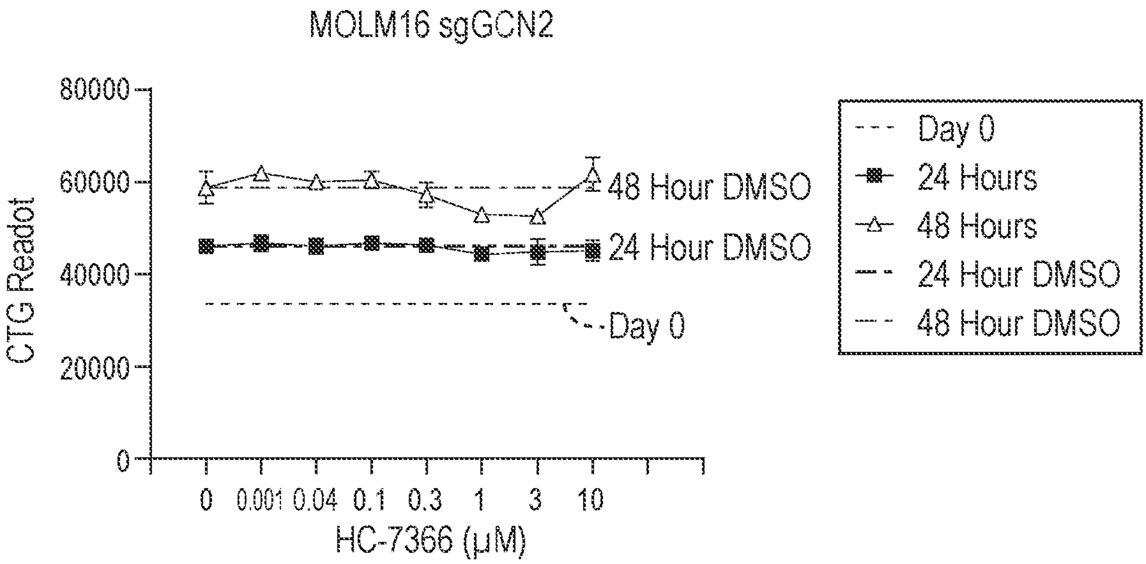
MOLM16 sgGCN2
CTG Readot
80000
60000
40000
20000
0
48 Hour DMSO
24 Hour DMSO
Day 0
0
0.001
0.04
0.1
0.3
1
3
10
HC-7366 (μM)
Day 0
24 Hours
48 Hours
24 Hour DMSO
48 Hour DMSO

FIG. 66

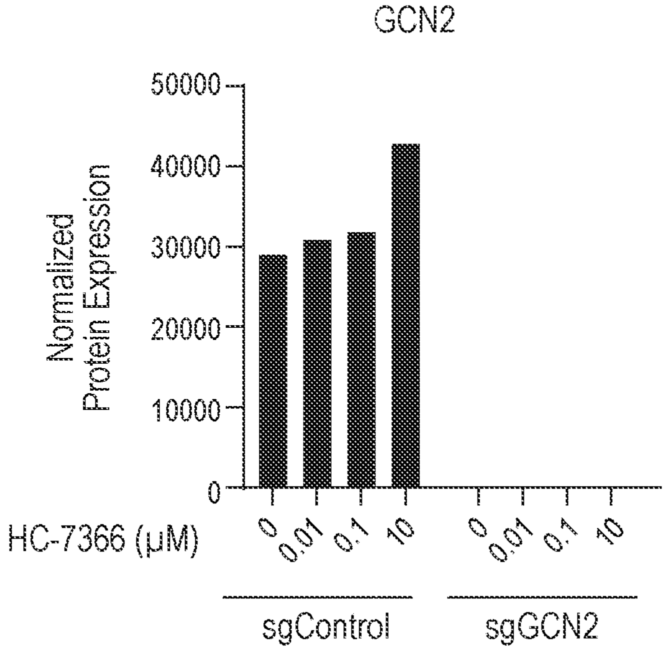
GCN2
Normalized Protein Expression
50000
40000
30000
20000
10000
0
HC-7366 (µM)
0
0.01
0.1
10
0
0.01
0.1
10
sgControl
sgGCN2

FIG. 67

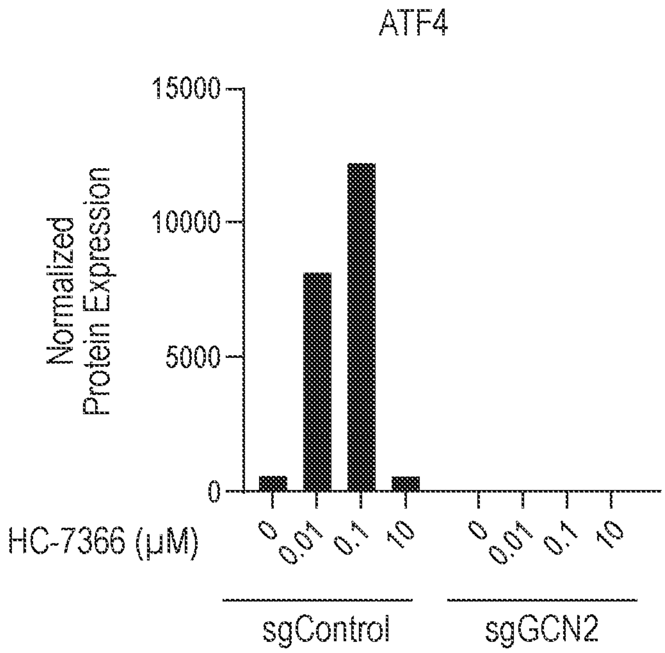
ATF4
Normalized Protein Expression
15000
10000
5000
0
HC-7366 (µM)
0
0.01
0.1
10
0
0.01
0.1
10
sgControl
sgGCN2

FIG. 68

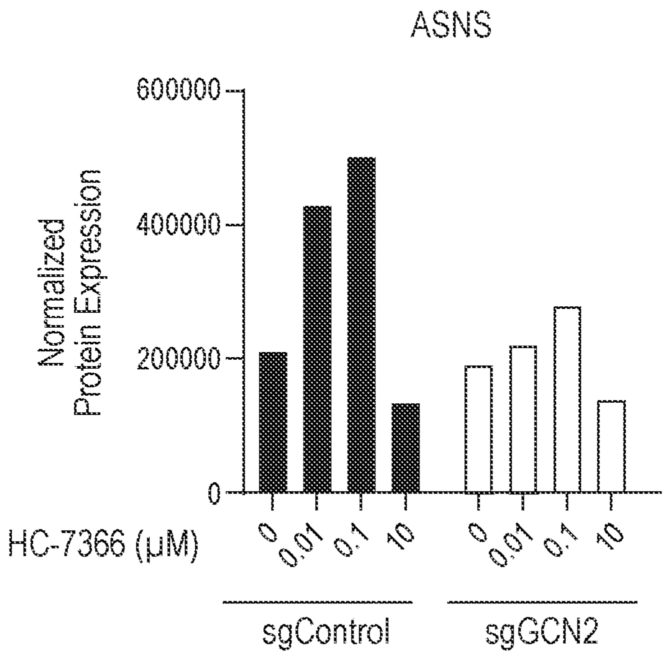
ASNS
600000
400000
200000
0
Normalized
Protein Expression
HC-7366 (µM)
0
0.01
0.1
10
0
0.01
0.1
10
sgControl
sgGCN2

FIG. 69

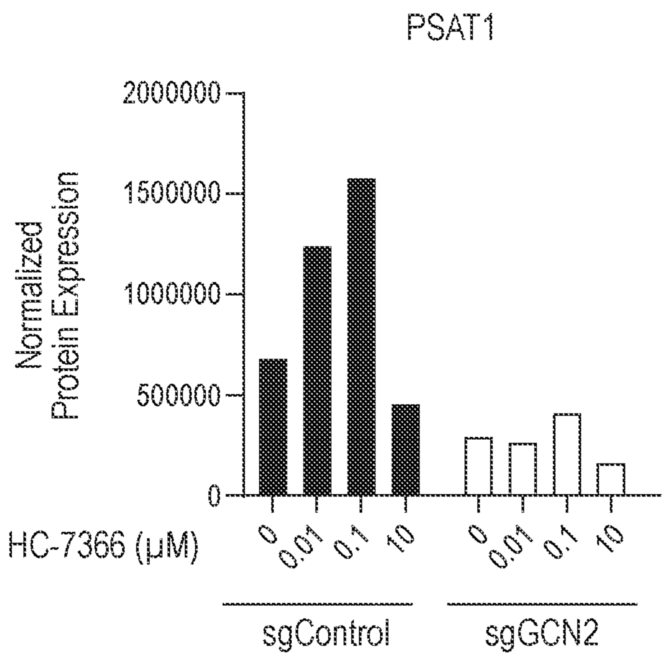
PSAT1
2000000
1500000
1000000
500000
0
Normalized
Protein Expression
HC-7366 (µM)
0
0.01
0.1
10
0
0.01
0.1
10
sgControl
sgGCN2

FIG. 70

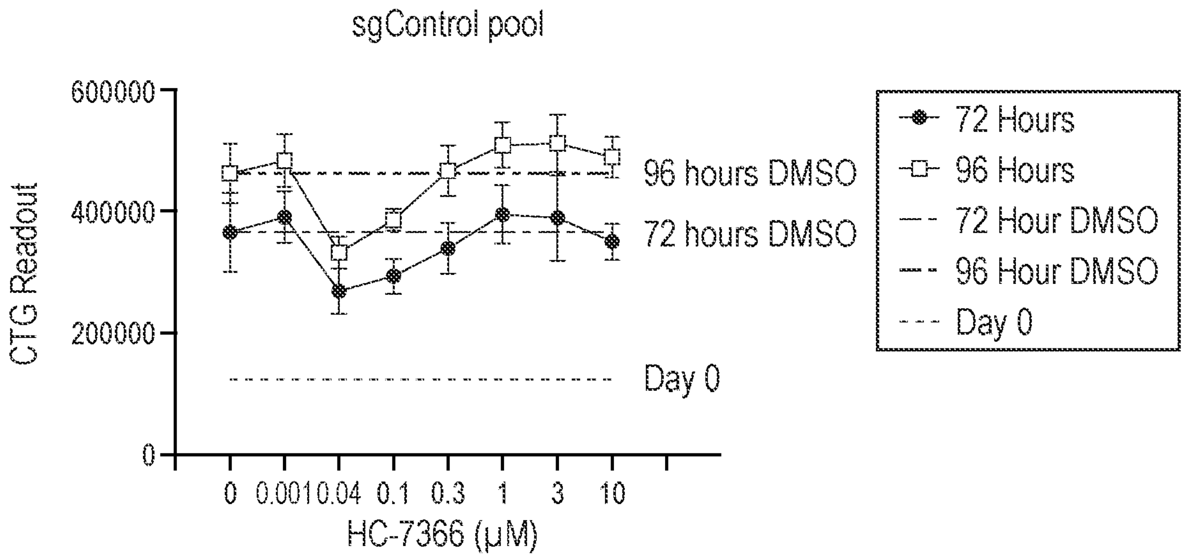
sgControl pool
600000
400000
200000
0
CTG Readout
96 hours DMSO
72 hours DMSO
Day 0
0
0.001
0.04
0.1
0.3
1
3
10
HC-7366 (µM)
72 Hours
96 Hours
72 Hour DMSO
96 Hour DMSO
Day 0

FIG. 71

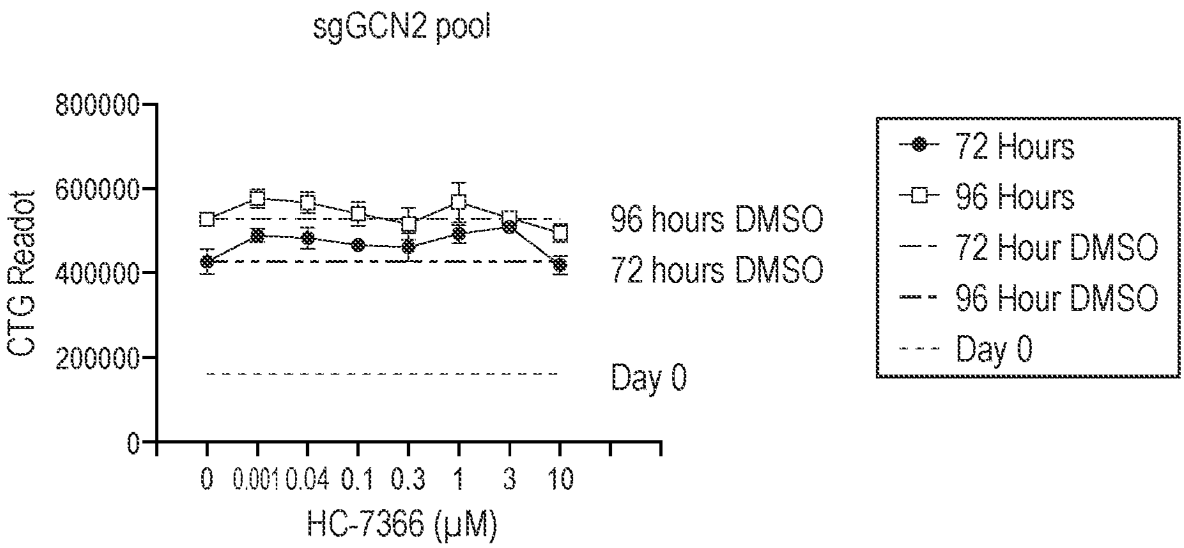
sgGCN2 pool
800000
600000
400000
200000
0
CTG Readot
96 hours DMSO
72 hours DMSO
Day 0
0
0.001
0.04
0.1
0.3
1
3
10
HC-7366 (µM)
72 Hours
96 Hours
72 Hour DMSO
96 Hour DMSO
Day 0

FIG. 72

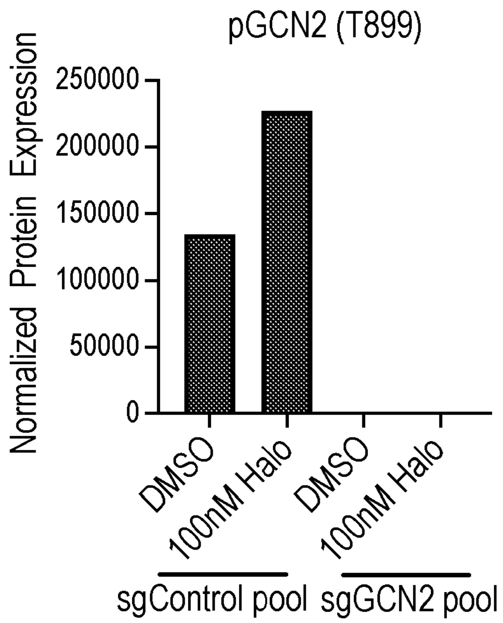
pGCN2 (T899)
Normalized Protein Expression
250000
200000
150000
100000
50000
0
DMSO
100nM Halo
DMSO
100nM Halo
sgControl pool
sgGCN2 pool

FIG. 73

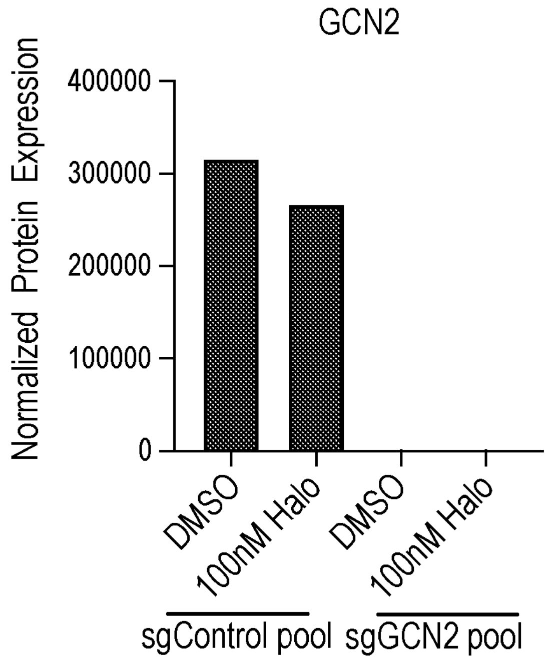
GCN2
Normalized Protein Expression
400000
300000
200000
100000
0
DMSO
100nM Halo
DMSO
100nM Halo
sgControl pool
sgGCN2 pool

FIG. 74

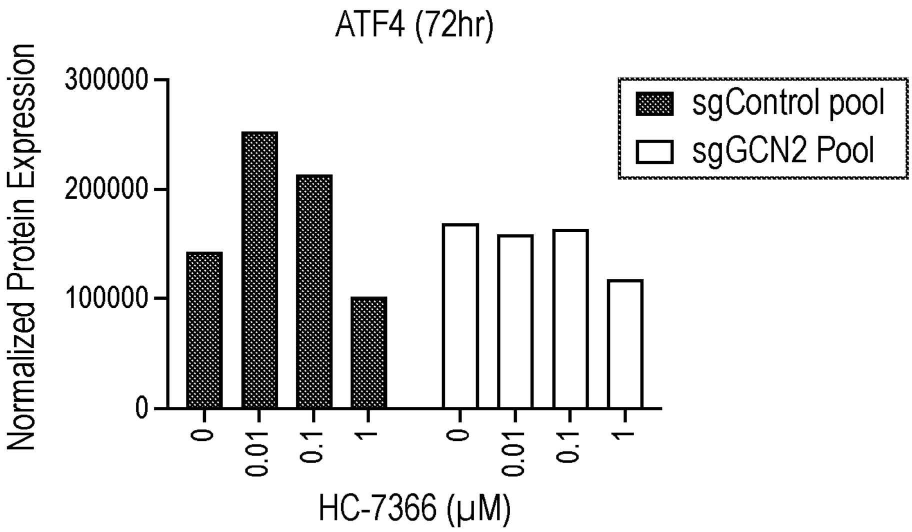
ATF4 (72hr)
Normalized Protein Expression
300000
200000
100000
0
sgControl pool
sgGCN2 Pool
0
0.01
0.1
1
0
0.01
0.1
1
HC-7366 (μM)

FIG. 75

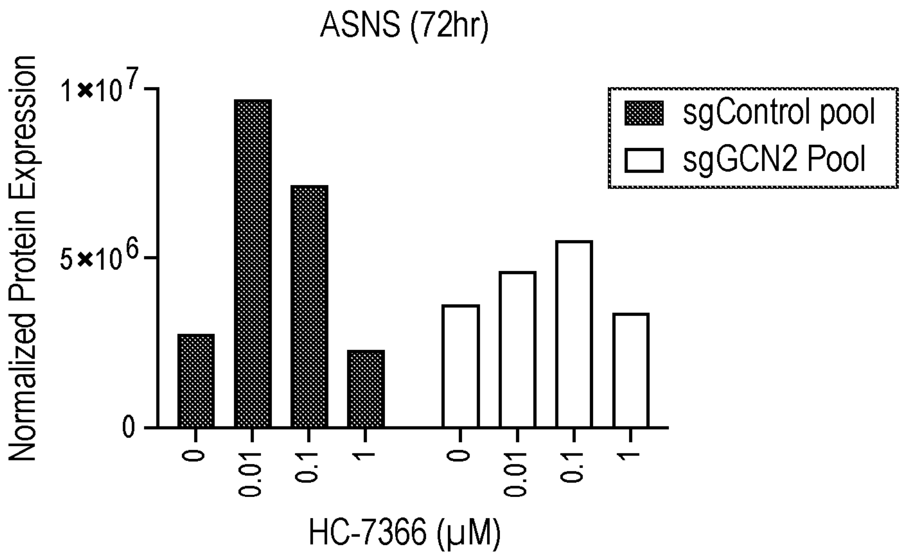
ASNS (72hr)
Normalized Protein Expression
1×10^7
5×10^6
0
sgControl pool
sgGCN2 Pool
0
0.01
0.1
1
0
0.01
0.1
1
HC-7366 (μM)

FIG. 76

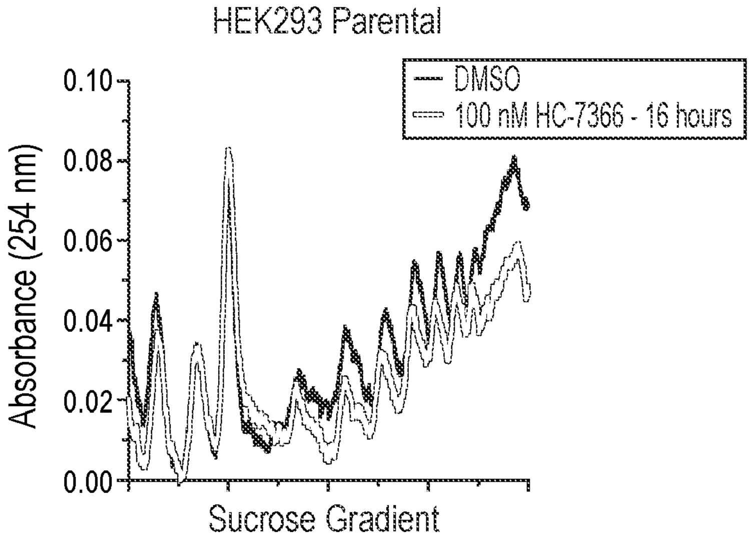


Polysome to monosome ratio
8.4
3.1

FIG. 77

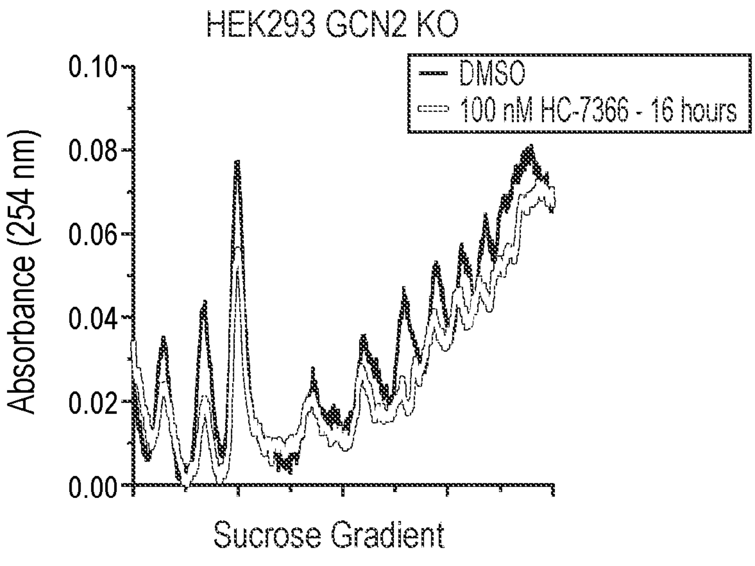


Polysome to monosome ratio
7.8
11.2

FIG. 78

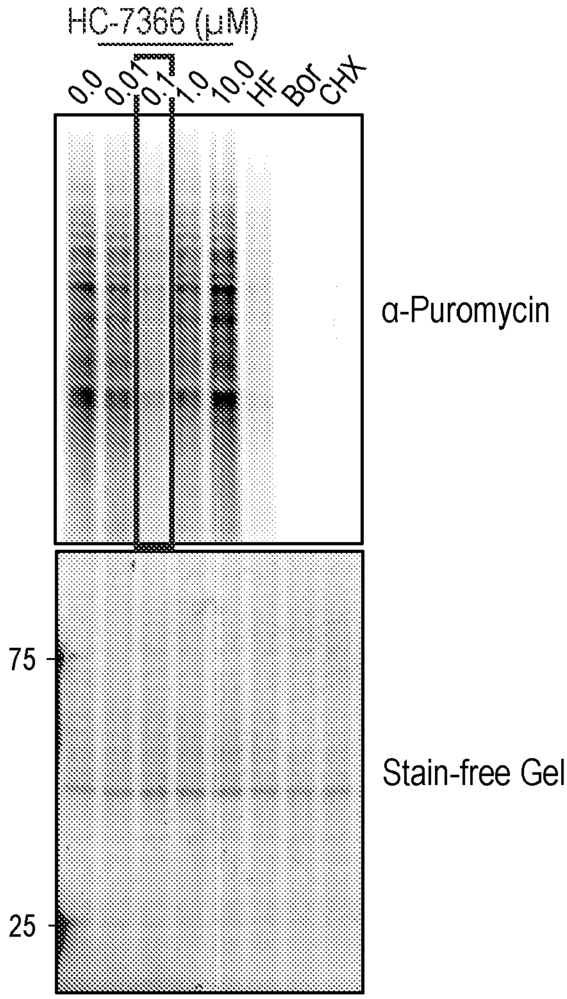
HC-7366 (μM)
0.0
0.01
0.1
1.0
10.0
HF
Bor
CHX
α-Puromycin
75
25
Stain-free Gel

FIG. 79

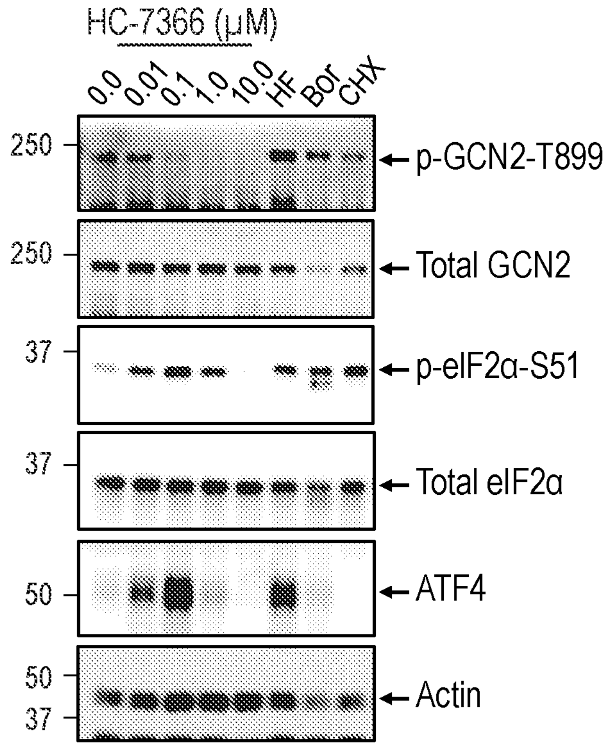


HC-7366; treatment for 6 hours
HF; 100 nM halofuginone for 6 hours
Bor; 1 µM borrelidin for 6 hours
CHX; 10 µg/ml for 2 hours
All samples pulsed with 0.5 µg/ml puromycin for 30 minutes

FIG. 80

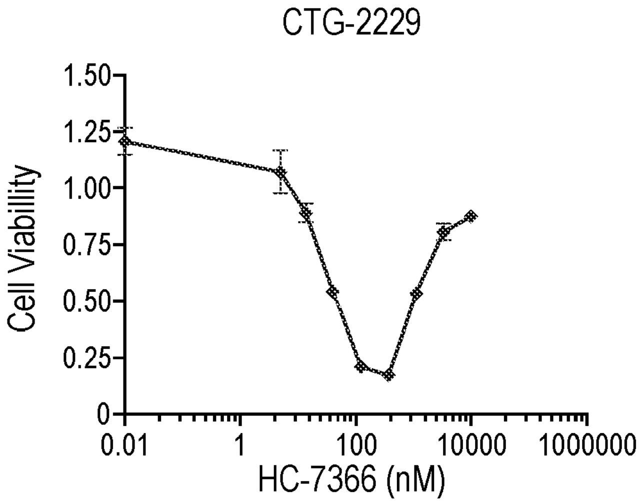


FIG. 81

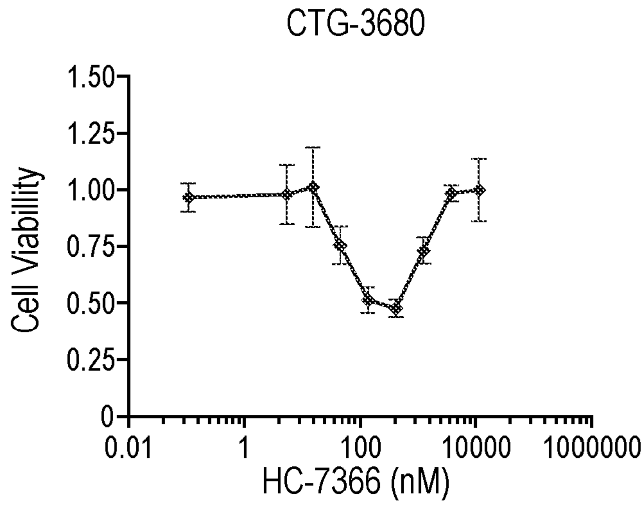
CTG-3680
Cell Viability
1.50
1.25
1.00
0.75
0.50
0.25
0
0.01
1
100
10000
1000000
HC-7366 (nM)

FIG. 82

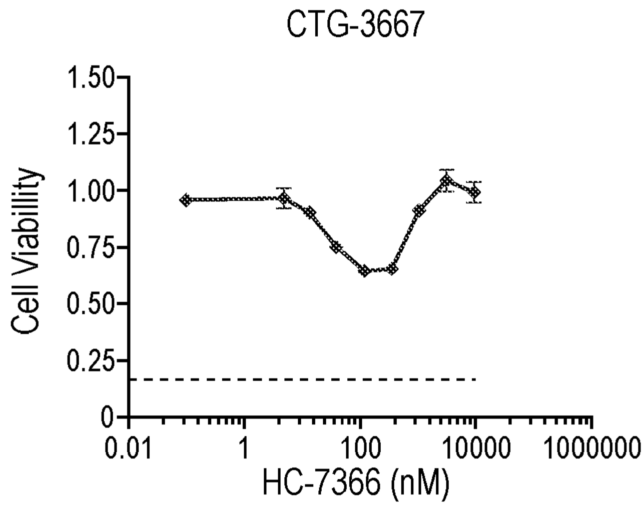
CTG-3667
Cell Viability
1.50
1.25
1.00
0.75
0.50
0.25
0
0.01
1
100
10000
1000000
HC-7366 (nM)

FIG. 83

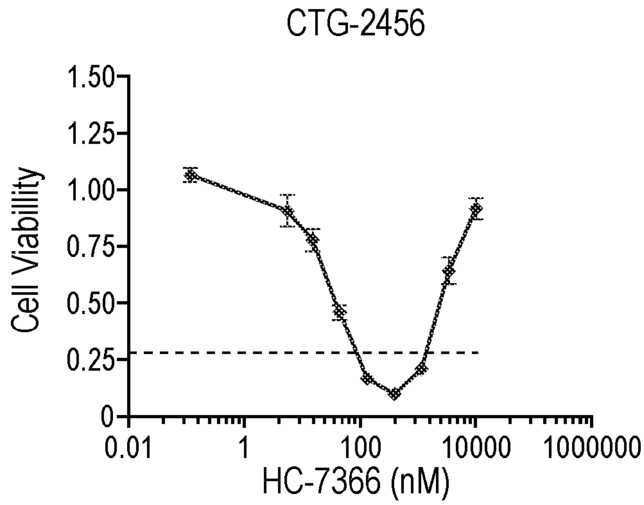
CTG-2456
1.50
1.25
1.00
0.75
0.50
0.25
0
Cell Viability
0.01
1
100
10000
1000000
HC-7366 (nM)

FIG. 84

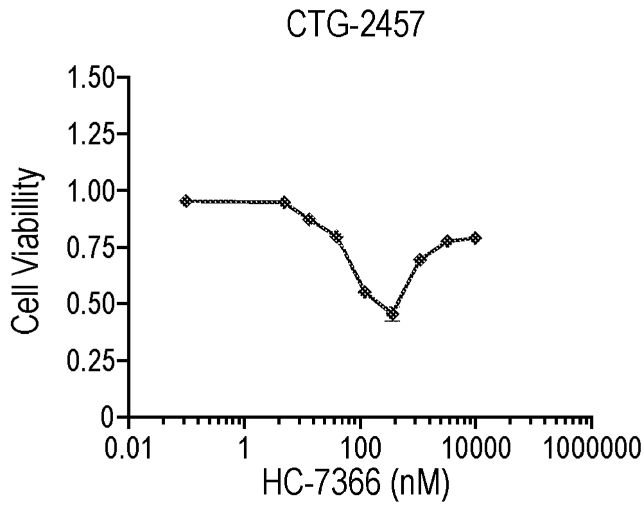
CTG-2457
1.50
1.25
1.00
0.75
0.50
0.25
0
Cell Viability
0.01
1
100
10000
1000000
HC-7366 (nM)

FIG. 85

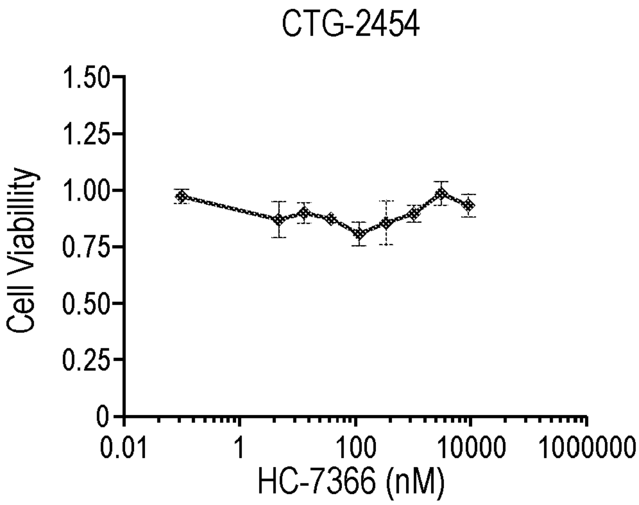
CTG-2454
Cell Viability
1.50
1.25
1.00
0.75
0.50
0.25
0
0.01
1
100
10000
1000000
HC-7366 (nM)

FIG. 86

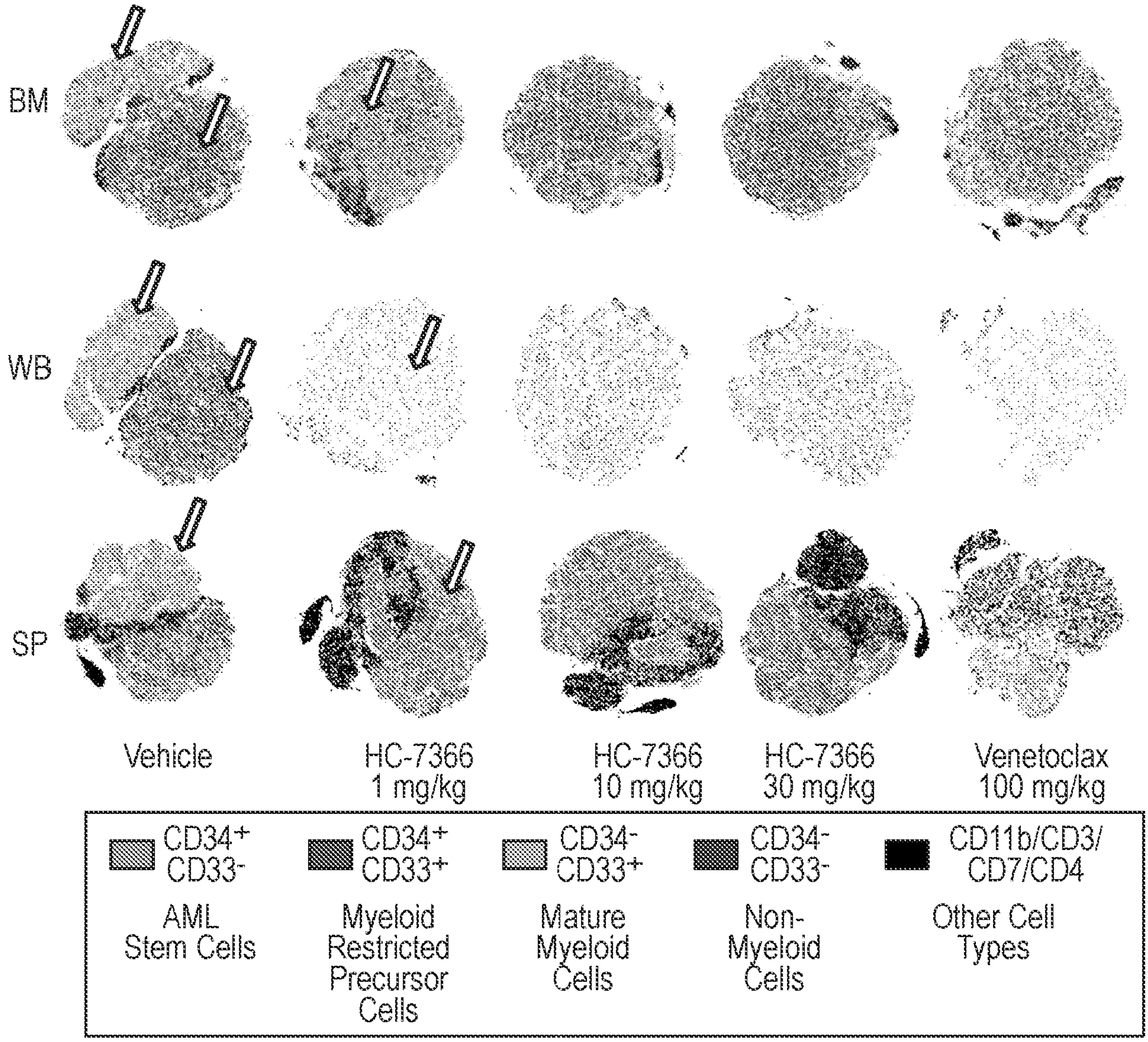
BM
WB
SP
Vehicle
HC-7366 1 mg/kg
HC-7366 10 mg/kg
HC-7366 30 mg/kg
Venetoclax 100 mg/kg
CD34+ CD33- AML Stem Cells
CD34+ CD33+ Myeloid Restricted Precursor Cells
CD34- CD33+ Mature Myeloid Cells
CD34- CD33- Non-Myeloid Cells
CD11b/CD3/CD7/CD4 Other Cell Types

FIG. 87

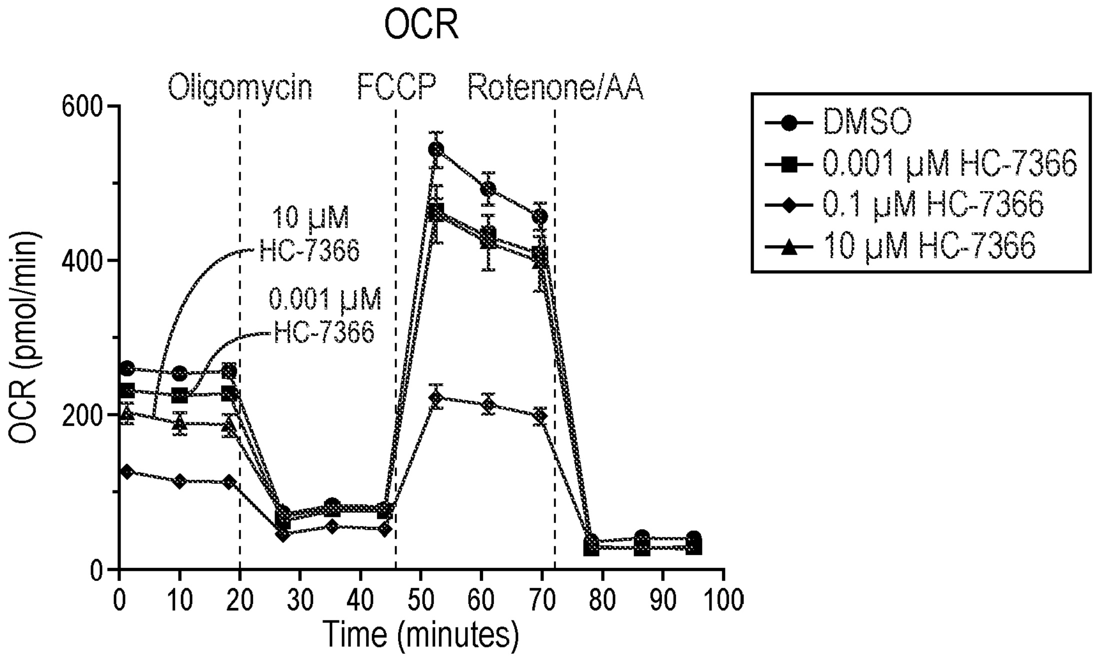

OCR
Oligomycin
FCCP
Rotenone/AA
600
400
200
0
OCR (pmol/min)
10 µM
HC-7366
0.001 µM
HC-7366
0
10
20
30
40
50
60
70
80
90
100
Time (minutes)
DMSO
0.001 µM HC-7366
0.1 µM HC-7366
10 µM HC-7366


FIG. 88

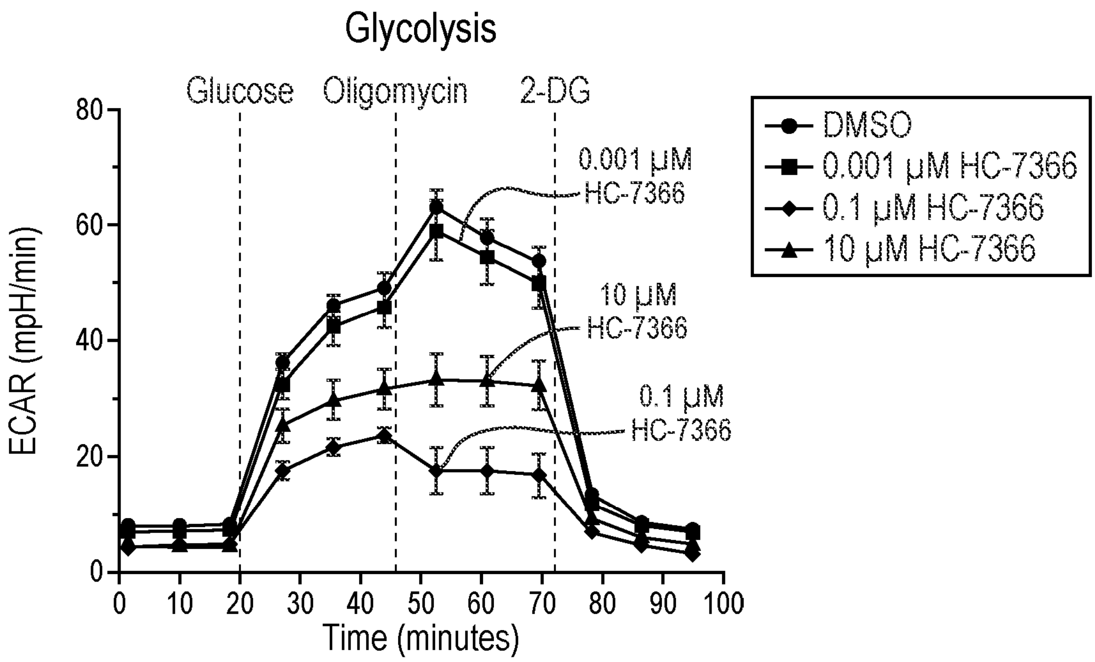

Glycolysis
Glucose
Oligomycin
2-DG
80
60
40
20
0
ECAR (mpH/min)
0.001 µM
HC-7366
10 µM
HC-7366
0.1 µM
HC-7366
0
10
20
30
40
50
60
70
80
90
100
Time (minutes)
DMSO
0.001 µM HC-7366
0.1 µM HC-7366
10 µM HC-7366


FIG. 89

Amino acids

| | | Fold of Change | | | | |
|---|---|---|---|---|---|---|
| | | Welch's Two-Sample t-Test | | | | |
| Sub Pathway | Biochemical Name | 0.3mg/kg / Vehicle | 1mg/kg / Vehicle | 3mg/kg / Vehicle | 10mg/kg / Vehicle | 30mg/kg / Vehicle |
| Free Amino Acids | alanine | 0.83 | 0.83 | 0.92 | 0.92 | 0.94 |
| | asparagine | 1.19 | 1.38 | 1.55 | 1.38 | 1.47 |
| | aspartate | 0.87 | 0.81 | 0.76 | 0.88 | 0.98 |
| | cysteine | 0.78 | 0.7 | 0.68 | 0.87 | 0.91 |
| | glutamine | 1.22 | 1.45 | 1.52 | 1.78 | 1.81 |
| | methionine | 0.76 | 0.93 | 1.05 | 0.78 | 0.85 |
| | serine | 1.56 | 1.91 | 2.07 | 1.77 | 1.71 |
| | theronine | 0.8 | 0.87 | 0.94 | 0.96 | 0.99 |
| | tryptophan | 1.06 | 1.38 | 1.5 | 1.18 | 1.22 |
| | tyrosine | 1.4 | 1.65 | 1.88 | 1.47 | 1.55 |
| | valine | 1.29 | 1.53 | 1.68 | 1.52 | 1.51 |
| | arginine | 1.16 | 1.35 | 1.44 | 1.37 | 1.29 |
| | glutamate | 0.96 | 0.96 | 0.95 | 0.98 | 1.01 |
| | glycine | 1.33 | 1.53 | 1.52 | 1.5 | 1.44 |
| | histidine | 1.04 | 1.11 | 1.16 | 1.14 | 1.23 |
| | isoleucine | 1.22 | 1.36 | 1.43 | 1.36 | 1.41 |
| | leucine | 1.12 | 1.31 | 1.39 | 1.22 | 1.26 |
| | lysine | 0.96 | 1.11 | 1.18 | 1.01 | 0.97 |
| | phenylalanine | 1.21 | 1.48 | 1.64 | 1.31 | 1.34 |
| | proline | 0.93 | 1.05 | 1.19 | 1.13 | 1.21 |

statistically significant decrease (p ≤ 0.05)

trending lower (0.05 < p < 0.10)

trending higher (0.05 < p < 0.10)

statistically significant increase (p ≤ 0.05)

FIG. 90

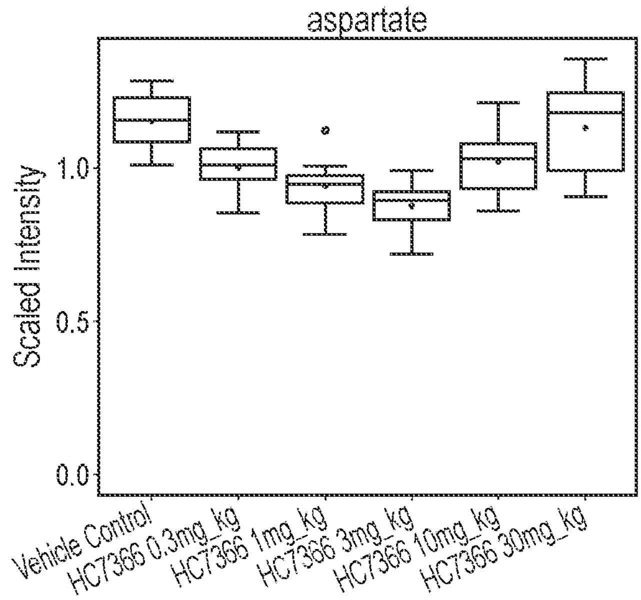


FIG. 91

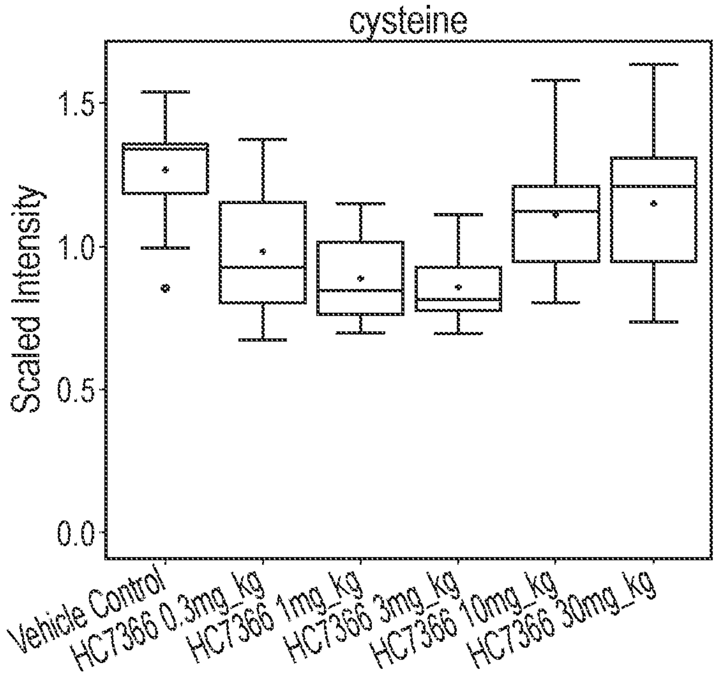
cysteine
Scaled Intensity
1.5
1.0
0.5
0.0
Vehicle Control
HC7366 0.3mg_kg
HC7366 1mg_kg
HC7366 3mg_kg
HC7366 10mg_kg
HC7366 30mg_kg

FIG. 92

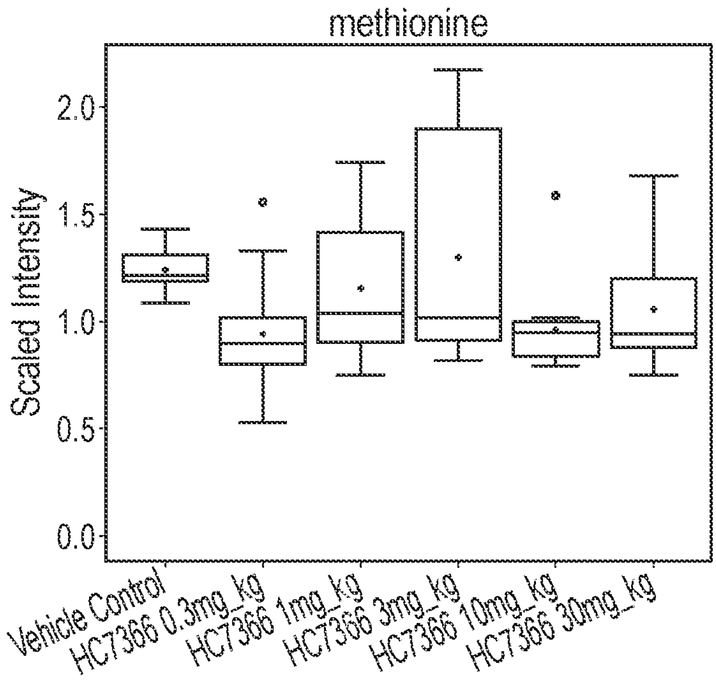
methionine
Scaled Intensity
2.0
1.5
1.0
0.5
0.0
Vehicle Control
HC7366 0.3mg_kg
HC7366 1mg_kg
HC7366 3mg_kg
HC7366 10mg_kg
HC7366 30mg_kg

FIG. 93

Oxidative stress

| | | Fold of Change | | | | |
|---|---|---|---|---|---|---|
| | | Welch's Two-Sample t-Test | | | | |
| Sub Pathway | Biochemical Name | 0.3mg/kg / Vehicle | 1mg/kg / Vehicle | 3mg/kg / Vehicle | 10mg/kg / Vehicle | 30mg/kg / Vehicle |
| Cysteine Metabolism | methionine | 0.76 | 0.93 | 1.05 | 0.78 | 0.85 |
| | S-adenosylmethionine (SAM) | 0.76 | 0.63 | 0.61 | 0.93 | 0.9 |
| | S-adenosylhomocysteine (SAH) | 0.81 | 0.78 | 0.7 | 0.72 | 0.81 |
| | cystathionine | 1.59 | 1.26 | 1.25 | 2.32 | 2.06 |
| | cysteine | 0.78 | 0.7 | 0.68 | 0.87 | 0.91 |
| Glutathione Metabolism | glutathione, reduced (GSH) | 0.06 | 0.04 | 0.02 | 0.06 | 0.1 |
| | glutathione, oxidized (GSSG) | 0.94 | 0.87 | 0.77 | 0.78 | 0.78 |
| | cysteinylglycine | 0.27 | 0.2 | 0.15 | 0.22 | 0.24 |
| | 5-oxoproline | 1.11 | 1.22 | 1.3 | 1.22 | 1.51 |
| | ophthalmate | 1.41 | 2.52 | 2.6 | 1.99 | 1.69 |
| Gamma-glutamyl Amino Acid | gamma-glutamylalanine | 0.42 | 0.33 | 0.39 | 0.48 | 0.79 |
| | gamma-glutamylglutamate | 0.86 | 0.86 | 0.78 | 0.76 | 0.89 |
| | gamma-glutamylglutamine | 0.8 | 0.63 | 0.69 | 1.03 | 1.23 |
| | gamma-glutamylglycine | 1.36 | 1.68 | 1.76 | 1.45 | 1.59 |
| | gamma-glutamylhistidine | 1.09 | 1.2 | 1.54 | 1.34 | 1.48 |
| | gamma-glutamylisoleucine* | 1.39 | 1.62 | 2.14 | 1.66 | 1.76 |
| | gamma-glutamylleucine | 1.17 | 1.45 | 1.93 | 1.32 | 1.44 |
| | gamma-glutamyl-epsilon-lysine | 1.16 | 2.04 | 2.71 | 1.97 | 1.72 |
| | gamma-glutamylphenylalanine | 1.09 | 1.42 | 2.03 | 1.37 | 1.49 |
| | gamma-glutamyltyrosine | 1.13 | 1.64 | 1.68 | 1.6 | 1.67 |
| | gamma-glutamylvaline | 1.58 | 1.8 | 2.35 | 2.03 | 2.06 |
| | gamma-glutamylserine | 1.35 | 1.65 | 1.98 | 1.31 | 1.47 |

statistically significant decrease ($p \leq 0.05$)
trending lower ($0.05 < p < 0.10$)
trending higher ($0.05 < p < 0.10$)
statistically significant increase ($p \leq 0.05$)

FIG. 94

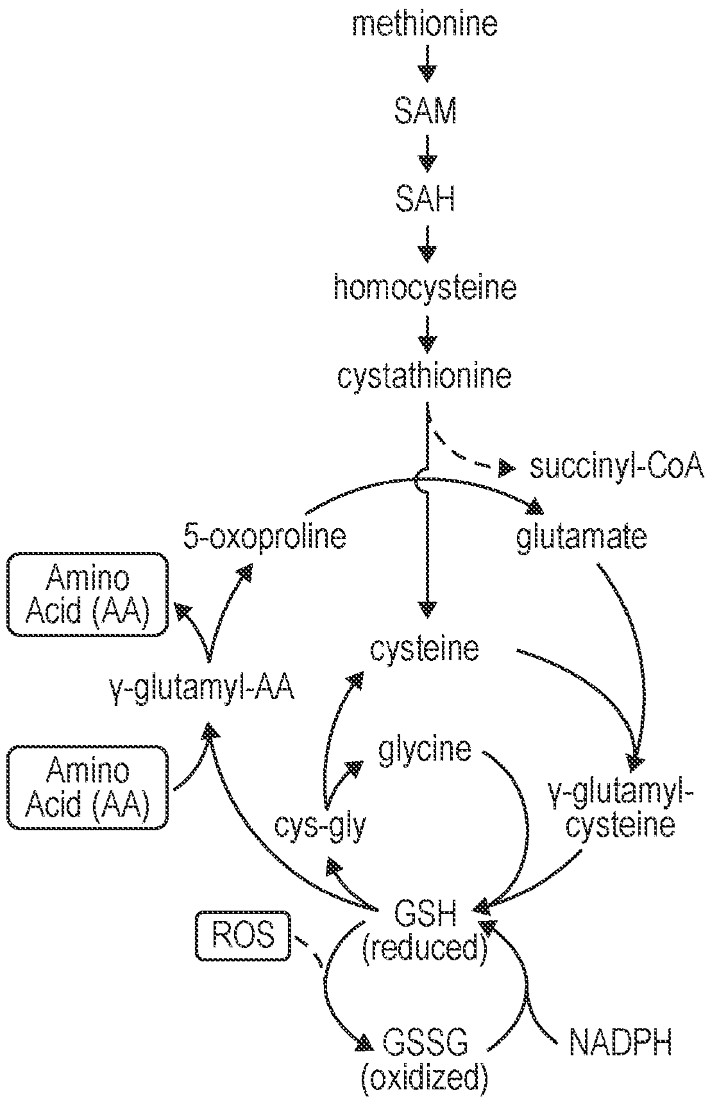
methionine
SAM
SAH
homocysteine
cystathionine
succinyl-CoA
5-oxoproline
glutamate
Amino Acid (AA)
cysteine
γ-glutamyl-AA
glycine
Amino Acid (AA)
γ-glutamyl-cysteine
cys-gly
GSH (reduced)
ROS
GSSG (oxidized)
NADPH

FIG. 95

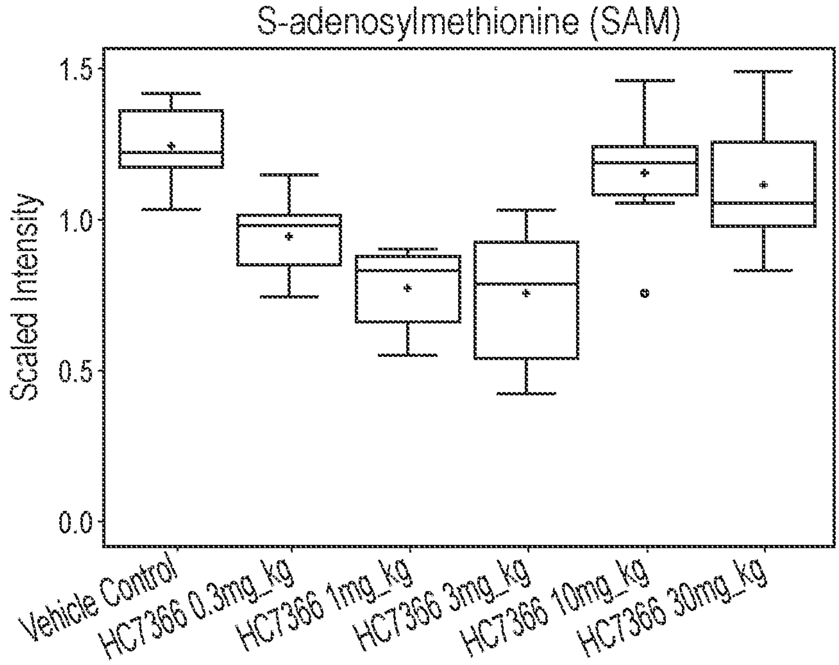
S-adenosylmethionine (SAM)
Scaled Intensity
1.5
1.0
0.5
0.0
Vehicle Control
HC7366 0.3mg_kg
HC7366 1mg_kg
HC7366 3mg_kg
HC7366 10mg_kg
HC7366 30mg_kg

FIG. 96

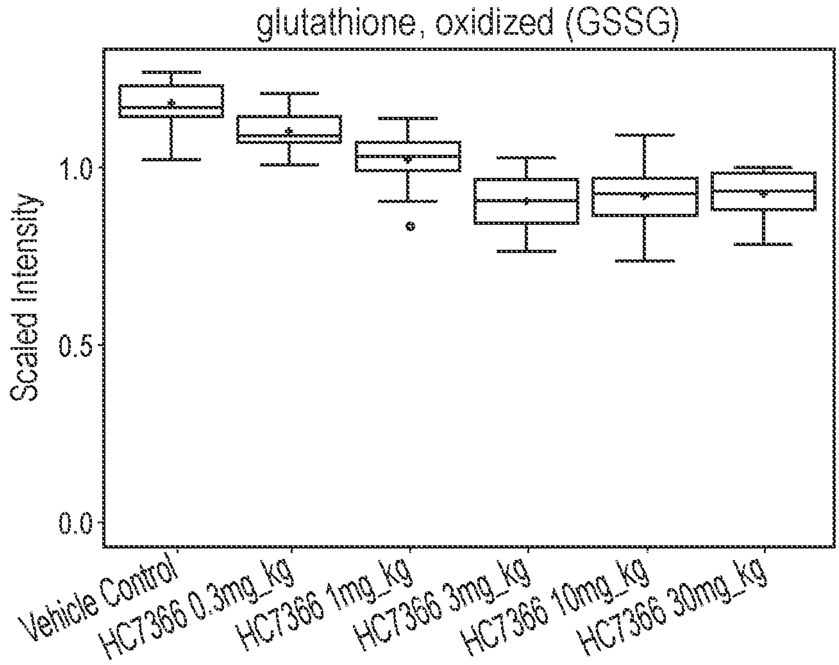
glutathione, oxidized (GSSG)
Scaled Intensity
1.0
0.5
0.0
Vehicle Control
HC7366 0.3mg_kg
HC7366 1mg_kg
HC7366 3mg_kg
HC7366 10mg_kg
HC7366 30mg_kg

FIG. 97

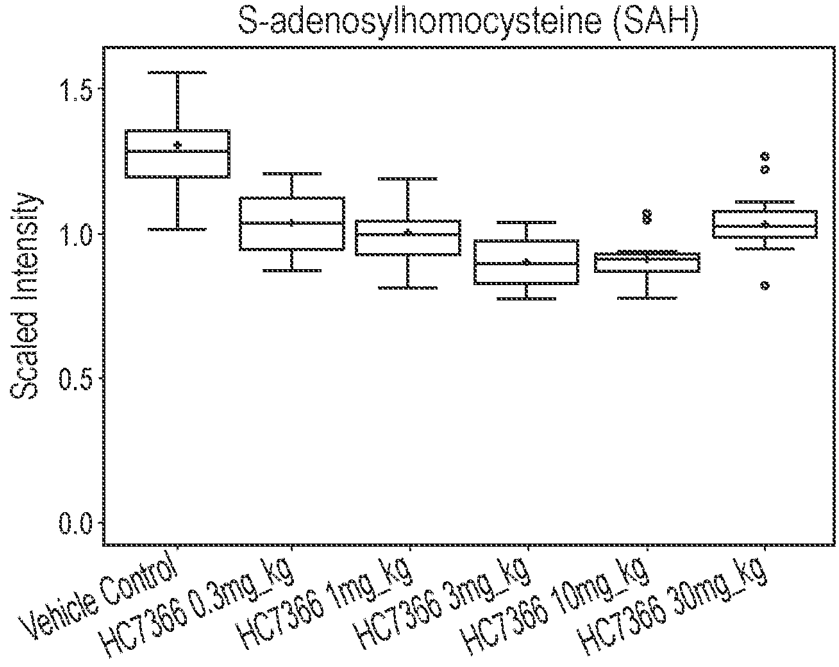
S-adenosylhomocysteine (SAH)
Scaled Intensity
1.5
1.0
0.5
0.0
Vehicle Control
HC7366 0.3mg_kg
HC7366 1mg_kg
HC7366 3mg_kg
HC7366 10mg_kg
HC7366 30mg_kg

FIG. 98

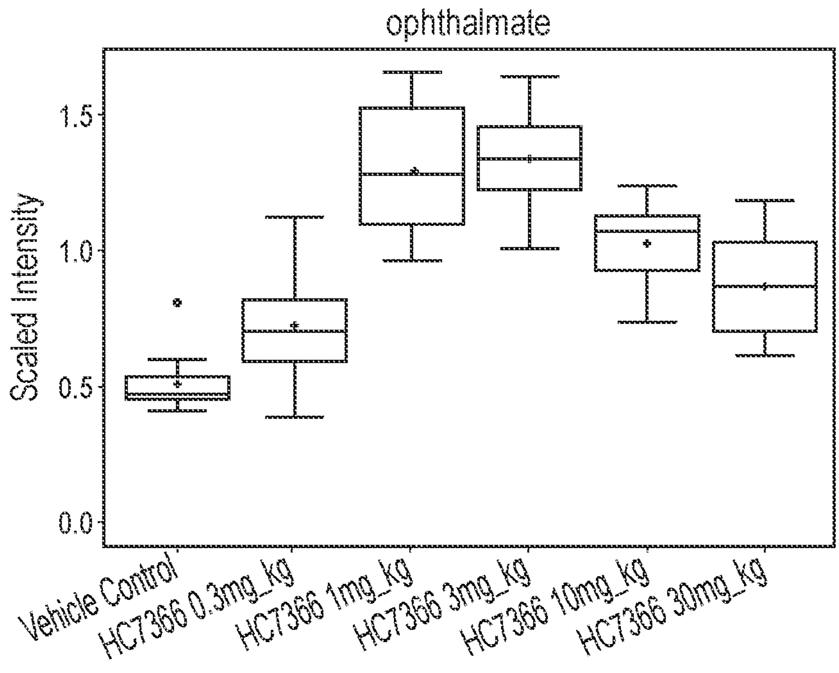
ophthalmate
Scaled Intensity
1.5
1.0
0.5
0.0
Vehicle Control
HC7366 0.3mg_kg
HC7366 1mg_kg
HC7366 3mg_kg
HC7366 10mg_kg
HC7366 30mg_kg

FIG. 99

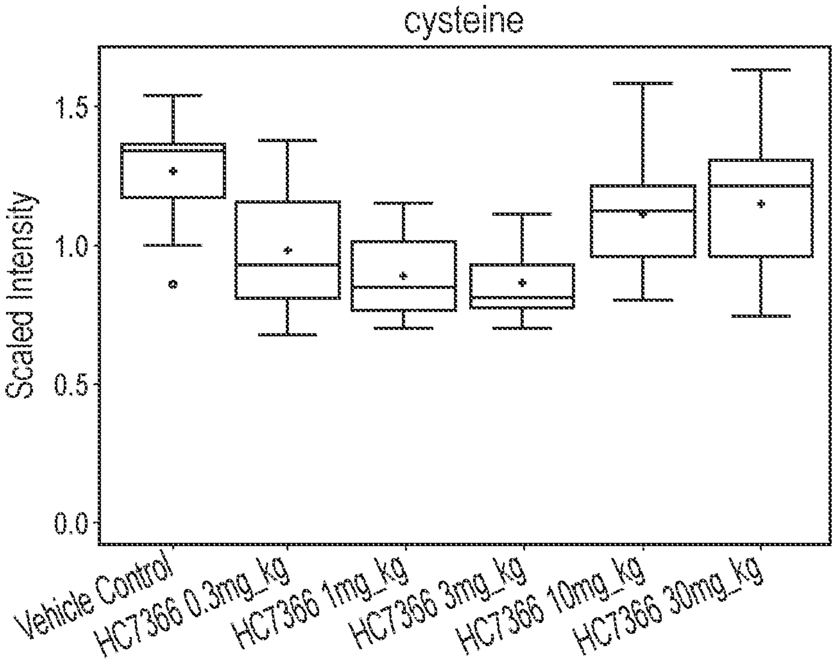


FIG. 100

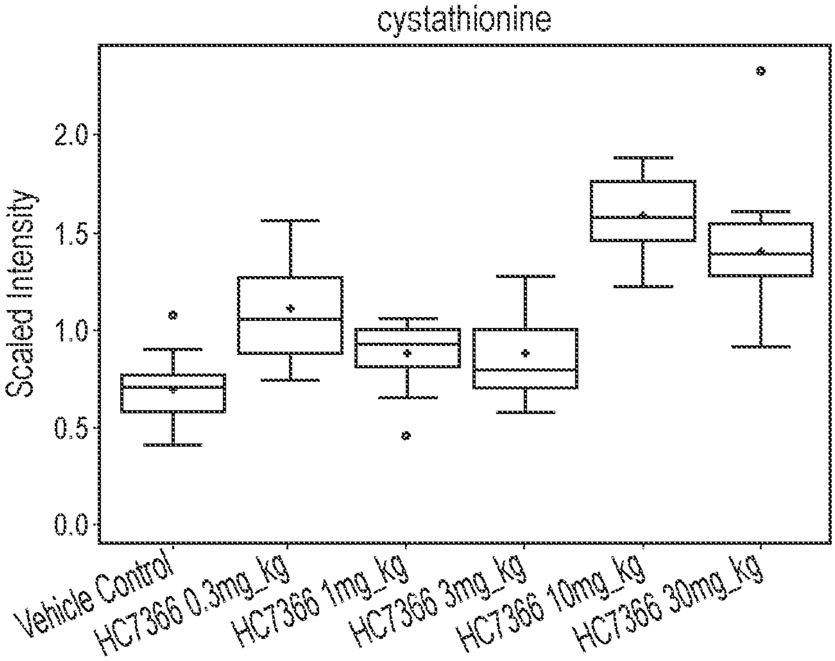


FIG. 101

Pyrimidine synthesis

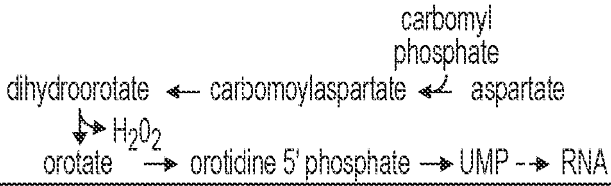


| | Fold of Change | | | | |
|---|---|---|---|---|---|
| | Welch's Two-Sample t-Test | | | | |
| Biochemical Name | 0.3mg/kg / Vehicle | 1mg/kg / Vehicle | 3mg/kg / Vehicle | 10mg/kg / Vehicle | 30mg/kg / Vehicle |
| aspartate | 0.87 | 0.81 | 0.76 | 0.88 | 0.98 |
| N-carbamoylaspartate | 0.32 | 0.22 | 0.21 | 0.22 | 0.32 |
| dihydroorotate | 0.29 | 0.12 | 0.07 | 0.13 | 0.26 |
| orotate | 0.70 | 0.48 | 0.29 | 0.32 | 0.53 |
| orotidine | 0.82 | 0.59 | 0.55 | 0.60 | 0.77 |
| uridine 5'-triphosphate (UTP) | 0.32 | 0.11 | 0.08 | 0.21 | 0.27 |
| uridine 5'-diphosphate (UDP) | 0.46 | 0.24 | 0.20 | 0.36 | 0.46 |
| uridine 5'-monophosphate (UMP) | 0.81 | 0.64 | 0.53 | 0.73 | 0.81 |
| uridine | 1.02 | 0.92 | 0.89 | 0.87 | 1.07 |

statistically significant decrease ($p \leq 0.05$)
trending lower ($0.05 < p < 0.10$)
trending higher ($0.05 < p < 0.10$)
statistically significant increase ($p \leq 0.05$)

FIG. 102

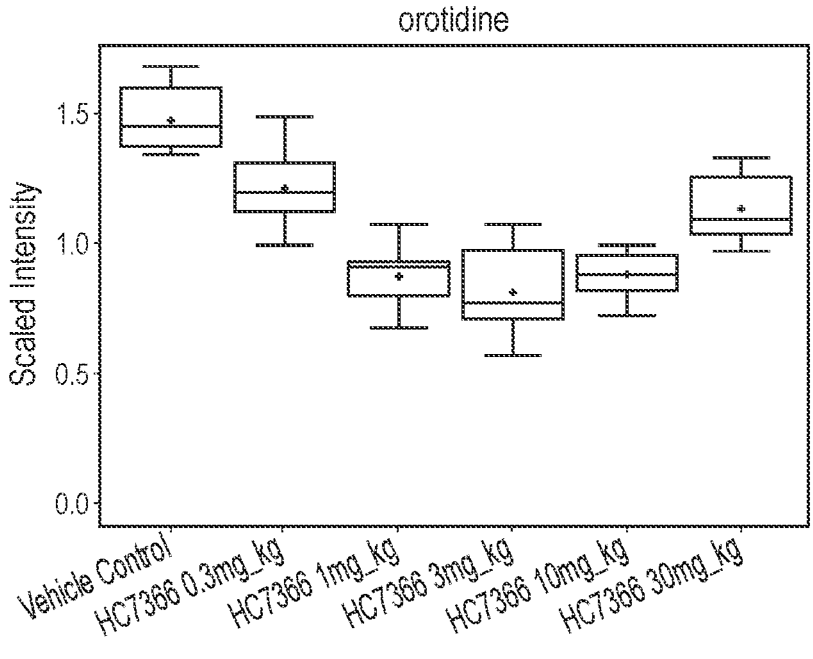


FIG. 103

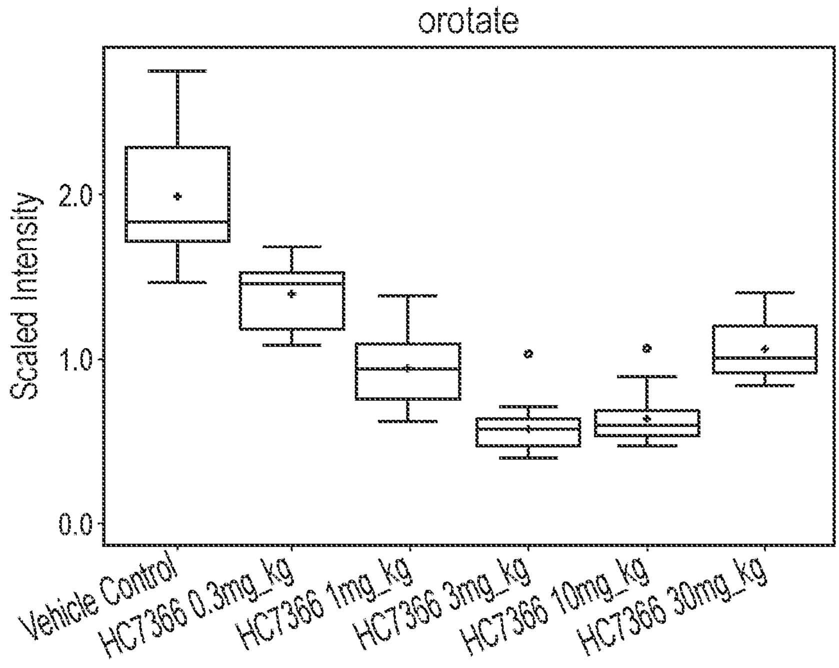


FIG. 104

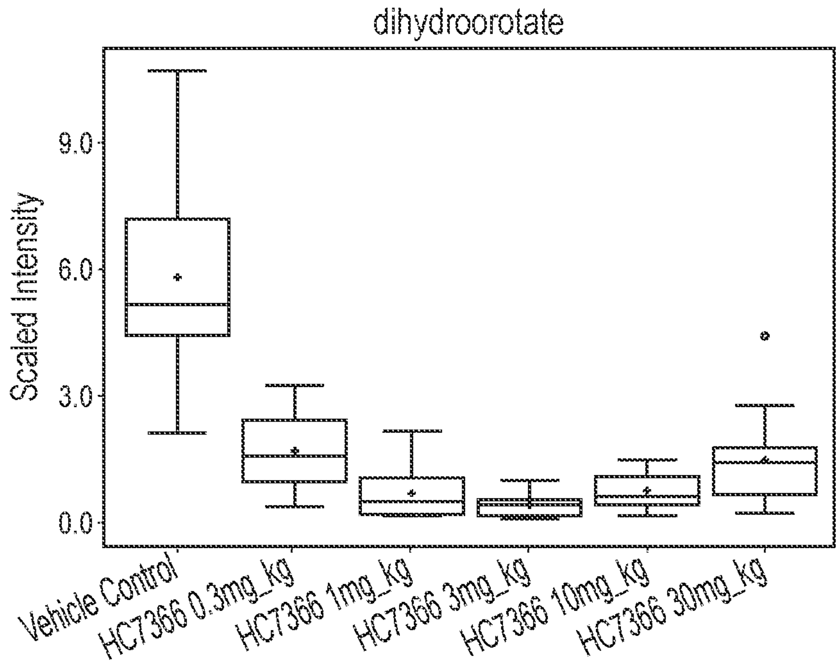


FIG. 105

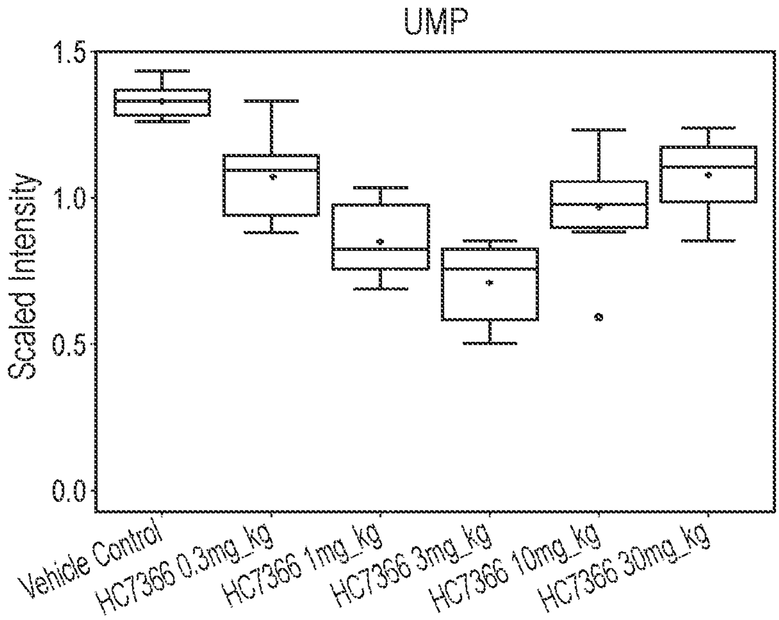


FIG. 106

Aspartate metabolism

| Biochemical Name | Fold of Change<br>Welch's Two-Sample t-Test<br>0.3mg/kg / Vehicle | 1mg/kg / Vehicle | 3mg/kg / Vehicle | 10mg/kg / Vehicle | 30mg/kg / Vehicle |
|---|---|---|---|---|---|
| aspartate | 0.87 | 0.81 | 0.76 | 0.88 | 0.98 |
| arginine | 1.16 | 1.35 | 1.44 | 1.37 | 1.29 |
| argininosuccinate | 1.43 | 1.77 | 2.04 | 1.46 | 1.76 |
| urea | 1.05 | 1.02 | 1.05 | 1.18 | 1.16 |
| ornithine | 1.45 | 1.88 | 1.78 | 1.33 | 1.39 |
| citrulline | 0.47 | 0.38 | 0.39 | 0.55 | 0.65 |
| N-monomethylarginine | 1.05 | 1.32 | 1.29 | 1.08 | 1.13 |
| fumarate | 1.20 | 1.13 | 1.15 | 1.02 | 1.21 |
| proline | 0.93 | 1.05 | 1.19 | 1.13 | 1.21 |
| putrescine | 0.96 | 0.64 | 0.56 | 0.81 | 1.08 |
| spermidine | 0.97 | 0.98 | 0.86 | 0.95 | 1.10 |
| spermine | 0.41 | 0.38 | 0.29 | 0.42 | 0.65 |
| 5-methylthioadenosine (MTA) | 0.78 | 0.66 | 0.62 | 0.93 | 0.90 |
| asparagine | 1.19 | 1.38 | 1.55 | 1.38 | 1.47 |

statistically significant decrease ($p \leq 0.05$) | trending lower ($0.05 < p < 0.10$) | trending higher ($0.05 < p < 0.10$) | statistically significant increase ($p \leq 0.05$)

FIG. 107

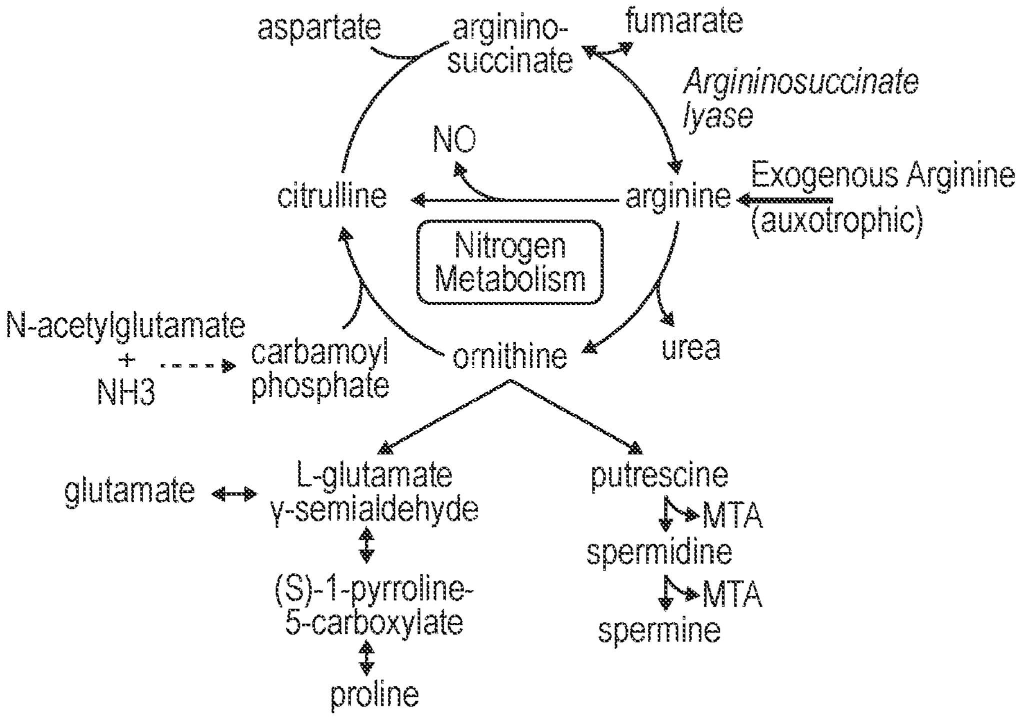


FIG. 108

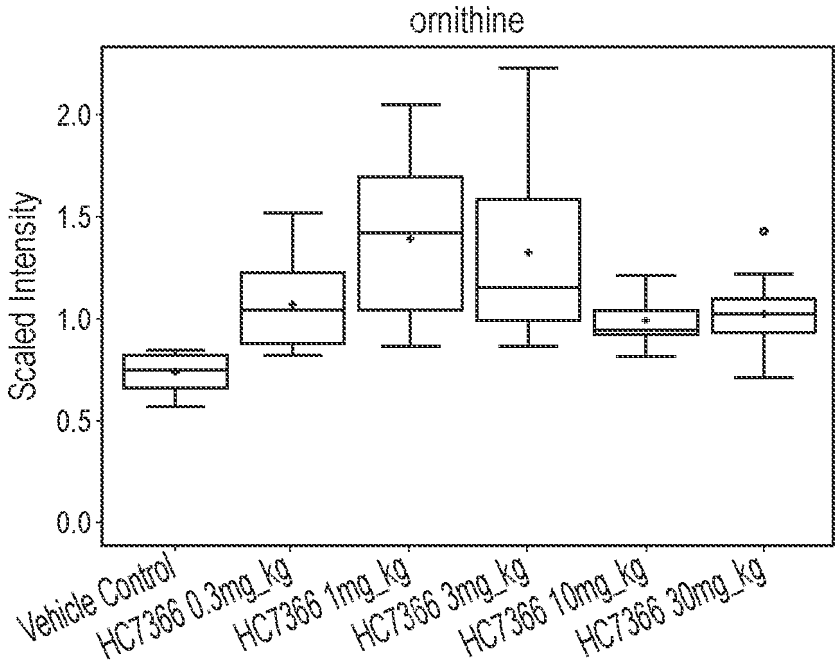


FIG. 109

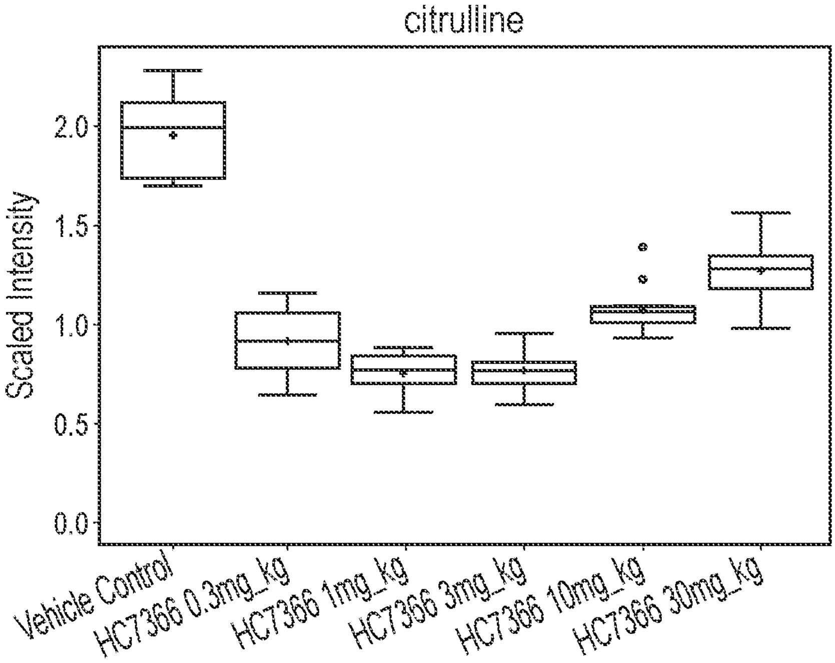


FIG. 110

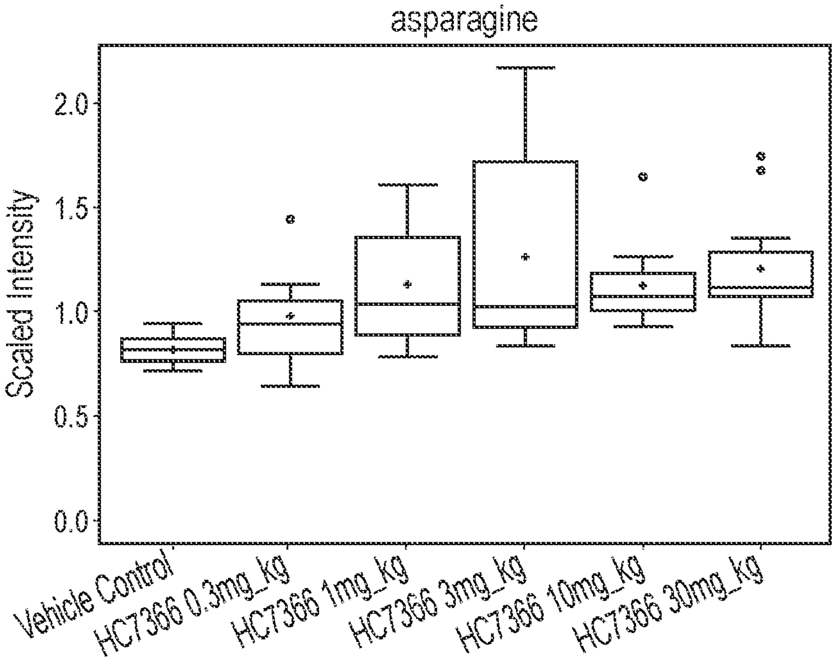


FIG. 111

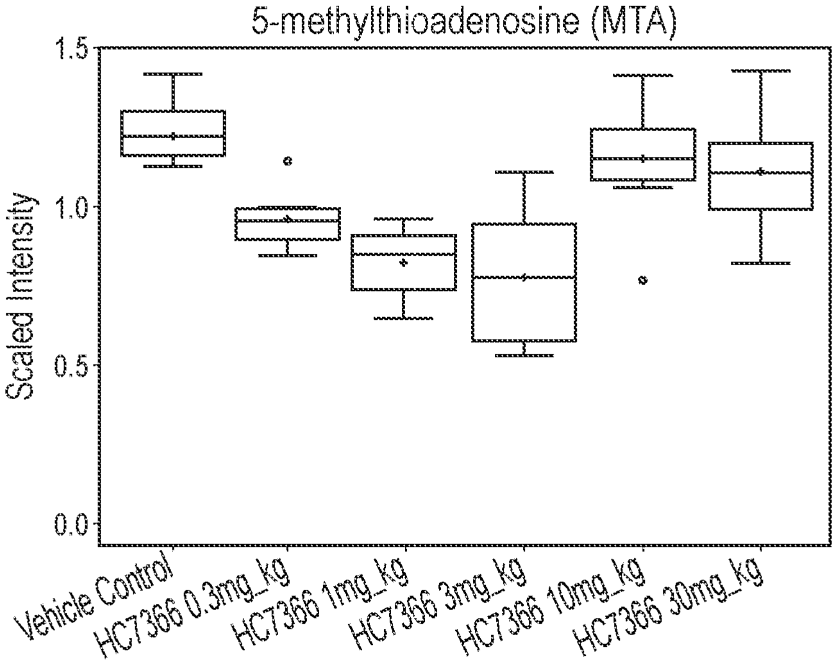


FIG. 112

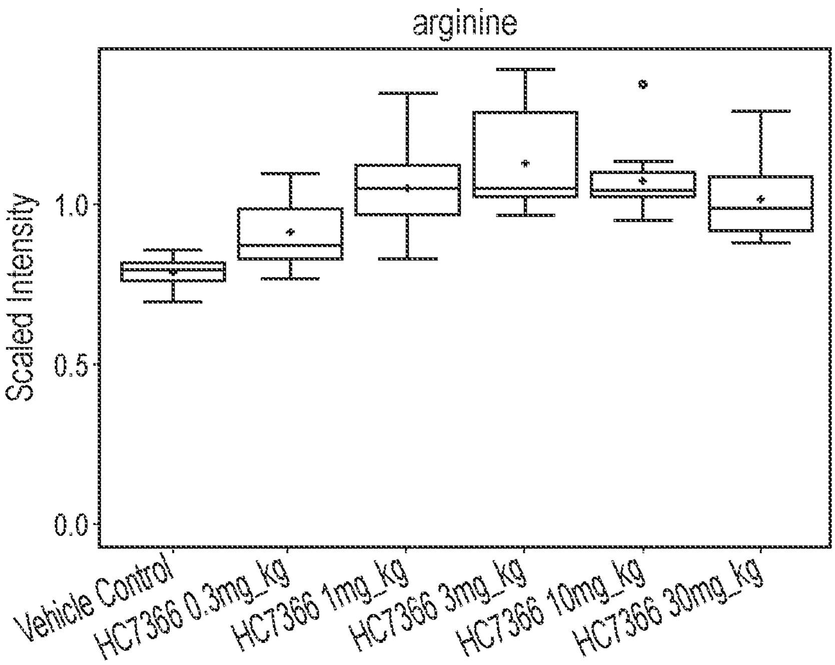


FIG. 113

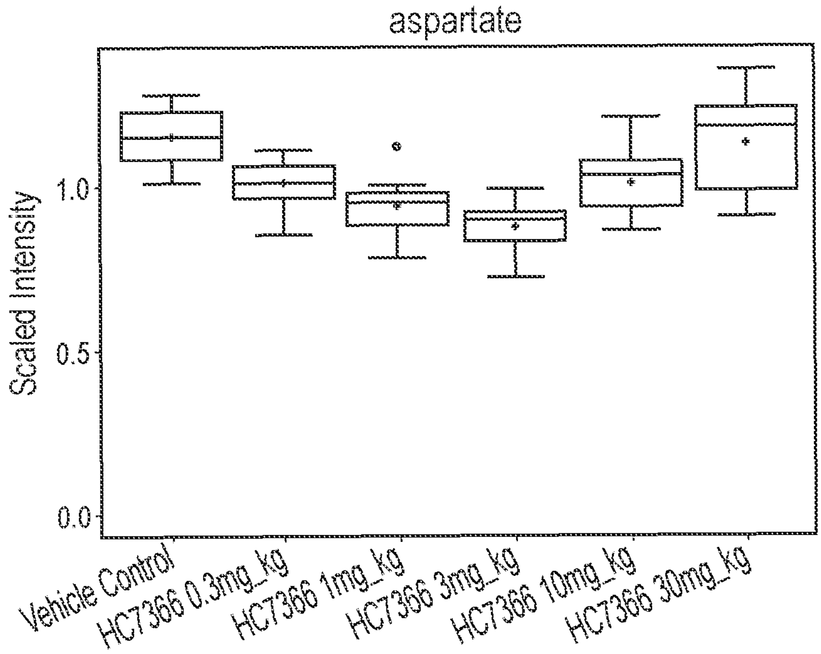


FIG. 114

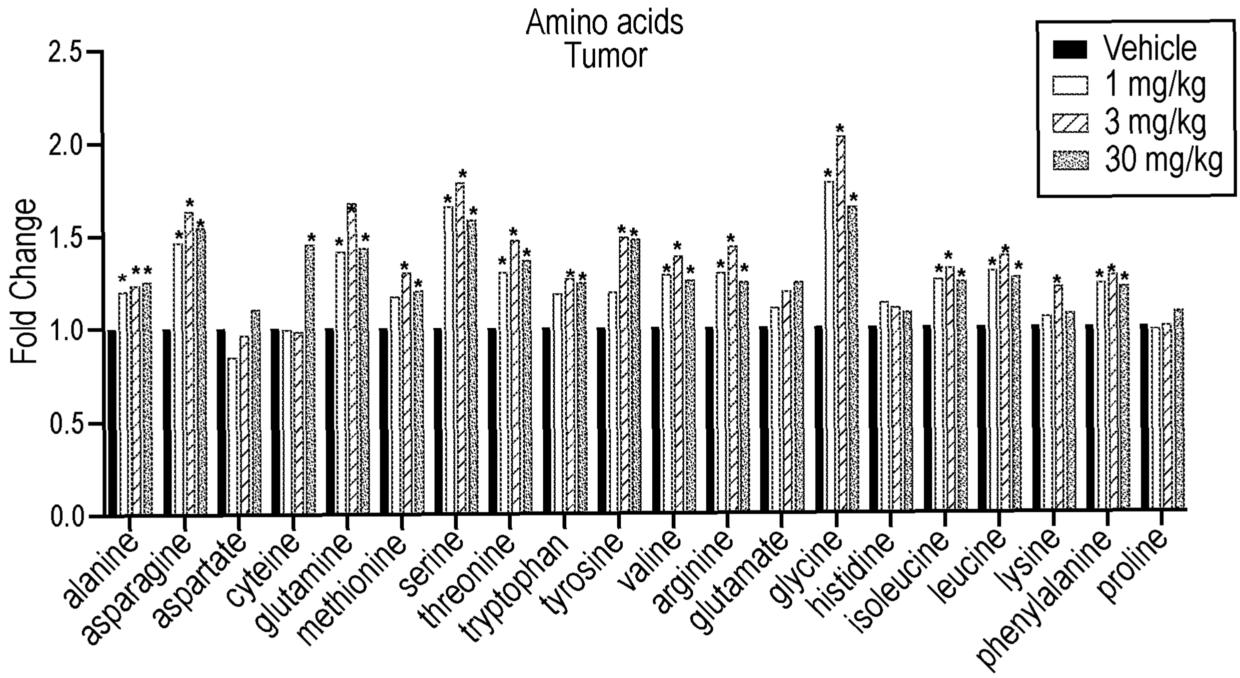


FIG. 115

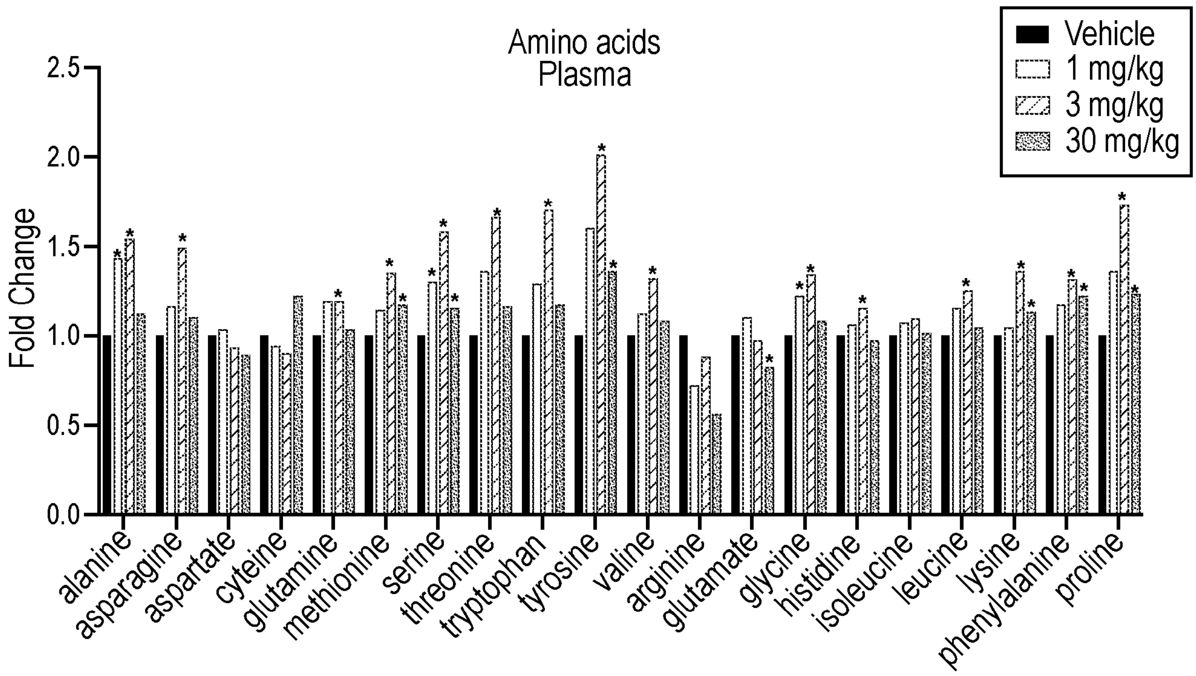


FIG. 116

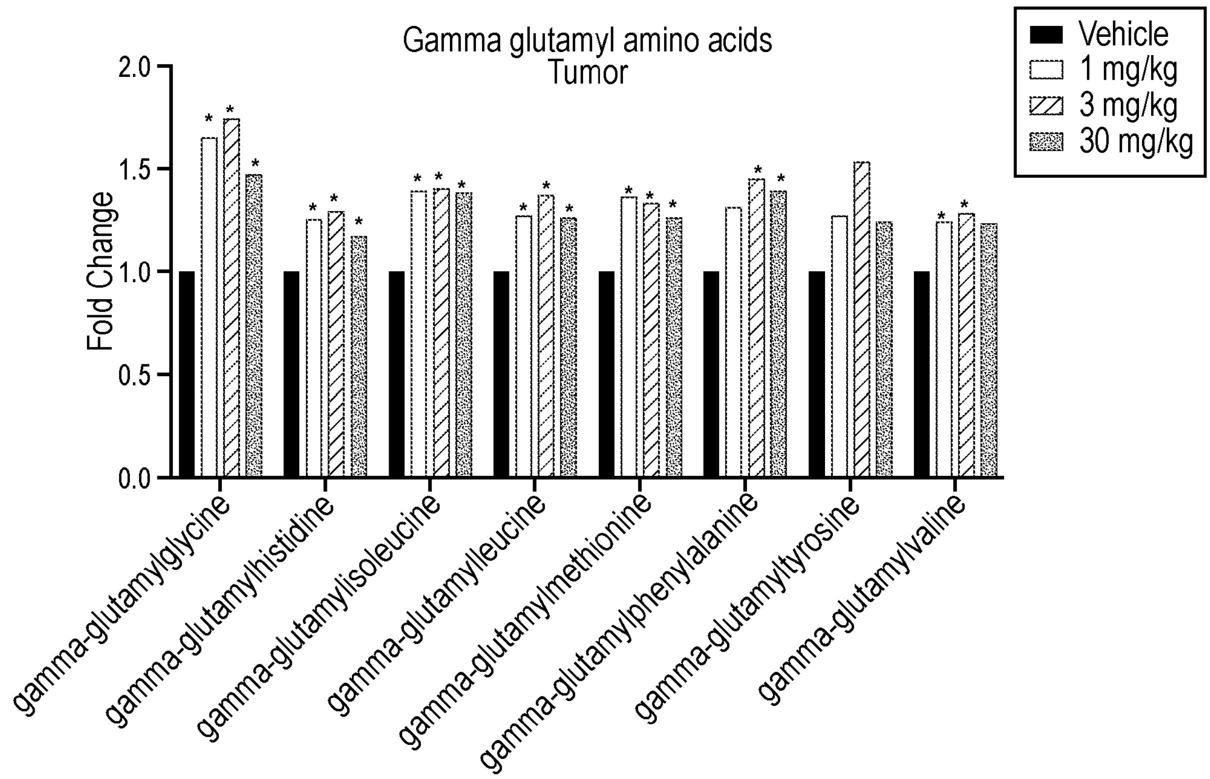


FIG. 117

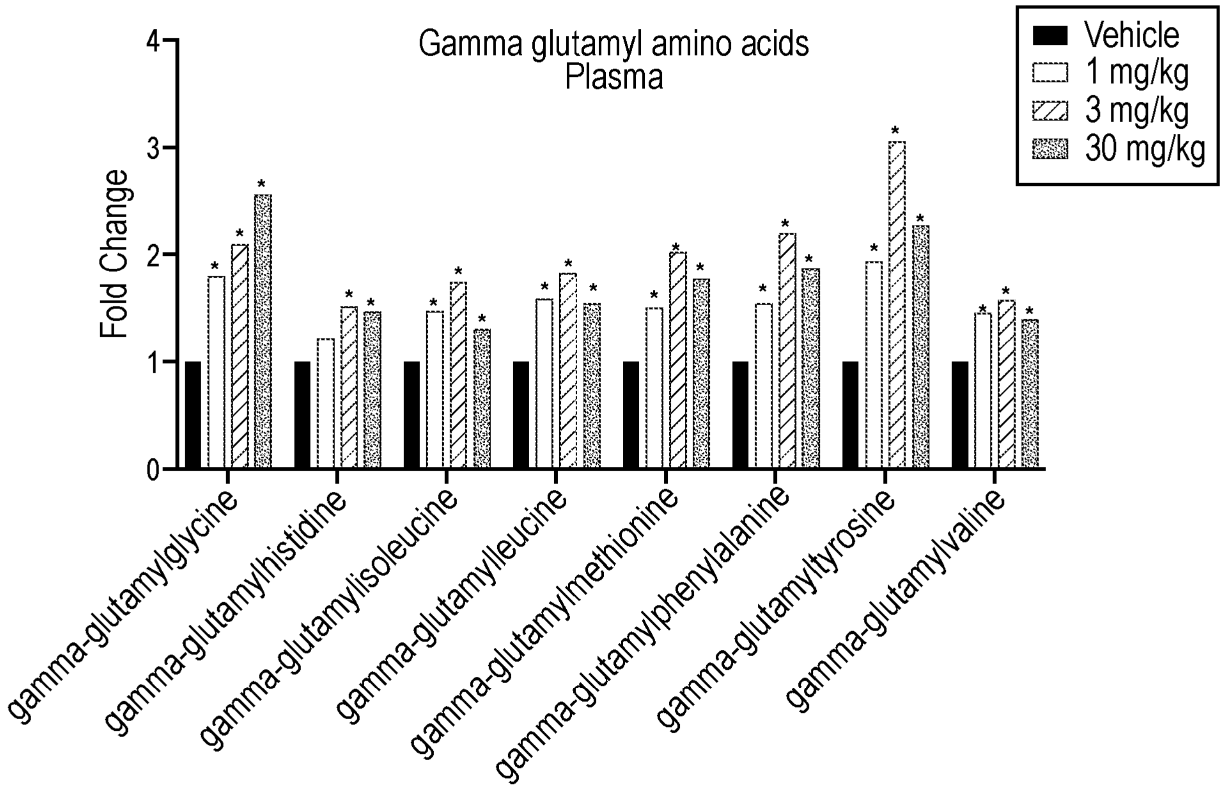


FIG. 118

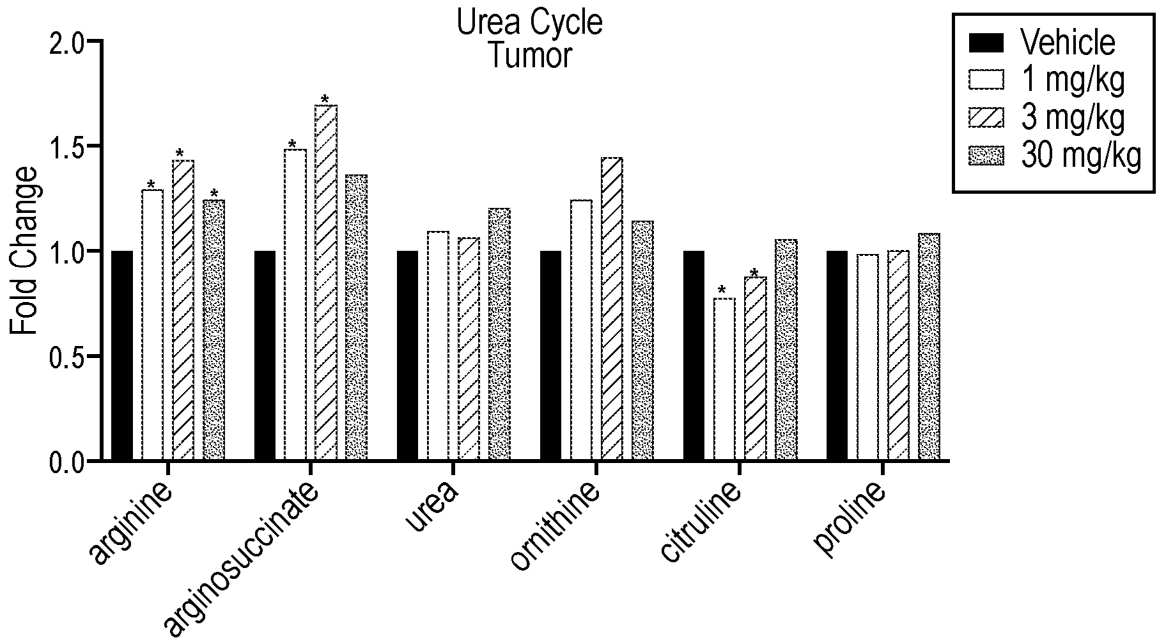


FIG. 119

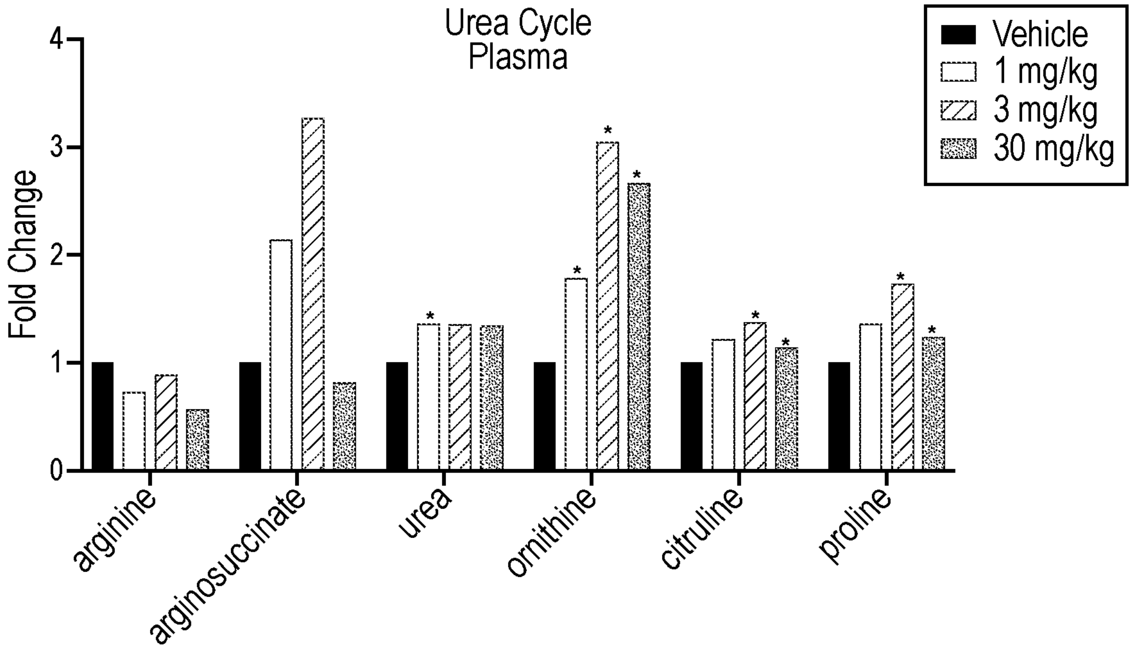


FIG. 120

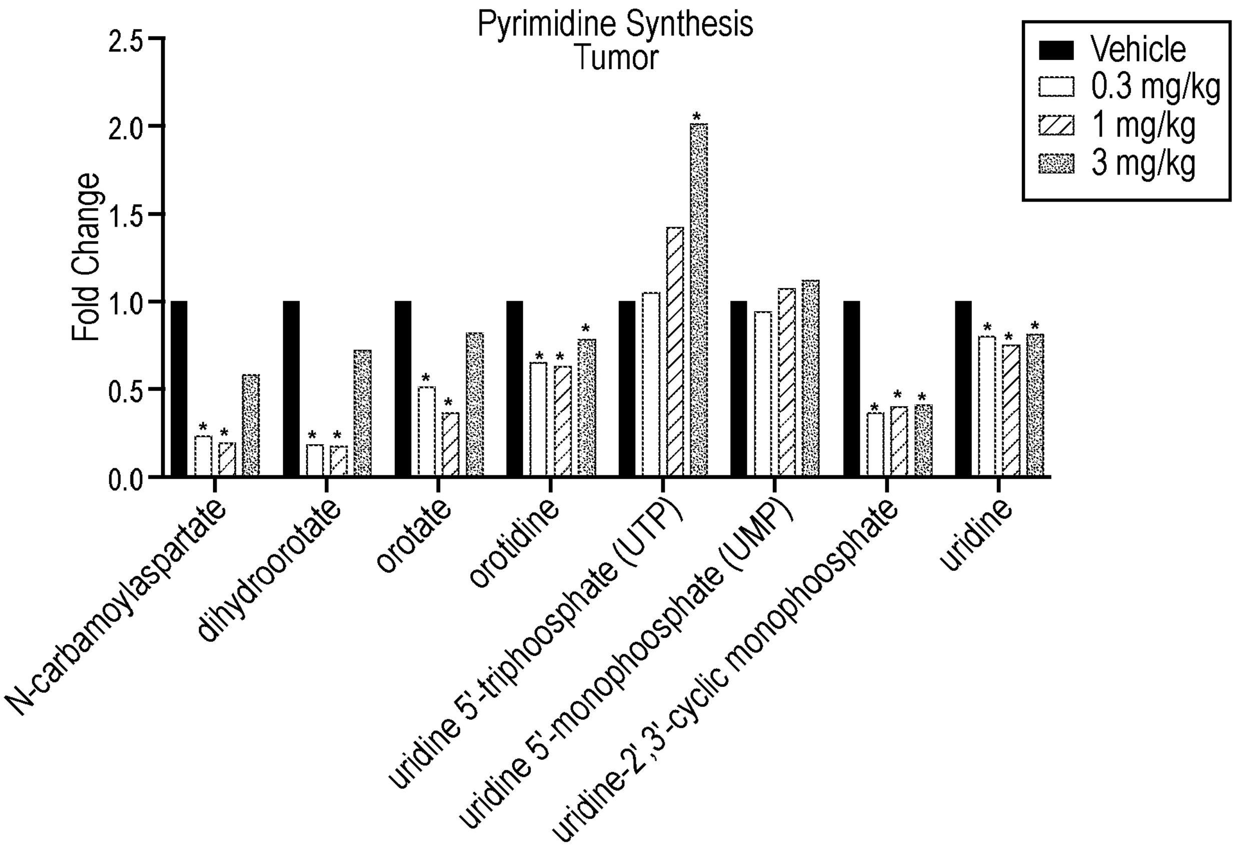


FIG. 121

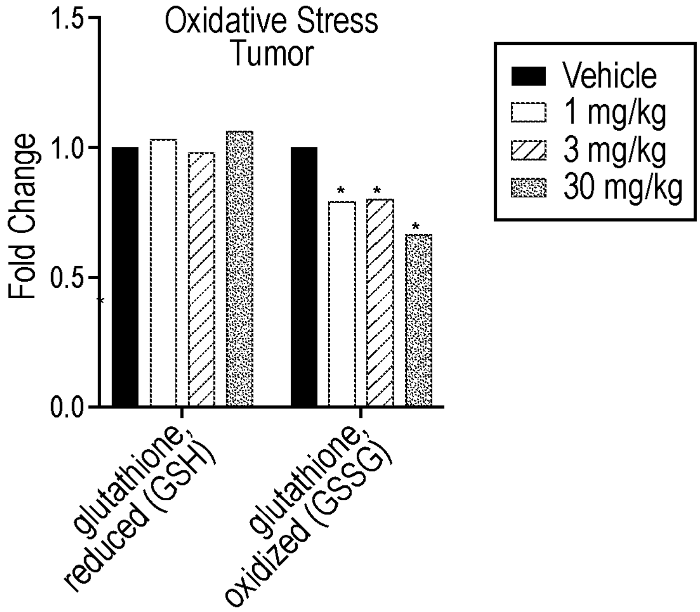


FIG. 122

| | Amino Acids | GCN2 WT + DMSO | GCN2 WT HC-7366 | GCN2 KO + DMSO | GCN2 KO HC-7366 |
|---|---|---|---|---|---|
| NEAA | Alanine* | 1.00 | **1.33** | 0.50* | 0.58 |
| | Aspartate* | 1.00 | **0.69** | 0.43* | 0.44 |
| | Glutamate | 1.00 | 1.15 | 0.47* | 0.53 |
| | Glycine | 1.00 | 1.14 | 0.54* | 0.55 |
| | Proline | 1.00 | 1.01 | 0.34* | 0.33 |
| | Serine | 1.00 | **1.87** | 0.84 | 1.08 |
| | Tyrosine* | 1.00 | **1.34** | 0.79 | 0.87 |
| EAA | Histidine | 1.00 | 1.20 | 0.89 | 0.88 |
| | Isoleucine* | 1.00 | **1.40** | 0.84 | 0.94 |
| | Leucine* | 1.00 | **1.36** | 0.82 | 0.93 |
| | Lysine | 1.00 | 1.09 | 0.87 | 1.13 |
| | Methionine* | 1.00 | **1.43** | 0.79 | 0.87 |
| | Phenylalanine* | 1.00 | **1.37** | 0.81 | 0.93 |
| | Taurine* | 1.00 | **0.61** | 0.08 | 0.07 |
| | Threonine* | 1.00 | **1.53** | 0.73* | 0.78 |
| | Tryptophan* | 1.00 | **1.33** | 0.74 | 0.82 |
| | Valine* | 1.00 | **1.40** | 0.85 | 0.96 |
| Conditionally EAA | Arginine | 1.00 | 1.13 | 0.94 | 1.18 |
| | Asparagine* | 1.00 | **1.62** | 0.64* | 0.75 |
| | Glutamine* | 1.00 | **1.58** | 0.16* | 0.17 |

FIG. 123

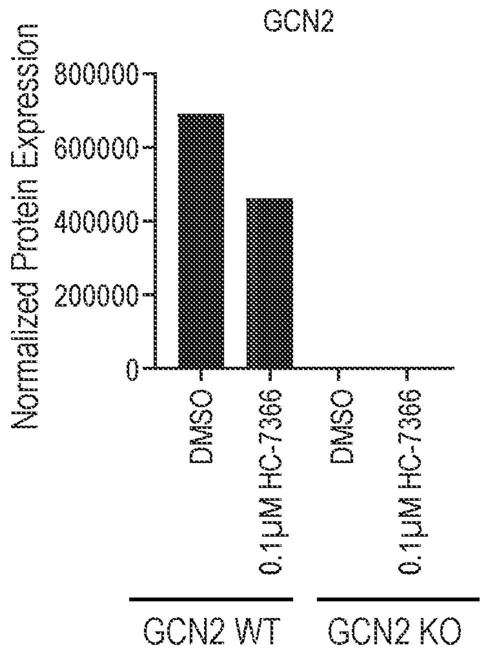


FIG. 124

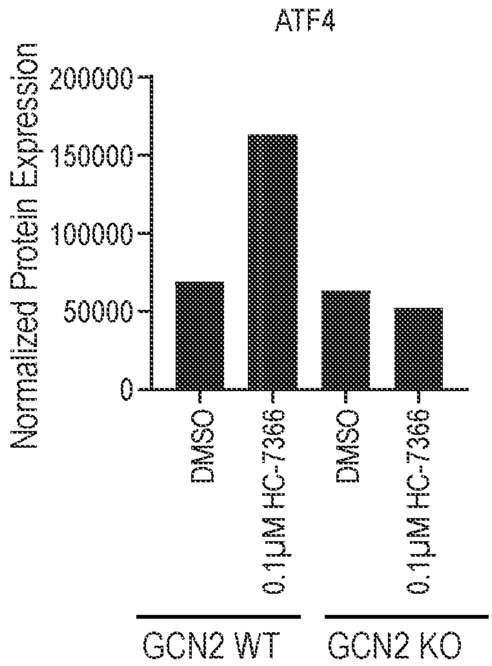


FIG. 125

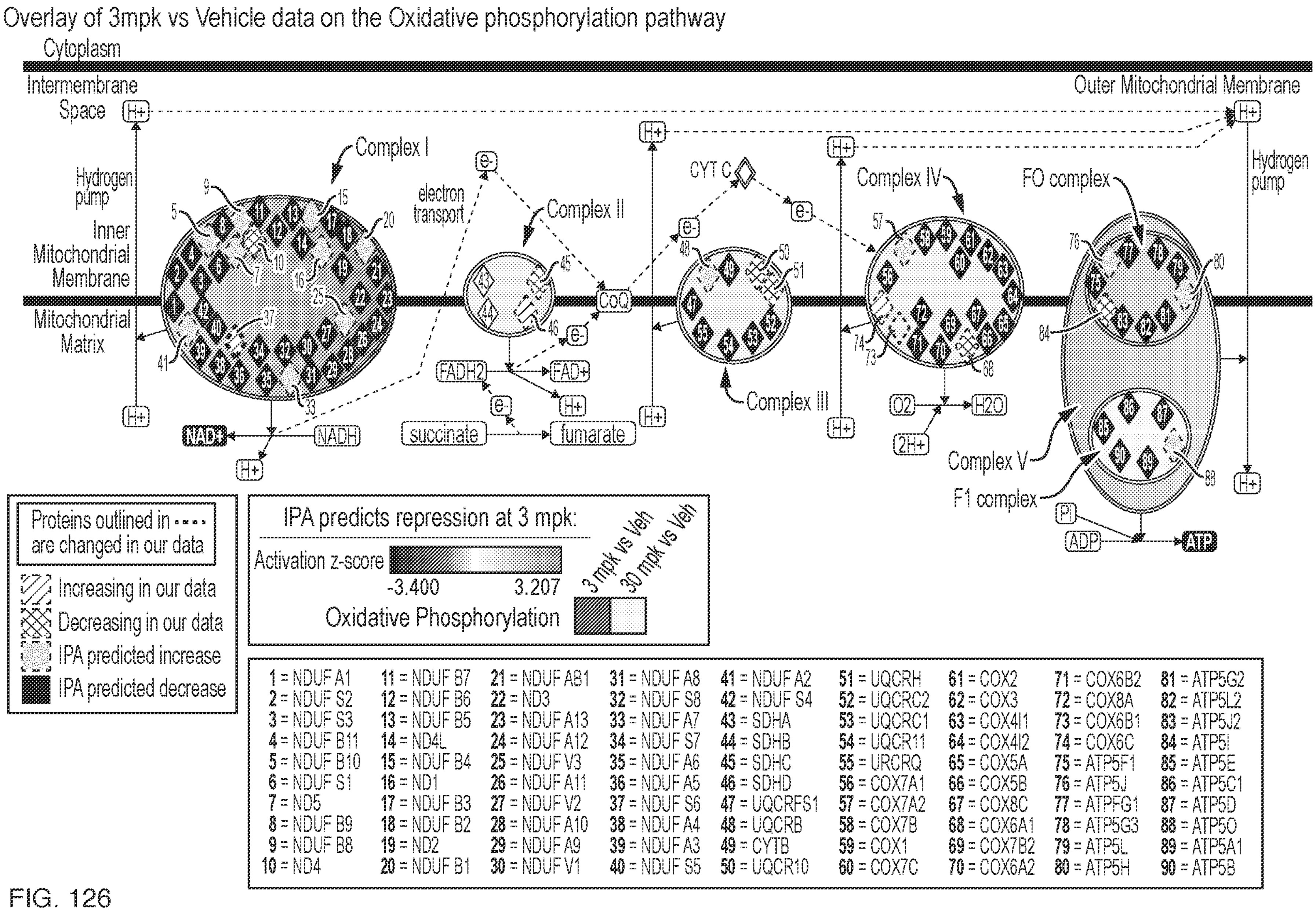
Overlay of 3mpk vs Vehicle data on the Oxidative phosphorylation pathway
Cytoplasm
Intermembrane Space
Outer Mitochondrial Membrane
Hydrogen pump
Inner Mitochondrial Membrane
Mitochondrial Matrix
Complex I
electron transport
Complex II
CYT C
Complex III
Complex IV
FO complex
Complex V
F1 complex
H+
e-
CoQ
FADH2
FAD+
succinate
fumarate
NAD+
NADH
O2
2H+
H2O
Pi
ADP
ATP
Proteins outlined in are changed in our data
Increasing in our data
Decreasing in our data
IPA predicted increase
IPA predicted decrease
IPA predicts repression at 3 mpk:
Activation z-score
-3.400
3.207
3 mpk vs Veh
30 mpk vs Veh
Oxidative Phosphorylation
1 = NDUF A1
2 = NDUF S2
3 = NDUF S3
4 = NDUF B11
5 = NDUF B10
6 = NDUF S1
7 = ND5
8 = NDUF B9
9 = NDUF B8
10 = ND4
11 = NDUF B7
12 = NDUF B6
13 = NDUF B5
14 = ND4L
15 = NDUF B4
16 = ND1
17 = NDUF B3
18 = NDUF B2
19 = ND2
20 = NDUF B1
21 = NDUF AB1
22 = ND3
23 = NDUF A13
24 = NDUF A12
25 = NDUF V3
26 = NDUF A11
27 = NDUF V2
28 = NDUF A10
29 = NDUF A9
30 = NDUF V1
31 = NDUF A8
32 = NDUF S8
33 = NDUF A7
34 = NDUF S7
35 = NDUF A6
36 = NDUF A5
37 = NDUF S6
38 = NDUF A4
39 = NDUF A3
40 = NDUF S5
41 = NDUF A2
42 = NDUF S4
43 = SDHA
44 = SDHB
45 = SDHC
46 = SDHD
47 = UQCRFS1
48 = UQCRB
49 = CYTB
50 = UQCR10
51 = UQCRH
52 = UQCRC2
53 = UQCRC1
54 = UQCR11
55 = URCRQ
56 = COX7A1
57 = COX7A2
58 = COX7B
59 = COX1
60 = COX7C
61 = COX2
62 = COX3
63 = COX4I1
64 = COX4I2
65 = COX5A
66 = COX5B
67 = COX8C
68 = COX6A1
69 = COX7B2
70 = COX6A2
71 = COX6B2
72 = COX8A
73 = COX6B1
74 = COX6C
75 = ATP5F1
76 = ATP5J
77 = ATPFG1
78 = ATP5G3
79 = ATP5L
80 = ATP5H
81 = ATP5G2
82 = ATP5L2
83 = ATP5J2
84 = ATP5I
85 = ATP5E
86 = ATP5C1
87 = ATP5D
88 = ATP5O
89 = ATP5A1
90 = ATP5B

FIG. 126

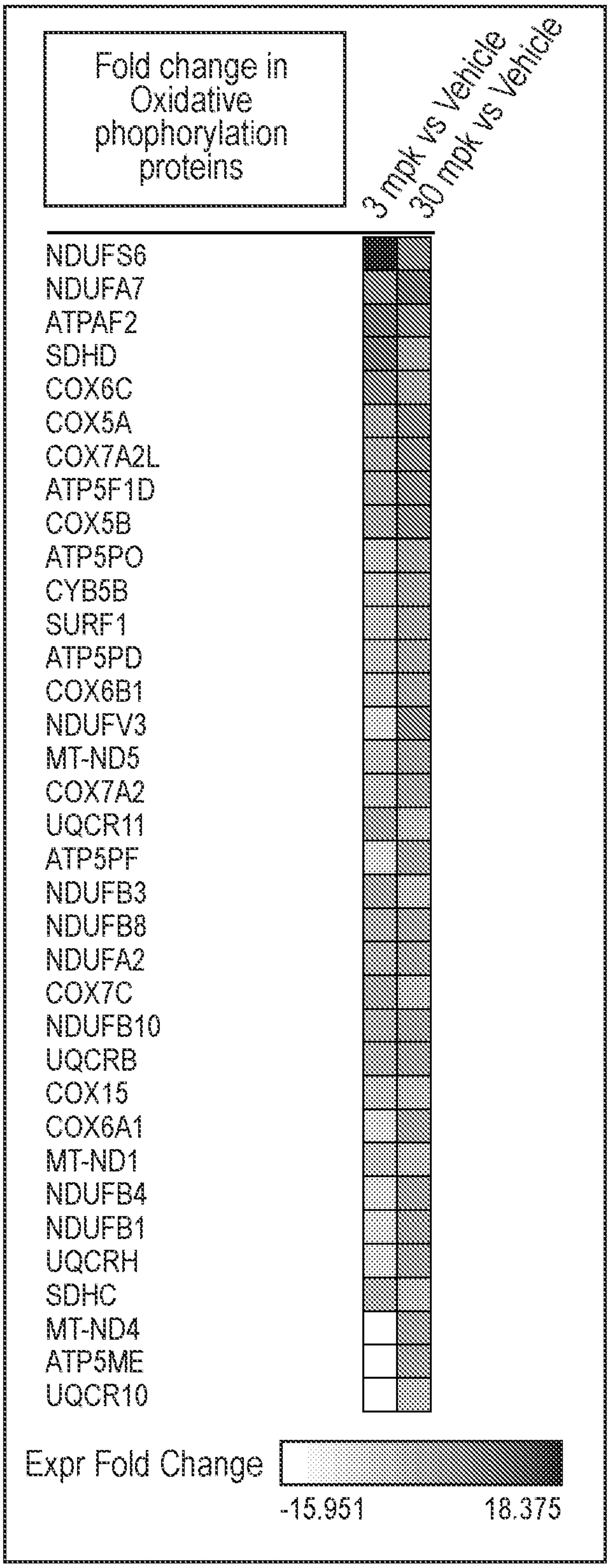
Fold change in Oxidative phophorylation proteins
3 mpk vs Vehicle
30 mpk vs Vehicle
NDUFS6
NDUFA7
ATPAF2
SDHD
COX6C
COX5A
COX7A2L
ATP5F1D
COX5B
ATP5PO
CYB5B
SURF1
ATP5PD
COX6B1
NDUFV3
MT-ND5
COX7A2
UQCR11
ATP5PF
NDUFB3
NDUFB8
NDUFA2
COX7C
NDUFB10
UQCRB
COX15
COX6A1
MT-ND1
NDUFB4
NDUFB1
UQCRH
SDHC
MT-ND4
ATP5ME
UQCR10
Expr Fold Change
-15.951
18.375

FIG. 127

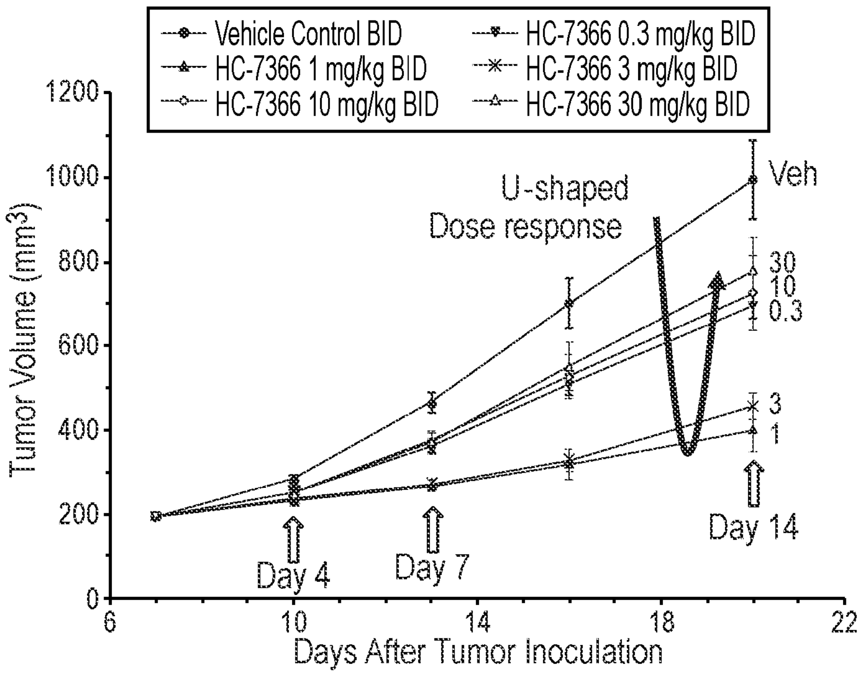


FIG. 128

| | | | | | **Most Efficacious** | | | | | | | | | | |
|---|---|---|---|---|---|---|---|---|---|---|---|---|---|---|---|
| HC-7366 | 0.3 mg/kg | | | 1 mg/kg | | | 3 mg/kg | | | 10 mg/kg | | | 30 mg/kg | | |
| Upstream Regulator | Day 4 | Day 7 | Day 14 | **Day 4** | **Day 7** | **Day 14** | **Day 4** | **Day 7** | **Day 14** | Day 4 | Day 7 | Day 14 | Day 4 | Day 7 | Day 14 |
| ARID1A | | 1.46 | 1.97 | | 3.23 | 3.22 | 0.36 | 3.39 | 2.57 | | 1.11 | | | | |
| **ATF4** | 2.58 | 1.84 | 2.15 | 3.48 | 2.20 | 2.11 | 4.13 | 2.44 | 3.38 | 3.37 | 1.65 | 1.97 | 0.80 | 1.51 | 0.80 |
| GLI1 | | 1.81 | 0.30 | 1.65 | 2.92 | 2.41 | -0.13 | 2.24 | 1.50 | | 1.67 | | | | |
| JUN | | 0.08 | 1.00 | 2.85 | 0.27 | -0.03 | 2.67 | -0.31 | 0.93 | | 0.66 | 1.45 | | | 1.96 |
| SREBF1 | | -2.49 | -1.50 | | -2.78 | -2.87 | -0.04 | -2.69 | -1.39 | | -1.99 | | | | |
| CREB1 | | -2.95 | -2.06 | -0.43 | -2.76 | -3.48 | 0.69 | -2.91 | -2.54 | | -2.60 | -0.02 | | | |
| SREBF2 | | -2.42 | -2.00 | | -2.48 | -2.23 | | -2.67 | -1.24 | | -1.98 | | | | |
| STAT4 | | -1.67 | -0.17 | | -2.34 | -2.68 | 2.80 | -2.49 | -1.85 | | -1.73 | | | | |
| KLF6 | | -2.96 | | | -2.55 | -3.18 | | -2.79 | | | -2.39 | | | | |
| HIF1A | 1.00 | -4.22 | -2.00 | 2.18 | -5.07 | -4.74 | | -4.84 | -2.51 | | -3.14 | | | | |
| SMAD4 | | | | | -1.78 | -0.75 | | -1.02 | | | | | | | |
| E2F1 | | | | | -3.07 | -1.94 | | -3.23 | | | | | | | |

TxF activation associated with efficacy

TxF inhibition associated with efficacy

Activation Z-score

Increased Gene Expression associated with Transcription Factor

Decreased Gene Expression associated with Transcription Factor

FIG. 129

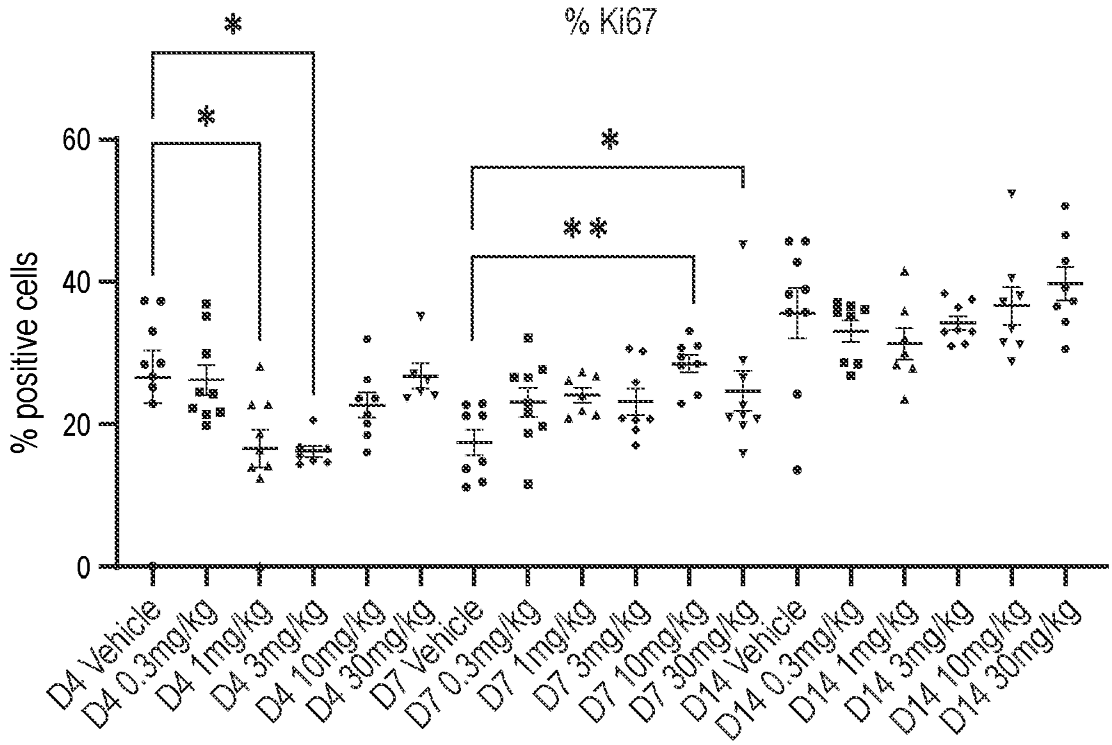


FIG. 130

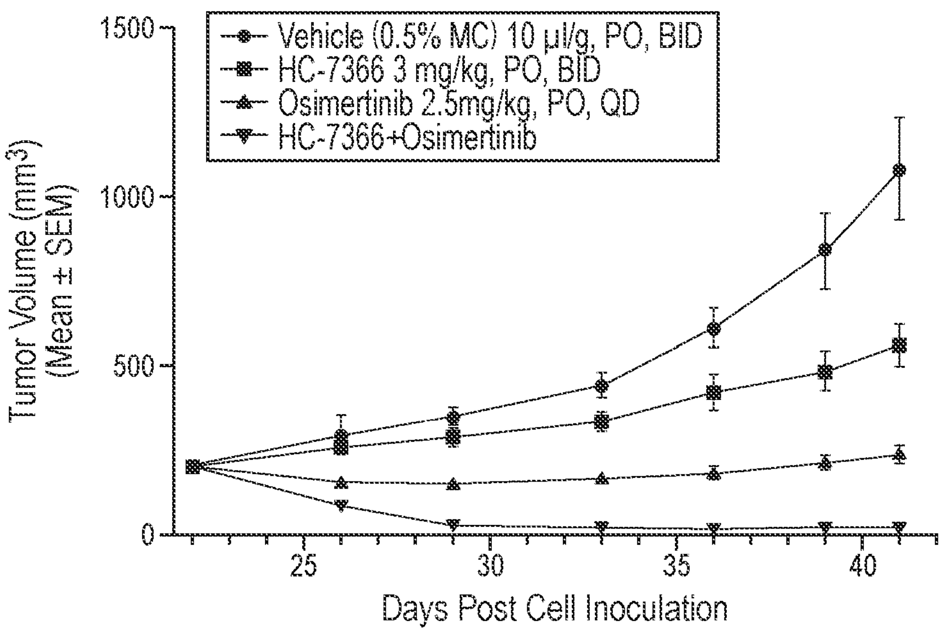


FIG. 131

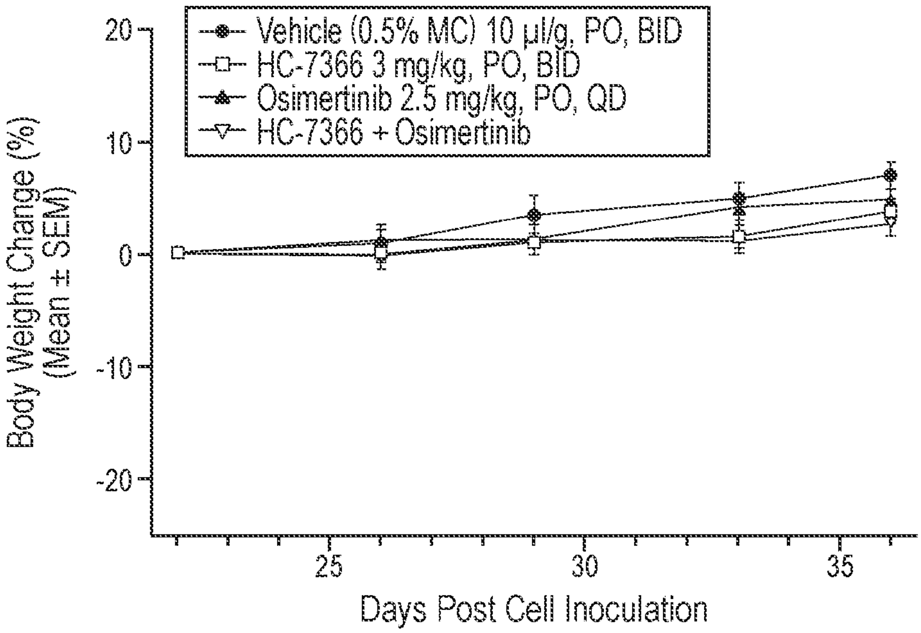


FIG. 132

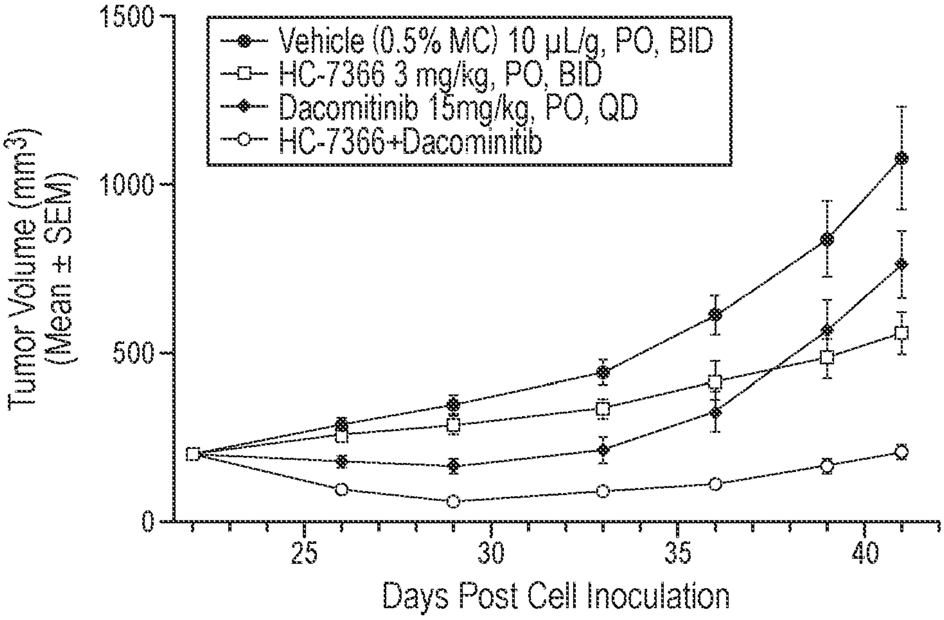


FIG. 133

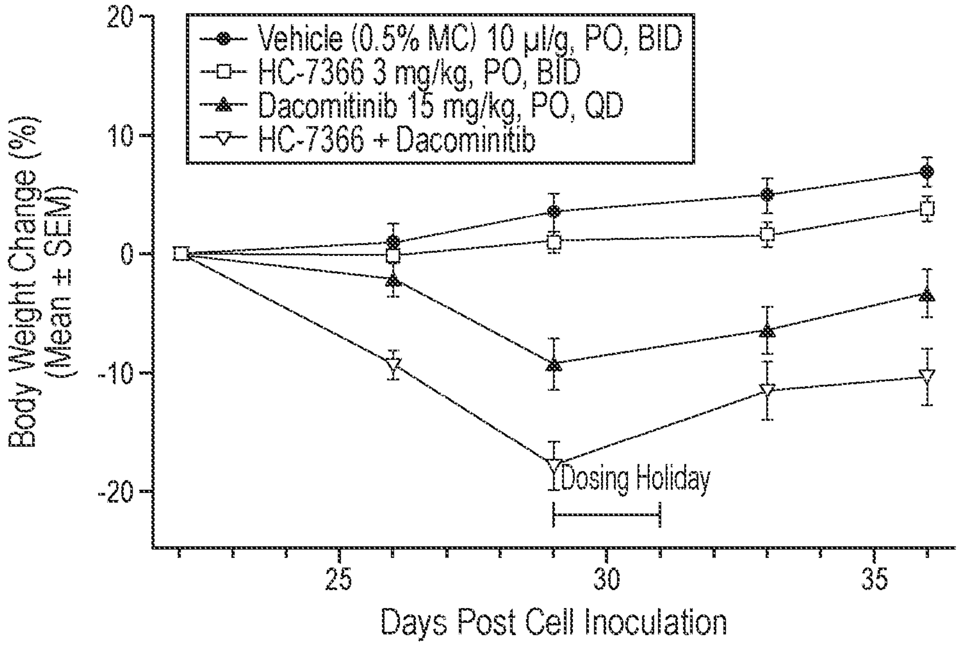


FIG. 134

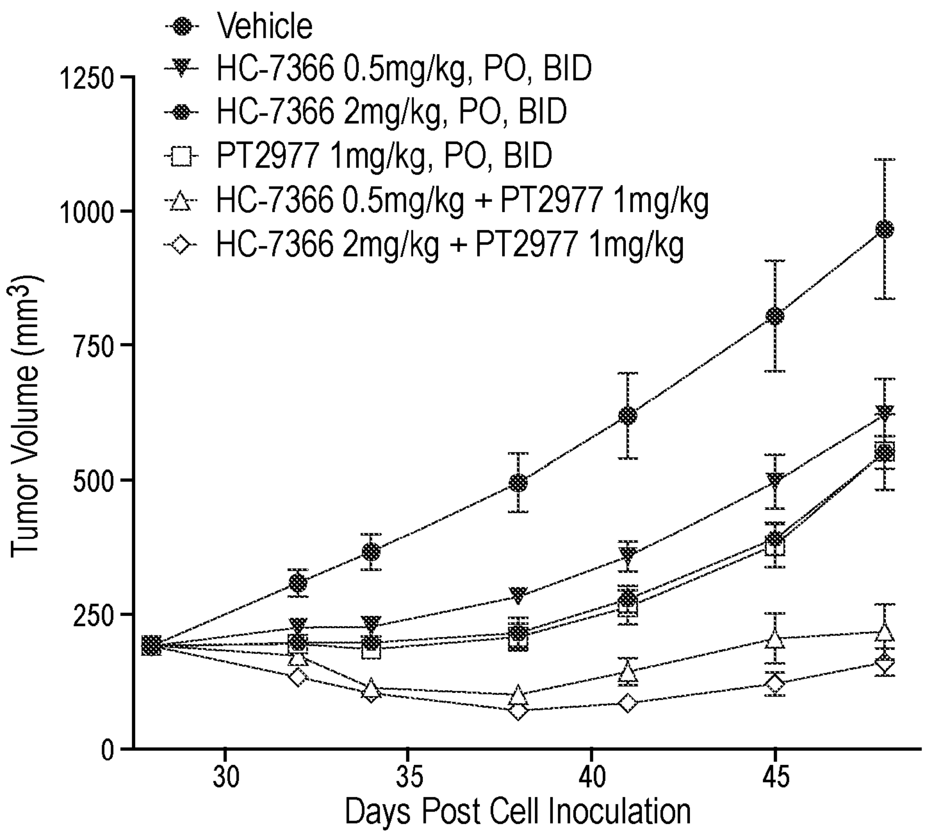


FIG. 135

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 63345727
- US 63440297 B
- US 63443269 B
- US 63455861 B


### Non-patent literature cited in the description


- **WANG et al.** Drug resistance and combating drug resistance in cancer. *Cancer Drug Resistance*, 2019, vol. 2 (2), 141-160
- **CHAKRABORTY et al.** The difficulties in cancer treatment. *Ecancermedicalscience*, 2012, vol. 6, ed16
- **YE et al.** The GCN2-ATF4 pathway is critical for tumor cell survival and proliferation in response to nutrient deprivation. *EMBO J.*, 2010, vol. 29 (12), 2082-2096
- **PAKOS-ZEBRUCKA et al.** The integrated stress response. *EMBO Rep*, 2016, vol. 17 (10), 1374-1395
- **DONNELLY et al.** The eIF2α kinases: their structures and functions. *Cell Mol Life Sci.*, 2013, vol. 70 (19), 3493-3511
- **ALBERT et al.** Adaptive Protein Translation by the Integrated Stress Response Maintains the Proliferative and Migratory Capacity of Lung Adenocarcinoma Cells. *Mol Cancer Res.*, 2019, vol. 17 (12), 2343-2355
- **ANDA et al.** Activation of Gen2 in response to different stresses. *PLOS ONE*, 2017, vol. 12 (8), E0182143
- **HARDING et al.** An integrated stress response regulates amino acid metabolism and resistance to oxidative stress. *Mol Cell*, 2003, vol. 11 (3), 619-633
- **COSTA-MATTIOLI et al.** The integrated stress response: From mechanism to disease. *Science*, 2020, vol. 368 (6489), eaat5314
- **WORTEL et al.** Surviving Stress: Modulation of ATF4-Mediated Stress Responses in Normal and Malignant Cells. *Trends Endocrinol Metabol.*, 2017, vol. 28 (11), 794-806
- **HARDING et al.** Ppplr14 gene knockout reveals an essential role for translation initiation factor 2 alpha (eIF2alpha) dephosphorylation in mammalian development. *Proc Natl Acad Sci USA*, 2009, vol. 106 (6), 1832-1837
- **MÜNCH**. The different axes of the mammalian mitochondrial unfolded protein response. *BMC Biology*, 2018, vol. 16 (1), 81
- **LEHMAN et al.** Translation Upregulation of an Individual p21Cip1 Transcript Variant by GCN2 Regulates Cell Proliferation and Survival under Nutrient Stress. *PLOS Genetics*, 2015, vol. 11 (6), e1005212
- Remington's Pharmaceutical Sciences. Mack Publ. Co., 1975
- Remington: The Science and Practice of Pharmacy. Lippincott Williams & Wilkins, 2005